# EUROPEAN PATENT APPLICATION

(11) **EP 4 775 992 A2**
(43) Date of publication of application: **15.07.2026**
(21) Application number: 26163638.5
(22) Date of filing: 28.01.2021
(51) Int. Cl.: G01N 33/66

(54) **TREATMENT OF MUCOPOLYSACCHARIDOSIS II WITH RECOMBINANT HUMAN IDURONATE-2-SULFATASE (IDS) PRODUCED BY HUMAN NEURAL OR GLIAL CELLS**

(30) Priority: 29.01.2020 US 202062967494 P; 17.08.2020 US 202063066625 P; 06.10.2020 US 202063088305 P
(62) Divisional of application: 21710357.1
(71) Applicant: RegenxBio Inc., Rockville, MD 20850 (US)
(72) Inventor: PAKOLA, Stephen, Joseph, Irvington, NY 10533 (US); FALABELLA, Paulo, Potomac, ME 20854 (US); NEVORET, Marie-Laure, Virginia Beach, VA 23452 (US)
(74) Representative: Jones Day

(57) **Abstract**

Compositions and methods are described for the delivery of recombinant human iduronate-2-sulfatase (IDS) produced by human neuronal or glial cells to the cerebrospinal fluid of the central nervous system (CNS) of a human subject diagnosed with mucopolysaccharidosis II (MPS II).

## Description

### CROSS-REFERENCE TO RELATED APPLICATIONS

This application claims the benefit of U.S. Provisional Application No. 63/088,305, filed October 06, 2020, U.S. Provisional Application No. 63/066,625, filed August 17, 2020 and U.S. Provisional Application No. 62/967,494, filed January 29, 2020, the content of each of which is incorporated herein by reference in its entirety.

### REFERENCE TO SEQUENCE LISTING SUBMITTED ELECTRONICALLY

This application incorporates by reference a Sequence Listing submitted with this application as text file entitled "Sequence_Listing_12656-129-228.TXT" created on January 12, 2021 and having a size of 172,339 bytes.

### 1. INTRODUCTION

Compositions and methods are described for the delivery of recombinant human iduronate-2-sulfatase (IDS) produced by human neuronal or glial cells to the cerebrospinal fluid (CSF) of the central nervous system (CNS) of a human subject diagnosed with mucopolysaccharidosis II (MPS II).

### 2. BACKGROUND OF THE INVENTION

Hunter syndrome/MPS II is a rare X-linked recessive genetic disease occurring in 0.5 to 1.3 per 100,000 male live births. This progressive and devastating disease is caused by genetic mutation in the IDS gene leading to deficiency of the lysosomal storage enzyme iduronate-2-sulfatase, an enzyme required for the lysosomal catabolism of heparan sulfate and dermatan sulfate. These ubiquitous polysaccharides, called GAGs (glycosaminoglycans), accumulate in tissues and organs of MPS II patients resulting in the characteristic storage lesions and diverse disease sequelae. Morbidity and mortality are high in this patient population; death has been reported to occur at a mean age of 11.7 years in patients with the severe phenotype (characterized by neurocognitive deterioration) and 21.7 years in patients with a mild or attenuated phenotype. (Young et al., 1982, A clinical and genetic study of Hunter's syndrome. 2 Differences between the mild and severe forms. J. Medical Genetics 19:408-411). The majority (two-thirds) of patients are reported to have the severe form of this disease. (Wraith JE, et al., 2007, Enzyme replacement therapy in patients who have mucopolysaccharidosis I and are younger than 5 years: Results of a multinational study of recombinant human alpha-L-Iduronidase (Laronidase). Pediatrics 120(1):E37-E46). While the disease primarily affects boys, affected females have been reported as a result of non-random x-inactivation and/or mutation in both alleles of the gene. (Martin et al., 2008, Recognition and diagnosis of mucopolysaccharidosis II (Hunter Syndrome). Pediatrics 121:e377). However, MPS II in females is extremely rare, occurring less than 2% of the time.

Patients with MPS II appear normal at birth, but signs and symptoms of disease typically present between the ages of 18 months and 4 years in the severe form and between the ages of 4 and 8 years in the attenuated form. Signs and symptoms common to all affected patients include short stature, coarse facial features, macrocephaly, macroglossia, hearing loss, hepato-and splenomegaly, dystosis multiplex, joint contractures, spinal stenosis and carpal tunnel syndrome. Frequent upper respiratory and ear infections occur in most patients and progressive airway obstruction is commonly found, leading to sleep apnea and often death. Cardiac disease is a major cause of death in this population and is characterized by valvular dysfunction leading to right and left ventricular hypertrophy and heart failure. Death is generally attributed to obstructive airway disease or cardiac failure.

In severe forms of the disease, early developmental milestones may be met, but developmental delay is readily apparent by 18-24 months. Some patients fail hearing screening tests in the first year and other milestones are delayed, including ability to sit unsupported, ability to walk, and speech. Developmental progression begins to plateau between 3 and 5 years of age, with regression reported to begin around 6.5 years. Of the ~50% of children with MPS II who become toilet trained, most, if not all, will lose this ability as the disease progresses. (Wraith et al., 2007, *supra;* Martin et al., 2008, *supra).*

Patients with significant neurologic involvement exhibit severe behavioral disturbances including hyperactivity, obstinacy, and aggression beginning in the second year of life and continuing until age 8-9, when neurodegeneration attenuates this behavior. (Muenzer, et al., 2009, Mucopolysaccharidosis I: Management and Treatment Guidelines, Pediatric 123(1): 19-29).

Seizures are reported in over half of severely affected patients who reach the age of 10, and by the time of death most patients with CNS involvement are severely mentally handicapped and require constant care. (Wraith et al., 2007, *supra;* Martin et al., 2008, *supra*)*.* Although patients with attenuated disease exhibit normal intellectual functioning, MRI imaging reveals gross brain abnormalities in all patients with MPS II including white matter lesions, enlarged ventricles, and brain atrophy. (Muenzer, et al., 2009, *supra*)*.*

Enzyme replacement therapy (ERT) with recombinant idursulfase produced by HT1080 (fibrosarcoma) cells (Elaprase^{®}, Shire Human Genetic Therapies) is the only approved product for the treatment of Hunter syndrome and is administered as a weekly infusion. (ELAPRASE (idursulfase) injection [package insert]. Lexington, MA: Shire Human Genetic Therapies, Inc; 2013, available at http://pi.shirecontent.com/PI/PDFs/Elaprase_USA_ENG.pdf).

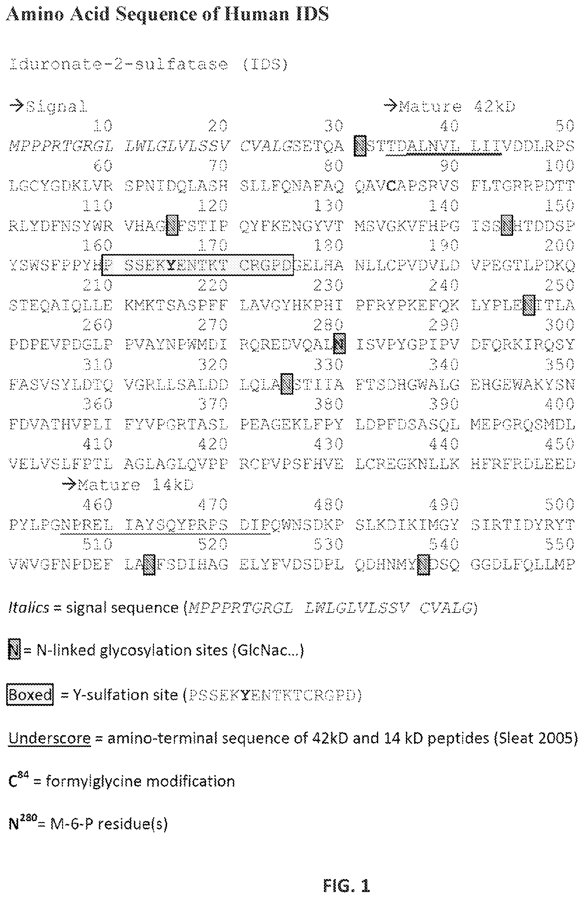

However, ERT as currently administered does not cross the blood brain barrier and is therefore unable to address the unmet need in patients with severe disease, *i.e.,* MPS II with CNS/neurocognitive and behavioral involvement. In a recent clinical trial designed to address this problem, idursulfase (Elaprase) formulated for intrathecal administration was administered once monthly to pediatric patients using an intrathecal drug delivery device implanted into the spine (insertion of the catheter at the level of L4/L5 with implantation of the access port via an incision on the lower ribs). The patients also received concurrent i.v. idursulfase once weekly. *See* Muenzer et al., 2016, Genetics in Med 18: 73-81, esp. p. 74; abstract available at https://www.ncbi.nlm.nih.gov/pubmed/25834948?dopt=Abstract). Device malfunction led to partial revision, total surgical revision, or removal in 6 of the 12 (50%) of the treated patients. Notably, 12 of 14 SAEs (serious adverse events) were device-related (complication of device insertion, device dislocation/connection issue, device breakage/malfunction/failure, implant site infection, procedural pain, and wound dehiscence). (Muenzer et al., 2016, p. 75, col. 2 and Fig. 1). Device breakage and catheter migration from the spinal canal was exacerbated by the high activity level of this pediatric population. (Muenzer et al., 2016 at p.78 Discussion).

### 3. SUMMARY OF THE INVENTION

The invention involves the delivery of recombinant human iduronate-2-sulfatase (rhIDS) produced by human neuronal or glial cells to the cerebrospinal fluid (CSF) of the central nervous system (CNS) of a human subject diagnosed with mucopolysaccharidosis II (MPS II), including, but not limited to patients diagnosed with Hunter syndrome.

In a preferred embodiment, the treatment is accomplished via gene therapy - *e.g.,* by administering a viral vector or other DNA expression construct encoding human IDS (hIDS), or a derivative of hIDS, to the CSF of a patient (human subject) diagnosed with MPS II, so that a permanent depot of transduced neuronal and/or glial cells is generated that continuously supplies the transgene product to the CNS. The rhIDS secreted from the neuronal/glial cell depot into the CSF will be endocytosed by cells in the CNS, resulting in "cross-correction" of the enzymatic defect in the recipient cells. Moreover, it has been found, unexpectedly, that the depot of transduced neural and glial cells in the CNS can deliver the recombinant enzyme to both the CNS and systemically, which may reduce or eliminate the need for systemic treatment, *e.g*., weekly i.v. injections of the enzyme.

In an alternative embodiment, the hIDS can be produced by human neuronal or glial cells in cell culture (*e.g*., bioreactors) and administered as an enzyme replacement therapy ("ERT"), *e.g.,* by injecting the enzyme - into the CSF, directly into the CNS, and/or systemically. However, the gene therapy approach offers several advantages over ERT since systemic delivery of the enzyme will not result in treating the CNS because the enzyme cannot cross the blood brain barrier; and, unlike the gene therapy approach of the invention, direct delivery of the enzyme to the CSF and/or CNS would require repeat injections which are not only burdensome, but pose a risk of infection.

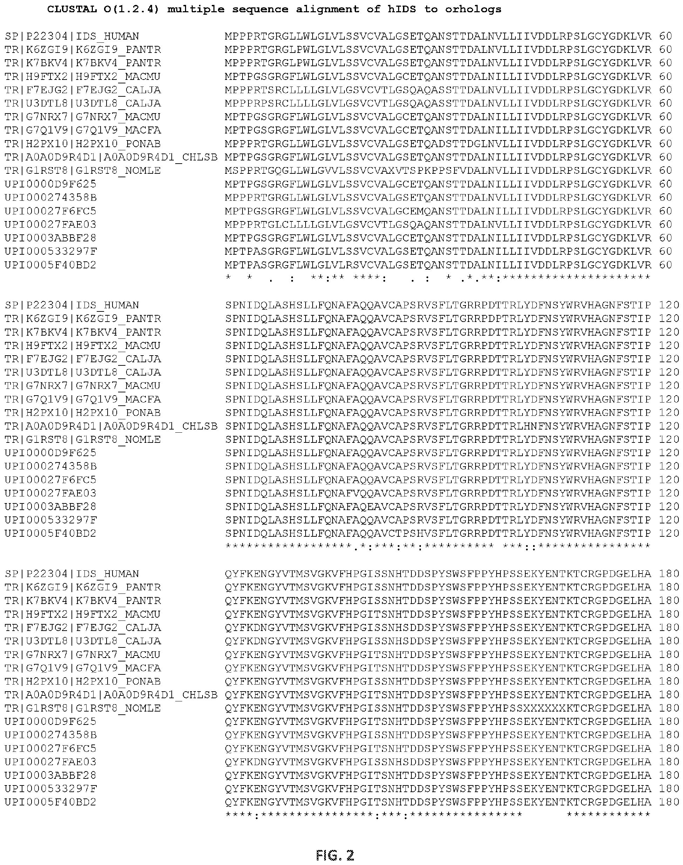

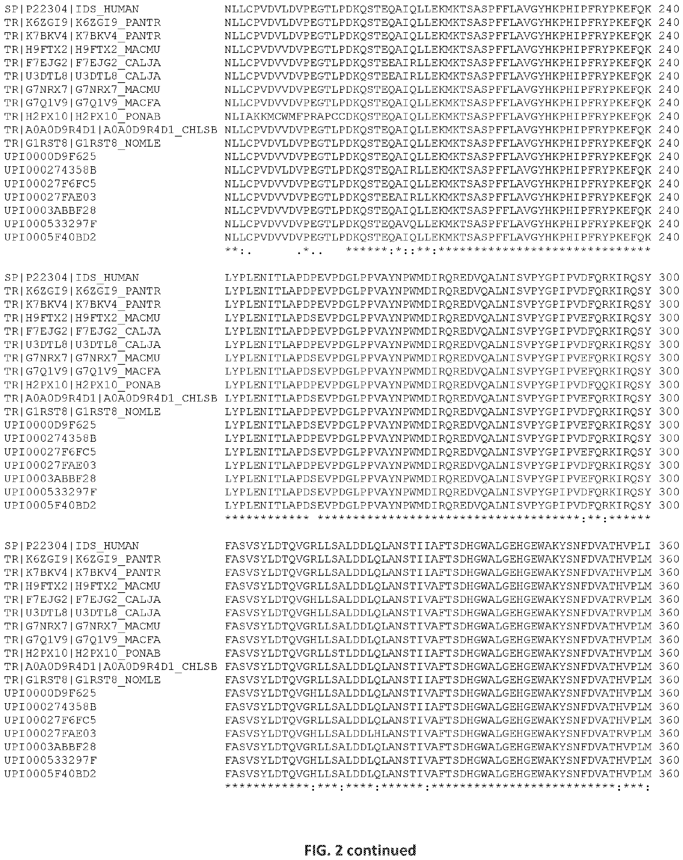

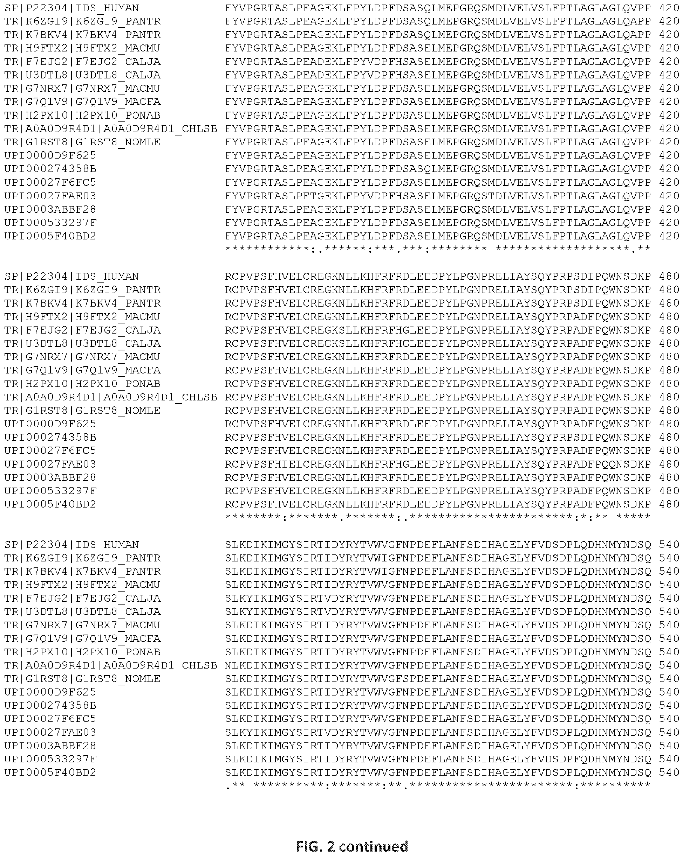

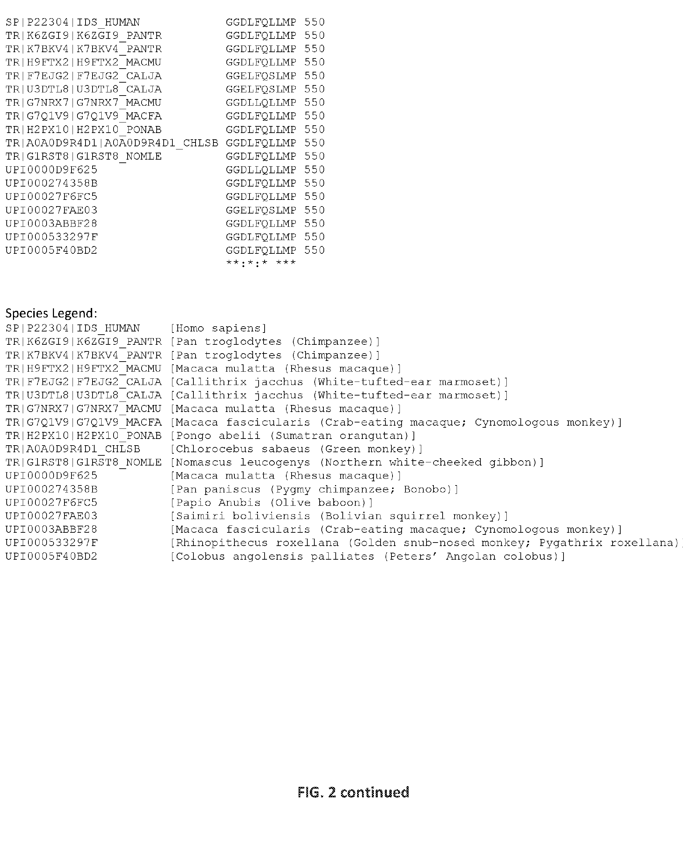

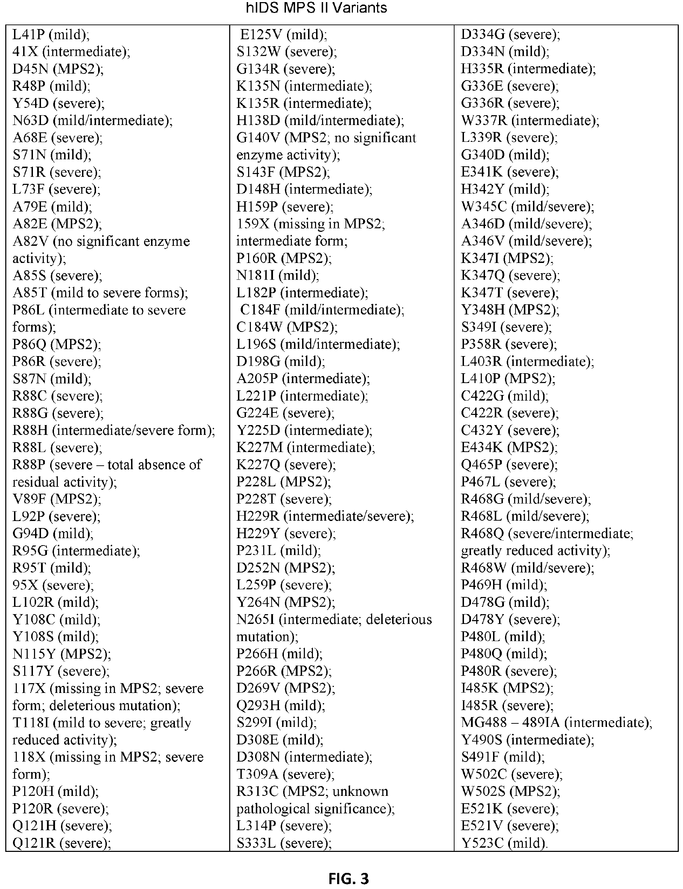

The hIDS encoded by the transgene can include, but is not limited to human IDS (hIDS) having the amino acid sequence of SEQ ID NO. 1 (as shown in FIG. 1), and derivatives of hIDS having amino acid substitutions, deletions, or additions, *e.g*., including but not limited to amino acid substitutions selected from corresponding non-conserved residues in orthologs of IDS shown in FIG. 2, with the *proviso* that such mutations do not include replacement of the cysteine residue at position 84 (C84) which is required for enzyme activity (Millat et al., 1997, Biochem J 326: 243-247); or a mutation that has been identified in severe, severe-intermediate, intermediate, or attenuated MPS II phenotypes *e.g*., as shown in FIG. 3, or as reported by Sukegawa-Hayasaka et al., 2006, J Inhert Metab Dis 29: 755-761 (reporting "attenuated" mutants R48P, A85T, W337R, and the truncated mutant Q531X; and "severe" mutants P86L, S333L, S349I, R468Q, R468L); Millat et al., 1998, BBA 1406: 214-218 (reporting "attenuated" mutants P480L and P480Q; and "severe" mutant P86L); and Bonucelli et al., 2001, BBA 1537:233-238, each of which is incorporated by reference herein in its entirety.

For example, amino acid substitutions at a particular position of hIDS can be selected from among corresponding non-conserved amino acid residues found at that position in the IDS orthologs aligned in FIG. 2, with the *proviso* that such substitutions do not include any of the deleterious mutations shown in FIG. 3 or as reported by Sukegawa-Hayasaka et al., 2006, *supra;* Millat et al., 1998, *supra;* or Bonucelli et al., 2001, *supra,* each of which is incorporated by reference herein in its entirety. The resulting transgene product can be tested using conventional assays *in vitro,* in cell culture or test animals to ensure that the mutation does not disrupt IDS function. Preferred amino acid substitutions, deletions or additions selected should be those that maintain or increase enzyme activity, stability or half-life of IDS, as tested by conventional assays *in vitro,* in cell culture or animal models for MPS II. For example, the enzyme activity of the transgene product can be assessed using a conventional enzyme assay with, for example, 4-Methylumbelliferyl α-L-idopyranosiduronic acid 2-sulfate or 4-methylumbelliferyl sulfate as the substrate *(see, e.g.,* Lee et al., 2015, Clin. Biochem. 48(18):1350-1353, Dean et al., 2006, Clin. Chem. 52(4):643-649 for exemplary IDS enzyme assays that can be used, each of which is incorporated by reference herein in its entirety). The ability of the transgene product to correct MPS II phenotype can be assessed in cell culture; *e.g.,* by transducing MPS II cells in culture with a viral vector or other DNA expression construct encoding hIDS or a derivative; by adding the transgene product or a derivative to MPS II cells in culture; or by co-culturing MPS II cells with human neuronal/glial host cells engineered to express and secrete rhIDS or a derivative, and determining correction of the defect in the MPS II cultured cells, *e.g.,* by detecting IDS enzyme activity and/or reduction in GAG storage in the MPS II cells in culture *(see, e.g.,* Stroncek et al., 1999, Transfusion 39(4):343-350, which is incorporated by reference herein in its entirety). In a preferred embodiment, the reduction in GAG storage is reduction in heparan sulfate (HS) storage. In another embodiment, the reduction in GAG storage is reduction in dermatan sulfate (DS) storage. In another embodiment, the reduction in GAG storage is reduction in both HS storage and DS storage.

Animal models for MPS II have been described that can be used to assess the therapeutics described herein. For example, a knockout mouse model (IDS-knockout) of MPS II was engineered by replacing exons 4 and 5 of the *IDS* gene with the neomycin resistance gene. (Garcia et al., 2007, J Inherit Metab Dis 30: 924-34). This IDS-knockout mouse exhibits many of the characteristics of MPS II, including skeletal abnormalities, hepatosplenomegaly, elevated urinary and tissue GAG, and brain storage lesions (Muenzer et al., 2001, Acta Paediatr Suppl 91:98-99) and was used to assess the effect of enzyme replacement therapy in MPS II in support of clinical trials for ERT. This mouse model, therefore, is a relevant model for studying the effects of gene therapy delivering rIDS produced by neuronal or glial cells as a treatment for MPS II *(see, e.g.,* Polito and Cosma, 2009, Am. J. Hum. Genet. 85(2):296-301, which is incorporated by reference herein in its entirety).

Preferably, the hIDS transgene produced by the human neuronal/glial cells should be controlled by expression control elements that function in neurons and/or glial cells, *e.g.,* the CB7 promoter (a chicken β-actin promoter and CMV enhancer), and can include other expression control elements that enhance expression of the transgene driven by the vector (*e.g.*, chicken β-actin intron and rabbit β-globin poly A signal). The cDNA construct for the hIDS transgene should include a coding sequence for a signal peptide that ensures proper co- and post-translational processing (glycosylation and protein sulfation) by the transduced CNS cells. Such signal peptides used by CNS cells may include but are not limited to:
Oligodendrocyte-myelin glycoprotein (hOMG) signal peptide:
   MEYQILKMSLCLFILLFLTPGILC (SEQ ID NO:2)
Cellular repressor of E1A-stimulated genes 2 (hCREG2) signal peptide:
   MSVRRGRRPARPGTRLSWLLCCSALLSPAAG (SEQ ID NO:3)
V-set and transmembrane domain containing 2B (hVSTM2B) signal peptide:
   MEQRNRLGALGYLPPLLLHALLLFV ADA (SEQ ID NO:4)
Protocadherin alpha-1 (hPCADHA1) signal peptide:
   MVFSRRGGLGARDLLLWLLLLAAWEVGSG (SEQ ID NO:5)
FAM19A1 (TAFA1) signal peptide:
   MAMVSAMSWVLYLWISACA (SEQ ID NO:6)
Interleukin-2 signal peptide:
   MYRMQLLSCIALILALVTNS (SEQ ID NO:14)
Signal peptides may also be referred to herein as leader sequences or leader peptides.

The recombinant vector used for delivering the transgene should have a tropism for cells in the CNS, including but limited to neurons and/or glial cells. Such vectors can include non-replicating recombinant adeno-associated virus vectors ("rAAV"), particularly those bearing an AAV9 or AAVrh10 capsid are preferred. AAV variant capsids can be used, including but not limited to those described by Wilson in US Patent No. 7,906,111 which is incorporated by reference herein in its entirety, with AAV/hu.31 and AAV/hu.32 being particularly preferred; as well as AAV variant capsids described by Chatterjee in US Patent No. 8,628,966, US Patent No. 8,927,514 and Smith et al., 2014, Mol Ther 22: 1625-1634, each of which is incorporated by reference herein in its entirety. However, other viral vectors may be used, including but not limited to lentiviral vectors, vaccinia viral vectors, or non-viral expression vectors referred to as "naked DNA" constructs.

In one embodiment, Construct 1 can be used for delivering the transgene. Construct 1 is a recombinant adeno-associated virus serotype 9 capsid containing human iduronate-2-sulfatase expression cassette wherein expression is driven by a hybrid of the cytomegalovirus (CMV) enhancer and the chicken beta actin promoter (CB7), wherein the IDS expression cassette is flanked by inverted terminal repeats (ITRs) and the transgene includes the chicken beta actin intron and a rabbit beta-globin polyadenylation (polyA) signal. In a preferred embodiment, the ITRs are AAV2 ITRs. In one embodiment, Construct 1 comprises a nucleic acid comprising the nucleotide sequence of SEQ ID NO:45.

Pharmaceutical compositions suitable for administration to the CSF comprise a suspension of the rhIDS vector in a formulation buffer comprising a physiologically compatible aqueous buffer, a surfactant and optional excipients. In certain embodiments, the pharmaceutical compositions are suitable for intrathecal administration. In certain embodiments, the pharmaceutical compositions are suitable for intracisternal administration (injection into the cisterna magna). In certain embodiments, the pharmaceutical compositions are suitable for injection into the subarachnoid space via a C1-2 puncture. In certain embodiments, the pharmaceutical compositions are suitable for intracerebroventricular administration. In certain embodiments, the pharmaceutical compositions are suitable for administration via lumbar puncture.

Therapeutically effective doses of the recombinant vector should be administered to the CSF via intrathecal administration (i.e., injection into the subarachnoid space so that the recombinant vectors distribute through the CSF and transduce cells in the CNS). This can be accomplished in a number of ways - e.g., by intracranial (cisternal or ventricular) injection , or injection into the lumbar cistern. For example, intracisternal (IC) injection (into the cisterna magna) can be performed by CT-guided suboccipital puncture; or injection into the subarachnoid space can be performed via a C1-2 puncture when feasible for the patient; or lumbar puncture (typically diagnostic procedures performed in order to collect a sample of CSF) can be used to access the CSF. Alternatively, intracerebroventricular (ICV) administration (a more invasive technique used for the introduction of antiinfective or anticancer drugs that do not penetrate the blood-brain barrier) can be used to instill the recombinant vectors directly into the ventricles of the brain. Alternatively, intranasal administration may be used to deliver the recombinant vector to the CNS.

CSF concentrations can be monitored by directly measuring the concentration of rhIDS in the CSF fluid obtained from occipital or lumbar punctures, or estimated by extrapolation from concentrations of the rhIDS detected in the patient's serum.

In certain embodiments, the recombinant nucleotide expression vector is administered at a dose that is dependent on the human subject's brain mass, and wherein the brain mass is determined by brain magnetic resonance imaging (MRI) of the human subject's brain. In certain embodiments, the recombinant nucleotide expression vector is administered at a dose of about 1.3 × 10¹⁰ GC/g brain mass as determined by MRI. In certain embodiments, the recombinant nucleotide expression vector is administered at a dose of about 6.5 × 10¹⁰ GC/g brain mass as determined by MRI. In certain embodiments, the recombinant nucleotide expression vector is administered at a dose of about 2.0 × 10¹¹ GC/g brain mass as determined by MRI. In certain embodiments, the human subject's brain mass is converted from the human subject's brain volume by multiplying the human subject's brain volume in cm³ by a factor of 1.046 g/cm³, wherein the human subject's brain volume is obtained from the human subject's brain MRI.

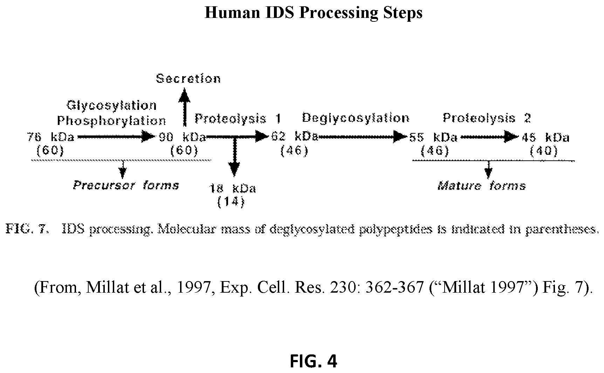

By way of background, human IDS is translated as a 550 amino acid polypeptide that contains eight potential N-glycosylation sites (N³¹, N¹¹⁵, N¹⁴⁴, N²⁴⁶, N²⁸⁰, N³²⁵, N⁵¹³ and N⁵³⁷) depicted in FIG.1 and includes a 25 amino acid signal sequence which is cleaved during processing. An initial 76 kDa intracellular precursor is converted into a phosphorylated 90 kDa precursor after modification of its oligosaccharide chains in the Golgi apparatus. This precursor is processed by glycosylation modifications and proteolytic cleavage through various intracellular intermediates to a major 55 kDa form. To summarize, after removal of the 25 aa signal sequence, proteolytic processing involves N-terminal proteolytic cleavage downstream of N³¹ removing a propeptide of eight amino acids (residues 26-33), and C-terminal proteolytic cleavage upstream of N⁵¹³ which releases an 18 kDa polypeptide and produces a 62 kDa intermediate that is converted to a 55 kDa mature form. Further proteolytic cleavage yields a 45 kDa mature form located in the lysosomal compartment. *(See* FIG. 4 for diagram reproduced from Millat et al., 1997, Exp Cell Res 230: 362-367 ("Millat 1997"); Millat et al. 1997, Biochem J. 326: 243-247 ("Millat 1997a"); and Froissart et al., 1995, Biochem J. 309:425-430, each of which is incorporated by reference herein in its entirety).

A formylglycine modification of C⁸⁴ (shown in bold in FIG. 1) required for enzyme activity probably occurs as an early post-translational or co-translational event, most probably in the endoplasmic reticulum. *(See,* Millat 1997a, citing Schmidt et al., 1995, Cell 82: 271-278). Post-translational processing continues in the Golgi to include addition of complex sialic acid-containing glycans and acquisition of mannose-6-phosphate residues which tag the enzyme for delivery to the lysosomal compartment. (*See,* Clarke, 2008, Expert Opin Pharmacother 9: 311-317 for a concise review which is incorporated by reference herein in its entirety). While no single glycosylation site is essential for IDS stability, glycosylation at position N²⁸⁰ is important for cellular internalization and lysosomal targeting via the mannose-6-phosphate (M6P) receptor. (Chung et al., 2014, Glycoconj J 31:309-315 at p. 310, first column). In the normal physiologic state, IDS is produced at very low levels and very little, if any, enzyme is secreted from the cell. (Clarke, 2008, *supra*)*.*

The invention is based, in part, on the following principles:
(i) Neuronal and glial cells in the CNS are secretory cells that possess the cellular machinery for post-translational processing of secreted proteins - including glycosylation, mannose-6-phosphorylation, and tyrosine-O-sulfation - robust processes in the CNS. *See, e.g.,* Sleat et al., 2005, Proteomics 5: 1520-1532, and Sleat 1996, J Biol Chem 271: 19191-98 which describes the human brain mannose-6-phosphate glycoproteome and notes that the brain contains more proteins with a much greater number of individual isoforms and mannose-6-phosphorylated proteins than found in other tissues; and Kanan et al., 2009, Exp. Eye Res. 89: 559-567 and Kanan & Al-Ubaidi, 2015, Exp. Eye Res. 133: 126-131 reporting the production of tyrosine-sulfated glycoproteins secreted by neuronal cells, each of which is incorporated by reference in its entirety for post-translational modifications made by human CNS cells.
(ii) The human brain produces multiple isoforms of natural/native IDS. In particular, N-terminal sequencing of human brain mannose-6-phosphorylated glycoproteins revealed that the N-terminal sequence of the mature 42 kDa chain of hIDS varies in the brain, starting at positions 34 or 36 as follows: T³⁴DALNVLLI; and A³⁶LNVLLIIV. (Sleat, 2005, Proteomics 5: 1520-1532, Table S2). Two of the eight N-linked glycosylation sites, namely N²⁸⁰ and N¹¹⁶, were found to be mannose-6-phophorylated in IDS obtained from human brain. (Sleat et al., 2006, Mol & Cell Proeomics 5.4: 686-701, reported at Table V).
(iii) During processing of hIDS, two polypeptides, 76 kDa and 90 kDa, are secreted by neural and glial cells, but only the 90 kDa polypeptide is mannose-6-phosphorylated, which is necessary for secreted forms of the enzyme to achieve cross correction. *(See,* Millat, 1997, Fig. 1 results for transduced lymphoblastoid cells, and Froissart 1995, Fig. 4 showing similar results for transduced fibroblasts - in culture medium, only the 90 kDa form is phosphorylated). Interestingly, it has been demonstrated that recombinant IDS produced by neuronal and glial cells may be endocytosed by recipient CNS cells more avidly than recombinant IDS produced by other cells such as kidney. Daniele 2002 (Biochimica et Biophysica Acta 1588(3):203-9) demonstrated M6P-receptor mediated endocytosis of recombinant IDS from conditioned media of transduced neuronal and glial cell cultures by a recipient population of non-transduced neuronal and glial cells which properly processed the precursor to the 45 kDa mature active form. Uptake of the recombinant IDS produced by the neuronal and glial cell lines (74% endocytosis) far exceeded uptake of the enzyme produced by a kidney cell line (5.6% endocytosis). In each case, uptake was inhibited by M6P, indicating that recombinant IDS uptake was M6P-receptor mediated. (See Daniele 2002, Tables 2 and 4 and accompanying description in Results at pp. 205-206 summarized in Table 1 below).

**Table 1. Summary of Results Reported in Daniele 2002**

| Cell Line Source of rIDS | Media Enzyme Units | Recipient Cells: Units Recovered | | % Endocytosis (mean value) |
|---|---|---|---|---|
| | | Neuronal | Glial | |
| Kidney ^{(transfected)} | 35 U | 1.7 U | 2.2 U | 5.6% |
| Neuronal ^{(Ad-transduced)} | 12 U | 8.8 U | 8.8 U | 74% |
| Glial ^{(Ad-transduced)} | 14 U | 10.5 U | 10.5 U | 74% |

(iv) The gene therapy approach described herein should result in the continuous secretion of an hIDS glycoprotein precursor of about 90 kDa as measured by polyacrylamide gel electrophoresis (depending on the assay used) that is enzymatically active. First, the enzyme responsible for the formylglycine modification of C⁸⁴ which is required for IDS activity -- the FGly-Generating Enzyme (FGE, aka SUMF1) -- is expressed in the cerebral cortex of the human brain (gene expression data for SUMF1 may be found, for example, at GeneCards, accessible at http://www.genecards.org). Second, the secreted glycosylated/phosphorylated rIDS produced by transduced neurons and glial cells *in situ* should be taken up and correctly processed by untransduced neural and glial cells in the CNS. Without being bound to any theory, it appears that the secreted rhIDS precursor produced *in situ* by gene therapy may be more avidly endocytosed by recipient cells in the CNS than would traditional recombinant enzymes used for ERT if administered to the CNS. For example, Elaprase^{®} (made in HT1080, a fibrosarcoma cell line) is a purified protein reported to have a molecular weight of about 76 kDa - not the 90 kDa species secreted by neuronal and glial cells that appears to be more heavily phosphorylated. While the eight N-linked glycosylation sites are reported to be fully occupied in Elaprase^{®} and contain two bis-mannose-6-phosphate terminated glycans as well as complex highly sialylated glycans, the post-translational modification of C⁸⁴ to FGly, which is an absolute requirement for enzyme activity, is only about 50%. (Clarke, 2008, Expert Opin Pharmacother 9:311-317; Elaprase^{®} Full Prescribing Information and EMA filing). Another recombinant product, Hunterase^{®} is made in CHO cells. While reported to have higher FGly and activity than Elaprase^{®}, mannose-6-phosphorylation and uptake did not differ. (Chung, 2014, Glycoconj J 31:309-315).
(v) The extracellular IDS efficacy *in vivo* depends on uptake (cell and lysosome internalization) through M6P and its active site formylglycine (FGly), which is converted from C⁸⁴ through post-translational modification by formylglycine-generating enzyme. As shown above in Table 1, brain cells (neuronal and glial cells) show higher enzyme activities when incubated with IDS precursor media secreted by transduced neuronal and glial cells than with IDS precursor media secreted by genetically engineered kidney cells. The resultant five-fold increase in activity can likely be attributed to the efficient uptake of IDS (See Daniele 2002, Tables 2 and 4). Commercial forms of IDS, which are generated by CHO cells or HT-1080 cells, have a FGly content of about about 50% to 70%, which determines the enzyme activity. However, neuronal and glial cells may improve upon this activity, due to improvement of IDS uptake.
(vi) The cellular and subcellular trafficking/uptake of lysosomal proteins, including IDS, is through M6P. IDS from brain cells may contain higher M6P content, as reported in Daniele 2002, and in Sleat, Proteomics, 2005 (indicating that the human brain contains more (in both a quantitative and qualitative sense) Man6-P glycoproteins than other tissues.). It is possible to measure the M6P content of an IDS precursor, as done in Daniele 2002. In the presence of inhibitory M6P (*e.g.,* 5 mM), the uptake of IDS precursor generated by non-neuronal or non-glial cells, such as the genetically engineered kidney cells of Daniele 2002, is predicted to decrease to levels close to that of the control cells, as was shown in Daniele 2002. While in the presence of inhibitory M6P, the uptake of IDS precursor generated by brain cells, such as neuronal and glial cells, is predicted to remain at a high level, as was shown in Daniele 2002, where the uptake was four times higher than control cells and comparable to the level of IDS activity (or uptake) of IDS precursor generated by genetically engineered kidney cells without the presence of inhibitory M6P. This assay allows for a way to predict the M6P content in IDS precursor generated by brain cells, and, in particular, to compare the M6P content in IDS precursors generated by different types of cells. The gene therapy approach described herein should result in the continuous secretion of an hIDS precursor that may be taken up into neuronal and glial cells at a high level in the presence of inhibitory M6P in such an assay.
(vii) The M6P content and uptake of IDS precursor may also be demonstrated by 90 kDa and 76 kDa gel bands (e.g., SDS-PAGE gel bands). The 90 kDa is reported to be highly glycosylated/phosphorylated and contains M6P, while 76 kDa is not. A very broad gel band with a range from 76 kDa to 95 kDa and with an average MW of 80-85 kDa, similar to the IDS precursor gel band generated from genetically engineered kidney cells (Daniele 2002, Figure 1), may be contrasted with a gel band of IDS precursor generated from brain cells. In Daniele 2002, the gel band cannot be obtained due to unsuccessful immunoprecipitation of the IDS precursor. The gene therapy approach described herein should result in the continuous secretion of an hIDS precursor that differs from the IDS precursor gel band generated from genetically engineered kidney cells.
(viii) The M6P content of commercial IDS precursor is 2 to 2.5 mol/mol, majority of which is present in a form of di-phosphorylated glycans. Although in average, every IDS precursor is phosphorylated, a normal distribution of glycans will have some IDS precursor with 2, 1 and 0 of di-phosphorylated M6P glycans assuming multiple phosphorylation sites. Uptake rate should be significant higher with multiple phosphorylation.
(ix) The glycosylation of hIDS by human cells of the CNS will result in the addition of glycans that can improve stability, half-life and reduce unwanted aggregation of the transgene product. Significantly, the glycans that are added to hIDS of the invention include 2,6-sialic acid, incorporating Neu5Ac ("NANA") but not its hydroxylated derivative, NeuGc (N-Glycolylneuraminic acid, *i.e.,* "NGNA" or "Neu5Gc"). Such glycans are not present in recombinant IDS products, such as Hunterase^{®}, made in CHO cells because CHO cells do not have the 2,6-sialyltransferase required to make this post-translational modification; nor do CHO cells produce bisecting GlcNAc, although they do add Neu5Gc (NGNA) as sialic acid not typical (and potentially immunogenic) to humans instead of Neu5Ac (NANA). *See, e.g.,* Dumont et al., 2016, Critical Rev in Biotech 36(6):1110-1122 (Early Online pp. 1-13 at p. 5); and Hague et al., 1998 Electrophor 19:2612-2630 ("[t]he CHO cell line is considered 'phenotypically restricted,' in terms of glycosylation, due to the lack of an α2,6-sialyl-transferase"). Moreover, CHO cells can also produce an immunogenic glycan, the α-Gal antigen, which reacts with anti-α-Gal antibodies present in most individuals, and at high concentrations can trigger anaphylaxis. *See, e.g.,* Bosques, 2010, Nat Biotech 28: 1153-1156. The human glycosylation pattern of the rhIDS of the invention should reduce immunogenicity of the transgene product and improve efficacy.
(x) Immunogenicity of a transgene product could be induced by various factors, including the immune condition of the patient, the structure and characteristics of the infused protein drug, the administration route, and the duration of treatment. Process-related impurities, such as host cell protein (HCP), host cell DNA, and chemical residuals, and product-related impurities, such as protein degradants and structural characteristics, such as glycosylation, oxidation and aggregation (sub-visible particles), may also increase immunogenicity by serving as an adjuvant that enhances the immune response. The amounts of process-related and product-related impurities can be affected by the manufacturing process: cell culture, purification, formulation, storage and handling, which can affect commercially manufactured IDS products. In gene therapy, proteins are produced *in vivo,* such that process-related impurities are not present and protein products are not likely to contain product-related impurities/degradants associated with proteins produced by recombinant technologies, such as protein aggregation and protein oxidation. Aggregation, for example, is associated with protein production and storage due to high protein concentration, surface interaction with manufacturing equipment and containers, and the purification process with certain buffer systems. But these conditions that promote aggregation are not present when a transgene is expressed *in vivo.* Oxidation, such as methionine, tryptophan and histidine oxidation, is also associated with protein production and storage, caused, for example, by stressed cell culture conditions, metal and air contact, and impurities in buffers and excipients. The proteins expressed *in vivo* may also oxidize in a stressed condition, but humans, like many organisms, are equipped with an antioxidation defense system, which not only reduces the oxidation stress, but can also repairs and/or reverses the oxidation. Thus, proteins produced *in vivo* are not likely to be in an oxidized form. Both aggregation and oxidation could affect the potency, PK (clearance) and can increase immunogenicity concerns. The gene therapy approach described herein should result in the continuous secretion of an hIDS precursor with a reduced immunogenicity compared to commercially manufactured products.
(xi) In addition to the N-linked glycosylation sites, hIDS contains a tyrosine ("Y") sulfation site (PSSEKY¹⁶⁵ENTKTCRGPD). (*See, e.g.,* Yang et al., 2015, Molecules 20:2138-2164, esp. at p. 2154 which is incorporated by reference in its entirety for the analysis of amino acids surrounding tyrosine residues subjected to protein tyrosine sulfation. The "rules" can be summarized as follows: Y residues with E or D within +5 to -5 position of Y, and where position -1 of Y is a neutral or acidic charged amino acid - but not a basic amino acid, *e.g.,* R, K, or H that abolishes sulfation). While not intending to be bound by any theory, sulfation of this site in hIDS may improve stability of the enzyme and binding affinity for substrate. Tyrosine-sulfation of hIDS - a robust post-translational process in human CNS cells - should result in improved processing and activity of transgene products. The significance of tyrosine-sulfation of lysosomal proteins has not been elucidated; but in other proteins it has been shown to increase avidity of protein-protein interactions (antibodies and receptors), and to promote proteolytic processing (peptide hormone). (See, Moore, 2003, J Biol. Chem. 278: 24243-46; and Bundegaard et al., 1995, The EMBO J 14: 3073-79). The tyrosylprotein sulfotransferase (TPST1) responsible for tyrosine-sulfation (which may occur as a final step in IDS processing) is apparently expressed at higher levels (based on mRNA) in the brain (gene expression data for TPST1 may be found, for example, at the EMBL-EBI Expression Atlas, accessible at http://www.ebi.ac.uk/gxa/home). Such post-translational modification, at best, is under-represented in CHO cell products. Unlike human CNS cells, CHO cells are not secretory cells and have a limited capacity for post-translational tyrosine-sulfation. *(See, e.g.,* Mikkelsen & Ezban, 1991, Biochemistry 30: 1533-1537, esp. discussion at p. 1537).

For the foregoing reasons, the production of rhIDS by human neuronal and/or glial cells should result in a "biobetter" molecule for the treatment of MPS II accomplished via gene therapy - *e.g.,* by administering a viral vector or other DNA expression construct encoding rhIDS to the CSF of a patient (human subject) diagnosed with an MPS II disease (including but not limited to Hunter) to create a permanent depot in the CNS that continuously supplies a fully human-glycosylated, mannose-6-phosphorylated, sulfated transgene product secreted by the transduced CNS cells. The hIDS transgene product secreted from the depot into the CSF will be endocytosed by cells in the CNS, resulting in "cross-correction" of the enzymatic defect in the MPS II recipient cells.

It is not essential that every rhIDS molecule produced either in the gene therapy or protein therapy approach be fully glycosylated, phosphorylated, and sulfated. Rather, the population of glycoproteins produced should have sufficient glycosylation (including 2,6-sialylation and mannose-6-phophorylation) and sulfation to demonstrate efficacy. The goal of gene therapy treatment of the invention is to slow or arrest the progression of disease. Efficacy may be monitored by measuring cognitive function (e.g., prevention or decrease in neurocognitive decline); reductions in biomarkers of disease (such as GAG) in CSF and or serum; and/or increase in IDS enzyme activity in CSF and/or serum. Signs of inflammation and other safety events may also be monitored.

As an alternative, or an additional treatment to gene therapy, the rhIDS glycoprotein can be produced in human neural or glial cell lines by recombinant DNA technology and the glycoprotein can be administered to patients diagnosed with MPS II systemically and/or into the CSF for ERT). Human cell lines that can be used for such recombinant glycoprotein production include but are not limited to HT-22, SK-N-MC, HCN-1A, HCN-2, NT2, SH-SY5y, hNSC11, or ReNcell VM *(see, e.g.,* Dumont et al., 2016, Critical Rev in Biotech 36(6):1110-1122 "Human cell lines for biopharmaceutical manufacturing: history, status, and future perspectives" which is incorporated by reference in its entirety for a review of the human cell lines that could be used for the recombinant production of the rHuGlyIDS glycoprotein). To ensure complete glycosylation, especially sialylation, and tyrosine-sulfation, the cell line used for production can be enhanced by engineering the host cells to co-express α-2,6-sialyltransferase (or both α-2,3-and α-2,6-sialyltransferases) and/or TPST-1 and TPST-2 enzymes responsible for tyrosine-O-sulfation.

While the delivery of rhIDS should minimize immune reactions, the clearest potential source of toxicity related to CNS-directed gene therapy is generating immunity against the expressed rhIDS protein in human subjects who are genetically deficient for IDS and, therefore, potentially not tolerant of the protein and/or the vector used to deliver the transgene.

Thus, in a preferred embodiment, it is advisable to co-treat the patient with immune suppression therapy -- especially when treating patients with severe disease who have close to zero levels of IDS. Immune suppression therapies involving a regimen of tacrolimus or rapamycin (sirolimus) in combination with mycophenolic acid, or other immune suppression regimens used in tissue transplantation procedures can be employed. Such immune suppression treatment may be administered during the course of gene therapy, and in certain embodiments, pre-treatment with immune suppression therapy may be preferred. Immune suppression therapy can be continued subsequent to the gene therapy treatment, based on the judgment of the treating physician, and may thereafter be withdrawn when immune tolerance is induced; *e.g*., after 180 days.

Combinations of delivery of the rhIDS to the CSF accompanied by delivery of other available treatments are encompassed by the methods of the invention. The additional treatments may be administered before, concurrently or subsequent to the gene therapy treatment. Available treatments for MPS II that could be combined with the gene therapy of the invention include but are not limited to enzyme replacement therapy using Elaprase^{®} administered systemically or to the CSF; and/or HSCT therapy.

In one aspect, provided herein is a method for treating a human subject diagnosed with MPS II, comprising delivering to the CSF of the human subject a therapeutically effective amount of a glycosylated recombinant human IDS precursor produced by human neuronal or human glial cells,wherein the glycosylated recombinant human IDS precursor is delivered by administration of a recombinant nucleotide expression vector encoding human IDS, wherein the recombinant nucleotide expression vector is administered at a dose that is dependent on the human subject's brain mass, and wherein the brain mass is determined by brain MRI of the human subject's brain.

In another aspect, provided herein is a method for treating a human subject diagnosed with MPS II, comprising, in the following order: (a) delivering to the CSF of the human subject a therapeutically effective amount of a glycosylated recombinant human IDS precursor produced by human neuronal or human glial cells; (b) measuring level of heparan sulfate in the CSF of the human subject; and (c) comparing the level of heparan sulfate in the CSF of the human subject with level of heparan sulfatae in a reference population; wherein the glycosylated recombinant human IDS precursor is delivered by administration of a recombinant nucleotide expression vector encoding human IDS, wherein the recombinant nucleotide expression vector is administered at a dose that is dependent on the human subject's brain mass, and wherein the brain mass is determined by brain magnetic resonance imaging (MRI) of the human subject's brain. In certain embodiments, the reference population consists of: (a) at least 1, 2, 3, 4, 5, 10, 25, 50, 75, 100, 200, 250, 300, 400, 500, or 1000 individual healthy people without MPS II, preferably of similar age, weight, and/or of the same gender as the human subject.

In another aspect, provided herein is a method for treating a human subject diagnosed with MPS II, comprising, in the following order: (a) taking a first measurement of the level of heparan sulfate in the CSF of the human subject; (b) delivering to the CSF of the human subject a therapeutically effective amount of a glycosylated recombinant human IDS precursor produced by human neuronal or human glial cells; and (c) after a period of time, taking a second measurement of the level of heparan sulfate; wherein the glycosylated recombinant human IDS precursor is delivered by administration of a recombinant nucleotide expression vector encoding human IDS, wherein the recombinant nucleotide expression vector is administered at a dose that is dependent on the human subject's brain mass, and wherein the brain mass is determined by brain magnetic resonance imaging (MRI) of the human subject's brain. In certain embodiments, the period of time is about 1 day, 2 days, 3 days, 4 days, 5 days, 6 days, 1 week, 2 weeks, 3 weeks, 4 weeks, 2 months, 3 moths, 4 months, 5 months, 6 months, 7 months, 8 months, 11 months, or 1 year.

In certain embodiments of the method for treating described herein, the glycosylated recombinant human IDS precursor is delivered to lysosomes of cells in the CNS of the human subject.

In certain embodiments of the method for treating described herein, the human subject's brain mass is converted from the human subject's brain volume by multiplying the human subject's brain volume in cm³ by a factor of 1.046 g/cm³, wherein the human subject's brain volume is determined by brain MRI of the subject's brain.

In certain embodiments of the method for treating described herein, the recombinant nucleotide expression vector is administered at a dose of about 1.3 × 10¹⁰ GC/g brain mass as determined by MRI, or about 6.5 × 10¹⁰ GC/g brain mass as determined by MRI. In certain embodiments of the method for treating described herein, the recombinant nucleotide expression vector is administered at a dose of about 2.0 × 10¹¹ GC/g brain mass as determined by MRI.

In various embodiments of the method for treating described herein, the human subject is 5 years old or older and less than 18 years old. In specific embodiments, the human subject is 5, 6, 7, 8, 9, 10, 11, 12, 13, 14, 15, 16, 17 or 18 years old. In specific embodiments, the human subject is about 5, 6, 7, 8, 9, 10, 11, 12, 13, 14, 15, 16, 17 or 18 years old. In specific embodiments, the human subject is 5-6, 6-7, 7-8, 8-9, 9-10, 10-11, 11-12, 12-13, 13-14, 14-15, 15-16, 16-17, 17-18 or 18-19 years old. In specific embodiments, the human subject is about 5-6, 6-7, 7-8, 8-9, 9-10, 10-11, 11-12, 12-13, 13-14, 14-15, 15-16, 16-17, 17-18 or 18-19 years old. In certain embodiments, the recombinant nucleotide expression vector is administered at a dose of about 6.5 × 10¹⁰ GC/g brain mass as determined by MRI. In certain embodiments, the recombinant nucleotide expression vector is administered at a dose according to Table 7.

In various embodiments of the method for treating described herein, the human subject is 4 months old or older and less than 5 years old. In specific embodiments, the human subject is 4, 5, 6, 7, 8, 9, 10, or 11 months old. In specific embodiments, the human subject is about 4, 5, 6, 7, 8, 9, 10, or 11 months old. In specific embodiments, the human subject is 4-5, 5-6, 6-7, 7-8, 8-9, 9-10, 10-11, or 11-12 months old. In specific embodiments, the human subject is about 4-5, 5-6, 6-7, 7-8, 8-9, 9-10, 10-11, or 11-12 months old. In specific embodiments, the human subject is 1, 2, 3, 4, or 5 years old. In specific embodiments, the human subject is about 1, 2, 3, 4, or 5 years old. In specific embodiments, the human subject is 1-2, 2-3, 3-4, 4-5, or 5-6 years old. In specific embodiments, the human subject is about 1-2, 2-3, 3-4, 4-5, or 5-6 years old. In certain embodiments, the recombinant nucleotide expression vector is administered at a dose of about 1.3 × 10¹⁰ GC/g brain mass as determined by MRI. In certain embodiments, the recombinant nucleotide expression vector is administered at a dose of about 6.5 × 10¹⁰ GC/g brain mass as determined by MRI. In certain embodiments, the recombinant nucleotide expression vector is administered at a dose of about 2.0 × 10¹¹ GC/g brain mass as determined by MRI. In certain embodiments, the recombinant nucleotide expression vector is administered at a dose chosen from Dose 1 or Dose 2 according to Table 5. In certain embodiments, the recombinant nucleotide expression vector is administered at a dose according to Table 6.

In some embodiments of the method for treating described herein, the recombinant nucleotide expression vector is administered via intracisternal (IC) administration. In other embodiments of the method for treating described herein, the recombinant nucleotide expression vector is administered via intracerebroventricular (ICV) administration.

In certain embodiments of the method for treating described herein, the recombinant nucleotide expression vector is administered at a volume that does not exceed 10% of the total cerebrospinal fluid volume of the human subject.

In certain embodiments of the method for treating described herein, the glycosylated recombinant human IDS precursor is secreted at a detectable level.

In certain embodiments of the method for treating described herein, the human neuronal or human glial cells carry at least one mutation in the endogenous gene encoding human IDS precursor.

In certain embodiments of the method for treating described herein, the human neuronal or human glial cells are transduced with a recombinant adeno-associated virus vector (rAAV).

In a preferred embodiment, the recombinant nucleotide expression vector is an AAV9 or AAVrh10 vector.

In certain embodiments of the method for treating described herein, the glycosylated recombinant human IDS precursor is expressed under the control of a CB7 promoter.

In certain embodiments of the method for treating described herein, the glycosylated recombinant human IDS precursor is expressed from a cDNA encoding human IDS precursor.

In certain embodiments of the method for treating described herein, the glycosylated recombinant human IDS precursor is about 90 kDa as measured by polyacrylamide gel electrophoresis.

In certain embodiments of the method for treating described herein, the glycosylated recombinant human IDS precursor contains a formylglycine.

In certain embodiments of the method for treating described herein, the glycosylated recombinant human IDS precursor (a) is α2,6-sialylated; (b) does not contain detectable NeuGc; (c) does not contain detectable α-Gal antigen; (d) contains tyrosine-sulfation; and/or (e) is mannose-6-phosphorylated.

In certain embodiments of the method for treating described herein, the glycosylated recombinant human IDS precursor comprises the amino acid sequence of SEQ ID NO. 1.

In certain embodiments provided herein, the method further comprising administering an immune suppression therapy to the human subject before or concurrently with the human IDS precursor treatment and optionally continuing immune suppression therapy thereafter.

In some embodiments, the immune suppression therapy comprises administering one or more corticosteroids, sirolimus, and/or tacrolimus. In a specific embodiment, the one or more corticosteroids are methylprednisolone and/or prednisone.

In some embodiments, the method further comprises administering one or more antibiotics to the human subject before or concurrently with the immune suppression therapy. In a specific embodiment, the one or more antibiotics are trimethoprim, sulfamethoxazole, pentamidine, dapsone, and/or atovaquone.

In some embodiments, the method further comprises administering one or more antifungal therapies to the human subject before or concurrently with the immune suppression therapy.

In some embodiments, the method further comprises a step of measuring one or more of the following biomarkers after administration of the recombinant nucleotide expression vector: (a) level of glycosaminoglycans (GAGs) in CSF; (b) level of iduronate-2-sulfatase (I2S) in CSF; (c) level of GAGs in plasma; (d) level of I2S in plasma; (e) level of leukocyte I2S enzyme activity; and (f) level of GAGs in urine. In a specific embodiment, the GAGs in CSF comprise heparin sulfate in CSF. In another specific embodiment, the GAGs in CSF are heparin sulfate in CSF. In another specific embodiment, the GAGs in plasma comprise heparin sulfate in plasma. In another specific embodiment, the GAGs in plasma are heparin sulfate in plasma. In another specific embodiment, the GAGs in urine comprise heparin sulfate in urine. In another specific embodiment, the GAGs in urine are heparin sulfate in urine. In a specific embodiment, the step of measuring comprises mearing level of heparin sulfate in CSF. In another specific embodiment, the step of measuring comprises measuring level of leukocyte I2S enzyme activity.

### 3.1 ILLUSTRATIVE EMBODIMENTS

### 3.1.1. Set 1

1. A method for treating a human subject diagnosed with mucopolysaccharidosis type II (MPS II), comprising delivering to the cerebrospinal fluid (CSF) of the human subject a therapeutically effective amount of a glycosylated recombinant human iduronate-2-sulfatase (IDS) precursor produced by human neuronal or human glial cells, wherein the glycosylated recombinant human IDS precursor is delivered by administration of a recombinant nucleotide expression vector encoding human IDS, wherein the recombinant nucleotide expression vector is administered at a dose that is dependent on the human subject's brain mass, and wherein the brain mass is determined by brain magnetic resonance imaging (MRI) of the human subject's brain.
2. The method of paragraph 1, wherein the glycosylated recombinant human IDS precursor is secreted at a detectable level.
3. The method of paragraph 1 or 2, wherein the human neuronal or human glial cells carry at least one mutation in the endogenous gene encoding human IDS precursor.
4. The method of any one of paragraphs 1-3, wherein the human neuronal or human glial cells are transduced with a recombinant adeno-associated virus vector (rAAV).
5. The method of any one of paragraphs 1-4, wherein the glycosylated recombinant human IDS precursor is expressed under the control of a CB7 promoter.
6. The method of any one of paragraphs 1-5, wherein the glycosylated recombinant human IDS precursor is expressed from a cDNA encoding human IDS precursor.
7. The method of any one of paragraphs 1-6, wherein the glycosylated recombinant human IDS precursor is about 90 kDa as measured by polyacrylamide gel electrophoresis.
8. The method of any one of paragraphs 1-7, wherein the glycosylated recombinant human IDS precursor contains a formylglycine.
9. The method of any one of paragraphs 1-8, wherein the glycosylated recombinant human IDS precursor (a) is α2,6-sialylated; (b) does not contain detectable NeuGc; (c) does not contain detectable α-Gal antigen; (d) contains tyrosine-sulfation; and/or (e) is mannose-6-phosphorylated.
10. The method of any one of paragraphs 1-9, in which the glycosylated recombinant human IDS precursor comprises the amino acid sequence of SEQ ID NO. 1.
11. The method of any one of paragraphs 1-10, wherein the recombinant nucleotide expression vector is an AAV9 or AAVrh10 vector.
12. The method of any one of paragraphs 1-11, wherein the human subject's brain mass is converted from the human subject's brain volume by multiplying the human subject's brain volume in cm³ by a factor of 1.046 g/cm³, wherein the human subject's brain volume is obtained from the human subject's brain MRI.
13. The method of any one of paragraphs 1-12, wherein the recombinant nucleotide expression vector is administered at a dose of about 1.3 × 10¹⁰ GC/g brain mass as determined by MRI, or about 6.5 × 10¹⁰ GC/g brain mass as determined by MRI.
14. The method of any one of paragraphs 1-13, wherein the recombinant nucleotide expression vector is administered via intracisternal (IC) administration.
15. The method of any one of paragraphs 1-13, wherein the recombinant nucleotide expression vector is administered via intracerebroventricular (ICV) administration.
16. The method of any one of paragraphs 1-15, wherein the recombinant nucleotide expression vector is administered at a volume that does not exceed 10% of the total cerebrospinal fluid volume of the human subject.
17. The method of any one of paragraphs 1-16, wherein the glycosylated recombinant human IDS precursor is delivered to lysosomes of cells in the CNS of the human subject.
18. The method of any one of paragraphs 1-17, further comprising administering an immune suppression therapy to the human subject before or concurrently with the human IDS precursor treatment and optionally continuing immune suppression therapy thereafter.
19. The method of paragraph 18, wherein the immune suppression therapy comprises administering one or more corticosteroids, sirolimus, and/or tacrolimus.
20. The method of paragraph 19, wherein the one or more corticosteroids are methylprednisolone and/or prednisone.
21. The method of any one of paragraphs 18-20, further comprising administering one or more antibiotics to the human subject before or concurrently with the immune suppression therapy.
22. The method of paragraph 21, wherein the one or more antibiotics are trimethoprim, sulfamethoxazole, pentamidine, dapsone, and/or atovaquone.
23. The method of any one of paragraphs 18-22, further comprising administering one or more antifungal therapies to the human subject before or concurrently with the immune suppression therapy.
24. The method of any one of paragraphs 1-23, further comprising a step of measuring one or more of the following biomarkers after administration of the recombinant nucleotide expression vector: (a) level of glycosaminoglycans (GAGs) in CSF; (b) level of iduronate-2-sulfatase (I2S) in CSF; (c) level of GAGs in plasma; (d) level of I2S in plasma; (e) level of leukocyte I2S enzyme activity; and (f) level of GAGs in urine.
25. The method of paragraph 24, wherein the GAGs in CSF comprise heparin sulfate in CSF.
26. The method of paragraph 24, wherein the GAGs in CSF are heparin sulfate in CSF.
27. The method of any one of paragraphs 24-26, wherein the GAGs in plasma comprise heparin sulfate in plasma.
28. The method of any one of paragraphs 24-26, wherein the GAGs in plasma are heparin sulfate in plasma.
29. The method of any one of paragraphs 24-28, wherein the GAGs in urine comprise heparin sulfate in urine.
30. The method of any one of paragraphs 24-28, wherein the GAGs in urine are heparin sulfate in urine.
31. The method of any one of paragraphs 24-30, wherein the step of measuring comprises mearing level of heparin sulfate in CSF.
32. The method of any one of paragraphs 24-31, wherein the step of measuring comprises measuring level of leukocyte I2S enzyme activity.

### 3.1.2. Set 2

1. A method for treating a human subject diagnosed with mucopolysaccharidosis type II (MPS II), comprising delivering to the cerebrospinal fluid (CSF) of the human subject a therapeutically effective amount of a glycosylated recombinant human iduronate-2-sulfatase (IDS) precursor produced by human neuronal or human glial cells, wherein the glycosylated recombinant human IDS precursor is delivered by administration of a recombinant nucleotide expression vector encoding human IDS, wherein the recombinant nucleotide expression vector is administered at a dose that is dependent on the human subject's brain mass, and wherein the brain mass is determined by brain magnetic resonance imaging (MRI) of the human subject's brain.
2. The method of paragraph 1, wherein the glycosylated recombinant human IDS precursor is secreted at a detectable level.
3. The method of paragraph 1 or 2, wherein the human neuronal or human glial cells carry at least one mutation in the endogenous gene encoding human IDS precursor.
4. The method of any one of paragraphs 1-3, wherein the human neuronal or human glial cells are transduced with a recombinant adeno-associated virus vector (rAAV).
5. The method of any one of paragraphs 1-4, wherein the glycosylated recombinant human IDS precursor is expressed under the control of a CB7 promoter.
6. The method of any one of paragraphs 1-5, wherein the glycosylated recombinant human IDS precursor is expressed from a cDNA encoding human IDS precursor.
7. The method of any one of paragraphs 1-6, wherein the glycosylated recombinant human IDS precursor is about 90 kDa as measured by polyacrylamide gel electrophoresis.
8. The method of any one of paragraphs 1-7, wherein the glycosylated recombinant human IDS precursor contains a formylglycine.
9. The method of any one of paragraphs 1-8, wherein the glycosylated recombinant human IDS precursor (a) is α2,6-sialylated; (b) does not contain detectable NeuGc; (c) does not contain detectable α-Gal antigen; (d) contains tyrosine-sulfation; and/or (e) is mannose-6-phosphorylated.
10. The method of any one of paragraphs 1-9, in which the glycosylated recombinant human IDS precursor comprises the amino acid sequence of SEQ ID NO. 1.
11. The method of any one of paragraphs 1-10, wherein the recombinant nucleotide expression vector is an AAV9 or AAVrh10 vector.
12. The method of any one of paragraphs 1-11, wherein the human subject's brain mass is converted from the human subject's brain volume by multiplying the human subject's brain volume in cm³ by a factor of 1.046 g/cm³, wherein the human subject's brain volume is obtained from the human subject's brain MRI.
13. The method of any one of paragraphs 1-12, wherein the recombinant nucleotide expression vector is administered at a dose of about 1.3 × 10¹⁰ GC/g brain mass as determined by MRI, or about 6.5 × 10¹⁰ GC/g brain mass as determined by MRI.
14. The method of any one of paragraphs 1-12, wherein the recombinant nucleotide expression vector is administered at a dose of about 2.0 × 10¹¹ GC/g brain mass as determined by MRI.
15. The method of any one of paragraphs 1-12, wherein the human subject is 5 years old or older and less than 18 years old.
16. The method of paragraph 15, wherein the recombinant nucleotide expression vector is administered at a dose of about 6.5 × 10¹⁰ GC/g brain mass as determined by MRI.
17. The method of paragraph 15, wherein the recombinant nucleotide expression vector is administered at a dose according to the table below:

| **Brain Mass (in g)** | | **Target Brain Mass (in g)** | **Dose: Total GC (6.5 × 10¹⁰ GC/g brain mass)** |
|---|---|---|---|
| **Min** | **Max** | | |
| 801 | 900 | 850 | 5.5 × 10¹³ |
| 901 | 1050 | 975 | 6.3 × 10¹³ |
| 1051 | 1200 | 1125 | 7.3 × 10¹³ |
| 1201 | - | 1300 | 8.5 × 10¹³ |

18. The method of any one of paragraphs 1-12, wherein the human subject is 4 months old or older and less than 5 years old.
19. The method of paragraph 18, wherein the recombinant nucleotide expression vector is administered at a dose chosen from Dose 1 or Dose 2 according to the table below:

| **Brain Mass (in g)** | | **Target** | **Dose Levels** | |
|---|---|---|---|---|
| **Min** | **Max** | | **Dose 1 Total GC** (1.3 × 10¹⁰ GC/g brain mass) | **Dose 2 Total GC** (6.5 × 10¹⁰ GC/g brain mass) |
| - | 700 | 650 | 8.5 × 10¹² | 4.2 × 10¹³ |
| 701 | 800 | 750 | 9.8 × 10¹² | 4.9 × 10¹³ |
| 801 | 900 | 850 | 1.1 × 10¹³ | 5.5 × 10¹³ |
| 901 | 1050 | 975 | 1.3 × 10¹³ | 6.3 × 10¹³ |
| 1051 | 1200 | 1125 | 1.5× 10¹³ | 7.3 × 10¹³ |
| 1201 | - | 1300 | 1.7 × 10¹³ | 8.5 × 10¹³ |

20. The method of paragraph 18, wherein the recombinant nucleotide expression vector is administered at a dose according to the table below:

| **Brain Mass (in g)** | | **Target** | **Dose 3 Total GC** (2.0 × 10¹¹ GC/g brain mass) |
|---|---|---|---|
| **Min** | **Max** | | |
| - | 474 | 450 | 9.0 × 10¹³ |
| 475 | 524 | 500 | 1.0 × 10¹⁴ |
| 525 | 574 | 550 | 1.1 × 10¹⁴ |
| 575 | 624 | 600 | 1.2 × 10¹⁴ |
| 625 | 674 | 650 | 1.3 × 10¹⁴ |
| 675 | 724 | 700 | 1.4 × 10¹⁴ |
| 725 | 774 | 750 | 1.5 × 10¹⁴ |
| 775 | 824 | 800 | 1.6 × 10¹⁴ |
| 825 | 874 | 850 | 1.7 × 10¹⁴ |
| 875 | 924 | 900 | 1.8 × 10¹⁴ |
| 925 | 974 | 950 | 1.9 × 10¹⁴ |
| 975 | 1024 | 1000 | 2.0 × 10¹⁴ |
| 1025 | 1074 | 1050 | 2.1 × 10¹⁴ |
| 1075 | 1124 | 1100 | 2.2 × 10¹⁴ |
| 1125 | 1174 | 1150 | 2.3 × 10¹⁴ |
| 1175 | 1224 | 1200 | 2.4 × 10¹⁴ |
| 1225 | 1274 | 1250 | 2.5 × 10¹⁴ |
| 1275 | >1300 | 1300 | 2.6 × 10¹⁴ |

21. The method of any one of paragraphs 1-20, wherein the recombinant nucleotide expression vector is administered via intracisternal (IC) administration.
22. The method of any one of paragraphs 1-20, wherein the recombinant nucleotide expression vector is administered via intracerebroventricular (ICV) administration.
23. The method of any one of paragraphs 1-22, wherein the recombinant nucleotide expression vector is administered at a volume that does not exceed 10% of the total cerebrospinal fluid volume of the human subject.
24. The method of any one of paragraphs 1-23, wherein the glycosylated recombinant human IDS precursor is delivered to lysosomes of cells in the CNS of the human subject.
25. The method of any one of paragraphs 1-24, further comprising administering an immune suppression therapy to the human subject before or concurrently with the human IDS precursor treatment and optionally continuing immune suppression therapy thereafter.
26. The method of paragraph 25, wherein the immune suppression therapy comprises administering one or more corticosteroids, sirolimus, and/or tacrolimus.
27. The method of paragraph 26, wherein the one or more corticosteroids are methylprednisolone and/or prednisone.
28. The method of any one of paragraphs 25-27, further comprising administering one or more antibiotics to the human subject before or concurrently with the immune suppression therapy.
29. The method of paragraph 28, wherein the one or more antibiotics are trimethoprim, sulfamethoxazole, pentamidine, dapsone, and/or atovaquone.
30. The method of any one of paragraphs 25-29, further comprising administering one or more antifungal therapies to the human subject before or concurrently with the immune suppression therapy.
31. The method of any one of paragraphs 1-30, further comprising a step of measuring one or more of the following biomarkers after administration of the recombinant nucleotide expression vector: (a) level of glycosaminoglycans (GAGs) in CSF; (b) level of iduronate-2-sulfatase (I2S) in CSF; (c) level of GAGs in plasma; (d) level of I2S in plasma; (e) level of leukocyte I2S enzyme activity; and (f) level of GAGs in urine.
32. The method of paragraph 31, wherein the GAGs in CSF comprise heparin sulfate in CSF.
33. The method of paragraph 31, wherein the GAGs in CSF are heparin sulfate in CSF.
34. The method of any one of paragraphs 31-33, wherein the GAGs in plasma comprise heparin sulfate in plasma.
35. The method of any one of paragraphs 31-33, wherein the GAGs in plasma are heparin sulfate in plasma.
36. The method of any one of paragraphs 31-35, wherein the GAGs in urine comprise heparin sulfate in urine.
37. The method of any one of paragraphs 31-35, wherein the GAGs in urine are heparin sulfate in urine.
38. The method of any one of paragraphs 31-37, wherein the step of measuring comprises mearing level of heparin sulfate in CSF.
39. The method of any one of paragraphs 31-38, wherein the step of measuring comprises measuring level of leukocyte I2S enzyme activity.

### 4. BRIEF DESCRIPTION OF THE DRAWINGS

**FIG. 1****.** The amino acid sequence of human IDS. A post-translational formylglycine modification of C⁸⁴ (shown in bold in FIG. 1) is required for enzyme activity. Eight N linked glycosylation sites (N³¹, N¹¹⁵, N¹⁴⁴, N²⁴⁶, N²⁸⁰, N³²⁵, N⁵¹³ and N⁵³⁷) are bold and boxed. One tyrosine-O-sulfation site (Y) is bold and the full sulfation site sequence (PSSEKY¹⁶⁵ENTKTCRGPD) is boxed. The N-terminus of the mature 42 kDa and mature 14 kDa polypeptides are indicated by horizontal arrows. In the brain, the N-terminus of the mature 42 kDa form starts at positions 34 or 36 as follows: T³⁴DALNVLLI; and A³⁶LNVLLIIV as indicated in FIG. 1. *(See,* Sleat, 2005, Proteomics 5: 1520-1532, Table S2). Two of the eight N-linked glycosylation sites, namely N²⁸⁰ and N¹¹⁶, are mannose-6-phophorylated in IDS obtained from human brain. (Sleat et al., 2006, Mol & Cell Proeomics 5.4: 686-701, reported at Table V).
**FIG. 2****.** Multiple sequence alignment of hIDS with known orthologs. The names of the species and protein IDs are as follows: SP|P22304|IDS_HUMAN [Homo sapiens]; TR|K6ZGI9_PANTR [Pan troglodytes (Chimpanzee)]; TR|K7BKV4_PANTR [Pan troglodytes (Chimpanzee)]; TR|H9FTX2_MACMU [Macaca mulatta (Rhesus macaque)]; TRF7EJG2_CALJA [Callithrix jacchus (White-tufted-ear marmoset)]; TR|U3DTL8_CALJA [Callithrix jacchus (White-tufted-ear marmoset)]; TR|G7NRX7_MACMU [Macaca mulatta (Rhesus macaque)]; TR|G7Q1V9_MACFA [Macaca fascicularis (Crab-eating macaque; Cynomologous monkey)]; TR|H2PX10_PONAB [Pongo abelii (Sumatran orangutan)]; TR|A0A0D9R4D1_CHLSB [Chlorocebus sabaeus (Green monkey)]; TR|G1RST8|G1RST8_NOMLE [Nomascus leucogenys (Northern white-cheeked gibbon)]; UPI0000D9F625 [Macaca mulatta (Rhesus macaque)]; UPI000274358B [Pan paniscus (Pygmy chimpanzee; Bonobo)]; UPI00027F6FC5 [Papio Anubis (Olive baboon)]; UPI00027FAE03 [Saimiri boliviensis (Bolivian squirrel monkey)]; UPI0003ABBF28 [Macaca fascicularis (Crab-eating macaque; Cynomologous monkey)]; UPI000533297F [Rhinopithecus roxellana (Golden snub-nosed monkey; Pygathrix roxellana)]; UPI0005F40BD2 [Colobus angolensis palliates (Peters' Angolan colobus)] (SEQ ID NOs: 27-44).
**FIG. 3****.** MPS II mutations in hIDS and corresponding disease phenotypes, mild, intermediate or severe. (from Uniprot).
**FIG. 4****.** Human IDS processing as reported in Millat et al., 1997, Exp. Cell. Res. 230: 362-367, at Fig.7.
**FIG. 5****.** Schematic Representation of Construct 1.
**FIG. 6****.** Clustal Multiple Sequence Alignment of AAV capsids 1 - 9 (SEQ ID NOs: 16-26). Amino acid substitutions (shown in bold in the bottom rows) can be made to AAV9 and AAV8 capsids by "recruiting" amino acid residues from the corresponding position of other aligned AAV capsids. Sequence regions designated by "HVR" = hypervariable regions.

### 5. DETAILED DESCRIPTION OF THE INVENTION

The invention involves the delivery of recombinant human iduronate-2-sulfatase (rhIDS) produced by human neuronal or glial cells to the cerebrospinal fluid (CSF) of the central nervous system (CNS) of a human subject diagnosed with mucopolysaccharidosis II (MPS II), including, but not limited to patients diagnosed with Hunter syndrome. See, also, International Patent Application No. PCT/US2017/027770, filed April 14, 2017 (published as WO/2017/181113 on October 19, 2017), which is incorporated by reference herein in its entirety, for compositions and methods that can be used according to the invention described herein.

In a preferred embodiment, the treatment is accomplished via gene therapy - *e.g.,* by administering a viral vector or other DNA expression construct encoding human IDS (hIDS), or a derivative of hIDS, to the CSF of a patient (human subject) diagnosed with MPS II, so that a permanent depot of transduced neuronal and/or glial cells is generated that continuously supplies the transgene product to the CNS. The rhIDS secreted from the neuronal/glial cell depot into the CSF will be endocytosed by cells in the CNS, resulting in "cross-correction" of the enzymatic defect in the recipient cells. Moreover, it has been found, unexpectedly, that the depot of transduced neural and glial cells in the CNS can deliver the recombinant enzyme to both the CNS and systemically, which may reduce or eliminate the need for systemic treatment, *e.g*., weekly i.v. injections of the enzyme.

In an alternative embodiment, the hIDS can be produced by human neuronal or glial cells in cell culture (e.g., bioreactors) and administered as an enzyme replacement therapy ("ERT"), *e.g.,* by injecting the enzyme - into the CSF, directly into the CNS, and/or systemically. However, the gene therapy approach offers several advantages over ERT since systemic delivery of the enzyme will not result in treating the CNS because the enzyme cannot cross the blood brain barrier; and, unlike the gene therapy approach of the invention, direct delivery of the enzyme to the CSF and/or CNS would require repeat injections which are not only burdensome, but pose a risk of infection.

The hIDS encoded by the transgene can include, but is not limited to human IDS (hIDS) having the amino acid sequence of SEQ ID NO. 1 (as shown in FIG. 1), and derivatives of hIDS having amino acid substitutions, deletions, or additions, *e.g*., including but not limited to amino acid substitutions selected from corresponding non-conserved residues in orthologs of IDS shown in FIG. 2, with the *proviso* that such mutations do not include replacement of the cysteine residue at position 84 (C84) which is required for enzyme activity (Millat et al., 1997, Biochem J 326: 243-247); or a mutation that has been identified in severe, severe-intermediate, intermediate, or attenuated MPS II phenotypes *e.g*., as shown in FIG. 3, or as reported by Sukegawa-Hayasaka et al., 2006, J Inhert Metab Dis 29: 755-761 (reporting "attenuated" mutants R48P, A85T, W337R, and the truncated mutant Q531X; and "severe" mutants P86L, S333L, S349I, R468Q, R468L); Millat et al., 1998, BBA 1406: 214-218 (reporting "attenuated" mutants P480L and P480Q; and "severe" mutant P86L); and Bonucelli et al., 2001, BBA 1537:233-238, each of which is incorporated by reference herein in its entirety.

For example, amino acid substitutions at a particular position of hIDS can be selected from among corresponding non-conserved amino acid residues found at that position in the IDS orthologs aligned in FIG. 2, with the *proviso* that such substitutions do not include any of the deleterious mutations shown in FIG. 3 or as reported by Sukegawa-Hayasaka et al., 2006, *supra;* Millat et al., 1998, *supra;* or Bonucelli et al., 2001, *supra,* each of which is incorporated by reference herein in its entirety. The resulting transgene product can be tested using conventional assays *in vitro,* in cell culture or test animals to ensure that the mutation does not disrupt IDS function. Preferred amino acid substitutions, deletions or additions selected should be those that maintain or increase enzyme activity, stability or half-life of IDS, as tested by conventional assays *in vitro,* in cell culture or animal models for MPS II. For example, the enzyme activity of the transgene product can be assessed using a conventional enzyme assay with, for example, 4-Methylumbelliferyl α-L-idopyranosiduronic acid 2-sulfate or 4-methylumbelliferyl sulfate as the substrate *(see, e.g.,* Lee et al., 2015, Clin. Biochem. 48(18):1350-1353, Dean et al., 2006, Clin. Chem. 52(4):643-649 for exemplary IDS enzyme assays that can be used, each of which is incorporated by reference herein in its entirety). The ability of the transgene product to correct MPS II phenotype can be assessed in cell culture; *e.g.,* by transducing MPS II cells in culture with a viral vector or other DNA expression construct encoding hIDS or a derivative; by adding the transgene product or a derivative to MPS II cells in culture; or by co-culturing MPS II cells with human neuronal/glial host cells engineered to express and secrete rhIDS or a derivative, and determining correction of the defect in the MPS II cultured cells, *e.g.,* by detecting IDS enzyme activity and/or reduction in GAG storage in the MPS II cells in culture *(see, e.g.,* Stroncek et al., 1999, Transfusion 39(4):343-350, which is incorporated by reference herein in its entirety).

Animal models for MPS II have been described that can be used to assess the therapeutics described herein. For example, a knockout mouse model (IDS-knockout) of MPS II was engineered by replacing exons 4 and 5 of the *IDS* gene with the neomycin resistance gene. (Garcia et al., 2007, J Inherit Metab Dis 30: 924-34). This IDS-knockout mouse exhibits many of the characteristics of MPS II, including skeletal abnormalities, hepatosplenomegaly, elevated urinary and tissue GAG, and brain storage lesions (Muenzer et al., 2001, Acta Paediatr Suppl 91:98-99) and was used to assess the effect of enzyme replacement therapy in MPS II in support of clinical trials for ERT. This mouse model, therefore, is a relevant model for studying the effects of gene therapy delivering rIDS produced by neuronal or glial cells as a treatment for MPS II *(see, e.g.,* Polito and Cosma, 2009, Am. J. Hum. Genet. 85(2):296-301, which is incorporated by reference herein in its entirety).

Preferably, the hIDS transgene produced by the human neuronal/glial cells should be controlled by expression control elements that function in neurons and/or glial cells, *e.g.,* the CB7 promoter (a chicken β-actin promoter and CMV enhancer), and can include other expression control elements that enhance expression of the transgene driven by the vector (*e.g*., chicken β-actin intron and rabbit β-globin poly A signal). The cDNA construct for the hIDS transgene should include a coding sequence for a signal peptide that ensures proper co- and post-translational processing (glycosylation and protein sulfation) by the transduced CNS cells. Such signal peptides used by CNS cells may include but are not limited to:
Oligodendrocyte-myelin glycoprotein (hOMG) signal peptide:
   MEYQILKMSLCLFILLFLTPGILC (SEQ ID NO:2)
Cellular repressor of E1A-stimulated genes 2 (hCREG2) signal peptide:
   MSVRRGRRPARPGTRLSWLLCCSALLSPAAG (SEQ ID NO:3)
V-set and transmembrane domain containing 2B (hVSTM2B) signal peptide:
   MEQRNRLGALGYLPPLLLHALLLFV ADA (SEQ ID NO:4)
Protocadherin alpha-1 (hPCADHA1) signal peptide:
   MVFSRRGGLGARDLLLWLLLLAAWEVGSG (SEQ ID NO:5)
FAM19A1 (TAFA1) signal peptide:
   MAMVSAMSWVLYLWISACA (SEQ ID NO:6)
Interleukin-2 signal peptide:
   MYRMQLLSCIALILALVTNS (SEQ ID NO:14)
Signal peptides may also be referred to herein as leader sequences or leader peptides.

The recombinant vector used for delivering the transgene should have a tropism for cells in the CNS, including but limited to neurons and/or glial cells. Such vectors can include non-replicating recombinant adeno-associated virus vectors ("rAAV"), particularly those bearing an AAV9 or AAVrh10 capsid are preferred. AAV variant capsids can be used, including but not limited to those described by Wilson in US Patent No. 7,906,111 which is incorporated by reference herein in its entirety, with AAV/hu.31 and AAV/hu.32 being particularly preferred; as well as AAV variant capsids described by Chatterjee in US Patent No. 8,628,966, US Patent No. 8,927,514 and Smith et al., 2014, Mol Ther 22: 1625-1634, each of which is incorporated by reference herein in its entirety. However, other viral vectors may be used, including but not limited to lentiviral vectors, vaccinia viral vectors, or non-viral expression vectors referred to as "naked DNA" constructs.

Pharmaceutical compositions suitable for administration to the CSF comprise a suspension of the rhIDS vector in a formulation buffer comprising a physiologically compatible aqueous buffer, a surfactant and optional excipients. In certain embodiments, the pharmaceutical compositions are suitable for intrathecal administration. In certain embodiments, the pharmaceutical compositions are suitable for intracisternal administration (injection into the cisterna magna). In certain embodiments, the pharmaceutical compositions are suitable for injection into the subarachnoid space via a C1-2 puncture. In certain embodiments, the pharmaceutical compositions are suitable for intracerebroventricular administration. In certain embodiments, the pharmaceutical compositions are suitable for administration via lumbar puncture.

Therapeutically effective doses of the recombinant vector should be administered to the CSF via intrathecal administration (i.e., injection into the subarachnoid space so that the recombinant vectors distribute through the CSF and transduce cells in the CNS). This can be accomplished in a number of ways - e.g., by intracranial (cisternal or ventricular) injection , or injection into the lumbar cistern. For example intracisternal (IC) injection (into the cisterna magna) can be performed by CT-guided suboccipital puncture; or injection into the subarachnoid space can be performed via a C1-2 puncture when feasible for the patient; or lumbar puncture (typically diagnostic procedures performed in order to collect a sample of CSF) can be used to access the CSF. Alternatively, intracerebroventricular (ICV) administration (a more invasive technique used for the introduction of antiinfective or anticancer drugs that do not penetrate the blood-brain barrier) can be used to instill the recombinant vectors directly into the ventricles of the brain. Alternatively, intranasal administration may be used to deliver the recombinant vector to the CNS.

Because of the relatively rapid brain growth that occurs early in a developing child, the total dose of AAV9.hIDS administered IC depends on the assumed brain mass across different age strata, *see, e.g.,* Table 2 below. For brain mass by age for the study subjects see, *e.g.,* AS Dekaban, Ann Neurol, 1978 Oct; 4(4): 345-56.

**Table 2: Total dose administered by age**

| **Subject Age** | **Assumed brain mass (g)** | **Dose 1 (total GC)** | **Dose 2 (total GC)** |
|---|---|---|---|
| ≥ 4 to < 9 months | 600 | 7.8 × 10¹² | 3.9 × 10¹³ |
| ≥ 9 to < 18 months | 1000 | 1.3 × 10¹³ | 6.5 × 10¹³ |
| ≥ 18 months to < 3 years | 1100 | 1.4 × 10¹³ | 7.2 × 10¹³ |
| ≥ 3 years | 1300 | 1.7 × 10¹³ | 8.5 × 10¹³ |

CSF concentrations can be monitored by directly measuring the concentration of rhIDS in the CSF fluid obtained from occipital or lumbar punctures, or estimated by extrapolation from concentrations of the rhIDS detected in the patient's serum.

By way of background, human IDS is translated as a 550 amino acid polypeptide that contains eight potential N-glycosylation sites (N³¹, N¹¹⁵, N¹⁴⁴, N²⁴⁶, N²⁸⁰, N³²⁵, N⁵¹³ and N⁵³⁷) depicted in FIG.1 and includes a 25 amino acid signal sequence which is cleaved during processing. An initial 76 kDa intracellular precursor is converted into a phosphorylated 90 kDa precursor after modification of its oligosaccharide chains in the Golgi apparatus. This precursor is processed by glycosylation modifications and proteolytic cleavage through various intracellular intermediates to a major 55 kDa form. To summarize, after removal of the 25 aa signal sequence, proteolytic processing involves N-terminal proteolytic cleavage downstream of N³¹ removing a propeptide of eight amino acids (residues 26-33), and C-terminal proteolytic cleavage upstream of N⁵¹³ which releases an 18 kDa polypeptide and produces a 62 kDa intermediate that is converted to a 55 kDa mature form. Further proteolytic cleavage yields a 45 kDa mature form located in the lysosomal compartment. *(See* FIG. 4 for diagram reproduced from Millat et al., 1997, Exp Cell Res 230: 362-367 ("Millat 1997"); Millat et al. 1997, Biochem J. 326: 243-247 ("Millat 1997a"); and Froissart et al., 1995, Biochem J. 309:425-430, each of which is incorporated by reference herein in its entirety).

A formylglycine modification of C⁸⁴ (shown in bold in FIG. 1) required for enzyme activity probably occurs as an early post-translational or co-translational event, most probably in the endoplasmic reticulum. *(See,* Millat 1997a, citing Schmidt et al., 1995, Cell 82: 271-278). Post-translational processing continues in the Golgi to include addition of complex sialic acid-containing glycans and acquisition of mannose-6-phosphate residues which tag the enzyme for delivery to the lysosomal compartment. *(See,* Clarke, 2008, Expert Opin Pharmacother 9: 311-317 for a concise review which is incorporated by reference herein in its entirety). While no single glycosylation site is essential for IDS stability, glycosylation at position N²⁸⁰ is important for cellular internalization and lysosomal targeting via the mannose-6-phosphate (M6P) receptor. (Chung et al., 2014, Glycoconj J 31:309-315 at p. 310, first column). In the normal physiologic state, IDS is produced at very low levels and very little, if any, enzyme is secreted from the cell. (Clarke, 2008, *supra).*

The invention is based, in part, on the following principles:
(i) Neuronal and glial cells in the CNS are secretory cells that possess the cellular machinery for post-translational processing of secreted proteins - including glycosylation, mannose-6-phosphorylation, and tyrosine-O-sulfation - robust processes in the CNS. *See, e.g.,* Sleat et al., 2005, Proteomics 5: 1520-1532, and Sleat 1996, J Biol Chem 271: 19191-98 which describes the human brain mannose-6-phosphate glycoproteome and notes that the brain contains more proteins with a much greater number of individual isoforms and mannose-6-phosphorylated proteins than found in other tissues; and Kanan et al., 2009, Exp. Eye Res. 89: 559-567 and Kanan & Al-Ubaidi, 2015, Exp. Eye Res. 133: 126-131 reporting the production of tyrosine-sulfated glycoproteins secreted by neuronal cells, each of which is incorporated by reference in its entirety for post-translational modifications made by human CNS cells.
(ii) The human brain produces multiple isoforms of natural/native IDS. In particular, N-terminal sequencing of human brain mannose-6-phosphorylated glycoproteins revealed that the N-terminal sequence of the mature 42 kDa chain of hIDS varies in the brain, starting at positions 34 or 36 as follows: T³⁴DALNVLLI; and A³⁶LNVLLIIY. (Sleat, 2005, Proteomics 5: 1520-1532, Table S2). Two of the eight N-linked glycosylation sites, namely N²⁸⁰ and N¹¹⁶, were found to be mannose-6-phophorylated in IDS obtained from human brain. (Sleat et al., 2006, Mol & Cell Proeomics 5.4: 686-701, reported at Table V).
(iii) During processing of hIDS, two polypeptides, 76 kDa and 90 kDa, are secreted by neural and glial cells, but only the 90 kDa polypeptide is mannose-6-phosphorylated, which is necessary for secreted forms of the enzyme to achieve cross correction. *(See,* Millat, 1997, Fig. 1 results for transduced lymphoblastoid cells, and Froissart 1995, Fig. 4 showing similar results for transduced fibroblasts - in culture medium, only the 90 kDa form is phosphorylated). Interestingly, it has been demonstrated that recombinant IDS produced by neuronal and glial cells may be endocytosed by recipient CNS cells more avidly than recombinant IDS produced by other cells such as kidney. Daniele 2002 demonstrated M6P-receptor mediated endocytosis of recombinant IDS from conditioned media of transduced neuronal and glial cell cultures by a recipient population of non-transduced neuronal and glial cells which properly processed the precursor to the 45 kDa mature active form. Uptake of the recombinant IDS produced by the neuronal and glial cell lines (74% endocytosis) far exceeded uptake of the enzyme produced by a kidney cell line (5.6% endocytosis). In each case, uptake was inhibited by M6P, indicating that recombinant IDS uptake was M6P-receptor mediated. (See Daniele 2002, Tables 2 and 4 and accompanying description in Results at pp. 205-206 summarized in Table 3 below).

**Table 3. Summary of Results Reported in Daniele 2002**

| Cell Line Source of rIDS | Media Enzyme Units | Recipient Cells: Units Recovered | | % Endocytosis (mean value) |
|---|---|---|---|---|
| | | Neuronal | Glial | |
| Kidney ^{(transfected)} | 35 U | 1.7 U | 2.2 U | 5.6% |
| Neuronal ^{(Ad-transduced)} | 12 U | 8.8 U | 8.8 U | 74% |
| Glial ^{(Ad-transduced)} | 14 U | 10.5 U | 10.5 U | 74% |

(iv) The gene therapy approach described herein should result in the continuous secretion of an hIDS glycoprotein precursor of about 90 kDa as measured by polyacrylamide gel electrophoresis (depending on the assay used) that is enzymatically active. First, the enzyme responsible for the formylglycine modification of C⁸⁴ which is required for IDS activity -- the FGly-Generating Enzyme (FGE, aka SUMF1) -- is expressed in the cerebral cortex of the human brain (gene expression data for SUMF1 may be found, for example, at GeneCards, accessible at http://www.genecards.org). Second, the secreted glycosylated/phosphorylated rIDS produced by transduced neurons and glial cells *in situ* should be taken up and correctly processed by untransduced neural and glial cells in the CNS. Without being bound to any theory, it appears that the secreted rhIDS precursor produced *in situ* by gene therapy may be more avidly endocytosed by recipient cells in the CNS than would traditional recombinant enzymes used for ERT if administered to the CNS. For example, Elaprase^{®} (made in HT1080, a fibrosarcoma cell line) is a purified protein reported to have a molecular weight of about 76 kDa - not the 90 kDa species secreted by neuronal and glial cells that appears to be more heavily phosphorylated. While the eight N-linked glycosylation sites are reported to be fully occupied in Elaprase^{®} and contain two bis-mannose-6-phosphate terminated glycans as well as complex highly sialylated glycans, the post-translational modification of C⁸⁴ to FGly, which is an absolute requirement for enzyme activity, is only about 50%. (Clarke, 2008, Expert Opin Pharmacother 9:311-317; Elaprase^{®} Full Prescribing Information and EMA filing). Another recombinant product, Hunterase^{®} is made in CHO cells. While reported to have higher FGly and activity than Elaprase^{®}, mannose-6-phosphorylation and uptake did not differ. (Chung, 2014, Glycoconj J 31:309-315).
(v) The extracellular IDS efficacy *in vivo* depends on uptake (cell and lysosome internalization) through mannose-6-phosphate (M6P) and its active site formylglycine (FGly), which is converted from C⁸⁴ through post-translational modification by formylglycine-generating enzyme. As shown above in Table 3, brain cells (neuronal and glial cells) show higher enzyme activities when incubated with IDS precursor media secreted by transduced neuronal and glial cells than with IDS precursor media secreted by genetically engineered kidney cells. The resultant five-fold increase in activity can likely be attributed to the efficient uptake of IDS (See Daniele 2002, Tables 2 and 4). Commercial forms of IDS, which are generated by CHO cells or HT-1080 cells, have a FGly content of about 50% to 70%, which determines the enzyme activity. However, neuronal and glial cells may improve upon this activity, due to improvement of IDS uptake.
(vi) The cellular and subcellular trafficking/uptake of lysosomal proteins, including IDS, is through M6P. IDS from brain cells may contain higher M6P content, as reported in Daniele 2002, and in Sleat, Proteomics, 2005 (indicating that the human brain contains more (in both a quantitative and qualitative sense) Man6-P glycoproteins than other tissues.). It is possible to measure the M6P content of an IDS precursor, as done in Daniele 2002. In the presence of inhibitory M6P *(e.g.,* 5 mM), the uptake of IDS precursor generated by non-neuronal or non-glial cells, such as the genetically engineered kidney cells of Daniele 2002, is predicted to decrease to levels close to that of the control cells, as was shown in Daniele 2002. While in the presence of inhibitory M6P, the uptake of IDS precursor generated by brain cells, such as neuronal and glial cells, is predicted to remain at a high level, as was shown in Daniele 2002, where the uptake was four times higher than control cells and comparable to the level of IDS activity (or uptake) of IDS precursor generated by genetically engineered kidney cells without the presence of inhibitory M6P. This assay allows for a way to predict the M6P content in IDS precursor generated by brain cells, and, in particular, to compare the M6P content in IDS precursors generated by different types of cells. The gene therapy approach described herein should result in the continuous secretion of an hIDS precursor that may be taken up into neuronal and glial cells at a high level in the presence of inhibitory M6P in such an assay.
(vii) The M6P content and uptake of IDS precursor may also be demonstrated by 90 kDa and 76 kDa gel bands (e.g., SDS-PAGE gel bands). The 90 kDa is reported to be highly glycosylated/phosphorylated and contains M6P, while 76 kDa is not. A very broad gel band with a range from 76 kDa to 95 kDa and with an average MW of 80-85 kDa, similar to the IDS precursor gel band generated from genetically engineered kidney cells (Daniele 2002, Figure 1), may be contrasted with a gel band of IDS precursor generated from brain cells. In Daniele 2002, the gel band cannot be obtained due to unsuccessful immunoprecipitation of the IDS precursor. The gene therapy approach described herein should result in the continuous secretion of an hIDS precursor that differs from the IDS precursor gel band generated from genetically engineered kidney cells.
(viii) The M6P content of commercial IDS precursor is 2 to 2.5 mol/mol, majority of which is present in a form of di-phosphorylated glycans. Although in average, every IDS precursor is phosphorylated, a normal distribution of glycans will have some IDS precursor with 2, 1 and 0 of di-phosphorylated M6P glycans assuming multiple phosphorylation sites. Uptake rate should be significant higher with multiple phosphorylation.
(ix) The glycosylation of hIDS by human cells of the CNS will result in the addition of glycans that can improve stability, half-life and reduce unwanted aggregation of the transgene product. Significantly, the glycans that are added to hIDS of the invention include 2,6-sialic acid, incorporating Neu5Ac ("NANA") but not its hydroxylated derivative, NeuGc (N-Glycolylneuraminic acid, *i.e.,* "NGNA" or "Neu5Gc"). Such glycans are not present in recombinant IDS products, such as Hunterase^{®}, made in CHO cells because CHO cells do not have the 2,6-sialyltransferase required to make this post-translational modification; nor do CHO cells produce bisecting GlcNAc, although they do add Neu5Gc (NGNA) as sialic acid not typical (and potentially immunogenic) to humans instead of Neu5Ac (NANA). *See, e.g.,* Dumont et al., 2016, Critical Rev in Biotech 36(6):1110-1122 (Early Online pp. 1-13 at p. 5); and Hague et al., 1998 Electrophor 19:2612-2630 ("[t]he CHO cell line is considered 'phenotypically restricted,' in terms of glycosylation, due to the lack of an α2,6-sialyl-transferase"). Moreover, CHO cells can also produce an immunogenic glycan, the α-Gal antigen, which reacts with anti-α-Gal antibodies present in most individuals, and at high concentrations can trigger anaphylaxis. *See, e.g.,* Bosques, 2010, Nat Biotech 28: 1153-1156. The human glycosylation pattern of the rhIDS of the invention should reduce immunogenicity of the transgene product and improve efficacy.
(x) Immunogenicity of a transgene product could be induced by various factors, including the immune condition of the patient, the structure and characteristics of the infused protein drug, the administration route, and the duration of treatment. Process-related impurities, such as host cell protein (HCP), host cell DNA, and chemical residuals, and product-related impurities, such as protein degradants and structural characteristics, such as glycosylation, oxidation and aggregation (sub-visible particles), may also increase immunogenicity by serving as an adjuvant that enhances the immune response. The amounts of process-related and product-related impurities can be affected by the manufacturing process: cell culture, purification, formulation, storage and handling, which can affect commercially manufactured IDS products. In gene therapy, proteins are produced *in vivo,* such that process-related impurities are not present and protein products are not likely to contain product-related impurities/degradants associated with proteins produced by recombinant technologies, such as protein aggregation and protein oxidation. Aggregation, for example, is associated with protein production and storage due to high protein concentration, surface interaction with manufacturing equipment and containers, and the purification process with certain buffer systems. But these conditions that promote aggregation are not present when a transgene is expressed *in vivo.* Oxidation, such as methionine, tryptophan and histidine oxidation, is also associated with protein production and storage, caused, for example, by stressed cell culture conditions, metal and air contact, and impurities in buffers and excipients. The proteins expressed *in vivo* may also oxidize in a stressed condition, but humans, like many organisms, are equipped with an antioxidation defense system, which not only reduces the oxidation stress, but can also repairs and/or reverses the oxidation. Thus, proteins produced *in vivo* are not likely to be in an oxidized form. Both aggregation and oxidation could affect the potency, pharmacokinetics (clearance) and can increase immunogenicity concerns. The gene therapy approach described herein should result in the continuous secretion of an hIDS precursor with a reduced immunogenicity compared to commercially manufactured products.
(xi) In addition to the N-linked glycosylation sites, hIDS contains a tyrosine ("Y") sulfation site (PSSEKY¹⁶⁵ENTKTCRGPD). (*See, e.g.,* Yang et al., 2015, Molecules 20:2138-2164, esp. at p. 2154 which is incorporated by reference in its entirety for the analysis of amino acids surrounding tyrosine residues subjected to protein tyrosine sulfation. The "rules" can be summarized as follows: Y residues with E or D within +5 to -5 position of Y, and where position -1 of Y is a neutral or acidic charged amino acid - but not a basic amino acid, *e.g.,* R, K, or H that abolishes sulfation). While not intending to be bound by any theory, sulfation of this site in hIDS may improve stability of the enzyme and binding affinity for substrate. Tyrosine-sulfation of hIDS - a robust post-translational process in human CNS cells - should result in improved processing and activity of transgene products. The significance of tyrosine-sulfation of lysosomal proteins has not been elucidated; but in other proteins it has been shown to increase avidity of protein-protein interactions (antibodies and receptors), and to promote proteolytic processing (peptide hormone). (See, Moore, 2003, J Biol. Chem. 278: 24243-46; and Bundegaard et al., 1995, The EMBO J 14: 3073-79). The tyrosylprotein sulfotransferase (TPST1) responsible for tyrosine-sulfation (which may occur as a final step in IDS processing) is apparently expressed at higher levels (based on mRNA) in the brain (gene expression data for TPST1 may be found, for example, at the EMBL-EBI Expression Atlas, accessible at http://www.ebi.ac.uk/gxa/home). Such post-translational modification, at best, is under-represented in CHO cell products. Unlike human CNS cells, CHO cells are not secretory cells and have a limited capacity for post-translational tyrosine-sulfation. *(See, e.g.,* Mikkelsen & Ezban, 1991, Biochemistry 30: 1533-1537, esp. discussion at p. 1537).

For the foregoing reasons, the production of rhIDS by human neuronal and/or glial cells should result in a "biobetter" molecule for the treatment of MPS II accomplished via gene therapy - *e.g.,* by administering a viral vector or other DNA expression construct encoding rhIDS to the CSF of a patient (human subject) diagnosed with an MPS II disease (including but not limited to Hunter) to create a permanent depot in the CNS that continuously supplies a fully human-glycosylated, mannose-6-phosphorylated, sulfated transgene product secreted by the transduced CNS cells. The hIDS transgene product secreted from the depot into the CSF will be endocytosed by cells in the CNS, resulting in "cross-correction" of the enzymatic defect in the MPS II recipient cells.

It is not essential that every rhIDS molecule produced either in the gene therapy or protein therapy approach be fully glycosylated, phosphorylated, and sulfated. Rather, the population of glycoproteins produced should have sufficient glycosylation (including 2,6-sialylation and mannose-6-phosphorylation) and sulfation to demonstrate efficacy. The goal of gene therapy treatment of the invention is to slow or arrest the progression of disease. Efficacy may be monitored by measuring cognitive function (*e.g*., prevention or decrease in neurocognitive decline); reductions in biomarkers of disease (such as GAG) in CSF and or serum; and/or increase in IDS enzyme activity in CSF and/or serum. Signs of inflammation and other safety events may also be monitored.

As an alternative, or an additional treatment to gene therapy, the rhIDS glycoprotein can be produced in human neural or glial cell lines by recombinant DNA technology and the glycoprotein can be administered to patients diagnosed with MPS II systemically and/or into the CSF for ERT). Human cell lines that can be used for such recombinant glycoprotein production include but are not limited to HT-22, SK-N-MC, HCN-1A, HCN-2, NT2, SH-SY5y, hNSC11, or ReNcell VM *(see, e.g.,* Dumont et al., 2016, Critical Rev in Biotech 36(6):1110-1122 "Human cell lines for biopharmaceutical manufacturing: history, status, and future perspectives" which is incorporated by reference in its entirety for a review of the human cell lines that could be used for the recombinant production of the rHuGlyIDS glycoprotein). To ensure complete glycosylation, especially sialylation, and tyrosine-sulfation, the cell line used for production can be enhanced by engineering the host cells to co-express α-2,6-sialyltransferase (or both α-2,3-and α-2,6-sialyltransferases) and/or TPST-1 and TPST-2 enzymes responsible for tyrosine-O-sulfation.

While the delivery of rhIDS should minimize immune reactions, the clearest potential source of toxicity related to CNS-directed gene therapy is generating immunity against the expressed rhIDS protein in human subjects who are genetically deficient for IDS and, therefore, potentially not tolerant of the protein and/or the vector used to deliver the transgene.

Thus, in a preferred embodiment, it is advisable to co-treat the patient with immune suppression therapy -- especially when treating patients with severe disease who have close to zero levels of IDS. Immune suppression therapies involving a regimen of tacrolimus or rapamycin (sirolimus) in combination with mycophenolic acid, or other immune suppression regimens used in tissue transplantation procedures can be employed. Such immune suppression treatment may be administered during the course of gene therapy, and in certain embodiments, pre-treatment with immune suppression therapy may be preferred. Immune suppression therapy can be continued subsequent to the gene therapy treatment, based on the judgment of the treating physician, and may thereafter be withdrawn when immune tolerance is induced; *e.g*., after 180 days.

Combinations of delivery of the rhIDS to the CSF accompanied by delivery of other available treatments are encompassed by the methods of the invention. The additional treatments may be administered before, concurrently or subsequent to the gene therapy treatment. Available treatments for MPS II that could be combined with the gene therapy of the invention include but are not limited to enzyme replacement therapy using Elaprase^{®} administered systemically or to the CSF; and/or HSCT therapy.

In certain embodiments, described herein is a method for treating a human subject diagnosed with mucopolysaccharidosis type II (MPS II), comprising delivering to the cerebrospinal fluid (CSF) of said human subject a therapeutically effective amount of a recombinant human iduronate-2-sulfatase (IDS) precursor produced by human neuronal or human glial cells.

In certain embodiments, described herein is a method of treating a human subject diagnosed with mucopolysaccharidosis type II (MPS II), comprising delivering to the cerebrospinal fluid (CSF) of said human subject, a therapeutically effective amount of a recombinant human iduronate-2-sulfatase (IDS) glycoprotein precursor that is about 90 kDa *(e.g.,* 85 kDa, 86 kDa, 87 kDa, 88 kDa, 89 kDa, 90 kDa, 91 kDa, 92 kDa, 93 kDa, 94 kDa, or 95 kDa) as measured by polyacrylamide gel electrophoresis, has a formylglycine residue at C⁸⁴ (Fig. 1), is α2,6-sialylated, does not contain detectable NeuGc, and is mannose-6-phosphorylated.

In certain embodiments, described herein is a method of treating a human subject diagnosed with mucopolysaccharidosis type II (MPS II), comprising delivering to the cerebrospinal fluid (CSF) of said human subject, a therapeutically effective amount of a recombinant human iduronate-2-sulfatase (IDS) glycoprotein precursor that is about 90 kDa *(e.g.,* 85 kDa, 86 kDa, 87 kDa, 88 kDa, 89 kDa, 90 kDa, 91 kDa, 92 kDa, 93 kDa, 94 kDa, or 95 kDa) as measured by polyacrylamide gel electrophoresis, has a formylglycine residue at C⁸⁴ (Fig. 1), is α2,6-sialylated, does not contain detectable NeuGc and/or α-Gal antigen, and is mannose-6-phosphorylated.

In certain embodiments, the human IDS precursor is delivered to the CSF from a depot of cells in the central nervous system genetically engineered to secrete said IDS precursor into the CSF. In certain embodiments, the depot is formed in the subject's brain. In certain embodiments, the human subject is deficient in IDS activity. In certain embodiments, the human IDS comprises the amino acid sequence of SEQ ID NO. 1.

In certain embodiments, described herein is a method of treating a human subject diagnosed with mucopolysaccharidosis type II (MPS II), comprising administering to the cerebrospinal fluid (CSF) of said human subject a recombinant nucleotide expression vector encoding human iduronate-2-sulfatase (IDS), wherein said expression vector when used to transduce a primary human neuronal cell in culture directs the expression of a secreted human IDS glycoprotein precursor that is about 90 kDa *(e.g.,* 85 kDa, 86 kDa, 87 kDa, 88 kDa, 89 kDa, 90 kDa, 91 kDa, 92 kDa, 93 kDa, 94 kDa, or 95 kDa) as measured by polyacrylamide gel electrophoresis, has a formylglycine residue at C⁸⁴ (Fig. 1), is α2,6-sialylated and mannose-6-phosphorylated.

In certain embodiments, described herein is a method of treating a human subject diagnosed with mucopolysaccharidosis type II (MPS II), comprising administering to the cerebrospinal fluid of the brain of said human subject, a therapeutically effective amount of a recombinant nucleotide expression vector encoding human IDS, so that a depot is formed in the subject's central nervous system that secretes a recombinant human IDS glycoprotein precursor that is α2,6-sialylated and mannose-6-phosphorylated.

In certain embodiments, secretion of said recombinant human IDS glycoprotein precursor that is α2,6-sialylated is confirmed by transducing a human neuronal cell line with said recombinant nucleotide expression vector in cell culture. In certain embodiments, secretion of said recombinant human IDS glycoprotein precursor that is mannose-6-phosphorylated is confirmed by transducing a human neuronal cell line with said recombinant nucleotide expression vector in cell culture. In certain embodiments, the secretion is confirmed in the presence and absence of mannose-6-phosphate.

In certain embodiments, described herein is a method of treating a human subject diagnosed with mucopolysaccharidosis type II (MPS II), comprising administering to the cerebrospinal fluid of the brain of said human subject, a therapeutically effective amount of a recombinant nucleotide expression vector encoding human IDS, so that a depot is formed that secretes a glycosylated IDS precursor containing a α2,6-sialylated glycan; wherein said recombinant vector, when used to transduce human neuronal cells in culture results in secretion of said glycosylated IDS precursor containing a α2,6-sialylated glycan in said cell culture.

In certain embodiments, described herein is a method of treating a human subject diagnosed with mucopolysaccharidosis type II (MPS II), comprising administering to the cerebrospinal fluid of the brain of said human subject, a therapeutically effective amount of a recombinant nucleotide expression vector encoding human IDS, so that a depot is formed that secretes a glycosylated IDS precursor that contains a mannose-6-phosphate; wherein said recombinant vector, when used to transduce human neuronal cells in culture results in secretion of said glycosylated IDS precursor that is mannose-6-phosphorylated in said cell culture.

In certain embodiments, described herein is a method of treating a human subject diagnosed with mucopolysaccharidosis type II (MPS II), comprising administering to the cerebrospinal fluid of the brain of said human subject, a therapeutically effective amount of a recombinant nucleotide expression vector encoding human IDS, so that a depot is formed that secretes a glycosylated IDS precursor that contains a formylglycine; wherein said recombinant vector, when used to transduce human neuronal cells in culture results in secretion of said glycosylated IDS precursor that contains a formylglycine in said cell culture.

In certain embodiments, the human IDS comprises the amino acid sequence of SEQ ID NO. 1. In certain embodiments, the IDS transgene encodes a leader peptide. In certain embodiments, the expression vector is a replication defective AAV vector. In certain embodiments, the expression vector is delivered to the CSF of the subject by intrathecal (e.g., intracisternal, C1-2 puncture if feasible for the patient, or lumbar puncture), intracerebroventricular, or intranasal administration. In certain embodiments, the human subject is deficient in IDS activity.

In preferred embodiments, the glycosylated IDS does not contain detectable NeuGc and/or α-Gal. The phrase "detectable NeuGc and/or α-Gal" used herein means NeuGc and/or α-Gal moieties detectable by standard assay methods known in the art. For example, NeuGc may be detected by HPLC according to Hara et al., 1989, "Highly Sensitive Determination of N-Acetyl-and N-Glycolylneuraminic Acids in Human Serum and Urine and Rat Serum by Reversed-Phase Liquid Chromatography with Fluorescence Detection." J. Chromatogr., B: Biomed. 377: 111-119, which is hereby incorporated by reference for the method of detecting NeuGc. Alternatively, NeuGc may be detected by mass spectrometry. The α-Gal may be detected using an ELISA, *see,* for example, Galili et al., 1998, "A sensitive assay for measuring alpha-Gal epitope expression on cells by a monoclonal anti-Gal antibody." Transplantation. 65(8):1129-32, or by mass spectrometry, *see,* for example, Ayoub et al., 2013, "Correct primary structure assessment and extensive glyco-profiling of cetuximab by a combination of intact, middle-up, middle-down and bottom-up ESI and MALDI mass spectrometry techniques." Landes Bioscience. 5(5): 699-710. *See* also the references cited in Platts-Mills et al., 2015, "Anaphylaxis to the Carbohydrate Side-Chain Alpha-gal" Immunol Allergy Clin North Am. 35(2): 247-260.

In one aspect, provided herein is a method for treating a human subject diagnosed with MPS II, comprising delivering to the CSF of the human subject a therapeutically effective amount of a glycosylated recombinant human IDS precursor produced by human neuronal or human glial cells, wherein the glycosylated recombinant human IDS precursor is delivered by administration of a recombinant nucleotide expression vector encoding human IDS, wherein the recombinant nucleotide expression vector is administered at a dose that is dependent on the human subject's brain mass, and wherein the brain mass is determined by brain MRI of the human subject's brain.

In another aspect, provided herein is a method for treating a human subject diagnosed with MPS II, comprising determining the human subject's brain mass from the human subject's brain MRI, and subsequently delivering to the CSF of the human subject a therapeutically effective amount of a glycosylated recombinant human IDS precursor produced by human neuronal cells or human glial cells, wherein the glycosylated recombinant human IDS precursor is delivered by administration of a recombinant nucleotide expression vector encoding human IDS, and wherein the recombinant nucleotide expression vector is administered at a dose that is dependent on the human subject's brain mass.

In another aspect, provided herein is a method for treating a human subject diagnosed with MPS II, comprising (a) determining the human subject's brain mass from the human subject's brain MRI, (b) calculating the dose based on the human subject's brain mass, and (c) subsequently administrating to the CSF of the subject the dose of recombinant nucleotide expression vector encoding human IDS.

In another aspect, provided herein is a method for treating a human subject diagnosed with MPS II, comprising, in the following order: (a) delivering to the CSF of the human subject a therapeutically effective amount of a glycosylated recombinant human IDS precursor produced by human neuronal or human glial cells; (b) measuring level of heparan sulfate in the CSF of the human subject; and (c) comparing the level of heparan sulfate in the CSF of the human subject with level of heapran sulfatae in a reference population; wherein the glycosylated recombinant human IDS precursor is delivered by administration of a recombinant nucleotide expression vector encoding human IDS, wherein the recombinant nucleotide expression vector is administered at a dose that is dependent on the human subject's brain mass, and wherein the brain mass is determined by brain magnetic resonance imaging (MRI) of the human subject's brain. In certain embodiments, the reference population consists of: (a) at least 1, 2, 3, 4, 5, 10, 25, 50, 75, 100, 200, 250, 300, 400, 500, or 1000 individual healthy people without MPS II, preferably of similar age, weight, and/or of the same gender as the human subject.

In another aspect, provided herein is a method for treating a human subject diagnosed with MPS II, comprising, in the following order: (a) taking a first measurement of the level of heparan sulfate in the CSF of the human subject; (b) delivering to the CSF of the human subject a therapeutically effective amount of a glycosylated recombinant human IDS precursor produced by human neuronal or human glial cells; and (c) after a period of time, taking a second measurement of the level of heparan sulfate; wherein the glycosylated recombinant human IDS precursor is delivered by administration of a recombinant nucleotide expression vector encoding human IDS, wherein the recombinant nucleotide expression vector is administered at a dose that is dependent on the human subject's brain mass, and wherein the brain mass is determined by brain magnetic resonance imaging (MRI) of the human subject's brain. In certain embodiments, the period of time is about 1 day, 2 days, 3 days, 4 days, 5 days, 6 days, 1 week, 2 weeks, 3 weeks, 4 weeks, 2 months, 3 moths, 4 months, 5 months, 6 months, 7 months, 8 months, 11 months, or 1 year.

In a preferred embodiment, the glycosylated recombinant human IDS precursor will be endocytosed by cells in the CNS. In a preferred embodiment, the glycosylated recombinant human IDS precursor is delivered to lysosomes of cells in the CNS of the human subject.

In certain embodiments of the method for treating described herein, the human subject's brain mass is converted from the human subject's brain volume by multiplying the human subject's brain volume in cm³ by a factor of 1.046 g/cm³, wherein the human subject's brain volume is determined by brain MRI of the subject's brain.

In certain embodiments of the method for treating described herein, the recombinant nucleotide expression vector is administered at a dose of about 1.3 × 10¹⁰ GC/g brain mass as determined by MRI, or about 6.5 × 10¹⁰ GC/g brain mass as determined by MRI.

In various embodiments of the method for treating described herein, the human subject is 5 years old or older and less than 18 years old. In specific embodiments, the human subject is 5, 6, 7, 8, 9, 10, 11, 12, 13, 14, 15, 16, 17 or 18 years old. In specific embodiments, the human subject is about 5, 6, 7, 8, 9, 10, 11, 12, 13, 14, 15, 16, 17 or 18 years old. In specific embodiments, the human subject is 5-6, 6-7, 7-8, 8-9, 9-10, 10-11, 11-12, 12-13, 13-14, 14-15, 15-16, 16-17, 17-18 or 18-19 years old. In specific embodiments, the human subject is about 5-6, 6-7, 7-8, 8-9, 9-10, 10-11, 11-12, 12-13, 13-14, 14-15, 15-16, 16-17, 17-18 or 18-19 years old. In certain embodiments, the recombinant nucleotide expression vector is administered at a dose of about 6.5 × 10¹⁰ GC/g brain mass as determined by MRI. In certain embodiments, the recombinant nucleotide expression vector is administered at a dose according to Table 7.

In various embodiments of the method for treating described herein, the human subject is 4 months old or older and less than 5 years old. In specific embodiments, the human subject is 4, 5, 6, 7, 8, 9, 10, or 11 months old. In specific embodiments, the human subject is about 4, 5, 6, 7, 8, 9, 10, or 11 months old. In specific embodiments, the human subject is 4-5, 5-6, 6-7, 7-8, 8-9, 9-10, 10-11, or 11-12 months old. In specific embodiments, the human subject is about 4-5, 5-6, 6-7, 7-8, 8-9, 9-10, 10-11, or 11-12 months old. In specific embodiments, the human subject is 1, 2, 3, 4, or 5 years old. In specific embodiments, the human subject is about 1, 2, 3, 4, or 5 years old. In specific embodiments, the human subject is 1-2, 2-3, 3-4, 4-5, or 5-6 years old. In specific embodiments, the human subject is about 1-2, 2-3, 3-4, 4-5, or 5-6 years old. In certain embodiments, the recombinant nucleotide expression vector is administered at a dose of about 1.3 × 10¹⁰ GC/g brain mass as determined by MRI. In certain embodiments, the recombinant nucleotide expression vector is administered at a dose of about 6.5 × 10¹⁰ GC/g brain mass as determined by MRI. In certain embodiments, the recombinant nucleotide expression vector is administered at a dose of about 2.0 × 10¹¹ GC/g brain mass as determined by MRI. In certain embodiments, the recombinant nucleotide expression vector is administered at a dose chosen from Dose 1 or Dose 2 according to Table 5. In certain embodiments, the recombinant nucleotide expression vector is administered at a dose according to Table 6.

In some embodiments of the method for treating described herein, the recombinant nucleotide expression vector is administered via intracisternal (IC) administration. In other embodiments of the method for treating described herein, the recombinant nucleotide expression vector is administered via intracerebroventricular (ICV) administration.

In certain embodiments of the method for treating described herein, the recombinant nucleotide expression vector is administered at a volume that does not exceed 10% of the total cerebrospinal fluid volume of the human subject.

In certain embodiments of the method for treating described herein, the glycosylated recombinant human IDS precursor is secreted at a detectable level.

In certain embodiments of the method for treating described herein, the human neuronal or human glial cells carry at least one mutation in the endogenous gene encoding human IDS precursor.

In certain embodiments of the method for treating described herein, the human neuronal or human glial cells are transduced with a recombinant adeno-associated virus vector (rAAV).

In a preferred embodiment, the recombinant nucleotide expression vector is an AAV9 or AAVrh10 vector.

In certain embodiments of the method for treating described herein, the glycosylated recombinant human IDS precursor is expressed under the control of a CB7 promoter.

In certain embodiments of the method for treating described herein, the glycosylated recombinant human IDS precursor is expressed from a cDNA encoding human IDS precursor.

In certain embodiments of the method for treating described herein, the glycosylated recombinant human IDS precursor is about 90 kDa as measured by polyacrylamide gel electrophoresis.

In certain embodiments of the method for treating described herein, the glycosylated recombinant human IDS precursor contains a formylglycine.

In certain embodiments of the method for treating described herein, the glycosylated recombinant human IDS precursor (a) is α2,6-sialylated; (b) does not contain detectable NeuGc; (c) does not contain detectable α-Gal antigen; (d) contains tyrosine-sulfation; and/or (e) is mannose-6-phosphorylated.

In certain embodiments of the method for treating described herein, the glycosylated recombinant human IDS precursor comprises the amino acid sequence of SEQ ID NO. 1.

In certain embodiments provided herein, the method further comprising administering an immune suppression therapy to the human subject before or concurrently with the human IDS precursor treatment and optionally continuing immune suppression therapy thereafter.

In some embodiments, the immune suppression therapy comprises administering one or more corticosteroids, sirolimus, and/or tacrolimus. In a specific embodiment, the one or more corticosteroids are methylprednisolone and/or prednisone.

In a specific embodiment, the immune suppression therapy comprises administering prednisone at a dose of about 0.10 mg/kg, 0.11 mg/kg, 0.12 mg/kg, 0.13 mg/kg, 0.14 mg/kg, 0.15 mg/kg, 0.16 mg/kg, 0.17 mg/kg, 0.18 mg/kg, 0.19 mg/kg, 0.20 mg/kg, 0.21 mg/kg, 0.22 mg/kg, 0.23 mg/kg, 0.24 mg/kg, 0.25 mg/kg, 0.26 mg/kg, 0.27 mg/kg, 0.28 mg/kg, 0.29 mg/kg, 0.30 mg/kg, 0.31 mg/kg, 0.32 mg/kg, 0.33 mg/kg, 0.34 mg/kg, 0.35 mg/kg, 0.36 mg/kg, 0.37 mg/kg, 0.38 mg/kg, 0.39 mg/kg, 0.40 mg/kg, 0.41 mg/kg, 0.42 mg/kg, 0.43 mg/kg, 0.44 mg/kg, 0.45 mg/kg, 0.46 mg/kg, 0.47 mg/kg, 0.48 mg/kg, 0.49 mg/kg, 0.5 mg/kg, 0.6 mg/kg, 0.7 mg/kg, 0.8 mg/kg, 0.9 mg/kg, or 1 mg/kg. In a specific embodiment, the immune suppression therapy comprises administering prednisone at a dose ranging from about 0.10 mg/kg to about 0.20 mg/kg. In a specific embodiment, the immune suppression therapy comprises administering prednisone at a dose ranging from about 0.20 mg/kg to about 0.30 mg/kg. In a specific embodiment, the immune suppression therapy comprises administering prednisone at a dose ranging from about 0.30 mg/kg to about 0.40 mg/kg. In a specific embodiment, the immune suppression therapy comprises administering prednisone at a dose ranging from about 0.40 mg/kg to about 0.50 mg/kg. In a specific embodiment, the immune suppression therapy comprises administering prednisone at a dose ranging from about 0.50 mg/kg to about 1 mg/kg. In a particular embodiment, the dose is administered daily. In a particular embodiment, the immune suppression therapy comprises administering prednisone at a dose of 0.5 mg/kg daily. In another particular embodiment, the immune suppression therapy comprises administering prednisone at a dose of 0.5 mg/kg daily with gradual tapering and discontinuation.

In a specific embodiment, the immune suppression therapy comprises administering methylprednisolone at a dose of about 0.5 mg/kg, 0.6 mg/kg, 0.7 mg/kg, 0.8 mg/kg, 0.9 mg/kg, 1 mg/kg, 1.5 mg/kg, 2 mg/kg, 2.5 mg/kg, 3 mg/kg, 3.5 mg/kg, 4mg/kg, 4.5 mg/kg, 5mg/kg, 5.5 mg/kg, 6mg/kg, 6.5 mg/kg, 7mg/kg, 7.5 mg/kg, 8mg/kg, 8.5 mg/kg, 9mg/kg, 9.5 mg/kg, 10mg/kg, 11 mg/kg, 12 mg/kg, 13 mg/kg, 14 mg/kg, 15 mg/kg, 16 mg/kg, 17 mg/kg, 18 mg/kg, 19 mg/kg, or 20 mg/kg. In a specific embodiment, the immune suppression therapy comprises administering methylprednisolone at a dose ranging from about 0.50 mg/kg to about 1.0 mg/kg. In a specific embodiment, the immune suppression therapy comprises administering methylprednisolone at a dose ranging from about 1.0 mg/kg to about 2.0 mg/kg. In a specific embodiment, the immune suppression therapy comprises administering methylprednisolone at a dose ranging from about 2.0 mg/kg to about 3.0 mg/kg. In a specific embodiment, the immune suppression therapy comprises administering methylprednisolone at a dose ranging from about 3.0 mg/kg to about 5.0 mg/kg. In a specific embodiment, the immune suppression therapy comprises administering methylprednisolone at a dose ranging from about 5.0 mg/kg to about 10.0 mg/kg. In a specific embodiment, the immune suppression therapy comprises administering methylprednisolone at a dose ranging from about 10.0 mg/kg to about 15.0 mg/kg. In a specific embodiment, the immune suppression therapy comprises administering methylprednisolone at a dose ranging from about 15.0 mg/kg to about 20.0 mg/kg. In a particular embodiment, the methylprednisolone is administered once. In a particular embodiment, the methylprednisolone is administered intravenously. In a particular embodiment, the methylprednisolone is administered for a maximum of 500 mg, In a particular embodiment, the methylprednisolone s administered over at least 30 minutes. In a particular embodiment, the immune suppression therapy comprises administering methylprednisolone at a dose of 10 mg/kg IV for maximum of 500 mg over at least 30 minutes.

In a specific embodiment, the immune suppression therapy comprises administering sirolimus at a dose to maintain a target blood level of 1-3 ng/mL. In a specific embodiment, the immune suppression therapy comprises administering sirolimus at a dose of about 0.25 mg/m²/day, 0.3 mg/m²/day, 0.4 mg/m²/day, 0.5 mg/m²/day, 0.6 mg/m²/day, 0.7 mg/m²/day, 0.8 mg/m²/day, 0.9 mg/m²/day, 1 mg/m²/day, 1.25 mg/m²/day, 1.5 mg/m²/day, 1.75 mg/m²/day, 2 mg/m²/day, 2.25 mg/m²/day, 2.5 mg/m²/day, 2.75 mg/m²/day, 3 mg/m²/day, 3.25 mg/m²/day, 3.5 mg/m²/day, 3.75 mg/m²/day, 4 mg/m²/day, 4.25 mg/m²/day, 4.5 mg/m²/day, 4.75 mg/m²/day, or 5 mg/m²/day. In a specific embodiment, the immune suppression therapy comprises administering sirolimus at a dose ranging from about 0.25 mg/m²/day to about 0.5 mg/m²/day. In a specific embodiment, the immune suppression therapy comprises administering sirolimus at a dose ranging from about 0.50 mg/m²/day to about 1.0 mg/m²/day. In a specific embodiment, the immune suppression therapy comprises administering sirolimus at a dose ranging from about 1.0 mg/m²/day to about 1.5 mg/m²/day. In a specific embodiment, the immune suppression therapy comprises administering sirolimus at a dose ranging from about 1.5 mg/m²/day to about 2 mg/m²/day. In a specific embodiment, the immune suppression therapy comprises administering sirolimus at a dose ranging from about 2 mg/m²/day to about 5mg/m²/day. In a particular embodiment, the dose is divided in BID dosing. In a particular embodiment, the immune suppression therapy comprises administering sirolimus at a dose of about 1 mg/m²/day every 4 hours. In a particular embodiment, the immune suppression therapy comprises administering sirolimus at a dose of about 0.5 mg/m²/day divided in BID dosing.

In a specific embodiment, the immune suppression therapy comprises administering tacrolimus at a dose to maintain a target blood level of 2-4 ng/mL. In a particular embodiment, the immune suppression therapy comprises administering tacrolimus at a dose of about 0.01 mg/kg, 0.02 mg/kg, 0.03 mg/kg 0.04 mg/kg 0.05 mg/kg, 0.06 mg/kg, 0.07 mg/kg, 0.08 mg/kg, 0.09 mg/kg, or 0.10 mg/kg. In a particular embodiment, the immune suppression therapy comprises administering tacrolimus at a dose ranging from 0.01 mg/kg to 0.02 mg/kg. In a particular embodiment, the immune suppression therapy comprises administering tacrolimus at a dose ranging from 0.02 mg/kg to 0.03 mg/kg. In a particular embodiment, the immune suppression therapy comprises administering tacrolimus at a dose ranging from 0.03 mg/kg to 0.05mg/kg. In a particular embodiment, the immune suppression therapy comprises administering tacrolimus at a dose ranging from 0.05mg/kg to 0.07mg/kg. In a particular embodiment, the immune suppression therapy comprises administering tacrolimus at a dose ranging from 0.07mg/kg to 0.10mg/kg. In a particular embodiment, the dose is administered twice daily. In a particular embodiment, the immune suppression therapy comprises administering tacrolimus at a dose of about 0.05mg/kg twice daily.

In some embodiments, the method further comprises administering one or more antibiotics to the human subject before or concurrently with the immune suppression therapy. In a specific embodiment, the one or more antibiotics are trimethoprim, sulfamethoxazole, pentamidine, dapsone, and/or atovaquone. In another specific embodiment, the one or more antibiotics are trimethoprim and/or sulfamethoxazole. In another specific embodiment, the one or more antibiotics are pentamidine, dapsone, and/or atovaquone. In specific embodiments, the one or more antibiotics are administered at a dose of about 1 mg/kg, 2 mg/kg, 3 mg/kg, 4 mg/kg, 5 mg/kg, 6 mg/kg, 7 mg/kg, 8 mg/kg, 9 mg/kg, or 10 mg/kg. In specific embodiments, the one or more antibiotics are administered at a dose ranging from about 1 mg/kg to 2 mg/kg. In specific embodiments, the one or more antibiotics are administered at a dose ranging from about 2 mg/kg to 3 mg/kg. In specific embodiments, the one or more antibiotics are administered at a dose ranging from about 3 mg/kg to 5 mg/kg. In specific embodiments, the one or more antibiotics are administered at a dose ranging from about 5 mg/kg to 7 mg/kg. In specific embodiments, the one or more antibiotics are administered at a dose ranging from about 7 mg/kg to 10 mg/kg. In a specific embodiment, the one or more antibiotics are administered at a dose of about three times a week. In certain embodiments, the one or more antibiotics are administered to prevent Pneumocystis carinii pneumonia.

In some embodiments, the method further comprises administering one or more antifungal therapies to the human subject before or concurrently with the immune suppression therapy. In certain embodiments, the one or more antifungal therapies are initiated if the absolute neutrophil count is < 500 mm³.

In some embodiments, the method further comprises a step of measuring one or more of the following biomarkers after administration of the recombinant nucleotide expression vector: (a) level of glycosaminoglycans (GAGs) in CSF; (b) level of iduronate-2-sulfatase (I2S) in CSF; (c) level of GAGs in plasma; (d) level of I2S in plasma; (e) level of leukocyte I2S enzyme activity; and (f) level of GAGs in urine. In a specific embodiment, the GAGs in CSF comprise heparin sulfate in CSF. In another specific embodiment, the GAGs in CSF are heparin sulfate in CSF. In another specific embodiment, the GAGs in plasma comprise heparin sulfate in plasma. In another specific embodiment, the GAGs in plasma are heparin sulfate in plasma. In another specific embodiment, the GAGs in urine comprise heparin sulfate in urine. In another specific embodiment, the GAGs in urine are heparin sulfate in urine. In a specific embodiment, the step of measuring comprises mearing level of heparin sulfate in CSF. In another specific embodiment, the step of measuring comprises measuring level of leukocyte I2S enzyme activity.

In certain embodiments of the method for treating described herein, the recombinant nucleotide expression vector is a liquid composition. In certain embodiments of the method for treating described herein, the recombinant nucleotide expression vector is a frozen composition. In certain embodiments of the method for treating described herein, the recombinant nucleotide expression vector is a lyophilized composition or a reconstituted lyophilized composition. In certain embodiments of the method for treating described herein, the recombinant nucleotide expression vector provided herein may be formulated in various dosage forms for IC or ICV administration. In certain embodiments of the method for treating described herein, the recombinant nucleotide expression vector provided herein may be provided in a unit-dosage form or multiple-dosage form. A unit-dosage form, as used herein, refers to a physically discrete unit suitable for administration to human and animal subjects, and packaged individually as is known in the art. Each unit-dose contains a predetermined quantity of the said recombinant nucleotide expression vector and/or other ingredient(s) sufficient to produce the desired therapeutic effect, in association with the required pharmaceutical carriers or excipients. Examples of a unit-dosage form include an ampoule, a vial, a prefilled syringe, or a cartridge.

In certain embodiments of the method for treating described herein, a unit-dosage form may be administered in fractions or multiples thereof. In certain embodiments of the method for treating described herein, a multiple-dosage form is a plurality of identical unit-dosage forms packaged in a single container to be administered in segregated unit-dosage form. Examples of a multiple-dosage form include a vial, a prefilled syringe, or a cartridge. In certain embodiments, the prefilled syringe comprises 8.5× 10¹² GC of the recombinant nucleotide expression vector In certain embodiments, the prefilled syringe comprises 9.8× 10¹² GC of the recombinant nucleotide expression vector. In certain embodiments, the prefilled syringe comprises 1.1× 10¹³ GC of the recombinant nucleotide expression vector. In certain embodiments, the prefilled syringe comprises 1.3× 10¹³ GC of the recombinant nucleotide expression vector. In certain embodiments, the prefilled syringe comprises 1.5× 10¹³ GC of the recombinant nucleotide expression vector. In certain embodiments, the prefilled syringe comprises 1.7× 10¹³ GC of the recombinant nucleotide expression vector. In certain embodiments, the prefilled syringe comprises 4.2× 10¹³ GC of the recombinant nucleotide expression vector In certain embodiments, the prefilled syringe comprises 4.9× 10¹³ GC of the recombinant nucleotide expression vector. In certain embodiments, the prefilled syringe comprises 5.5× 10¹³ GC of the recombinant nucleotide expression vector. In certain embodiments, the prefilled syringe comprises 6.3× 10¹³ GC of the recombinant nucleotide expression vector. In certain embodiments, the prefilled syringe comprises 7.3× 10¹³ GC of the recombinant nucleotide expression vector. In certain embodiments, the prefilled syringe comprises 8.5× 10¹³ GC of the recombinant nucleotide expression vector. In certain embodiments, the prefilled syringe comprises 9.0× 10¹³ GC of the recombinant nucleotide expression vector. In certain embodiments, the prefilled syringe comprises 1.0× 10¹⁴ GC of the recombinant nucleotide expression vector. In certain embodiments, the prefilled syringe comprises 1.1× 10¹⁴ GC of the recombinant nucleotide expression vector. In certain embodiments, the prefilled syringe comprises 1.2× 10¹⁴ GC of the recombinant nucleotide expression vector. In certain embodiments, the prefilled syringe comprises 1.3× 10¹⁴ GC of the recombinant nucleotide expression vector. In certain embodiments, the prefilled syringe comprises 1.4× 10¹⁴ GC of the recombinant nucleotide expression vector. In certain embodiments, the prefilled syringe comprises 1.5× 10¹⁴ GC of the recombinant nucleotide expression vector. In certain embodiments, the prefilled syringe comprises 1.6× 10¹⁴ GC of the recombinant nucleotide expression vector. In certain embodiments, the prefilled syringe comprises 1.7× 10¹⁴ GC of the recombinant nucleotide expression vector. In certain embodiments, the prefilled syringe comprises 1.8× 10¹⁴ GC of the recombinant nucleotide expression vector. In certain embodiments, the prefilled syringe comprises 1.9× 10¹⁴ of the recombinant nucleotide expression vector. In certain embodiments, the prefilled syringe comprises 2.0× 10¹⁴ GC of the recombinant nucleotide expression vector. In certain embodiments, the prefilled syringe comprises 2.1× 10¹⁴ GC of the recombinant nucleotide expression vector. In certain embodiments, the prefilled syringe comprises 2.2× 10¹⁴ GC of the recombinant nucleotide expression vector. In certain embodiments, the prefilled syringe comprises 2.3× 10¹⁴ GC of the recombinant nucleotide expression vector. In certain embodiments, the prefilled syringe comprises 2.4× 10¹⁴ GC of the recombinant nucleotide expression vector. In certain embodiments, the prefilled syringe comprises 2.5× 10¹⁴ GC of the recombinant nucleotide expression vector. In certain embodiments, the prefilled syringe comprises 2.6× 10¹⁴ GC of the recombinant nucleotide expression vector.

As used herein, the term "about" means within plus or minus 10% of a given value or range. In certain embodiments, the term "about" means within plus or minus 1% of a given value or range, wherein the value is a dose that is dependent on the human subject's brain mass, and wherein the brain mass is determined by brain MRI of the human subject's brain. In certain embodiments, the term "about" means within plus or minus 2% of a given value or range, wherein the value is a dose that is determined by brain MRI of the subject's brain, and wherein the brain mass is determined by brain MRI of the human subject's brain. In certain embodiments, the term "about" means within plus or minus 5% of a given value or range, wherein the value is a dose that is dependent on the human subject's brain mass, and wherein the brain mass is determined by brain MRI of the human subject's brain. In certain embodiments, the term "about" means within plus or minus 7% of a given value or range, wherein the value is a dose that is dependent on the human subject's brain mass, and wherein the brain mass is determined by brain MRI of the human subject's brain. In certain embodiments, the term "about" means within plus or minus 10% of a given value or range, wherein the value is a dose that is dependent on the human subject's brain mass, and wherein the brain mass is determined by brain MRI of the human subject's brain. However, it is to be understood that in this specification, the term "about" also affords support for recitation of the exact value with which the term is connected. For example, "about 10" also provides support for the number "10" exactly.

### 5.1 PROCESSING, N-GLYCOSYLATION AND TYROSINE SULFATION

### 5.1.1. Processing

Human IDS includes a 25 amino acid signal sequence which is cleaved during processing. An initial 76 kDa intracellular IDS precursor is converted into a phosphorylated 90 kDa IDS precursor after modification of its oligosaccharide chains in the Golgi apparatus. This precursor is processed by glycosylation modifications and proteolytic cleavage through various intracellular intermediates to a major 55 kDa form. To summarize, after removal of the 25 aa signal sequence, proteolytic processing involves N-terminal proteolytic cleavage downstream of N³¹ removing a propeptide of eight amino acids (residues 26-33), and C-terminal proteolytic cleavage upstream of N⁵¹³ which releases an 18 kDa polypeptide and produces a 62 kDa intermediate that is converted to a 55 kDa mature form. Further proteolytic cleavage yields a 45 kDa mature form located in the lysosomal compartment. (*See* FIG. 4 for diagram reproduced from Millat et al., 1997, Exp Cell Res 230: 362-367 ("Millat 1997"); Millat et al. 1997, Biochem J. 326: 243-247 ("Millat 1997a"); and Froissart et al., 1995, Biochem J. 309:425-430, each of which is incorporated by reference herein in its entirety).

A formylglycine modification of C⁸⁴ (shown in bold in FIG. 1) required for enzyme activity probably occurs as an early post-translational or co-translational event, most probably in the endoplasmic reticulum. *(See,* Millat 1997a, citing Schmidt et al., 1995, Cell 82: 271-278). Post-translational processing continues in the Golgi to include addition of complex sialic acid-containing glycans and acquisition of mannose-6-phosphate residues which tag the enzyme for delivery to the lysosomal compartment. (*See*, Clarke, 2008, Expert Opin Pharmacother 9: 311-317 for a concise review which is incorporated by reference herein in its entirety).

In a specific embodiment, HuGlyIDS used in accordance with the methods described herein, when expressed in a neuronal or glial cell, *in vivo* or *in vitro,* can be the 90 kDa (*e.g.*, 85 kDa, 86 kDa, 87 kDa, 88 kDa, 89 kDa, 90 kDa, 91 kDa, 92 kDa, 93 kDa, 94 kDa, or 95 kDa) mannose-6-phosphorylated form of the enzyme. IDS produced from neuronal and glial cells may contain higher M6P content, as reported in Daniele 2002, and in Sleat, Proteomics, 2005 (indicating that the human brain contains more (in both a quantitative and qualitative sense) M6P glycoproteins than other tissues.). It is possible to measure the M6P content of an IDS precursor, as done in Daniele 2002.

Accordingly, in certain embodiments, HuGlyIDS used in accordance with the methods described herein, when expressed in a neuronal or glial cell, *in vivo* or *in vitro,* is mannose-6-phosphorylated at a higher level than IDS expressed in a non-neuronal or glial cell. In particular, HuGlyIDS used in accordance with the methods described herein, when expressed in a neuronal or glial cell, *in vivo* or *in vitro,* is mannose-6-phosphorylated at a higher level than IDS expressed in a HT1080 or CHO cell. In certain embodiments, the mannose-6-phosphorylation level of the expresssed IDS is measured by uptake of the IDS by a human neuronal cell in the presence of M6P *(e.g.,* 5 mM M6P). In certain embodiments, when expressed in a neuronal or glial cell, *in vivo* or *in vitro,* 10% - 20%, 20% - 30%, 30% - 40%, 40% - 50%, 50% - 60%, 60% - 70%, 70% - 80%, 80% - 90%, or 90% - 100% of HuGlyIDS molecules used in accordance with the methods described herein are mannose-6-phosphorylated.

### 5.1.2. N-Glycosylation

Neuronal and glial cells in the CNS are secretory cells that possess the cellular machinery for post-translational processing of secreted proteins - including glycosylation and tyrosine-O-sulfation. hIDS has eight asparaginal ("N") glycosylation sites identified in FIG. 1 (N³¹ST; N¹¹⁵FS; N¹⁴⁴HT; N²⁴⁶IT; N²⁸⁰IS; N³²⁵ST; N⁵¹³FS; N⁵³⁷DS). Two of the eight N-linked glycosylation sites, namely N²⁸⁰ and N¹¹⁶, are mannose-6-phophorylated in IDS obtained from human brain. (Sleat et al., 2006, Mol & Cell Proeomics 5.4: 686-701, reported at Table V). While no single glycosylation site is essential for IDS stability, glycosylation at position N²⁸⁰ is important for cellular internalization and lysosomal targeting via the mannose-6-phosphate (M6P) receptor. (Chung et al., 2014, Glycoconj J 31:309-315 at p. 310, first column). In the normal physiologic state, IDS is produced at very low levels and very little, if any, enzyme is secreted from the cell. (Clarke, 2008, *supra*)*.*

It is not essential that every molecule produced either in the gene therapy or protein therapy approach be fully glycosylated and sulfated. Rather, the population of glycoproteins produced should have sufficient glycosylation and sulfation to demonstrate efficacy.

In a specific embodiment, HuGlyIDS used in accordance with the methods described herein, when expressed in a neuronal or glial cell, *in vivo* or *in vitro,* could be glycosylated at 100% of its N-glycosylation sites. However, one of skill in the art will appreciate that not every N-glycosylation site of HuGlyIDS need be N-glycosylated in order for benefits of glycosylation to be attained. Rather, benefits of glycosylation can be realized when only a percentage of N-glycosylation sites are glycosylated, and/or when only a percentage of expressed IDS molecules are glycosylated. Accordingly, in certain embodiments, HuGlyIDS used in accordance with the methods described herein, when expressed in a neuronal or glial cell, *in vivo* or *in vitro,* is glycosylated at 10% - 20%, 20% - 30%, 30% - 40%, 40% - 50%, 50% - 60%, 60% - 70%, 70% - 80%, 80% - 90%, or 90% - 100% of its available N-glycosylation sites. In certain embodiments, when expressed in a neuronal or glial cell, *in vivo* or *in vitro,* 10% - 20%, 20% - 30%, 30% - 40%, 40% - 50%, 50% - 60%, 60% - 70%, 70% - 80%, 80% - 90%, or 90% - 100% of HuGlyIDS molecules used in accordance with the methods described herein are glycosylated at least one of their available N-glycosylation sites.

In a specific embodiment, at least 10%, 20% 30%, 40%, 50%, 60%, 70%, 75%, 80%, 85%, 90%, 95%, or 99% of the N-glycosylation sites present in HuGlyIDS used in accordance with the methods described herein are glycosylated at an Asn residue (or other relevant residue) present in an N-glycosylation site, when the HuGlyIDS is expressed in a neuronal or glial cell, *in vivo* or *in vitro.* That is, at least 50%, 60%, 70%, 75%, 80%, 85%, 90%, 95%, or 99% of the N-glycosylation sites of the resultant HuGlyIDS are glycosylated.

In another specific embodiment, at least 10%, 20% 30%, 40%, 50%, 60%, 70%, 75%, 80%, 85%, 90%, 95%, or 99% of the N-glycosylation sites present in a HuGlyIDS molecule used in accordance with the methods described herein are glycosylated with an identical attached glycan linked to the Asn residue (or other relevant residue) present in an N-glycosylation site, when the HuGlyIDS is expressed in a neuronal or glial cell, *in vivo* or *in vitro.* That is, at least 50%, 60%, 70%, 75%, 80%, 85%, 90%, 95%, or 99% of the N-glycosylation sites of the resultant HuGlyIDS have an identical attached glycan.

Importantly, when the IDS proteins used in accordance with the methods described herein are expressed in neuronal or glial cells, the need for *in vitro* production in prokaryotic host cells (e.g., *E. coli*) or eukaryotic host cells (e.g., CHO cells) is circumvented. Instead, as a result of the methods described herein (e.g., use of neuronal or glial cells to express IDS), N-glycosylation sites of the IDS proteins are advantageously decorated with glycans relevant to and beneficial to treatment of humans, and, in particular, at the target location of treatment. Such an advantage is unattainable when CHO cells or *E. coli* are utilized in protein production, because e.g., CHO cells (1) do not express 2,6 sialyltransferase and thus cannot add 2,6 sialic acid during N-glycosylation and (2) can add Neu5Gc as sialic acid instead of Neu5Ac; and because *E. coli* does not naturally contain components needed for N-glycosylation. Furthermore, such an advantage may be unattainable when human cells that are not neuronal or glial cells are utilized in protein production. Accordingly, in one embodiment, an IDS protein expressed in a neuronal or glial cell to give rise to a HuGlyIDS used in the methods of treatment described herein is glycosylated in the manner in which a protein is N-glycosylated in human neuronal or glial cells, but is not glycosylated in the manner in which proteins are glycosylated in CHO cells. In another embodiment, an IDS protein expressed in a neuronal or glial cell to give rise to a HuGlyIDS used in the methods of treatment described herein is glycosylated in the manner in which a protein is N-glycosylated in a neuronal or glial cells, wherein such glycosylation is not naturally possible using a prokaryotic host cell, e.g., using *E. coli.* In one embodiment, an IDS protein expressed in a human neuronal or glial cell to give rise to a HuGlyIDS used in the methods of treatment described herein is glycosylated in the manner in which a protein is N-glycosylated in human neuronal or glial cells, but is not glycosylated in the manner in which proteins are glycosylated in human cells which are not neuronal or glial cells.

Assays for determining the glycosylation pattern of proteins are known in the art. For example, hydrazinolysis can be used to analyze glycans. First, polysaccharides are released from their associated protein by incubation with hydrazine (the Ludger Liberate Hydrazinolysis Glycan Release Kit, Oxfordshire, UK can be used). The nucleophile hydrazine attacks the glycosidic bond between the polysaccharide and the carrier protein and allows release of the attached glycans. N-acetyl groups are lost during this treatment and have to be reconstituted by re-N-acetylation. The free glycans can be purified on carbon columns and subsequently labeled at the reducing end with the fluorophor 2-amino benzamide. The labeled polysaccharides can be separated on a GlycoSep-N column (GL Sciences) according to the HPLC protocol of Royle et al, Anal Biochem 2002, 304(1):70-90. The resulting fluorescence chromatogram indicates the polysaccharide length and number of repeating units. Structural information can be gathered by collecting individual peaks and subsequently performing MS/MS analysis. Thereby the monosaccharide composition and sequence of the repeating unit can be confirmed and additionally in homogeneity of the polysaccharide composition can be identified. Specific peaks of low molecular weight can be analyzed by MALDI-MS/MS and the result used to confirm the glycan sequence. Each peak corresponds to a polymer consisting of a certain number of repeat units and fragments thereof. The chromatogram thus allows measurement of the polymer length distribution. The elution time is an indication for polymer length, while fluorescence intensity correlates with molar abundance for the respective polymer.

Homogeneity of the glycan patterns associated with proteins, as it relates to both glycan length and numbers glycans present across glycosylation sites, can be assessed using methods known in the art, e.g., methods that measure glycan length and hydrodynamic radius. Size exclusion-HPLC allows the measurement of the hydrodynamic radius. Higher numbers of glycosylation sites in a protein lead to higher variation in hydrodynamic radius compared to a carrier with less glycosylation sites. However, when single glycan chains are analyzed, they may be more homogenous due to the more controlled length. Glycan length can measured by hydrazinolysis, SDS PAGE, and capillary gel electrophoresis. In addition, homogeneity can also mean that certain glycosylation site usage patterns change to a broader/narrower range. These factors can be measured by Glycopeptide LC-MS/MS.

N-glycosylation confers numerous benefits on the HuGlyIDS used in the methods described herein. Such benefits are unattainable by production of proteins in *E. coli,* because *E. coli* does not naturally possess components needed for N-glycosylation. Further, some benefits are unattainable through protein production in, e.g., CHO cells, because CHO cells lack components needed for addition of certain glycans (e.g., 2,6 sialic acid) and because CHO cells can add glycans, e.g., Neu5Gc not typical to humans, and the α-Gal antigen which is immunogenic in most individuals and at high concentrations can trigger anaphylaxis. Even further, some benefits are unattainable through protein production in human cells that are not neuronal or glial cells. Thus, the expression of IDS in human neuronal or glial cells results in the production of HuGlyIDS comprising beneficial glycans that otherwise would not be associated with the protein if produced in CHO cells, in *E. coli,* or in human cells which are not neuronal or glial cells.

### 5.1.3. Tyrosine Sulfation

In addition to the N-linked glycosylation sites, hIDS contains a tyrosine ("Y") sulfation site (PSSEKY¹⁶⁵ENTKTCRGPD). (*See*, *e.g.*, Yang et al., 2015, Molecules 20:2138-2164, esp. at p. 2154 which is incorporated by reference in its entirety for the analysis of amino acids surrounding tyrosine residues subjected to protein tyrosine sulfation. The "rules" can be summarized as follows: Y residues with E or D within +5 to -5 position of Y, and where position -1 of Y is a neutral or acidic charged amino acid - but not a basic amino acid, *e.g.,* R, K, or H that abolishes sulfation).

Importantly, tyrosine-sulfated proteins cannot be produced in *E. coli,* which naturally does not possess the enzymes required for tyrosine-sulfation. Further, CHO cells are deficient for tyrosine sulfation-they are not secretory cells and have a limited capacity for post-translational tyrosine-sulfation. See, e.g., Mikkelsen & Ezban, 1991, Biochemistry 30: 1533-1537. Advantageously, the methods provided herein call for expression of IDS, e.g., HuGlyIDS, in neurons or glial cells, which are secretory and do have capacity for tyrosine sulfation. Assays for detection tyrosine sulfation are known in the art. See, e.g., Yang et al., 2015, Molecules 20:2138-2164.

Tyrosine-sulfation of hIDS - a robust post-translational process in human CNS cells - should result in improved processing and activity of transgene products. The significance of tyrosine-sulfation of lysosomal proteins has not been elucidated; but in other proteins it has been shown to increase avidity of protein-protein interactions (antibodies and receptors), and to promote proteolytic processing (peptide hormone). (See, Moore, 2003, J Biol. Chem. 278:24243-46; and Bundegaard et al., 1995, The EMBO J 14: 3073-79). The tyrosylprotein sulfotransferase (TPST1) responsible for tyrosine-sulfation (which may occur as a final step in IDS processing) is apparently expressed at higher levels (based on mRNA) in the brain (gene expression data for TPST1 may be found, for example, at the EMBL-EBI Expression Atlas, accessible at http://www.ebi.ac.uk/gxa/home).

### 5.2 CONSTRUCTS AND FORMULATIONS

For use in the methods provided herein are viral vectors or other DNA expression constructs encoding iduronate-2-sulfatase (IDS), *e.g*., human IDS (hIDS). The viral vectors and other DNA expression constructs provided herein include any suitable method for delivery of a transgene to the cerebrospinal fluid (CSF). The means of delivery of a transgene include viral vectors, liposomes, other lipid-containing complexes, other macromolecular complexes, synthetic modified mRNA, unmodified mRNA, small molecules, non-biologically active molecules (*e.g.*, gold particles), polymerized molecules *(e.g.,* dendrimers), naked DNA, plasmids, phages, transposons, cosmids, or episomes. In some embodiments, the vector is a targeted vector, *e.g.,* a vector targeted to neuronal cells.

In some aspects, the disclosure provides for a nucleic acid for use, wherein the nucleic acid encodes an IDS, *e.g.,* hIDS, operatively linked to a promoter selected from the group consisting of: cytomegalovirus (CMV) promoter, Rous sarcoma virus (RSV) promoter, MMT promoter, EF-1 alpha promoter, UB6 promoter, chicken beta-actin promoter, CAG promoter, RPE65 promoter and opsin promoter.

In certain embodiments, provided herein are recombinant vectors that comprise one or more nucleic acids (e.g. polynucleotides). The nucleic acids may comprise DNA, RNA, or a combination of DNA and RNA. In certain embodiments, the DNA comprises one or more of the sequences selected from the group consisting of promoter sequences, the sequence of the gene of interest (the transgene, *e.g*., IDS), untranslated regions, and termination sequences. In certain embodiments, viral vectors provided herein comprise a promoter operably linked to the gene of interest.

In certain embodiments, nucleic acids (*e.g*., polynucleotides) and nucleic acid sequences disclosed herein may be codon-optimized, for example, via any codon-optimization technique known to one of skill in the art (*see, e.g.*, review by Quax et al., 2015, Mol Cell 59:149-161).

In another aspect, the disclosure provides for a formulation comprising a recombinant nucleotide expression vector encoding human IDS, wherein the formulation is suitable for administration to the cerebrospinal fluid of human brain, so that a depot is formed in the human central nervous system that secretes a recombinant human IDS glycoprotein precursor that is about 90 kDa (*e.g.*, 85 kDa, 86 kDa, 87 kDa, 88 kDa, 89 kDa, 90 kDa, 91 kDa, 92 kDa, 93 kDa, 94 kDa, or 95 kDa) as measured by polyacrylamide gel electrophoresis, contains a formylglycine, is α2,6-sialylated, does not contain detectable NeuGc, does not contain α-Gal antigen, and/or is mannose-6-phosphorylated. For example, the formulation may contain buffer (such as, a buffer having a particular pH, or a buffer containing a particular ingredient) that makes it suitable for administration to the cerebrospinal fluid of human brain, so that a depot is formed in the human central nervous system that secretes a recombinant human IDS glycoprotein precursor that is about 90 kDa (*e.g.*, 85 kDa, 86 kDa, 87 kDa, 88 kDa, 89 kDa, 90 kDa, 91 kDa, 92 kDa, 93 kDa, 94 kDa, or 95 kDa) as measured by polyacrylamide gel electrophoresis, contains a formylglycine, is α2,6-sialylated, does not contain detectable NeuGc, does not contain α-Gal antigen, and/or is mannose-6-phosphorylated. In a specific embodiment, the buffer comprises a physiologically compatible aqueous buffer, a surfactant and optional excipients.

In another aspect, the disclosure provides for a kit comprising a recombinant nucleotide expression vector encoding human IDS and a pharmaceutically acceptable carrier, wherein the recombinant nucleotide expression vector is suitable for administration to the cerebrospinal fluid (CSF) of human brain, so that a depot is formed in the human central nervous system that secretes a recombinant human IDS glycoprotein precursor that is about 90 kDa (*e.g.*, 85 kDa, 86 kDa, 87 kDa, 88 kDa, 89 kDa, 90 kDa, 91 kDa, 92 kDa, 93 kDa, 94 kDa, or 95 kDa) as measured by polyacrylamide gel electrophoresis, contains a formylglycine, is α2,6-sialylated, does not contain detectable NeuGc, does not contain detectable α-Gal antigen, and/or is mannose-6-phosphorylated. In another aspect, the disclosure provides for a kit comprising a formulation comprising a recombinant nucleotide expression vector encoding human IDS, wherein the formulation is suitable for administration to the CSF of human brain, so that a depot is formed in the human central nervous system that secretes a recombinant human IDS glycoprotein precursor that is about 90 kDa (*e.g.*, 85 kDa, 86 kDa, 87 kDa, 88 kDa, 89 kDa, 90 kDa, 91 kDa, 92 kDa, 93 kDa, 94 kDa, or 95 kDa) as measured by polyacrylamide gel electrophoresis, contains a formylglycine, is α2,6-sialylated, does not contain detectable NeuGc, does not contain detectable α-Gal antigen, and/or is mannose-6-phosphorylated. A kit described herein comprises the recombinant nucleotide expression vector or the formulation in one or more containers. Optionally associated with such one or more containers can be a notice in the form prescribed by a governmental agency regulating the manufacture, use or sale of pharmaceuticals or biological products, which notice reflects approval by the agency of manufacture, use or sale for human administration.

The formulations and kits encompassed herein can be used in accordance with the methods for treating a human patient as provided in this disclosure.

### 5.2.1. mRNA

In certain embodiments, the vectors provided herein are modified mRNA encoding for the gene of interest (*e.g*., the transgene, for example, IDS). The synthesis of modified and unmodified mRNA for delivery of a transgene to the CSF is taught, for example, in Hocquemiller et al., 2016, Human Gene Therapy 27(7):478-496, which is incorporated by reference herein in its entirety. In certain embodiments, provided herein is a modified mRNA encoding for IDS, *e.g.,* hIDS.

### 5.2.2. Viral vectors

Viral vectors include adenovirus, adeno-associated virus (AAV, *e.g*., AAV9, AAVrh10), lentivirus, helper-dependent adenovirus, herpes simplex virus, poxvirus, hemagglutinin virus of Japan (HVJ), alphavirus, vaccinia virus, and retrovirus vectors. Retroviral vectors include murine leukemia virus (MLV)- and human immunodeficiency virus (HIV)-based vectors. Alphavirus vectors include semliki forest virus (SFV) and sindbis virus (SIN). In certain embodiments, the viral vectors provided herein are recombinant viral vectors. In certain embodiments, the viral vectors provided herein are altered such that they are replication-deficient in humans. In certain embodiments, the viral vectors are hybrid vectors, *e.g.,* an AAV vector placed into a "helpless" adenoviral vector. In certain embodiments, provided herein are viral vectors comprising a viral capsid from a first virus and viral envelope proteins from a second virus. In specific embodiments, the second virus is vesicular stomatitus virus (VSV). In more specific embodiments, the envelope protein is VSV-G protein.

In certain embodiments, the viral vectors provided herein are HIV based viral vectors. In certain embodiments, HIV-based vectors provided herein comprise at least two polynucleotides, wherein the gag and pol genes are from an HIV genome and the env gene is from another virus.

In certain embodiments, the viral vectors provided herein are herpes simplex virus-based viral vectors. In certain embodiments, herpes simplex virus-based vectors provided herein are modified such that they do not comprise one or more immediately early (IE) genes, rendering them non-cytotoxic.

In certain embodiments, the viral vectors provided herein are MLV based viral vectors. In certain embodiments, MLV-based vectors provided herein comprise up to 8 kb of heterologous DNA in place of the viral genes.

In certain embodiments, the viral vectors provided herein are lentivirus-based viral vectors. In certain embodiments, lentiviral vectors provided herein are derived from human lentiviruses. In certain embodiments, lentiviral vectors provided herein are derived from non-human lentiviruses. In certain embodiments, lentiviral vectors provided herein are packaged into a lentiviral capsid. In certain embodiments, lentiviral vectors provided herein comprise one or more of the following elements: long terminal repeats, a primer binding site, a polypurine tract, att sites, and an encapsidation site.

In certain embodiments, the viral vectors provided herein are alphavirus-based viral vectors. In certain embodiments, alphavirus vectors provided herein are recombinant, replication-defective alphaviruses. In certain embodiments, alphavirus replicons in the alphavirus vectors provided herein are targeted to specific cell types by displaying a functional heterologous ligand on their virion surface.

In certain embodiments, the viral vectors provided herein are AAV based viral vectors. In preferred embodiments, the viral vectors provided herein are AAV9 or AAVrh10 based viral vectors. In certain embodiments, the AAV9 or AAVrh10 based viral vectors provided herein retain tropism for CNS cells. Multiple AAV serotypes have been identified. In certain embodiments, AAV-based vectors provided herein comprise components from one or more serotypes of AAV. In certain embodiments, AAV based vectors provided herein comprise components from one or more of AAV1, AAV2, AAV3, AAV4, AAV5, AAV6, AAV7, AAV8, AAV9, AAVrh10, AAV10 or AAV11. In preferred embodiments, AAV based vectors provided herein comprise components from one or more of AAV8, AAV9, AAVrh10, AAV10, or AAV11 serotypes. AAV9-based viral vectors are used in the methods described herein. Nucleic acid sequences of AAV based viral vectors and methods of making recombinant AAV and AAV capsids are taught, for example, in United States Patent No. 7,282,199 B2, United States Patent No. 7,790,449 B2, United States Patent No. 8,318,480 B2, United States Patent No. 8,962,332 B2 and International Patent Application No. PCT/EP2014/076466, each of which is incorporated herein by reference in its entirety. In one aspect, provided herein are AAV (e.g., AAV9 or AAVrh10)-based viral vectors encoding a transgene (*e.g*., IDS). In specific embodiments, provided herein are AAV9-based viral vectors encoding IDS. In more specific embodiments, provided herein are AAV9-based viral vectors encoding hIDS.

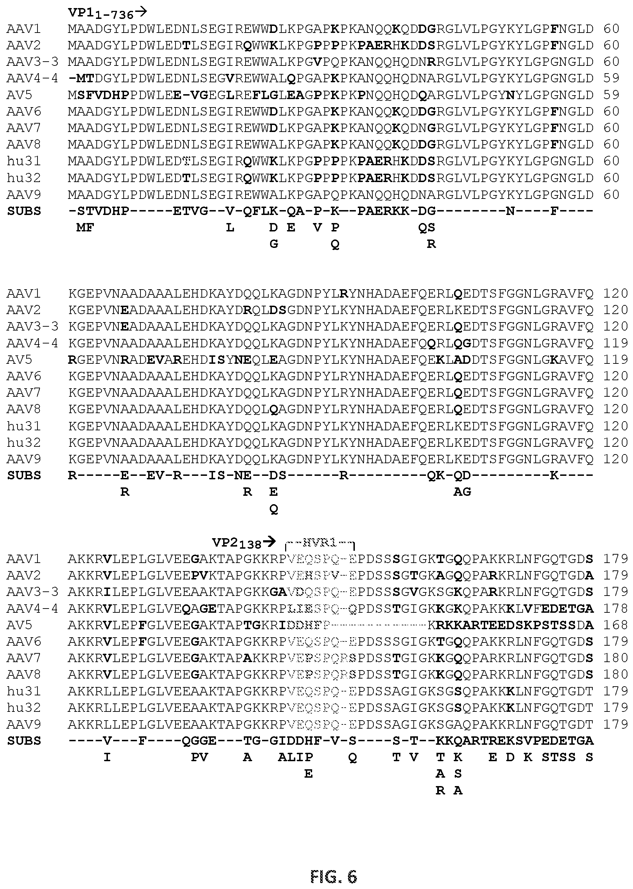

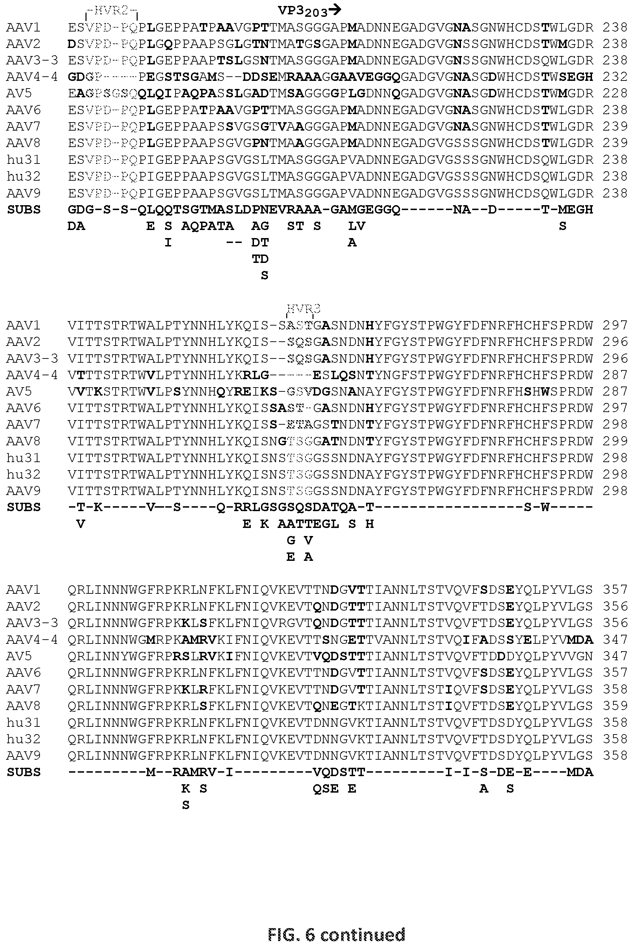

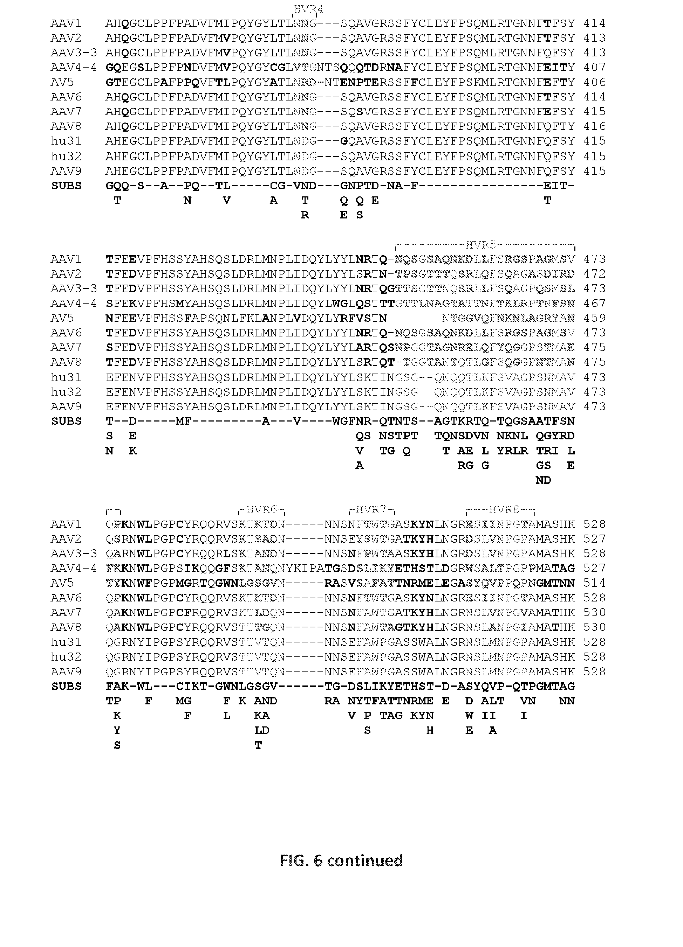

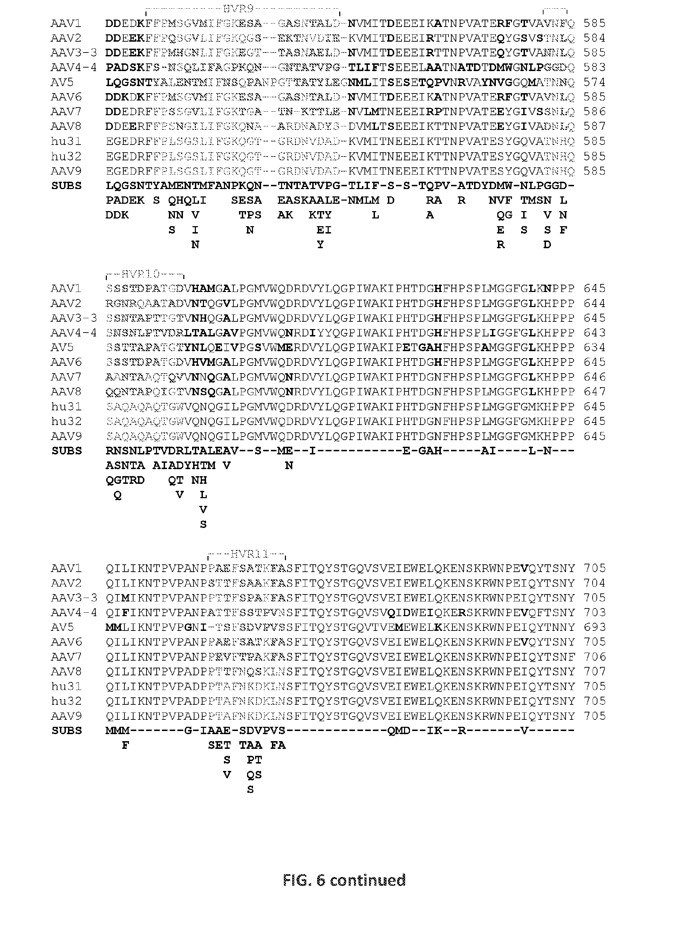

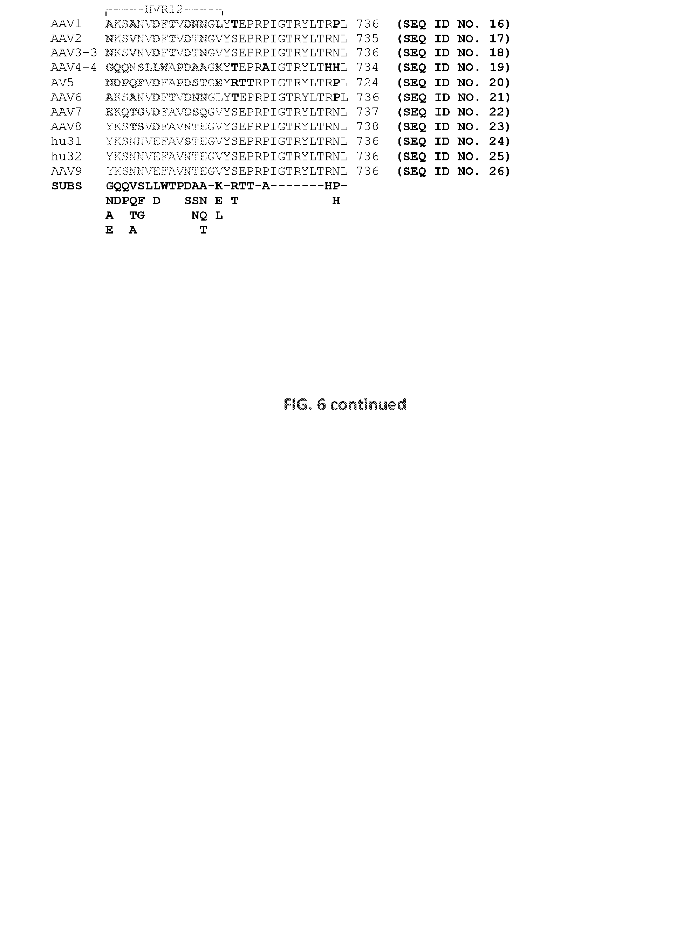

Provided in particular embodiments are AAV9 vectors comprising an artificial genome comprising (i) an expression cassette containing the transgene under the control of regulatory elements and flanked by ITRs; and (ii) a viral capsid that has the amino acid sequence of the AAV9 capsid protein or is at least 95%, 96%, 97%, 98%, 99% or 99.9% identical to the amino acid sequence of the AAV9 capsid protein (SEQ ID NO: 26) while retaining the biological function of the AAV9 capsid. In certain embodiments, the encoded AAV9 capsid has the sequence of SEQ ID NO: 26 with 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acid substitutions and retaining the biological function of the AAV9 capsid. FIG. 6 provides a comparative alignment of the amino acid sequences of the capsid proteins of different AAV serotypes with potential amino acids that may be substituted at certain positions in the aligned sequences based upon the comparison in the row labeled SUBS. Accordingly, in specific embodiments, the AAV9 vector comprises an AAV9 capsid variant that has 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acid substitutions identified in the SUBS row of FIG. 6 that are not present at that position in the native AAV9 sequence.

In certain embodiments, the AAV that is used in the methods described herein is Anc80 or Anc80L65, as described in Zinn et al., 2015, Cell Rep. 12(6): 1056-1068, which is incorporated by reference in its entirety. In certain embodiments, the AAV that is used in the methods described herein comprises one of the following amino acid insertions: LGETTRP or LALGETTRP, as described in United States Patent Nos. 9,193,956; 9458517; and 9,587,282 and US patent application publication no. 2016/0376323, each of which is incorporated herein by reference in its entirety. In certain embodiments, the AAV that is used in the methods described herein is AAV.7m8, as described in United States Patent Nos. 9,193,956; 9,458,517; and 9,587,282 and US patent application publication no. 2016/0376323, each of which is incorporated herein by reference in its entirety. In certain embodiments, the AAV that is used in the methods described herein is any AAV disclosed in United States Patent No. 9,585,971, such as AAV-PHP.B. In certain embodiments, the AAV that is used in the methods described herein is an AAV disclosed in any of the following patents and patent applications, each of which is incorporated herein by reference in its entirety: United States Patent Nos. 7,906,111; 8,524,446; 8,999,678; 8,628,966; 8,927,514; 8,734,809; US 9,284,357; 9,409,953; 9,169,299; 9,193,956; 9458517; and 9,587,282 US patent application publication nos. 2015/0374803; 2015/0126588; 2017/0067908; 2013/0224836; 2016/0215024; 2017/0051257; and International Patent Application Nos. PCT/US2015/034799; PCT/EP2015/053335.

In certain embodiments, a single-stranded AAV (ssAAV) may be used *supra.* In certain embodiments, a self-complementary vector, e.g., scAAV, may be used (*see*, *e.g.,* Wu, 2007, Human Gene Therapy, 18(2):171-82, McCarty et al, 2001, Gene Therapy, Vol 8, Number 16, Pages 1248-1254; and U.S. Patent Nos. 6,596,535; 7,125,717; and 7,456,683, each of which is incorporated herein by reference in its entirety).

In certain embodiments, the viral vectors used in the methods described herein are adenovirus based viral vectors . A recombinant adenovirus vector may be used to transfer in the IDS. The recombinant adenovirus can be a first generation vector, with an E1 deletion, with or without an E3 deletion, and with the expression cassette inserted into either deleted region. The recombinant adenovirus can be a second generation vector, which contains full or partial deletions of the E2 and E4 regions. A helper-dependent adenovirus retains only the adenovirus inverted terminal repeats and the packaging signal (phi). The transgene is inserted between the packaging signal and the 3'ITR, with or without stuffer sequences to keep the artificial genome close to wild-type size of approx. 36 kb. An exemplary protocol for production of adenoviral vectors may be found in Alba et al., 2005, "Gutless adenovirus: last generation adenovirus for gene therapy," Gene Therapy 12:S18-S27, which is incorporated by reference herein in its entirety.

In certain embodiments, the viral vectors used in the methods described herein are lentivirus based viral vectors . A recombinant lentivirus vector may be used to transfer in the IDS. Four plasmids are used to make the construct: Gag/pol sequence containing plasmid, Rev sequence containing plasmids, Envelope protein containing plasmid (i.e. VSV-G), and Cis plasmid with the packaging elements and the IDS gene.

For lentiviral vector production, the four plasmids are co-transfected into cells (i.e., HEK293 based cells), whereby polyethylenimine or calcium phosphate can be used as transfection agents, among others. The lentivirus is then harvested in the supernatant (lentiviruses need to bud from the cells to be active, so no cell harvest needs/should be done). The supernatant is filtered (0.45 µm) and then magnesium chloride and benzonase added. Further downstream processes can vary widely, with using TFF and column chromatography being the most GMP compatible ones. Others use ultracentrifugation with/without column chromatography. Exemplary protocols for production of lentiviral vectors may be found in Lesch et al., 2011, "Production and purification of lentiviral vector generated in 293T suspension cells with baculoviral vectors," Gene Therapy 18:531-538, and Ausubel et al., 2012, "Production of CGMP-Grade Lentiviral Vectors," Bioprocess Int. 10(2):32-43, both of which are incorporated by reference herein in their entireties.

In a specific embodiment, a vector for use in the methods described herein is one that encodes an IDS (e.g., hIDS) such that, upon transduction of cells in the CNS, or a relevant cell (e.g., a neuronal cell *in vivo* or *in vitro*), a glycosylated variant of IDS is expressed by the transduced cell. In a specific embodiment, a vector for use in the methods described herein is one that encodes an IDS (e.g., hIDS) such that, upon transduction of a cell in the CNS, or a relevant cell (e.g., a neuronal cell *in vivo* or *in vitro*), a sulfated variant of IDS is expressed by the cell.

### 5.2.3. Promoters and Modifiers of Gene Expression

In certain embodiments, the vectors provided herein comprise components that modulate gene delivery or gene expression (*e.g*., "expression control elements"). In certain embodiments, the vectors provided herein comprise components that modulate gene expression. In certain embodiments, the vectors provided herein comprise components that influence binding or targeting to cells. In certain embodiments, the vectors provided herein comprise components that influence the localization of the polynucleotide (*e.g*., the transgene) within the cell after uptake. In certain embodiments, the vectors provided herein comprise components that can be used as detectable or selectable markers, *e.g*., to detect or select for cells that have taken up the polynucleotide.

In certain embodiments, the viral vectors provided herein comprise one or more promoters. In certain embodiments, the promoter is a constitutive promoter. In alternate embodiments, the promoter is an inducible promoter. The native IDS gene, like most housekeeping genes, primarily uses a GC-rich promoter. In a preferred embodiment, strong constitutive promoters that provide for sustained expression of hIDS are used. Such promoters include "CAG" synthetic promoters that contain: "C" - the cytomegalovirus (CMV) early enhancer element; "A" - the promoter as well as the first exon and intron of the chicken beta-actin gene; and "G" - the splice acceptor of the rabbit beta-globin gene (see, Miyazaki et al., 1989, Gene 79: 269-277; and Niwa et al., Gene 108: 193-199).

In certain embodiments, the promoter is a CB7 promoter (*see* Dinculescu et al., 2005, Hum Gene Ther 16: 649-663, incorporated by reference herein in its entirety). In some embodiments, the CB7 promoter includes other expression control elements that enhance expression of the transgene driven by the vector. In certain embodiments, the other expression control elements include chicken β-actin intron and/or rabbit β-globin polA signal. In certain embodiments, the promoter comprises a TATA box. In certain embodiments, the promoter comprises one or more elements. In certain embodiments, the one or more promoter elements may be inverted or moved relative to one another. In certain embodiments, the elements of the promoter are positioned to function cooperatively. In certain embodiments, the elements of the promoter are positioned to function independently. In certain embodiments, the viral vectors provided herein comprise one or more promoters selected from the group consisting of the human CMV immediate early gene promoter, the SV40 early promoter, the Rous sarcoma virus (RS) long terminal repeat, and rat insulin promoter. In certain embodiments, the vectors provided herein comprise one or more long terminal repeat (LTR) promoters selected from the group consisting of AAV, MLV, MMTV, SV40, RSV, HIV-1, and HIV-2 LTRs. In certain embodiments, the vectors provided herein comprise one or more tissue specific promoters (e.g., a neuronal cell-specific promoter).

In certain embodiments, the viral vectors provided herein comprise one or more regulatory elements other than a promoter. In certain embodiments, the viral vectors provided herein comprise an enhancer. In certain embodiments, the viral vectors provided herein comprise a repressor. In certain embodiments, the viral vectors provided herein comprise an intron or a chimeric intron. In certain embodiments, the viral vectors provided herein comprise a polyadenylation sequence.

### 5.2.4. Signal Peptides

In certain embodiments, the vectors provided herein comprise components that modulate protein delivery. In certain embodiments, the viral vectors provided herein comprise one or more signal peptides. In certain embodiments, the signal peptides allow for the transgene product (*e.g.*, IDS) to achieve the proper packaging (*e.g.* glycosylation) in the cell. In certain embodiments, the signal peptides allow for the transgene product (*e.g.*, IDS) to achieve the proper localization in the cell. In certain embodiments, the signal peptides allow for the transgene product (*e.g.*, IDS) to achieve secretion from the cell. Examples of signal peptides to be used in connection with the vectors and transgenes provided herein may be found in Table 4. Signal peptides may also be referred to herein as leader sequences or leader peptides.

**Table 4. Signal peptides for use with the vectors provided herein.**

| **SEQ ID NO.** | **Signal Peptide** | **Sequence** |
|---|---|---|
| 2 | Oligodendrocyte-myelin glycoprotein (hOMG) signal peptide | MEYQILKMSLCLFILLFLTPGILC |
| 3 | Cellular repressor of E1A-stimulated genes 2 (hCREG2) signal peptide | MSVRRGRRPARPGTRLSWLLCCSALLSP AAG |
| 4 | V-set and transmembrane domain containing 2B (hVSTM2B) signal peptide | MEQRNRLGALGYLPPLLLHALLLFVADA |
| 5 | Protocadherin alpha-1 (hPCADHA1) signal peptide | MVFSRRGGLGARDLLLWLLLLAAWEVG SG |
| 6 | FAM19A1 (TAFA1) signal peptide | MAMVSAMSWVLYLWISACA |
| 7 | VEGF-A signal peptide | MNFLLSWVHW SLALLLYLHH AKWSQA |
| 8 | Fibulin-1 signal peptide | MERAAPSRRV PLPLLLLGGL ALLAAGVDA |
| 9 | Vitronectin signal peptide | MAPLRPLLIL ALLAWVALA |
| 10 | Complement Factor H signal peptide | MRLLAKIICLMLWAICVA |
| 11 | Opticin signal peptide | MRLLAFLSLL ALVLQETGT |
| 12 | Albumin signal peptide | MKWVTFISLLFLFSSAYS |
| 13 | Chymotrypsinogen signal peptide | MAFLWLLSCWALLGTTFG |
| 14 | Interleukin-2 signal peptide | MYRMQLLSCIALILALVTNS |
| 15 | Trypsinogen-2 signal peptide | MNLLLILTFVAAAVA |

### 5.2.5. Untranslated regions

In certain embodiments, the viral vectors provided herein comprise one or more untranslated regions (UTRs), *e.g.,* 3' and/or 5' UTRs. In certain embodiments, the UTRs are optimized for the desired level of protein expression. In certain embodiments, the UTRs are optimized for the mRNA half life of the transgene. In certain embodiments, the UTRs are optimized for the stability of the mRNA of the transgene. In certain embodiments, the UTRs are optimized for the secondary structure of the mRNA of the transgene.

### 5.2.6. Inverted terminal repeats

In certain embodiments, the viral vectors provided herein comprise one or more inverted terminal repeat (ITR) sequences. ITR sequences may be used for packaging the recombinant gene expression cassette into the virion of the viral vector. In certain embodiments, the ITR is from an AAV, *e.g.*, AAV9 (*see*, *e.g.,* Yan et al., 2005, J. Virol., 79(1):364-379; United States Patent No. 7,282,199 B2, United States Patent No. 7,790,449 B2, United States Patent No. 8,318,480 B2, United States Patent No. 8,962,332 B2 and International Patent Application No. PCT/EP2014/076466, each of which is incorporated herein by reference in its entirety).

### 5.2.7. Transgenes

In certain embodiments, the vectors provided herein encode an IDS transgene. In specific embodiments, the IDS is controlled by appropriate expression control elements for expression in neuronal cells: In certain embodiments, the IDS (*e.g.*, hIDS) transgene comprises the amino acid sequence of SEQ ID NO: 1. In certain embodiments, the IDS (*e.g.*, hIDS) transgene comprises an amino acid sequence that is at least 85%, 86%, 87%, 88%, 89%, 90%, 91%, 92%, 93%, 94%, 95%, 96%, 97%, 98% or 99% identical to the sequence set forth in SEQ ID NO: 1.

The HuGlyIDS encoded by the transgene can include, but is not limited to human IDS (hIDS) having the amino acid sequence of SEQ ID NO. 1 (as shown in FIG. 1), and derivatives of hIDS having amino acid substitutions, deletions, or additions, *e.g*., including but not limited to amino acid substitutions selected from corresponding non-conserved residues in orthologs of IDS shown in FIG. 2, with the *proviso* with the *proviso* that such mutations do not include replacement of the cysteine residue at position 84 (C84) which is required for enzyme activity (Millat et al., 1997, Biochem J 326: 243-247); or a mutation that has been identified in severe, severe-intermediate, intermediate, or attenuated MPS II phenotypes *e.g*., as shown in FIG. 3, or as reported by Sukegawa-Hayasaka et al., 2006, J Inhert Metab Dis 29: 755-761 (reporting "attenuated" mutants R48P, A85T, W337R, and the truncated mutant Q531X; and "severe" mutants P86L, S333L, S349I, R468Q, R468L); Millat et al., 1998, BBA 1406: 214-218 (reporting "attenuated" mutants P480L and P480Q; and "severe" mutant P86L); and Bonucelli et al., 2001, BBA 1537:233-238, each of which is incorporated by reference herein in its entirety.

For example, amino acid substitutions at a particular position of hIDS can be selected from among corresponding non-conserved amino acid residues found at that position in the IDS orthologs aligned in FIG. 2, with the *proviso* that such substitutions do not include any of the deleterious mutations shown in FIG. 3 or as reported by Sukegawa-Hayasaka et al., 2006, *supra*; Millat et al., 1998, *supra*; or Bonucelli et al., 2001, *supra*, each of which is incorporated by reference herein in its entirety. The resulting transgene product can be tested using conventional assays *in vitro,* in cell culture or test animals to ensure that the mutation does not disrupt IDS function. Preferred amino acid substitutions, deletions or additions selected should be those that maintain or increase enzyme activity, stability or half-life of IDS, as tested by conventional assays *in vitro,* in cell culture or animal models for MPS II. For example, the enzyme activity of the transgene product can be assessed using a conventional enzyme assay with, for example, 4-Methylumbelliferyl α-L-idopyranosiduronic acid 2-sulfate or 4-methylumbelliferyl sulfate as the substrate (*see*, *e.g.,* Lee et al., 2015, Clin. Biochem. 48(18):1350-1353, Dean et al., 2006, Clin. Chem. 52(4):643-649 for exemplary IDS enzyme assays that can be used, each of which is incorporated by reference herein in its entirety). The ability of the transgene product to correct MPS II phenotype can be assessed in cell culture; *e.g.*, by transducing MPS II cells in culture with a viral vector or other DNA expression construct encoding hIDS or a derivative; by adding the transgene product or a derivative to MPS II cells in culture; or by co-culturing MPS II cells with human neuronal/glial host cells engineered to express and secrete rhIDS or a derivative, and determining correction of the defect in the MPS II cultured cells, *e.g.*, by detecting IDS enzyme activity and/or reduction in GAG storage in the MPS II cells in culture (*see*, *e.g.*, Stroncek et al., 1999, Transfusion 39(4):343-350, which is incorporated by reference herein in its entirety).

### 5.2.8. Constructs

In certain embodiments, the viral vectors provided herein comprise the following elements in the following order: a) a first ITR sequence, b) a first linker sequence, c) a promoter sequence, d) a second linker sequence, e) an intron sequence, f) a third linker sequence, g) a sequence encoding the transgene (*e.g*., IDS), h) a fourth linker sequence, i) a poly A sequence, j) a fifth linker sequence, and k) a second ITR sequence.

In certain embodiments, the viral vectors provided herein comprise the following elements in the following order: a) a promoter sequence, and b) a sequence encoding the transgene (*e.g*., IDS). In certain embodiments, the viral vectors provided herein comprise the following elements in the following order: a) a promoter sequence, and b) a sequence encoding the transgene (*e.g*., IDS), wherein the transgene comprises a signal peptide.

In certain embodiments, the viral vectors provided herein comprise the following elements in the following order: a) a first ITR sequence, b) a first linker sequence, c) a promoter sequence, d) a second linker sequence, e) an intron sequence, f) a third linker sequence, g) a first UTR sequence, h) a sequence encoding the transgene (*e.g*., IDS), i) a second UTR sequence, j) a fourth linker sequence, k) a poly A sequence, 1) a fifth linker sequence, and m) a second ITR sequence.

In certain embodiments, the viral vectors provided herein comprise the following elements in the following order: a) a first ITR sequence, b) a first linker sequence, c) a promoter sequence, d) a second linker sequence, e) an intron sequence, f) a third linker sequence, g) a first UTR sequence, h) a sequence encoding the transgene (*e.g*., IDS), i) a second UTR sequence, j) a fourth linker sequence, k) a poly A sequence, 1) a fifth linker sequence, and m) a second ITR sequence, wherein the transgene comprises a signal peptide, and wherein the transgene encodes hIDS.

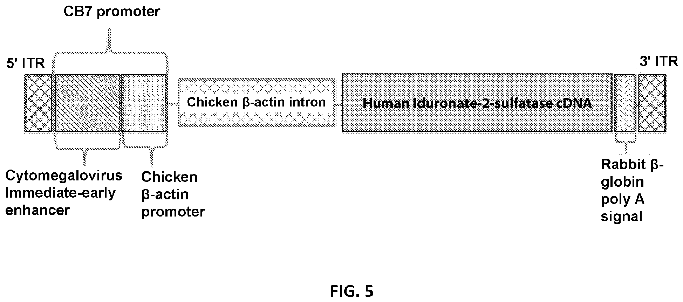

In a specific embodiment, the viral vector described herein comprises the elements and in the order as illustrated in FIG. 5.

### 5.2.9. Manufacture and testing of vectors

The viral vectors provided herein may be manufactured using host cells. The viral vectors provided herein may be manufactured using mammalian host cells, for example, A549 , WEHI, 10T1/2, BHK, MDCK, COS1, COS7, BSC 1, BSC 40, BMT 10, VERO, W138, HeLa, 293, Saos, C2C12, L, HT1080, HepG2, primary fibroblast, hepatocyte, and myoblast cells. The viral vectors provided herein may be manufactured using host cells from human, monkey, mouse, rat, rabbit, or hamster.

The host cells are stably transformed with the sequences encoding the transgene and associated elements (i.e., the vector genome), and the means of producing viruses in the host cells, for example, the replication and capsid genes (*e.g.*, the rep and cap genes of AAV). For a method of producing recombinant AAV vectors with AAV8 capsids, *see* Section IV of the Detailed Description of U.S. Patent No. 7,282,199 B2, which is incorporated herein by reference in its entirety. Genome copy titers of said vectors may be determined, for example, by TAQMAN^{®} analysis. Virions may be recovered, for example, by CsCl₂ sedimentation.

In vitro assays, e.g., cell culture assays, can be used to measure transgene expression from a vector described herein, thus indicating, *e.g*., potency of the vector. For example, the HT-22, SK-N-MC, HCN-1A, HCN-2, NT2, SH-SY5y, hNSC11, or ReNcell VM cell lines, or other cell lines that are derived from neuronal or glial cells or progenitors of neuronal or glial cells can be used to assess transgene expression. Once expressed, characteristics of the expressed product (i.e., HuGlyIDS) can be determined, including determination of the glycosylation and tyrosine sulfation patterns associated with the HuGlyIDS.

### 5.2.10. Compositions

Compositions are described comprising a vector encoding a transgene described herein and a suitable carrier. A suitable carrier (*e.g.*, for administration to the CSF, and, for example, to neuronal cells) would be readily selected by one of skill in the art.

### 5.3 GENE THERAPY

Methods are described for the administration of a therapeutically effective amount of a transgene construct to human subjects having MPS II. More particularly, methods for administration of a therapeutically effective amount of a transgene construct to patients having MPS II, in particular, for administration to the CSF are described. In particular embodiments, such methods for administration to the CSF of a therapeutically effective amount of a transgene construct can be used to treat to patients having Hunter's syndrome.

### 5.3.1. Target Patient Populations

In certain embodiments, therapeutically effective doses of the recombinant vector are administered to patients diagnosed with MPS II. In specific embodiments, the patients have been diagnosed with mild MPS II. In specific embodiments, the patients have been diagnosed with severe MPS II. In specific embodiments, the patients have been diagnosed with Hunter's syndrome. In specific embodiments, the patients have been diagnosed with neuronopathic MPS II.

In certain embodiments, therapeutically effective doses of the recombinant vector are administered to patients diagnosed with MPS II who have been identified as responsive to treatment with IDS, *e.g.*, hIDS.

In certain embodiments, therapeutically effective doses of the recombinant vector are administered to pediatric patients. In certain embodiments, therapeutically effective doses of the recombinant vector are administered to patients that are less than three years old. In certain embodiments, therapeutically effective doses of the recombinant vector are administered to patients that are aged 2 to 4 years old. In certain embodiments, therapeutically effective doses of the recombinant vector are administered to patients that are 4 months old or older and less than 5 years old. In a specific embodiment, therapeutically effective doses of the recombinant vector are administered to patients that have severe MPS II and are 4 months old or older and less than 5 years old. In certain embodiments, therapeutically effective doses of the recombinant vector are administered to patients that are 5 years old or older and less than 18 years old. In a specific embodiment, therapeutically effective doses of the recombinant vector are administered to patients that have neuronopathic MPS II and are 5 years old or older and less than 18 years old. In certain embodiments, therapeutically effective doses of the recombinant vector are administered to patients that are 18 months old or older and 8 years old or younger. In a specific embodiment, therapeutically effective doses of the recombinant vector are administered to patients that are pediatric male patients and are 18 months old or older and 8 years old or younger. In certain embodiments, therapeutically effective doses of the recombinant vector are administered to patients that are aged 3 to 8 years old. In certain embodiments, therapeutically effective doses of the recombinant vector are administered to patients that are aged 8 to 16 years old. In certain embodiments, therapeutically effective doses of the recombinant vector are administered to patients that are aged 5 to 18 years old. In certain embodiments, therapeutically effective doses of the recombinant vector are administered to patients that are 10 years old or younger. In a specific embodiment, therapeutically effective doses of the recombinant vector are administered to patients that have severe MPS II and are 10 years old or younger. In certain embodiments, therapeutically effective doses of the recombinant vector are administered to patients that are 18 years old or younger. In certain embodiments, therapeutically effective doses of the recombinant vector are administered to patients that are more than 5 years old. In certain embodiments, therapeutically effective doses of the recombinant vector are administered to patients that are more than 10 years old.

In certain embodiments, therapeutically effective doses of the recombinant vector are administered to patients that are 4, 5, 6, 7, 8, 9, 10, or 11 months old. In certain embodiments, therapeutically effective doses of the recombinant vector are administered to patients that are about 4, 5, 6, 7, 8, 9, 10, or 11 months old. In certain embodiments, therapeutically effective doses of the recombinant vector are administered to patients that are 4-5, 5-6, 6-7, 7-8, 8-9, 9-10, 10-11, or 11-12 months old. In certain embodiments, therapeutically effective doses of the recombinant vector are administered to patients that are about 4-5, 5-6, 6-7, 7-8, 8-9, 9-10, 10-11, or 11-12 months old. In certain embodiments, therapeutically effective doses of the recombinant vector are administered to patients that are 1, 2, 3, 4, or 5 years old. In certain embodiments, therapeutically effective doses of the recombinant vector are administered to patients that are about 1, 2, 3, 4, or 5 years old. In certain embodiments, therapeutically effective doses of the recombinant vector are administered to patients that are 1-2, 2-3, 3-4, 4-5, or 5-6 years old. In certain embodiments, therapeutically effective doses of the recombinant vector are administered to patients that are about 1-2, 2-3, 3-4, 4-5, or 5-6 years old. In certain embodiments, therapeutically effective doses of the recombinant vector are administered to patients that are 5-6, 6-7, 7-8, 8-9, 9-10, 10-11, 11-12, 12-13, 13-14, 14-15, 15-16, 16-17, 17-18, or 18-19 years old. In certain embodiments, therapeutically effective doses of the recombinant vector are administered to patients that are about 5-6, 6-7, 7-8, 8-9, 9-10, 10-11, 11-12, 12-13, 13-14, 14-15, 15-16, 16-17, 17-18, or 18-19 years old.

In certain embodiments, therapeutically effective doses of the recombinant vector are administered to adolescent patients. In certain embodiments, therapeutically effective doses of the recombinant vector are administered to adult patients. In some embodiments, therapeutically effective doses of the recombinant vector are administered to male patients. In other embodiments, therapeutically effective doses of the recombinant vector are administered to female patients.

In certain embodiments, therapeutically effective doses of the recombinant vector are administered to patients diagnosed with MPS II who have been identified as responsive to treatment with IDS, *e.g.*, hIDS, injected into the CSF prior to treatment with gene therapy.

### 5.3.2. Dosage and Mode of Administration

In certain embodiments, therapeutically effective doses of the recombinant vector are administered to the CSF via intrathecal administration (*i.e.*, injection into the subarachnoid space so that the recombinant vectors distribute through the CSF and transduce cells in the CNS). This can be accomplished in a number of ways - *e.g.*, by intracranial (cisternal or ventricular) injection , or injection into the lumbar cistern. In certain embodiments, intrathecal administration is performed via intracisternal (IC) injection (e.g., into the cisterna magna). In specific embodiments, intracisternal injection is performed by CT-guided suboccipital puncture. In specific embodiments, intrathecal injection is performed by lumbar puncture. In specific embodiments, injection into the subarachnoid space is performed by C1-2 puncture if feasible for the patient. Alternatively, intracerebroventricular (ICV) administration (a more invasive technique used for the introduction of antiinfective or anticancer drugs that do not penetrate the blood-brain barrier), for example, image-assisted ICV injection, can be used to instill the recombinant vectors directly into the ventricles of the brain. In a specific embodiment, the recombinant vector is administered via a single image-assisted ICV injection. In a further specific embodiment, the recombinant vector is administered via a single image-assisted ICV injection with immediate removal of the administration catheter. In certain embodiments, therapeutically effective doses of the recombinant vector are administered to the CNS via intranasal administration. In certain embodiments, therapeutically effective doses of the recombinant vector are administered to the CNS via intraparenchymal injection. In certain embodiments, intraparenchymal injection is targeted to the striatum. In certain embodiments, intraparenchymal injection is targeted to the white matter. In certain embodiments, therapeutically effective doses of the recombinant vector are administered to the CSF by any means known to the art, for example, by any means disclosed in Hocquemiller et al., 2016, Human Gene Therapy 27(7):478-496, which is hereby incorporated by reference in its entirety.

In preferred embodiments, for intrathecal administration (including IC and ICV administration), therapeutically effective doses of the recombinant vector are administered to the CSF in an injection volume that does not exceed 10% of the total CSF volume, which total CSF volume is about 50 mL in infants and about 150 mL in adults. A carrier suitable for intrathecal injection, such as Elliotts B Solution, should be used as a vehicle for the recombinant vectors. Elliots B Solution (generic name: sodium chloride, sodium bicarbonate, anhydrous dextrose, magnesium sulfate, potassium chloride, calcium chloride and sodium phosphate) is a sterile, nonpyrogenic, isotonic solution containing no bacteriostatic preservatives and is used as a diluent for intrathecal administration of chemotherapeutics.

In one embodiment, a non-replicating recombinant AAV9 vector expressing human iduronate-2-sulfatase (IDS) is used for treatment. In certain embodiments, the IDS expression cassette is flanked by inverted terminal repeats (ITRs) and expression is driven by a hybrid of the cytomegalovirus (CMV) enhancer and the chicken beta actin promoter (CB7). In certain embodiments, the transgene includes the chicken beta actin intron and a rabbit beta-globin polyadenylation (polyA) signal.

In certain embodiments, the recombinant nucleotide expression vector is administered at a dose that is dependent on the human subject's brain mass. In preferred embodiments, the brain mass is determined by brain magnetic resonance imaging (MRI) of the human subject's brain. In certain embodiments, the human subject's brain mass is converted from the human subject's brain volume by multiplying the human subject's brain volume in cm³ by a factor of 1.046 g/cm3, wherein the human subject's brain volume is obtained from the human subject's brain MRI.

In certain embodiments, the rAAV9.hIDS is administered IC (by suboccipital injection) as a single flat dose ranging from 1.4 × 10¹³ GC (1.1 × 10¹⁰ GC/g brain mass) to 7.0 × 10¹³ GC (5.6 × 10¹⁰ GC/g brain mass) in a volume of about 5 to 20 ml. In the event the patient has neutralizing antibodies to AAV, doses at the high range may be used.

In certain embodiments, the recombinant vector described herein may be administered intrathecally as a single flat dose ranging from about 1.3 × 10¹⁰ GC/g brain mass to about 6.5 × 10¹⁰ GC/g brain mass (for example, when the human patient is 4 months old or older and less than 5 years old). In a specific embodiment, the recombinant vector described herein may be administered intrathecally as a single flat dose at about 1.3 × 10¹⁰ GC/g brain mass (for example, when the human patient is 4 months old or older and less than 5 years old). In another specific embodiment, the recombinant vector described herein may be administered intrathecally as a single flat dose at about 6.5 × 10¹⁰ GC/g brain mass (for example, when the human patient is 4 months old or older and less than 5 years old). In another specific embodiment, the recombinant vector described herein may be administered intrathecally as a single flat dose at Dose 1 or Dose 2 as listed in and according to Table 5 below (for example, when the human patient is 4 months old or older and less than 5 years old).

In certain embodiments, the recombinant vector described herein may be administered intrathecally as a single flat dose ranging from about 1.3 × 10¹⁰ GC/g brain mass to about 2.0 × 10¹¹ GC/g brain mass (for example, when the human patient is 4 months old or older and less than 5 years old). In a specific embodiment, the recombinant vector described herein may be administered intrathecally as a single flat dose at about 2.0 × 10¹¹ GC/g brain mass (for example, when the human patient is 4 months old or older and less than 5 years old). In another specific embodiment, the recombinant vector described herein may be administered intrathecally as a single flat dose at Dose 3 as listed in and according to Table 6 below (for example, when the human patient is 4 months old or older and less than 5 years old).

In certain embodiments, the recombinant vector described herein may be administered by IC administration as a single flat dose ranging from about 1.3 × 10¹⁰ GC/g brain mass to about 6.5 × 10¹⁰ GC/g brain mass (for example, when the human patient is 4 months old or older and less than 5 years old). In a specific embodiment, the recombinant vector described herein may be administered by IC administration as a single flat dose at about 1.3 × 10¹⁰ GC/g brain mass (for example, when the human patient is 4 months old or older and less than 5 years old). In another specific embodiment, the recombinant vector described herein may be administered by IC administration as a single flat dose at about 6.5 × 10¹⁰ GC/g brain mass (for example, when the human patient is 4 months old or older and less than 5 years old). In another specific embodiment, the recombinant vector described herein may be administered by IC administration as a single flat dose at Dose 1 or Dose 2 as listed in and according to Table 5 below (for example, when the human patient is 4 months old or older and less than 5 years old).

In certain embodiments, the recombinant vector described herein may be administered by IC administration as a single flat dose ranging from about 1.3 × 10¹⁰ GC/g brain mass to about 2.0 × 10¹¹ GC/g brain mass (for example, when the human patient is 4 months old or older and less than 5 years old). In a specific embodiment, the recombinant vector described herein may be administered by IC administration as a single flat dose at about 2.0 × 10¹¹ GC/g brain mass (for example, when the human patient is 4 months old or older and less than 5 years old). In another specific embodiment, the recombinant vector described herein may be administered by IC administration as a single flat dose at Dose 3 as listed in and according to Table 6 below (for example, when the human patient is 4 months old or older and less than 5 years old).

In certain embodiments, the recombinant vector described herein may be administered by ICV administration as a single flat dose ranging from about 1.3 × 10¹⁰ GC/g brain mass to about 6.5 × 10¹⁰ GC/g brain mass (for example, when the human patient is 4 months old or older and less than 5 years old). In a specific embodiment, the recombinant vector described herein may be administered by ICV administration as a single flat dose at about 1.3 × 10¹⁰ GC/g brain mass (for example, when the human patient is 4 months old or older and less than 5 years old). In another specific embodiment, the recombinant vector described herein may be administered by ICV administration as a single flat dose at about 6.5 × 10¹⁰ GC/g brain mass (for example, when the human patient is 4 months old or older and less than 5 years old). In another specific embodiment, the recombinant vector described herein may be administered by ICV administration as a single flat dose at Dose 1 or Dose 2 as listed in and according to Table 5 below (for example, when the human patient is 4 months old or older and less than 5 years old).

In certain embodiments, the recombinant vector described herein may be administered by ICV administration as a single flat dose ranging from about 1.3 × 10¹⁰ GC/g brain mass to about 2.0 × 10¹¹ GC/g brain mass (for example, when the human patient is 4 months old or older and less than 5 years old). In a specific embodiment, the recombinant vector described herein may be administered by ICV administration as a single flat dose at about 2.0 × 10¹¹ GC/g brain mass (for example, when the human patient is 4 months old or older and less than 5 years old). In another specific embodiment, the recombinant vector described herein may be administered by ICV administration as a single flat dose at Dose 3 as listed in and according to Table 6 below (for example, when the human patient is 4 months old or older and less than 5 years old).

**Table 5. Total Dose Administered by Brain Mass (Dose 1 or Dose 2)**

| **Brain Mass (in g)** | | **Target** | **Dose Levels** | |
|---|---|---|---|---|
| **Min** | **Max** | | **Dose 1 Total GC** (1.3 × 10¹⁰ GC/g brain mass) | **Dose 2 Total GC** (6.5 × 10¹⁰ GC/g brain mass) |
| - | 700 | 650 | 8.5 × 10¹² | 4.2 × 10¹³ |
| 701 | 800 | 750 | 9.8 × 10¹² | 4.9 × 10¹³ |
| 801 | 900 | 850 | 1.1 × 10¹³ | 5.5 × 10¹³ |
| 901 | 1050 | 975 | 1.3 × 10¹³ | 6.3 × 10¹³ |
| 1051 | 1200 | 1125 | 1.5 × 10¹³ | 7.3 × 10¹³ |
| 1201 | - | 1300 | 1.7 × 10¹³ | 8.5 × 10¹³ |

**Table 6. Total Dose Administered by Brain Mass (Dose 3)**

| **Brain Mass (in g)** | | **Target** | **Dose 3 Total GC** (2.0 × 10¹¹ GC/g brain mass) |
|---|---|---|---|
| **Min** | **Max** | | |
| - | 474 | 450 | 9.0 x 10¹³ |
| 475 | 524 | 500 | 1.0 × 10¹⁴ |
| 525 | 574 | 550 | 1.1 × 10¹⁴ |
| 575 | 624 | 600 | 1.2 × 10¹⁴ |
| 625 | 674 | 650 | 1.3 × 10¹⁴ |
| 675 | 724 | 700 | 1.4 × 10¹⁴ |
| 725 | 774 | 750 | 1.5 × 10¹⁴ |
| 775 | 824 | 800 | 1.6 × 10¹⁴ |
| 825 | 874 | 850 | 1.7 × 10¹⁴ |
| 875 | 924 | 900 | 1.8 × 10¹⁴ |
| 925 | 974 | 950 | 1.9 × 10¹⁴ |
| 975 | 1024 | 1000 | 2.0 × 10¹⁴ |
| 1025 | 1074 | 1050 | 2.1 × 10¹⁴ |
| 1075 | 1124 | 1100 | 2.2 × 10¹⁴ |
| 1125 | 1174 | 1150 | 2.3 × 10¹⁴ |
| 1175 | 1224 | 1200 | 2.4 × 10¹⁴ |
| 1225 | 1274 | 1250 | 2.5 × 10¹⁴ |
| 1275 | >1300 | 1300 | 2.6 × 10¹⁴ |

In a specific embodiment, the recombinant vector described herein may be administered intrathecally as a single flat dose at about 6.5 × 10¹⁰ GC/g brain mass (for example, when the human patient is 5 years old or older and less than 18 years old). In another specific embodiment, the recombinant vector described herein may be administered intrathecally as a single flat dose as listed in and according to Table 7 below (for example, when the human patient is 5 years old or older and less than 18 years old).

In a specific embodiment, the recombinant vector described herein may be administered by IC administration as a single flat dose at about 6.5 × 10¹⁰ GC/g brain mass (for example, when the human patient is 5 years old or older and less than 18 years old). In another specific embodiment, the recombinant vector described herein may be administered by IC administration as a single flat dose as listed in and according to Table 7 below (for example, when the human patient is 5 years old or older and less than 18 years old).

In a specific embodiment, the recombinant vector described herein may be administered by ICV administration as a single flat dose at about 6.5 × 10¹⁰ GC/g brain mass (for example, when the human patient is 5 years old or older and less than 18 years old). In another specific embodiment, the recombinant vector described herein may be administered by ICV administration as a single flat dose as listed in and according to Table 7 below (for example, when the human patient is 5 years old or older and less than 18 years old).

**Table 7. Total Dose Administered by Brain Mass**

| **Brain Mass (in g)** | | **Target Brain Mass (in g)** | **Dose: Total GC (6.5** × **10¹⁰ GC/g brain mass)** |
|---|---|---|---|
| **Min** | **Max** | | |
| 801 | 900 | 850 | 5.5 × 10¹³ |
| 901 | 1050 | 975 | 6.3 × 10¹³ |
| 1051 | 1200 | 1125 | 7.3 × 10¹³ |
| 1201 | - | 1300 | 8.5 × 10¹³ |

### 5.4 COMBINATION THERAPIES

Combinations of administration of the HuGlyIDS to the CSF accompanied by administration of other available treatments are encompassed by the methods of the invention. The additional treatments may be administered before, concurrently or subsequent to the gene therapy treatment. Available treatments for MPS II that could be combined with the gene therapy of the invention include but are not limited to enzyme replacement therapy (ERT) using idursulfase administered systemically or to the CSF; and/or HSCT therapy. In another embodiment, ERT can be administered using the rHuGlyIDS glycoprotein produced in human neuronal and glial cell lines by recombinant DNA technology. Human neuronal and glial cell lines that can be used for such recombinant glycoprotein production include but are not limited to HT-22, SK-N-MC, HCN-1A, HCN-2, NT2, SH-SY5y, hNSC11, or ReNcell VM to name a few. To ensure complete glycosylation, especially sialylation, and tyrosine-sulfation, the cell line used for production can be enhanced by engineering the host cells to co-express α-2,6-sialyltransferase (or both α-2,3- and α-2,6-sialyltransferases) and/or TPST-1 and TPST-2 enzymes responsible for tyrosine-O-sulfation.

### 5.5 BIOMARKERS/SAMPLING/MONITORING EFFICACY

Efficacy may be monitored by measuring cognitive function (e.g., prevention or decrease in neurocognitive decline); reductions in biomarkers of disease (such as GAG, including heparan sulfate and dermatan sulfate) in CSF and or serum; and/or increase in IDS enzyme activity in CSF and/or serum. Signs of inflammation and other safety events may also be monitored.

### 5.5.1. Disease Markers

In certain embodiments, efficacy of treatment with the recombinant nucleotide expression vector is monitored by measuring the level of a disease biomarker in the patient. In certain embodiments, the level of the disease biomarker is measured in the CSF of the patient. In certain embodiments, the level of the disease biomarker is measured in the serum of the patient. In certain embodiments, the level of the disease biomarker is measured in the plasma of the patient. In certain embodiments, the level of the disease biomarker is measured in the urine of the patient. In certain embodiments, the disease biomarker is GAG. In preferred embodiments, the disease biomarker is heparan sulfate. In certain embodiments, the disease biomarker is dermatan sulfate. In certain embodiments, the disease biomarker is IDS enzyme activity. In certain embodiments, the disease biomarker is inflammation. In certain embodiments, the disease biomarker is a safety event.

In certain embodiments, efficacy of treatment with the recombinant nucleotide expression vector is monitored by measuring one or more of the following biomarkers in a sample from the patient: (a) level of GAGs in CSF; (b) level of I2S in CSF; (c) level of GAGs in plasma; (d) level of I2S in plasma; (e) level of leukocyte I2S enzyme activity; (f) level of GAGs in urine, (g) level of heparan sulfate in CSF, and (h) level of dermatan sulfate in CSF.

In some embodiments, heparan sulfate (HS) glycosaminoglycan (CAGs) in CSF is measured using a bioanalytical LC/MS. In some embodiments, heparan sulfate non-reducing ends and total heparan sulfate in CNS is measured using a bioanalytical LC/MS. In some embodiments, heparan sulfate non-reducing ends and total heparan sulfate CAGs in plasma is measured using a bioanalytical LC/MS. In some embodiments, HS CAGs in urine is measured using a bioanalytical LC/MS. In some embodiments, urine total CAGs concentration is measured using a colorimetric assay. In some embodiments, more details about the assays that can be used is provided in Section 6 of the disclosure.

### 5.5.2. Tests for Neurocognitive function

In certain embodiments, efficacy of treatment with the recombinant vector is monitored by measuring the level of cognitive function in the patient. Cognitive function may be measured by any method known to one of skill in the art. In certain embodiments, cognitive function is measured via a validated instrument for measuring intelligence quotient (IQ). In specific embodiments, IQ is measured by Wechsler Abbreviated Scale of Intelligence, Second Edition (WASI-II). In certain embodiments, cognitive function is measured via a validated instrument for measuring memory. In specific embodiments, memory is measured by Hopkins Verbal Learning Test (HVLT). In certain embodiments, cognitive function is measured via a validated instrument for measuring attention. In specific embodiments, attention is measured by Test Of Variables of Attention (TOVA). In certain embodiments, cognitive function is measured via a validated instrument for measuring one or more of IQ, memory, and attention.

### 5.5.3. Physical changes

In certain embodiments, efficacy of treatment with the recombinant vector is monitored by measuring physical characteristics associated with lysosomal storage deficiency in the patient. In certain embodiments, the physical characteristics are storage lesions. In certain embodiments, the physical characteristic is short stature. In certain embodiments, the physical characteristic is coarsened facial features. In certain embodiments, the physical characteristic is obstructive sleep apnea. In certain embodiments, the physical characteristic is hearing impairment. In certain embodiments, the physical characteristic is vision impairment. In specific embodiments, the visual impairment is due to corneal clouding. In certain embodiments, the physical characteristic is hydrocephalus. In certain embodiments, the physical characteristic is spinal cord compression. In certain embodiments, the physical characteristic is hepatosplenomegaly. In certain embodiments, the physical characteristics are bone and joint deformities. In certain embodiments, the physical characteristic is cardiac valve disease. In certain embodiments, the physical characteristics are recurrent upper respiratory infections. In certain embodiments, the physical characteristic is carpal tunnel syndrome. In certain embodiments, the physical characteristic is macroglossia (enlarged tongue). In certain embodiments, the physical characteristic is enlarged vocal cords and/or change in voice. Such physical characteristics may be measured by any method known to one of skill in the art.

**TABLE OF SEQUENCES**

| **SEQ ID NO:** | **Description** | **Sequence** |
|---|---|---|
| 1 | Human IDS amino acid sequence | MPPPRTGRGL LWLGLVLSSV CVALGSETQA NSTTDALNVL LIIVDDLRPS LGCYGDKLVR SPNIDQLASH SLLFQNAFAQ QAVCAPSRVS FLTGRRPDTT RLYDFNSYWR VHAGNFSTIP QYFKENGYVT MSVGKVFHPG ISSNHTDDSP YSWSFPPYHP SSEKYENTKT CRGPDGELHA NLLCPVDVLD VPEGTLPDKQ STEQAIQLLE KMKTSASPFF LAVGYHKPHI PFRYPKEFQK LYPLENITLA PDPEVPDGLP PVAYNPWMDI RQREDVQALN ISVPYGPIPV DFQRKIRQSY FASVSYLDTQ VGRLLSALDD LQLANSTIIA FTSDHGWALG EHGEWAKYSN FDVATHVPLI FYVPGRTASL PEAGEKLFPY LDPFDSASQL MEPGRQSMDL VELVSLFPTL AGLAGLQVPP RCPVPSFHVE LCREGKNLLK HFRFRDLEED PYLPGNPREL IAYSQYPRPS DIPQWNSDKP SLKDIKIMGY SIRTIDYRYT VWVGFNPDEF LANFSDIHAG ELYFVDSDPL QDHNMYNDSQ GGDLFQLLMP |
| 2 | Oligodendrocyte-myelin glycoprotein (hOMG) signal peptide | MEYQILKMSL CLFILLFLTP GILC |
| 3 | Cellular repressor of E1A-stimulated genes 2 (hCREG2) signal peptide | MSVRRGRRPA RPGTRLSWLL CCSALLSPAA G |
| 4 | V-set and transmembrane domain containing 2B (hVSTM2B) signal peptide | MEQRNRLGAL GYLPPLLLHA LLLFVADA |
| 5 | Protocadherin alpha-1 (hPCADHA1) signal peptide | MVFSRRGGLG ARDLLLWLLL LAAWEVGSG |
| 6 | FAM19A1 (TAFA1) signal peptide | MAMVSAMSWV LYLWISACA |
| 7 | VEGF-A signal peptide | MNFLLSWVHW SLALLLYLHH AKWSQA |
| 8 | Fibulin-1 signal peptide | MERAAPSRRV PLPLLLLGGL ALLAAGVDA |
| 9 | Vitronectin signal peptide | MAPLRPLLIL ALLAWVALA |
| 10 | Complement Factor H signal peptide | MRLLAKIICL MLWAICVA |
| 11 | Opticin signal peptide | MRLLAFLSLL ALVLQETGT |
| 12 | Albumin signal peptide | MKWVTFISLL FLFSSAYS |
| 13 | Chymotrypsinogen signal peptide | MAFLWLLSCW ALLGTTFG |
| 14 | Interleukin-2 signal peptide | MYRMQLLSCI ALILALVTNS |
| 15 | Trypsinogen-2 signal peptide | MNLLLILTFV AAAVA |
| 16 | AAV1 | MAADGYLPDWLEDNLSEGIREWWDLKPGAPKPKANQQKQDDG RGLVLPGYKYLGPFNGLDKGEPVNAADAAALEHDKAYDQQLK |
| | | AGDNPYLRYNHADAEFQERLQEDTSFGGNLGRAVFQAKKRVL EPLGLVEEGAKTAPGKKRPVEQSPQEPDSSSGIGKTGQQPAK KRLNFGQTGDSESVPDPQPLGEPPATPAAVGPTTMASGGGAP MADNNEGADGVGNASGNWHCDSTWLGDRVITTSTRTWALPTY NNHLYKQISSASTGASNDNHYFGYSTPWGYFDFNRFHCHFSP RDWQRLINNNWGFRPKRLNFKLFNIQVKEVTTNDGVTTIANN LTSTVQVFSDSEYQLPYVLGSAHQGCLPPFPADVFMIPQYGY LTLNNGSQAVGRSSFYCLEYFPSQMLRTGNNFTFSYTFEEVP FHSSYAHSQSLDRLMNPLIDQYLYYLNRTQNQSGSAQNKDLL FSRGSPAGMSVQPKNWLPGPCYRQQRVSKTKTDNNNSNFTWT GASKYNLNGRESIINPGTAMASHKDDEDKFFPMSGVMIFGKE SAGASNTALDNVMITDEEEIKATNPVATERFGTVAVNFQSSS TDPATGDVHAMGALPGMVWQDRDVYLQGPIWAKIPHTDGHFH PSPLMGGFGLKNPPPQILIKNTPVPANPPAEFSATKFASFIT QYSTGQVSVEIEWELQKENSKRWNPEVQYTSNYAKSANVDFT VDNNGLYTEPRPIGTRYLTRPL |
| 17 | AAV2 | |
| 18 | AAV3-3 | MAADGYLPDWLEDNLSEGIREWWALKPGVPQPKANQQHQDNR RGLVLPGYKYLGPGNGLDKGEPVNEADAAALEHDKAYDQQLK AGDNPYLKYNHADAEFQERLQEDTSFGGNLGRAVFQAKKRIL EPLGLVEEAAKTAPGKKGAVDQSPQEPDSSSGVGKSGKQPAR KRLNFGQTGDSESVPDPQPLGEPPAAPTSLGSNTMASGGGAP MADNNEGADGVGNSSGNWHCDSQWLGDRVITTSTRTWALPTY NNHLYKQISSQSGASNDNHYFGYSTPWGYFDFNRFHCHFSPR |
| | | DWQRLINNNWGFRPKKLSFKLFNIQVRGVTQNDGTTTIANNL TSTVQVFTDSEYQLPYVLGSAHQGCLPPFPADVFMVPQYGYL TLNNGSQAVGRSSFYCLEYFPSQMLRTGNNFQFSYTFEDVPF HSSYAHSQSLDRLMNPLIDQYLYYLNRTQGTTSGTTNQSRLL FSQAGPQSMSLQARNWLPGPCYRQQRLSKTANDNNNSNFPWT AASKYHLNGRDSLVNPGPAMASHKDDEEKFFPMHGNLIFGKE GTTASNAELDNVMITDEEEIRTTNPVATEQYGTVANNLQSSN TAPTTGTVNHQGALPGMVWQDRDVYLQGPIWAKIPHTDGHFH PSPLMGGFGLKHPPPQIMIKNTPVPANPPTTFSPAKFASFIT QYSTGQVSVEIEWELQKENSKRWNPEIQYTSNYNKSVNVDFT VDTNGVYSEPRPIGTRYLTRNL |
| 19 | AAV4-4 | MTDGYLPDWLEDNLSEGVREWWALQPGAPKPKANQQHQDNAR GLVLPGYKYLGPGNGLDKGEPVNAADAAALEHDKAYDQQLKA GDNPYLKYNHADAEFQQRLQGDTSFGGNLGRAVFQAKKRVLE PLGLVEQAGETAPGKKRPLIESPQQPDSSTGIGKKGKQPAKK KLVFEDETGAGDGPPEGSTSGAMSDDSEMRAAAGGAAVEGGQ GADGVGNASGDWHCDSTWSEGHVTTTSTRTWVLPTYNNHLYK RLGESLQSNTYNGFSTPWGYFDFNRFHCHFSPRDWQRLINNN WGMRPKAMRVKIFNIQVKEVTTSNGETTVANNLTSTVQIFAD SSYELPYVMDAGQEGSLPPFPNDVFMVPQYGYCGLVTGNTSQ QQTDRNAFYCLEYFPSQMLRTGNNFEITYSFEKVPFHSMYAH SQSLDRLMNPLIDQYLWGLQSTTTGTTLNAGTATTNFTKLRP TNFSNFKKNWLPGPSIKQQGFSKTANQNYKIPATGSDSLIKY ETHSTLDGRWSALTPGPPMATAGPADSKFSNSQLIFAGPKQN GNTATVPGTLIFTSEEELAATNATDTDMWGNLPGGDQSNSNL PTVDRLTALGAVPGMVWQNRDIYYQGPIWAKIPHTDGHFHPS PLIGGFGLKHPPPQIFIKNTPVPANPATTFSSTPVNSFITQY STGQVSVQIDWEIQKERSKRWNPEVQFTSNYGQQNSLLWAPD AAGKYTEPRAIGTRYLTHHL |
| 20 | AAV5 | MSFVDHPPDWLEEVGEGLREFLGLEAGPPKPKPNQQHQDQAR GLVLPGYNYLGPGNGLDRGEPVNRADEVAREHDISYNEQLEA GDNPYLKYNHADAEFQEKLADDTSFGGNLGKAVFQAKKRVLE PFGLVEEGAKTAPTGKRIDDHFPKRKKARTEEDSKPSTSSDA EAGPSGSQQLQIPAQPASSLGADTMSAGGGGPLGDNNQGADG VGNASGDWHCDSTWMGDRVVTKSTRTWVLPSYNNHQYREIKS GSVDGSNANAYFGYSTPWGYFDFNRFHSHWSPRDWQRLINNY WGFRPRSLRVKIFNIQVKEVTVQDSTTTIANNLTSTVQVFTD DDYQLPYVVGNGTEGCLPAFPPQVFTLPQYGYATLNRDNTEN PTERSSFFCLEYFPSKMLRTGNNFEFTYNFEEVPFHSSFAPS QNLFKLANPLVDQYLYRFVSTNNTGGVQFNKNLAGRYANTYK NWFPGPMGRTQGWNLGSGVNRASVSAFATTNRMELEGASYQV |
| | | PPQPNGMTNNLQGSNTYALENTMIFNSQPANPGTTATYLEGN MLITSESETQPVNRVAYNVGGQMATNNQSSTTAPATGTYNLQ EIVPGSVWMERDVYLQGPIWAKIPETGAHFHPSPAMGGFGLK HPPPMMLIKNTPVPGNITSFSDVPVSSFITQYSTGQVTVEME WELKKENSKRWNPEIQYTNNYNDPQFVDFAPDSTGEYRTTRP IGTRYLTRPL |
| 21 | AAV6 | MAADGYLPDWLEDNLSEGIREWWDLKPGAPKPKANQQKQDDG RGLVLPGYKYLGPFNGLDKGEPVNAADAAALEHDKAYDQQLK AGDNPYLRYNHADAEFQERLQEDTSFGGNLGRAVFQAKKRVL EPFGLVEEGAKTAPGKKRPVEQSPQEPDSSSGIGKTGQQPAK KRLNFGQTGDSESVPDPQPLGEPPATPAAVGPTTMASGGGAP MADNNEGADGVGNASGNWHCDSTWLGDRVITTSTRTWALPTY NNHLYKQISSASTGASNDNHYFGYSTPWGYFDFNRFHCHFSP RDWQRLINNNWGFRPKRLNFKLFNIQVKEVTTNDGVTTIANN LTSTVQVFSDSEYQLPYVLGSAHQGCLPPFPADVFMIPQYGY LTLNNGSQAVGRSSFYCLEYFPSQMLRTGNNFTFSYTFEDVP FHSSYAHSQSLDRLMNPLIDQYLYYLNRTQNQSGSAQNKDLL FSRGSPAGMSVQPKNWLPGPCYRQQRVSKTKTDNNNSNFTWT GASKYNLNGRESIINPGTAMASHKDDKDKFFPMSGVMIFGKE SAGASNTALDNVMITDEEEIKATNPVATERFGTVAVNLQSSS TDPATGDVHVMGALPGMVWQDRDVYLQGPIWAKIPHTDGHFH PSPLMGGFGLKHPPPQILIKNTPVPANPPAEFSATKFASFIT QYSTGQVSVEIEWELQKENSKRWNPEVQYTSNYAKSANVDFT VDNNGLYTEPRPIGTRYLTRPL |
| 22 | AAV7 | MAADGYLPDWLEDNLSEGIREWWDLKPGAPKPKANQQKQDNG RGLVLPGYKYLGPFNGLDKGEPVNAADAAALEHDKAYDQQLK AGDNPYLRYNHADAEFQERLQEDTSFGGNLGRAVFQAKKRVL EPLGLVEEGAKTAPAKKRPVEPSPQRSPDSSTGIGKKGQQPA RKRLNFGQTGDSESVPDPQPLGEPPAAPSSVGSGTVAAGGGA PMADNNEGADGVGNASGNWHCDSTWLGDRVITTSTRTWALPT YNNHLYKQISSETAGSTNDNTYFGYSTPWGYFDFNRFHCHFS PRDWQRLINNNWGFRPKKLRFKLFNIQVKEVTTNDGVTTIAN NLTSTIQVFSDSEYQLPYVLGSAHQGCLPPFPADVFMIPQYG YLTLNNGSQSVGRSSFYCLEYFPSQMLRTGNNFEFSYSFEDV PFHSSYAHSQSLDRLMNPLIDQYLYYLARTQSNPGGTAGNRE LQFYQGGPSTMAEQAKNWLPGPCFRQQRVSKTLDQNNNSNFA WTGATKYHLNGRNSLVNPGVAMATHKDDEDRFFPSSGVLIFG KTGATNKTTLENVLMTNEEEIRPTNPVATEEYGIVSSNLQAA NTAAQTQVVNNQGALPGMVWQNRDVYLQGPIWAKIPHTDGNF HPSPLMGGFGLKHPPPQILIKNTPVPANPPEVFTPAKFASFI |
| | | TQYSTGQVSVEIEWELQKENSKRWNPEIQYTSNFEKQTGVDF AVDSQGVYSEPRPIGTRYLTRNL |
| 23 | AAV8 | MAADGYLPDWLEDNLSEGIREWWALKPGAPKPKANQQKQDDG RGLVLPGYKYLGPFNGLDKGEPVNAADAAALEHDKAYDQQLQ AGDNPYLRYNHADAEFQERLQEDTSFGGNLGRAVFQAKKRVL EPLGLVEEGAKTAPGKKRPVEPSPQRSPDSSTGIGKKGQQPA RKRLNFGQTGDSESVPDPQPLGEPPAAPSGVGPNTMAAGGGA PMADNNEGADGVGSSSGNWHCDSTWLGDRVITTSTRTWALPT YNNHLYKQISNGTSGGATNDNTYFGYSTPWGYFDFNRFHCHF SPRDWQRLINNNWGFRPKRLSFKLFNIQVKEVTQNEGTKTIA NNLTSTIQVFTDSEYQLPYVLGSAHQGCLPPFPADVFMIPQY GYLTLNNGSQAVGRSSFYCLEYFPSQMLRTGNNFQFTYTFED VPFHSSYAHSQSLDRLMNPLIDQYLYYLSRTQTTGGTANTQT LGFSQGGPNTMANQAKNWLPGPCYRQQRVSTTTGQNNNSNFA WTAGTKYHLNGRNSLANPGIAMATHKDDEERFFPSNGILIFG KQNAARDNADYSDVMLTSEEEIKTTNPVATEEYGIVADNLQQ QNTAPQIGTVNSQGALPGMVWQNRDVYLQGPIWAKIPHTDGN FHPSPLMGGFGLKHPPPQILIKNTPVPADPPTTFNQSKLNSF ITQYSTGQVSVEIEWELQKENSKRWNPEIQYTSNYYKSTSVD FAVNTEGVYSEPRPIGTRYLTRNL |
| 24 | hu31 | MAADGYLPDWLEDTLSEGIRQWWKLKPGPPPPKPAERHKDDS RGLVLPGYKYLGPGNGLDKGEPVNAADAAALEHDKAYDQQLK AGDNPYLKYNHADAEFQERLKEDTSFGGNLGRAVFQAKKRLL EPLGLVEEAAKTAPGKKRPVEQSPQEPDSSAGIGKSGSQPAK KKLNFGQTGDTESVPDPQPIGEPPAAPSGVGSLTMASGGGAP VADNNEGADGVGSSSGNWHCDSQWLGDRVITTSTRTWALPTY NNHLYKQISNSTSGGSSNDNAYFGYSTPWGYFDFNRFHCHFS PRDWQRLINNNWGFRPKRLNFKLFNIQVKEVTDNNGVKTIAN NLTSTVQVFTDSDYQLPYVLGSAHEGCLPPFPADVFMIPQYG YLTLNDGGQAVGRSSFYCLEYFPSQMLRTGNNFQFSYEFENV PFHSSYAHSQSLDRLMNPLIDQYLYYLSKTINGSGQNQQTLK FSVAGPSNMAVQGRNYIPGPSYRQQRVSTTVTQNNNSEFAWP GASSWALNGRNSLMNPGPAMASHKEGEDRFFPLSGSLIFGKQ GTGRDNVDADKVMITNEEEIKTTNPVATESYGQVATNHQSAQ AQAQTGWVQNQGILPGMVWQDRDVYLQGPIWAKIPHTDGNFH PSPLMGGFGMKHPPPQILIKNTPVPADPPTAFNKDKLNSFIT QYSTGQVSVEIEWELQKENSKRWNPEIQYTSNYYKSNNVEFA VSTEGVYSEPRPIGTRYLTRNL |
| 25 | hu32 | MAADGYLPDWLEDTLSEGIRQWWKLKPGPPPPKPAERHKDDS RGLVLPGYKYLGPGNGLDKGEPVNAADAAALEHDKAYDQQLK AGDNPYLKYNHADAEFQERLKEDTSFGGNLGRAVFQAKKRLL EPLGLVEEAAKTAPGKKRPVEQSPQEPDSSAGIGKSGSQPAK KKLNFGQTGDTESVPDPQPIGEPPAAPSGVGSLTMASGGGAP VADNNEGADGVGSSSGNWHCDSQWLGDRVITTSTRTWALPTY NNHLYKQISNSTSGGSSNDNAYFGYSTPWGYFDFNRFHCHFS PRDWQRLINNNWGFRPKRLNFKLFNIQVKEVTDNNGVKTIAN NLTSTVQVFTDSDYQLPYVLGSAHEGCLPPFPADVFMIPQYG YLTLNDGSQAVGRSSFYCLEYFPSQMLRTGNNFQFSYEFENV PFHSSYAHSQSLDRLMNPLIDQYLYYLSKTINGSGQNQQTLK FSVAGPSNMAVQGRNYIPGPSYRQQRVSTTVTQNNNSEFAWP GASSWALNGRNSLMNPGPAMASHKEGEDRFFPLSGSLIFGKQ GTGRDNVDADKVMITNEEEIKTTNPVATESYGQVATNHQSAQ AQAQTGWVQNQGILPGMVWQDRDVYLQGPIWAKIPHTDGNFH PSPLMGGFGMKHPPPQILIKNTPVPADPPTAFNKDKLNSFIT QYSTGQVSVEIEWELQKENSKRWNPEIQYTSNYYKSNNVEFA VNTEGVYSEPRPIGTRYLTRNL |
| 26 | AAV9 | MAADGYLPDWLEDNLSEGIREWWALKPGAPQPKANQQHQDNA RGLVLPGYKYLGPGNGLDKGEPVNAADAAALEHDKAYDQQLK AGDNPYLKYNHADAEFQERLKEDTSFGGNLGRAVFQAKKRLL EPLGLVEEAAKTAPGKKRPVEQSPQEPDSSAGIGKSGAQPAK KRLNFGQTGDTESVPDPQPIGEPPAAPSGVGSLTMASGGGAP VADNNEGADGVGSSSGNWHCDSQWLGDRVITTSTRTWALPTY NNHLYKQISNSTSGGSSNDNAYFGYSTPWGYFDFNRFHCHFS PRDWQRLINNNWGFRPKRLNFKLFNIQVKEVTDNNGVKTIAN NLTSTVQVFTDSDYQLPYVLGSAHEGCLPPFPADVFMIPQYG YLTLNDGSQAVGRSSFYCLEYFPSQMLRTGNNFQFSYEFENV PFHSSYAHSQSLDRLMNPLIDQYLYYLSKTINGSGQNQQTLK FSVAGPSNMAVQGRNYIPGPSYRQQRVSTTVTQNNNSEFAWP GASSWALNGRNSLMNPGPAMASHKEGEDRFFPLSGSLIFGKQ GTGRDNVDADKVMITNEEEIKTTNPVATESYGQVATNHQSAQ AQAQTGWVQNQGILPGMVWQDRDVYLQGPIWAKIPHTDGNFH PSPLMGGFGMKHPPPQILIKNTPVPADPPTAFNKDKLNSFIT QYSTGQVSVEIEWELQKENSKRWNPEIQYTSNYYKSNNVEFA VNTEGVYSEPRPIGTRYLTRNL |
| 27 | SP\|P22304\|IDS_HU MAN [Homo sapiens] | MPPPRTGRGLLWLGLVLSSVCVALGSETQANSTTDALNVLLI IVDDLRPSLGCYGDKLVRSPNIDQLASHSLLFQNAFAQQAVC APSRVSFLTGRRPDTTRLYDFNSYWRVHAGNFSTIPQYFKEN GYVTMSVGKVFHPGISSNHTDDSPYSWSFPPYHPSSEKYENT KTCRGPDGELHANLLCPVDVLDVPEGTLPDKQSTEQAIQLLE |
| | | KMKTSASPFFLAVGYHKPHIPFRYPKEFQKLYPLENITLAPD PEVPDGLPPVAYNPWMDIRQREDVQALNISVPYGPIPVDFQR KIRQSYFASVSYLDTQVGRLLSALDDLQLANSTIIAFTSDHG WALGEHGEWAKYSNFDVATHVPLIFYVPGRTASLPEAGEKLF PYLDPFDSASQLMEPGRQSMDLVELVSLFPTLAGLAGLQVPP RCPVPSFHVELCREGKNLLKHFRFRDLEEDPYLPGNPRELIA YSQYPRPSDIPQWNSDKPSLKDIKIMGYSIRTIDYRYTVWVG FNPDEFLANFSDIHAGELYFVDSDPLQDHNMYNDSQGGDLFQ LLMP |
| 28 | TR\|K6ZGI9_PANT R [Pan troglodytes (Chimpanzee)] | MPPPRTGRGLPWLGLVLSSVCVALGSETQANSTTDALNVLLI IVDDLRPSLGCYGDKLVRSPNIDQLASHSLLFQNAFAQQAVC APSRVSFLTGRRPDPTRLYDFNSYWRVHAGNFSTIPQYFKEN GYVTMSVGKVFHPGISSNHTDDSPYSWSFPPYHPSSEKYENT KTCRGPDGELHANLLCPVDVLDVPEGTLPDKQSTEQAIQLLE KMKTSASPFFLAVGYHKPHIPFRYPKEFQKLYPLENITLAPD PEVPDGLPPVAYNPWMDIRQREDVQALNISVPYGPIPVDFQR KIRQSYFASVSYLDTQVGRLLSALDDLQLANSTIIAFTSDHG WALGEHGEWAKYSNFDVATHVPLMFYVPGRTASLPEAGEKLF PYLDPFDSASQLMEPGRQSMDLVELVSLFPTLAGLAGLQAPP RCPVPSFHVELCREGKNLLKHFRFRDLEEDPYLPGNPRELIA YSQYPRPSDIPQWNSDKPSLKDIKIMGYSIRTIDYRYTVWIG FNPDEFLANFSDIHAGELYFVDSDPLQDHNMYNDSQGGDLFQ LLMP |
| 29 | TR\|K7BKV4_PANT R [Pan troglodytes (Chimpanzee)] | MPPPRTGRGLPWLGLVLSSVCVALGSETQANSTTDALNVLLI IVDDLRPSLGCYGDKLVRSPNIDQLASHSLLFQNAFAQQAVC APSRVSFLTGRRPDTTRLYDFNSYWRVHAGNFSTIPQYFKEN GYVTMSVGKVFHPGISSNHTDDSPYSWSFPPYHPSSEKYENT KTCRGPDGELHANLLCPVDVLDVPEGTLPDKQSTEQAIQLLE KMKTSASPFFLAVGYHKPHIPFRYPKEFQKLYPLENITLAPD PEVPDGLPPVAYNPWMDIRQREDVQALNISVPYGPIPVDFQR KIRQSYFASVSYLDTQVGRLLSALDDLQLANSTIIAFTSDHG WALGEHGEWAKYSNFDVATHVPLMFYVPGRTASLPEAGEKLF PYLDPFDSASQLMEPGRQSMDLVELVSLFPTLAGLAGLQAPP RCPVPSFHVELCREGKNLLKHFRFRDLEEDPYLPGNPRELIA YSQYPRPSDIPQWNSDKPSLKDIKIMGYSIRTIDYRYTVWIG FNPDEFLANFSDIHAGELYFVDSDPLQDHNMYNDSQGGDLFQ LLMP |
| 30 | TR\|H9FTX2_MAC MU [Macaca mulatta (Rhesus macaque)] | MPTPGSGRGFLWLGLVLSSVCVALGCETQANSTTDALNILLI IVDDLRPSLGCYGDKLVRSPNIDQLASHSLLFQNAFAQQAVC APSRVSFLTGRRPDTTRLYDFNSYWRVHAGNFSTIPQYFKEN GYVTMSVGKVFHPGITSNHTDDSPYSWSFPPYHPSSEKYENT KTCRGPDGELHANLLCPVDVVDVPEGTLPDKQSTEQAIQLLE KMKTSASPFFLAVGYHKPHIPFRYPKEFQKLYPLENITLAPD SEVPDGLPPVAYNPWMDIRQREDVQALNISVPYGPIPVEFQR |
| | | KIRQSYFASVSYLDTQVGRLLSALDDLQLANSTIVAFTSDHG WALGEHGEWAKYSNFDVATHVPLMFYVPGRTASLPEAGEKLF PYLDPFDSASELMEPGRQSMDLVELVSLFPTLAGLAGLQVPP RCPVPSFHVELCREGKNLLKHFRFRDLEEDPYLPGNPRELIA YSQYPRPADFPQWNSDKPSLKDIKIMGYSIRTIDYRYTVWVG FNPDEFLANFSDIHAGELYFVDSDPLQDHNMYNDSQGGDLFQ LLMP |
| 31 | TRF7EJG2_CALJA [Callithrix jacchus (White-tufted-ear marmoset)] | MPPPRTSRCLLLLGLVLGSVCVTLGSQAQASSTTDALNVLLI IVDDLRPSLGCYGDKLVRSPNIDQLASHSLLFQNAFAQQAVC APSRVSFLTGRRPDTTRLYDFNSYWRVHAGNFSTIPQYFKDN GYVTMSVGKVFHPGISSNHSDDSPYSWSFPPYHPSSEKYENT KTCRGPDGELHANLLCPVDVVDVPEGTLPDKQSTEEAIRLLE KMKTSASPFFLAVGYHKPHIPFRYPKEFQKLYPLENITLAPD PEVPDGLPPVAYNPWMDIRQREDVQALNISVPYGPIPVDFQR KIRQSYFASVSYLDTQVGHLLSALDDLQLANSTIVAFTSDHG WALGEHGEWAKYSNFDVATRVPLMFYVPGRTASLPEADEKLF PYVDPFHSASELMEPGRQSMDLVELVSLFPTLAGLAGLQVPP RCPVPSFHVELCREGKSLLKHFRFHGLEEDPYLPGNPRELIA YSQYPRPADFPQWNSDKPSLKYIKIMGYSIRTVDYRYTVWVG FNPDEFLANFSDIHAGELYFVDSDPLQDHNMYNDSQGGELFQ SLMP |
| 32 | TR\|U3DTL8_CALJ A [Callithrix jacchus (White-tufted-ear marmoset)] | MPPPRPSRCLLLLGLVLGSVCVTLGSQAQASSTTDALNVLLI IVDDLRPSLGCYGDKLVRSPNIDQLASHSLLFQNAFAQQAVC APSRVSFLTGRRPDTTRLYDFNSYWRVHAGNFSTIPQYFKDN GYVTMSVGKVFHPGISSNHSDDSPYSWSFPPYHPSSEKYENT KTCRGPDGELHANLLCPVDVVDVPEGTLPDKQSTEEAIRLLE KMKTSASPFFLAVGYHKPHIPFRYPKEFQKLYPLENITLAPD PEVPDGLPPVAYNPWMDIRQREDVQALNISVPYGPIPVDFQR KIRQSYFASVSYLDTQVGHLLSALDDLQLANSTIVAFTSDHG WALGEHGEWAKYSNFDVATRVPLMFYVPGRTASLPEADEKLF PYVDPFHSASELMEPGRQSMDLVELVSLFPTLAGLAGLQVPP RCPVPSFHVELCREGKSLLKHFRFHGLEEDPYLPGNPRELIA YSQYPRPADFPQWNSDKPSLKYIKIMGYSIRTVDYRYTVWVG FNPDEFLANFSDIHAGELYFVDSDPLQDHNMYNDSQGGELFQ SLMP |
| 33 | TR\|G7NRX7 _MAC MU [Macaca mulatta (Rhesus macaque)] | MPTPGSGRGFLWLGLVLSSVCVALGCETQANSTTDALNILLI IVDDLRPSLGCYGDKLVRSPNIDQLASHSLLFQNAFAQQAVC APSRVSFLTGRRPDTTRLYDFNSYWRVHAGNFSTIPQYFKEN GYVTMSVGKVFHPGITSNHTDDSPYSWSFPPYHPSSEKYENT KTCRGPDGELHANLLCPVDVVDVPEGTLPDKQSTEQAIQLLE KMKTSASPFFLAVGYHKPHIPFRYPKEFQKLYPLENITLAPD SEVPDGLPPVAYNPWMDIRQREDVQALNISVPYGPIPVEFQR KIRQSYFASVSYLDTQVGRLLSALDDLQLANSTIVAFTSDHG WALGEHGEWAKYSNFDVATHVPLMFYVPGRTASLPEAGEKLF |
| | | PYLDPFDSASELMEPGRQSMDLVELVSLFPTLAGLAGLQVPP RCPVPSFHVELCREGKNLLKHFRFRDLEEDPYLPGNPRELIA YSQYPRPADFPQWNSDKPSLKDIKIMGYSIRTIDYRYTVWVG FNPDEFLANFSDIHAGELYFVDSDPLQDHNMYNDSQGGDLLQ LLMP |
| 34 | TR\|G7Q1V9_MAC FA [Macaca fascicularis (Crab-eating macaque; Cynomologous monkey)] | MPTPGSGRGFLWLGLVLSSVCVALGCETQANSTTDALNILLI IVDDLRPSLGCYGDKLVRSPNIDQLASHSLLFQNAFAQQAVC APSRVSFLTGRRPDTTRLYDFNSYWRVHAGNFSTIPQYFKEN GYVTMSVGKVFHPGITSNHTDDSPYSWSFPPYHPSSEKYENT KTCRGPDGELHANLLCPVDVVDVPEGTLPDKQSTEQAIQLLE KMKTSASPFFLAVGYHKPHIPFRYPKEFQKLYPLENITLAPD SEVPDGLPPVAYNPWMDIRQREDVQALNISVPYGPIPVEFQR KIRQSYFASVSYLDTQVGHLLSALDDLQLANSTIVAFTSDHG WALGEHGEWAKYSNFDVATHVPLMFYVPGRTASLPEAGEKLF PYLDPFDSASELMEPGRQSMDLVELVSLFPTLAGLAGLQVPP RCPVPSFHVELCREGKNLLKHFRFRDLEEDPYLPGNPRELIA YSQYPRPADFPQWNSDKPSLKDIKIMGYSIRTIDYRYTVWVG FNPDEFLANFSDIHAGELYFVDSDPLQDHNMYNDSQGGDLFQ LLMP |
| 35 | TR\|H2PX10_PONA B [Pongo abelii (Sumatran orangutan)] | MPPPRTGRGLLWLGLVLSSVCVALGSETQADSTTDGLNVLLI IVDDLRPSLGCYGDKLVRSPNIDQLASHSLLFQNAFAQQAVC APSRVSFLTGRRPDTTRLYDFNSYWRVHAGNFSTIPQYFKEN GYVTMSVGKVFHPGISSNHTDDSPYSWSFPPYHPSSEKYENT KTCRGPDGELHANLIAKKMCWMFPRAPCCDKQSTEQAIQLLE KMKTSASPFFLAVGYHKPHIPFRYPKEFQKLYPLENITLAPD PEVPDGLPPVAYNPWMDIRQREDVQALNISVPYGPIPVDFQQ KIRQSYFASVSYLDTQVGRLLSTLDDLQLANSTIIAFTSDHG WALGEHGEWAKYSNFDVATHVPLMFYVPGRTASLPEAGEKLF PYLDPFDSASELMEPGRQSMDLVELVSLFPTLAGLAGLQVPP RCPVPSFHVELCREGKNLLKHFRFRDLEEDPYLPGNPRELIA YSQYPRPADIPQWNSDKPSLKDIKIMGYSIRTIDYRYTVWVG FNPDEFLANFSDIHAGELYFVDSDPLQDHNMYNDSQGGDLFQ LLMP |
| 36 | TR\|A0A0D9R4D1_ CHLSB [Chlorocebus sabaeus (Green monkey)] | MPTPGSGRGFLWLGLVLSSVCVALGSETQANSTTDALNILLI IVDDLRPSLGCYGDKLVRSPNIDQLASHSLLFQNAFAQQAVC APSRVSFLTGRRPDTTRLHNFNSYWRVHAGNFSTIPQYFKEN GYVTMSVGKVFHPGITSNHTDDSPYSWSFPPYHPSSEKYENT KTCRGPDGELHANLLCPVDVVDVPEGTLPDKQSTEQAIQLLE KMKTSASPFFLAVGYHKPHIPFRYPKEFQKLYPLENITLAPD SEVPDGLPPVAYNPWMDIRQREDVQALNISVPYGPIPVEFQR KIRQSYFASVSYLDTQVGRLLSALDDLQLANSTIVAFTSDHG WALGEHGEWAKYSNFDVATHVPLMFYVPGRTASLPEAGEKLF PYLDPFDSASELMEPGRQSMDLVELVSLFPTLAGLAGLQVPP RCPVPSFHVELCREGKNLLKHFRFRDLEEDPYLPGNPRELIA |
| | | YSQYPRPADFPQWNSDKPNLKDIKIMGYSIRTIDYRYTVWVG FNPDEFLANFSDIHAGELYFVDSDPLQDHNMYNDSQGGDLFQ LLMP |
| 37 | TR\|G1RST8\|G1RST 8_NOMLE [Nomascus leucogenys (Northern white-cheeked gibbon)] | MSPPRTGQGLLWLGVVLSSVCVAXVTSPKPPSFVDALNVLLI IVDDLRPSLGCYGDKLVRSPNIDQLASHSLLFQNAFAQQAVC APSRVSFLTGRRPDTTRLYDFNSYWRVHAGNFSTIPQYFKEN GYVTMSVGKVFHPGISSNHTDDSPYSWSFPPYHPSSXXXXXX KTCRGPDGELHANLLCPVDVLDVPEGTLPDKQSTEQAIQLLE KMKTSASPFFLAVGYHKPHIPFRYPKEFQKLYPLENITLAPD PEVPDGLPPVAYNPWMDIRQREDVQALNISVPYGPIPVDFQR KIRQSYFASVSYLDTQVGRLLSALDDLQLANSTIIAFTSDHG WALGEHGEWAKYSNFDVATHVPLMFYVPGRTASLPEAGEKLF PYLDPFDSASELMEPGRQSMDLVELVSLFPTLAGLAGLQVPP RCPVPSFHVELCREGKNLLKHFRFRDLEEDPYLPGNPRELIA YSQYPRPADIPQWNSDKPSLKDIKIMGYSIRTIDYRYTVWVG FSPDEFLANFSDIHAGELYFVDSDPLQDHNMYNDSQGGDLFQ LLMP |
| 38 | UPI0000D9F625 [Macaca mulatta (Rhesus macaque)] | MPTPGSGRGFLWLGLVLSSVCVALGCETQANSTTDALNILLI IVDDLRPSLGCYGDKLVRSPNIDQLASHSLLFQNAFAQQAVC APSRVSFLTGRRPDTTRLYDFNSYWRVHAGNFSTIPQYFKEN GYVTMSVGKVFHPGITSNHTDDSPYSWSFPPYHPSSEKYENT KTCRGPDGELHANLLCPVDVVDVPEGTLPDKQSTEQAIQLLE KMKTSASPFFLAVGYHKPHIPFRYPKEFQKLYPLENITLAPD SEVPDGLPPVAYNPWMDIRQREDVQALNISVPYGPIPVEFQR KIRQSYFASVSYLDTQVGHLLSALDDLQLANSTIVAFTSDHG WALGEHGEWAKYSNFDVATHVPLMFYVPGRTASLPEAGEKLF PYLDPFDSASELMEPGRQSMDLVELVSLFPTLAGLAGLQVPP RCPVPSFHVELCREGKNLLKHFRFRDLEEDPYLPGNPRELIA YSQYPRPADFPQWNSDKPSLKDIKIMGYSIRTIDYRYTVWVG FNPDEFLANFSDIHAGELYFVDSDPLQDHNMYNDSQGGDLLQ LLMP |
| 39 | UPI000274358B [Pan paniscus (Pygmy chimpanzee; Bonobo)] | MPPPRTGRGLLWLGLVLSSVCVALGSETQANSTTDALNVLLI IVDDLRPSLGCYGDKLVRSPNIDQLASHSLLFQNAFAQQAVC APSRVSFLTGRRPDTTRLYDFNSYWRVHAGNFSTIPQYFKEN GYVTMSVGKVFHPGISSNHTDDSPYSWSFPPYHPSSEKYENT KTCRGPDGELHANLLCPVDVLDVPEGTLPDKQSTEQAIRLLE KMKTSASPFFLAVGYHKPHIPFRYPKEFQKLYPLENITLAPD PEVPDGLPPVAYNPWMDIRQREDVQALNISVPYGPIPVDFQR KIRQSYFASVSYLDTQVGRLLSALDDLQLANSTIIAFTSDHG WALGEHGEWAKYSNFDVATHVPLMFYVPGRTASLPEAGEKLF PYLDPFDSASQLMEPGRQSMDLVELVSLFPTLAGLAGLQVPP RCPVPSFHVELCREGKNLLKHFRFRDLEEDPYLPGNPRELIA YSQYPRPSDIPQWNSDKPSLKDIKIMGYSIRTIDYRYTVWVG |
| | | FNPDEFLANFSDIHAGELYFVDSDPLQDHNMYNDSQGGDLFQ LLMP |
| 40 | UPI00027F6FC5 [Papio Anubis (Olive baboon)] | MPTPGSGRGFLWLGLVLSSVCVALGCEMQANSTTDALNILLI IVDDLRPSLGCYGDKLVRSPNIDQLASHSLLFQNAFAQQAVC APSRVSFLTGRRPDTTRLYDFNSYWRVHAGNFSTIPQYFKEN GYVTMSVGKVFHPGITSNHTDDSPYSWSFPPYHPSSEKYENT KTCRGPDGELHANLLCPVDVVDVPEGTLPDKQSTEQAIQLLE KMKTSASPFFLAVGYHKPHIPFRYPKEFQKLYPLENITLAPD SEVPDGLPPVAYNPWMDIRQREDVQALNISVPYGPIPVEFQR KIRQSYFASVSYLDTQVGRLLSALDDLQLANSTIVAFTSDHG WALGEHGEWAKYSNFDVATHVPLMFYVPGRTASLPEAGEKLF PYLDPFDSASELMEPGRQSMDLVELVSLFPTLAGLAGLQVPP RCPVPSFHVELCREGKNLLKHFRFRDLEEDPYLPGNPRELIA YSQYPRPADFPQWNSDKPSLKDIKIMGYSIRTIDYRYTVWVG FNPDEFLANFSDIHAGELYFVDSDPLQDHNMYNDSQGGDLFQ LLMP |
| 41 | UPI00027FAE03 [Saimiri boliviensis (Bolivian squirrel monkey)] | MPPPRTGLCLLLLGLVLGSVCVTLGSQAQANSTTDALNVLLI IVDDLRPSLGCYGDKLVRSPNIDQLASHSLLFQNAFVQQAVC APSRVSFLTGRRPDTTRLYDFNSYWRVHAGNFSTIPQYFKDN GYVTMSVGKVFHPGISSNHSDDSPYSWSFPPYHPSSEKYENT KTCRGPDGELHANLLCPVDVVDVPEGTLPDKQSTEEAIRLLK KMKTSASPFFLAVGYHKPHIPFRYPKEFQKLYPLENITLAPD PEVPDGLPPVAYNPWMDIRQREDVQALNISVPYGPIPVDFQR KIRQSYFASVSYLDTQVGHLLSALDDLHLANSTIVAFTSDHG WALGEHGEWAKYSNFDVATRVPLMFYVPGRTASLPETGEKLF PYVDPFHSASELMEPGRQSTDLVELVSLFPTLAGLAGLQVPP RCPVPSFHIELCREGKNLLKHFRFHGLEEDPYLPGNPRELIA YSQYPRPADFPQQNSDKPSLKYIKIMGYSIRTVDYRYTVWVG FNPDEFLANFSDIHAGELYFVDSDPLQDHNMYNDSQGGELFQ SLMP |
| 42 | UPI0003ABBF28 [Macaca fascicularis (Crab-eating macaque; Cynomologous monkey)] | MPTPGSGRGFLWLGLVLSSVCVALGCETQANSTTDALNILLI IVDDLRPSLGCYGDKLVRSPNIDQLASHSLLFQNAFAQEAVC APSRVSFLTGRRPDTTRLYDFNSYWRVHAGNFSTIPQYFKEN GYVTMSVGKVFHPGITSNHTDDSPYSWSFPPYHPSSEKYENT KTCRGPDGELHANLLCPVDVVDVPEGTLPDKQSTEQAIQLLE KMKTSASPFFLAVGYHKPHIPFRYPKEFQKLYPLENITLAPD SEVPDGLPPVAYNPWMDIRQREDVQALNISVPYGPIPVEFQR KIRQSYFASVSYLDTQVGRLLSALDDLQLANSTIVAFTSDHG WALGEHGEWAKYSNFDVATHVPLMFYVPGRTASLPEAGEKLF PYLDPFDSASELMEPGRQSMDLVELVSLFPTLAGLAGLQVPP RCPVPSFHVELCREGKNLLKHFRFRDLEEDPYLPGNPRELIA YSQYPRPADFPQWNSDKPSLKDIKIMGYSIRTIDYRYTVWVG FNPDEFLANFSDIHAGELYFVDSDPLQDHNMYNDSQGGDLFQ LLMP |
| 43 | UPI000533297F [Rhinopithecus roxellana (Golden snub-nosed monkey; Pygathrix roxellana)] | MPTPASGRGFLWLGLVLSSVCVALGSETQANSTTDALNILLI IVDDLRPSLGCYGDKLVRSPNIDQLASHSLLFQNAFAQQAVC APSRVSFLTGRRPDTTRLYDFNSYWRVHAGNFSTIPQYFKEN GYVTMSVGKVFHPGITSNHTDDSPYSWSFPPYHPSSEKYENT KTCRGPDGELHANLLCPVDVVDVPEGTLPDKQSTEQAVQLLE KMKTSASPFFLAVGYHKPHIPFRYPKEFQKLYPLENITLAPD SEVPDGLPPVAYNPWMDIRQREDVQALNISVPYGPIPVDFQR KIRQSYFASVSYLDTQVGHLLSALDDLQLANSTIVAFTSDHG WALGEHGEWAKYSNFDVATHVPLMFYVPGRTASLPEAGEKLF PYLDPFDSASELMEPGRQSMDLVELVSLFPTLAGLAGLQVPP RCPVPSFHVELCREGKNLLKHFRFRDLEEDPYLPGNPRELIA YSQYPRPADFPQWNSDKPSLKDIKIMGYSIRTIDYRYTVWVG FNPDEFLANFSDIHAGELYFVDSDPFQDHNMYNDSQGGDLFQ LLMP |
| 44 | UPI0005F40BD2 [Colobus angolensis palliates (Peters' Angolan colobus)] | MPTPASGRGFLWLGLVLRSVCVALGSETQANSTTDALNILLI IVDDLRPSLGCYGDKLVRSPNIDQLASHSLLFQNAFAQQAVC TPSHVSFLTGRRPDTTRLYDFNSYWRVHAGNFSTIPQYFKEN GYVTMSVGKVFHPGITSNHTDDSPYSWSFPPYHPSSEKYENT KTCRGPDGELHANLLCPVDVVDVPEGTLPDKQSTEQAIQLLE KMKTSASPFFLAVGYHKPHIPFRYPKEFQKLYPLENITLAPD SEVPDGLPPVAYNPWMDIRQREDVQALNISVPYGPIPVDFQR KIRQSYFASVSYLDTQVGHLLSALDDLQLANSTIVAFTSDHG WALGEHGEWAKYSNFDVATHVPLMFYVPGRTASLPEAGEKLF PYLDPFDSASELMEPGRQSMDLVELVSLFPTLAGLAGLQVPP RCPVPSFHVELCREGKNLLKHFRFRDLEEDPYLPGNPRELIA YSQYPRPADFPQWNSDKPSLKDIKIMGYSIRTIDYRYTVWVG FNPDEFLANFSDIHAGELYFVDSDPLQDHNMYNDSQGGDLFQ LLMP |
| 45 | CB7.CI.hIDS.RBG | gctgcgcgct cgctcgctca ctgaggccgc ccgggcaaag cccgggcgtc gggcgacctt 60 tggtcgcccg gcctcagtga gcgagcgagc gcgcagagag ggagtggcca actccatcac 120 taggggttcc ttgtagttaa tgattaaccc gccatgctac ttatctacca gggtaatggg 180 gatcctctag aactatagct agtcgacatt gattattgac tagttattaa tagtaatcaa 240 ttacggggtc attagttcat agcccatata tggagttccg cgttacataa cttacggtaa 300 |
| | (2)..(131) 5' ITR | |
| | (199)..(580) | |
| | CMV IE promoter | |
| | (583)..(864) | |
| | CB promoter | |
| | (837)..(840) | |
| | TATA_signal | atggcccgcc tggctgaccg cccaacgacc cccgcccatt gacgtcaata atgacgtatg     360 ttcccatagt aacgccaata gggactttcc attgacgtca atgggtggac tatttacggt     420 aaactgccca cttggcagta catcaagtgt atcatatgcc aagtacgccc cctattgacg     480 tcaatgacgg taaatggccc gcctggcatt atgcccagta catgacctta tgggactttc     540 ctacttggca gtacatctac gtattagtca tcgctattac catggtcgag gtgagcccca     600 cgttctgctt cactctcccc atctcccccc cctccccacc cccaattttg tatttattta     660 ttttttaatt attttgtgca gcgatggggg cggggggggg gggggggcgc gcgccaggcg     720 gggcggggcg gggcgagggg cggggcgggg cgaggcggag aggtgcggcg gcagccaatc     780 agagcggcgc gctccgaaag tttcctttta tggcgaggcg gcggcggcgg cggccctata     840 aaaagcgaag cgcgcggcgg gcggggagtc gctgcgacgc tgccttcgcc ccgtgccccg     900 ctccgccgcc gcctcgcgcc gcccgccccg gctctgactg accgcgttac tcccacaggt     960 gagcgggcgg gacggccctt ctcctccggg ctgtaattag cgcttggttt aatgacggct     1020 tgtttctttt ctgtggctgc gtgaaagcct tgaggggctc cggagggccc tttgtgcgg     1080 ggggagcggc tcggggggtg cgtgcgtgtg tgtgtgcgtg gggagcgccg cgtgcggctc     1140 cgcgctgccc ggcggctgtg agcgctgcgg gcgcggcgcg gggctttgtg cgctccgcag     1200 |
| | (959)..(1930) | |
| | chicken beta-actin intron | |
| | (1937)..(3589) | |
| | hIDS | |
| | (3623)..(3749) | |
| | rabbit globin polyA | |
| | (3838)..(3967) | |
| | 3' ITR | |
| | | |
| | | tgtgcgcgag gggagcgcgg ccgggggcgg tgccccgcgg tgcggggggg gctgcgaggg 1260 gaacaaaggc tgcgtgcggg gtgtgtgcgt gggggggtga gcagggggtg tgggcgcgtc 1320 ggtcgggctg caaccccccc tgcacccccc tccccgagtt gctgagcacg gcccggcttc 1380 gggtgcgggg ctccgtacgg ggcgtggcgc ggggctcgcc gtgccgggcg gggggtggcg 1440 gcaggtgggg gtgccgggcg gggcggggcc gcctcgggcc ggggagggct cgggggaggg 1500 gcgcggcggc ccccggagcg ccggcggctg tcgaggcgcg gcgagccgca gccattgcct 1560 tttatggtaa tcgtgcgaga gggcgcaggg acttcctttg tcccaaatct gtgcggagcc 1620 gaaatctggg aggcgccgcc gcaccccctc tagcgggcgc ggggcgaagc ggtgcggcgc 1680 cggcaggaag gaaatgggcg gggagggcct tcgtgcgtcg ccgcgccgcc gtccccttct 1740 ccctctccag cctcggggct gtccgcgggg ggacggctgc cttcgggggg gacggggcag 1800 ggcggggttc ggcttctggc gtgtgaccgg cggctctaga gcctctgcta accatgttca 1860 tgccttcttc tttttcctac agctcctggg caacgtgctg gttattgtgc tgtctcatca 1920 ttttggcaaa gaattcatgc cgccaccccg gaccggccga ggccttctct ggctgggtct 1980 ggttctgagc tccgtctgcg tcgccctcgg atccgaaacg caggccaact cgaccacaga 2040 |
| | | tgctctgaac gttcttctca tcatcgtgga tgacctgcgc ccctccctgg gctgttatgg 2100 ggataagctg gtgaggtccc caaatattga ccaactggca tcccacagcc tcctcttcca 2160 gaatgccttt gcgcagcaag cagtgtgcgc cccgagccgc gtttctttcc tcactggcag 2220 gagacctgac accacccgcc tgtacgactt caactcctac tggagggtgc acgctggaaa 2280 cttctccacc atcccccagt acttcaagga gaatggctat gtgaccatgt cggtgggaaa 2340 agtctttcac cctgggatat cttctaacca taccgatgat tctccgtata gctggtcttt 2400 tccaccttat catccttcct ctgagaagta tgaaaacact aagacatgtc gagggccaga 2460 tggagaactc catgccaacc tgctttgccc tgtggatgtg ctggatgttc ccgagggcac 2520 cttgcctgac aaacagagca ctgagcaagc catacagttg ttggaaaaga tgaaaacgtc 2580 agccagtcct ttcttcctgg ccgttgggta tcataagcca cacatcccct tcagataccc 2640 caaggaattt cagaagttgt atcccttgga gaacatcacc ctggcccccg atcccgaggt 2700 ccctgatggc ctaccccctg tggcctacaa cccctggatg gacatcaggc aacgggaaga 2760 cgtccaagcc ttaaacatca gtgtgccgta tggtccaatt cctgtggact ttcagcggaa 2820 aatccgccag agctactttg cctctgtgtc atatttggat acacaggtcg gccgcctctt 2880 gagtgctttg gacgatcttc agctggccaa cagcaccatc attgcattta cctcggatca 2940 |
| | | tgggtgggct ctaggtgaac atggagaatg ggccaaatac agcaattttg atgttgctac 3000 ccatgttccc ctgatattct atgttcctgg aaggacggct tcacttccgg aggcaggcga 3060 gaagcttttc ccttacctcg acccttttga ttccgcctca cagttgatgg agccaggcag 3120 gcaatccatg gaccttgtgg aacttgtgtc tctttttccc acgctggctg gacttgcagg 3180 actgcaggtt ccacctcgct gccccgttcc ttcatttcac gttgagctgt gcagagaagg 3240 caagaacctt ctgaagcatt ttcgattccg tgacttggaa gaggatccgt acctccctgg 3300 taatccccgt gaactgattg cctatagcca gtatccccgg ccttcagaca tccctcagtg 3360 gaattctgac aagccgagtt taaaagatat aaagatcatg ggctattcca tacgcaccat 3420 agactatagg tatactgtgt gggttggctt caatcctgat gaatttctag ctaacttttc 3480 tgacatccat gcaggggaac tgtattttgt ggattctgac ccattgcagg atcacaatat 3540 gtataatgat tcccaaggtg gagatctttt ccagttgttg atgccttgac tcgaggacgg 3600 ggtgaactac gcctgaggat ccgatctttt tccctctgcc aaaaattatg gggacatcat 3660 gaagcccctt gagcatctga cttctggcta ataaaggaaa tttattttca ttgcaatagt 3720 gtgttggaat tttttgtgtc tctcactcgg aagcaattcg ttgatctgaa tttcgaccac 3780 |
| | | ccataatacc cattaccctg gtagataagt agcatggcgg gttaatcatt aactacaagg     3840 aacccctagt gatggagttg gccactccct ctctgcgcgc tcgctcgctc actgaggccg     3900 ggcgaccaaa ggtcgcccga cgcccgggct ttgcccgggc ggcctcagtg agcgagcgag     3960 cgcgcag 3967 |

### 6. EXAMPLES

### 6.1 EXAMPLE 1: hIDS cDNA

A hIDS cDNA-based vector is constructed comprising a transgene comprising hIDS (SEQ ID NO: 1). The transgene also comprises nucleic acids comprising a signal peptide chosen from the group listed in Table 4. Optionally, the vector additionally comprises a promoter.

### 6.2 EXAMPLE 2: Substituted hIDS cDNAs

A hIDS cDNA-based vector is constructed comprising a transgene comprising hIDS having amino acid substitutions, deletions, or additions compared to the hIDS sequence of SEQ ID NO: 1, e.g., including but not limited to amino acid substitutions selected from corresponding non-conserved residues in orthologs of IDS shown in FIG. 2, with the *proviso* that such mutations do not include replacement of the cysteine residue at position 84 (C84) which is required for enzyme activity (Millat et al., 1997, Biochem J 326: 243-247); or a mutation that has been identified in severe, severe-intermediate, intermediate, or attenuated MPS II phenotypes *e.g.,* as shown in FIG. 3, or as reported by Sukegawa-Hayasaka et al., 2006, J Inhert Metab Dis 29: 755-761 (reporting "attenuated" mutants R48P, A85T, W337R, and the truncated mutant Q531X; and "severe" mutants P86L, S333L, S349I, R468Q, R468L); Millat et al., 1998, BBA 1406: 214-218 (reporting "attenuated" mutants P480L and P480Q; and "severe" mutant P86L); and Bonucelli et al., 2001, BBA 1537:233-238, each of which is incorporated by reference herein in its entirety. The transgene also comprises nucleic acids comprising a signal peptide chosen from the group listed in Table 4. Optionally, the vector additionally comprises a promoter.

### 6.3 EXAMPLE 3: Treatment of MPS II in animals models with hIDS or substituted hIDS

An hIDS cDNA-based vector is deemed useful for treatment of MPS II when expressed as a transgene. An animal model for MPS II, for example a mouse model described in Garcia et al., 2007, J Inherit Metab Dis 30: 924-34 or Muenzer et al., 2001, Acta Paediatr Suppl 91:98-99 is administered a recombinant vector that encodes hIDS intrathecally at a dose sufficient to deliver and maintain a therapeutically effective concentration of the transgene product in the CSF of the animal. Following treatment, the animal is evaluated for improvement in symptoms consistent with the disease in the particular animal model.

### 6.4 EXAMPLE 4: Treatment of MPS II with hIDS or substituted hIDS

An hIDS cDNA-based vector is deemed useful for treatment of MPS II when expressed as a transgene. A subject presenting with MPS II is administered a cDNA-based vector that encodes hIDS (*e.g.,* such as Construct 1 (see below) intrathecally at a dose sufficient to deliver and maintain a therapeutic concentration of the transgene product in the CSF. Following treatment, the subject is evaluated for improvement in symptoms of MPS II.

### 6.5 EXAMPLE 5: A Phase I/II Multicenter, Open-Label Study to Evaluate the Safety, Tolerability, and Pharmacodynamics of Construct 1 in Pediatric Subjects with MPS II (Hunter Syndrome)

### 6.5.1. SYNOPSIS

### INVESTIGATIONAL PRODUCT, DOSE, AND Route OF ADMINISTRATION

Construct 1: AAV9.CB7.hIDS (recombinant adeno-associated virus serotype 9 capsid containing human iduronate-2-sulfatase expression cassette). See paragraph [0019] and Figure 5.

### Product will be delivered as a single intracisternal (IC) dose.

Two dose levels will be evaluated, 1.3 × 10¹⁰ genome copies (GC)/g brain mass (Dose 1) and 6.5 × 10¹⁰ GC/g brain mass (Dose 2). Total dose administered will account for estimated brain size of study subjects based on their age. Total volume of product administered will not exceed 5 mL.

### OBJECTIVES

### Primary Objective:

To evaluate the safety and tolerability of Construct 1 through 24 weeks following a single IC dose administered to pediatric subjects who have severe MPS II

### Secondary Objectives:

To evaluate the long-term safety and tolerability of Construct 1

To evaluate the effect of Construct 1 on biomarkers in cerebrospinal fluid (CSF), plasma, and urine

To evaluate the effect of Construct 1 on neurodevelopmental parameters of cognitive, behavioral, and adaptive function

To evaluate vector shedding in CSF, plasma, and urine

### Exploratory Objectives:

- To evaluate immunogenicity of Construct 1
- To explore the effect of Construct 1 on physical changes to the CNS
- To explore the effect of Construct 1 on systemic manifestations of disease
- To explore the effect of Construct 1 on auditory capacity
- To explore the effect of Construct 1 on biomarkers in plasma and urine in subjects who temporarily discontinue IV ERT (ELAPRASE^{®})
- To explore the effect of Construct 1 on quality of life (QOL) and sleep measures.

### STUDY DESIGN AND METHODOLOGY

This is a Phase I/II, first-in-human, multicenter, open-label, single arm dose escalation study of Construct 1. No control group is included. Approximately 6 pediatric subjects who have severe MPS II could be enrolled into 2 dose cohorts, 1.3 × 10¹⁰ GC/g brain mass (Dose 1) or 6.5 × 10¹⁰ GC/g brain mass (Dose 2) and will receive a single dose of Construct 1 administered by IC injection. Safety will be the primary focus for the initial 24 weeks after treatment (primary study period). Following completion of the primary study period, subjects will continue to be assessed (safety and efficacy) for up to a total of 104 weeks following treatment with Construct 1. At the end of the study, subjects will be invited to participate in a long-term follow-up study.

The first 3 eligible subjects will be enrolled into the Dose 1 cohort (1.3 × 10¹⁰ GC/g brain mass). After Construct 1 administration to the first subject, there will be an 8-week observation period for safety. The Internal Safety Committee (ISC) will review the safety data obtained during the first 8 weeks (including data obtained during the Week 8 visit) for this subject, and if there are no safety concerns, the 2^{nd} subject may be enrolled. The same process will be used to enroll the 3^{rd} subject. If no safety review trigger (SRT) event is observed, all available safety data for the Dose 1 cohort obtained up to and including the Week 8 visit for the 3^{rd} subject will be evaluated by the Independent Data Monitoring Committee (IDMC). If the decision is to proceed to the second dose (6.5 × 10¹⁰ GC/g brain mass), the subsequent 2 subjects will follow the same dosing scheme as the initial dose cohort with dosing of each subsequent subject occurring after all safety data obtained during the first 8 weeks (including data obtained during the Week 8 visit) for the last dosed subject have been reviewed. The ISC will review all subject safety data obtained up to and including the Week 2 visit of the 2^{nd} subject and may determine that it is safe to proceed with dosing of the 3^{rd} subject immediately after this assessment. All available safety data for the Dose 2 cohort will be evaluated by the IDMC after the Week 8 visit for the 3^{rd} subject in the Dose 2 cohort.

Potential subjects will be screened up to 35 days prior to dosing to determine eligibility for the study. Those subjects who meet the eligibility criteria will be admitted to the hospital between Day -2 and the morning of Day 1 (according to institutional practice), and baseline assessments will be performed pre-dose. Subjects will receive a single IC dose of Construct 1 on Day 1 and will remain in the hospital for approximately 30-36 hours after dosing for observation. Subsequent assessments in the primary study period (i.e., through Week 24) will be performed weekly through Week 4 and at Weeks 8, 12, 16, 20, and 24. After the primary study period, visits will be at Weeks 28, 32, 40, 48, 52, 56, 64, 78, and 104. The Week 12, 40, and 64 visits may be performed by a home health nurse. The Week 20 and 28 assessments will be limited to evaluation of AEs and concomitant therapies by telephone contact.

All subjects will initially receive immune suppression (IS) in the study based on findings of potential immunogenicity in the nonclinical safety/toxicology study conducted in animals and will include corticosteroids (methylprednisolone 10 mg/kg intravenously [IV] once on Day 1 predose and oral prednisone starting at 0.5 mg/kg/day on Day 2 with gradual tapering and discontinuation by Week 12), tacrolimus (1 mg twice daily [BID] by mouth [PO] Day 2 to Week 24 with target blood level of 4-8 ng/mL and tapering over 8 weeks between Week 24 and 32) and sirolimus (a loading dose of 1 mg/m² every 4 hours x 3 doses on Day -2 and then from Day -1: sirolimus 0.5 mg/m²/day divided in BID dosing with target blood level of 4-8 ng/ml until Week 48). Neurologic assessments and tacrolimus/sirolimus blood level monitoring will be conducted. The doses of sirolimus and tacrolimus will be adjusted to maintain blood levels in the target range.

No IS therapy is planned after Week 48. If IS is required after Week 48 to control a clinically-relevant immune response, the appropriate immunosuppressive regimen will be determined by the principal investigator (PI), in discussion with the Medical Monitor and Sponsor, as clinically indicated.

Efficacy assessments will include neurocognitive function, auditory capacity, brain MRI, liver and spleen size, and measurements of levels of pharmacodynamic (PD) biomarkers in CSF, plasma, and urine. Neurocognitive or adaptive scales performed as part of subjects' standard of care while participating in the trial may also be collected, as determined by the study sponsor after discussing with the site.

### ENDPOINTS

### Primary Endpoints:

- Safety through Week 24: AEs and serious adverse events (SAEs)

### Secondary Endpoints:

- Safety through Week 104: AE reporting, laboratory evaluations, vital signs, ECGs, physical examinations, and neurologic assessments
   - Biomarkers in CSF (GAGs, I2S activity), plasma (GAGs, I2S activity), and urine (GAGs)
- Neurodevelopmental parameters of cognitive, behavioral, and adaptive function:
   ▪ Bayley Scales of Infant and Toddler Development, 3rd Edition (BSID-III) (Bayley, 2005, Scales of Infant and Toddler Development, 3rd Ed., Springer, New York, NY) or Kaufman Assessment Battery for Children, 2nd Edition (KABC-II) (Kaufman, 2004, Kaufman Assessment Battery for Children, 2nd Ed.)
   ▪ Vineland Adaptive Behavior Scales, 2nd Edition, Comprehensive Interview Form (VABS-II) (Sparrow et al., 2005, Vineland Adaptive Behavior Scales, 2nd Ed. )
- Vector concentration in CSF, plasma, and urine by quantitative polymerase chain reaction (PCR) to Construct 1 deoxyribonucleic acid (DNA)

### Exploratory Endpoints:

- Immunogenicity measurements
   o Neutralizing antibody titers to AAV9 and binding antibody titers to I2S in CSF and serum
   o Enzyme-linked immunospot (ELISPOT) assay: T-cell response to AAV9 and I2S
   o Flow cytometry: AAV- and I2S-specific regulatory T cells
- CNS structural abnormalities assessed by magnetic resonance imaging (MRI) of the brain
- Liver and spleen size assessed by MRI and ultrasound of the abdomen
- Auditory capacity changes measured by auditory brainstem response (ABR) testing
- Plasma and urinary GAGs in subjects who temporarily discontinue IV ERT (ELAPRASE^{®})
- PedsQL (Version 4)
- Global impression of sleep scale

The total duration of the study may be 104 weeks post-dose with a primary safety evaluation time point of 24 weeks. Screening may take up to 35 days.

### DIAGNOSIS AND CRITERIA FOR INCLUSION AND EXCLUSION

To be eligible to participate in this study, a subject must meet all the following inclusion criteria:
1. The subject's legal guardian(s) is(are) willing and able to provide written, signed informed consent after the nature of the study has been explained, and prior to any research-related procedures.
2. Is a male
3. Meets one of the following criteria:
   a. Has a documented diagnosis of MPS II *AND* is ≥ 4 months to <5 years of age *AND* a has a neurocognitive testing score > 55 and ≤ 77 (BSID-III or KABC-II), *OR*
   b. Has a documented diagnosis of MPS II *AND* is ≥ 4 months and <5 years of age *AND* has a decline of ≥ 1 standard deviation on sequential neurocognitive testing (BSID-III or KABC-II) and a testing score > 55, *OR*
   c. Has a relative diagnosed with severe MPS II who has the same *IDS* mutation as the subject AND in the opinion of a geneticist has inherited a severe form of MPS II
4. Has sufficient auditory and visual capacity, with or without aids, to complete the required protocol testing, and be compliant with wearing the aid, if applicable, on testing days

Subjects who meet any of the following exclusion criteria will not be eligible to participate in the study:
1. Has a contraindication for an IC injection, including any of the following:
   a. Review of baseline MRI testing by the team of neuroradiologists/neurosurgeons participating in study (1 per site) shows a contraindication for an IC injection
   b. History of prior head/neck surgery, which resulted in a contraindication to IC injection, based on review of available information by the team of neuroradiologists/neurosurgeons participating in study
   c. Has any contraindication to computed tomography (CT), contrast agent, or to general anesthesia
   d. Has any contraindication to MRI or gadolinium
   e. Has estimated glomerular filtration rate (eGFR) <30 mL/min/1.73 m²
2. Has any condition that would contraindicate treatment with prednisone, tacrolimus or sirolimus
3. Has any neurocognitive deficit not attributable to MPS II or diagnosis of a neuropsychiatric condition that may in the opinion of the PI confound interpretation of study results
4. Has any contraindication to lumbar puncture
5. Has a ventricular shunt
6. Has undergone hematopoietic stem cell transplantation (HSCT)
7. Has had prior treatment with an AAV-based gene therapy product
8. Has received idursulfase [ELAPRASE^{®}] via intrathecal (IT) administration
9. Has received idursulfase [ELAPRASE^{®}] IV and experienced a serious hypersensitivity reaction, including anaphylaxis, deemed related to IV idursulfase [ELAPRASE^{®}] administration.
10. Has received any investigational product within 30 days of Day 1 or 5 half-lives before signing of the Informed Consent Form (ICF), whichever is longer
11. Has any history of lymphoma or history of another cancer, other than squamous cell or basal cell carcinoma of the skin, that has not been in full remission for at least 3 months before screening
12. Has a platelet count <100,000 per microliter (µL)
13. Has aminotransferase (ALT) or aspartate aminotransferase (AST) >3 × ULN or total bilirubin >1.5 × ULN at screening unless the subject has a previously known history of Gilbert's syndrome and a fractionated bilirubin that shows conjugated bilirubin <35% of total bilirubin
14. Uncontrolled hypertension (systolic blood pressure [BP] >180 mmHg, diastolic BP >100 mmHg) despite maximal medical treatment
15. Has a history of human immunodeficiency virus (HIV) or hepatitis B or hepatitis C virus infection, or positive screening tests for hepatitis B surface antigen or hepatitis B core antibody, or hepatitis C or HIV antibodies
16. Is a first-degree family member of a clinical site employee or any other individual involved with the conduct of the study or is a clinical site employee or other individual involved with the conduct of the study
17. Has a clinically significant ECG abnormality that, in the opinion of the PI, would compromise the subject's safety
18. Has a serious or unstable medical or psychological condition that, in the opinion of the PI, would compromise the subject's safety or successful participation in the study or interpretation of study results
19. Has uncontrolled seizures that in opinion of the PI would put the subject at undue risk

Exclusion criteria related to immunosuppressive therapy:
20. Has a history of a hypersensitivity reaction to tacrolimus, sirolimus, or prednisone
21. Has a history of a primary immunodeficiency (e.g., common variable immunodeficiency syndrome), splenectomy, or any underlying condition that predisposes the subject to infection
22. Has herpes zoster (VZV), cytomegalovirus (CMV), or Epstein-Barr virus (EBV) infection that has not completely resolved at least 12 weeks prior to screening
23. Has any infection requiring hospitalization or treatment with parenteral anti-infectives not resolved at least 8 weeks prior to Visit 2
24. Has any active infection requiring oral anti-infectives (including antivirals) within 10 days prior to Visit 2
25. Has a history of active tuberculosis (TB) or a positive Quantiferon-TB Gold test during screening
26. Has any live vaccine within 8 weeks prior to signing the ICF
27. Had major surgery within 8 weeks before signing the ICF or major surgery planned during the study period
28. Anticipate the need for adenoidectomy or tonsillectomy within 6 months of enrollment
29. Has an absolute neutrophil count <1.3 × 10³/µL
30. Has any condition or laboratory abnormality that the PI believes would not be appropriate for immunosuppressive therapy

### STATISTICAL METHODS

All data will be presented in subject data listings. Categorical variables will be summarized using frequencies and percentages, and continuous variables will be summarized using descriptive statistics (n, mean, standard deviation, median, minimum, and maximum). Graphical displays will be presented as appropriate. Safety and PD endpoints will be reported by dose group and may also be reported for the 2 dose groups combined.

Sample Size and Power Calculation: No formal calculation was performed to determine sample size.

### 6.5.2. ABBREVIATIONS AND TERMS

**Table 8: Abbreviations and Specialist Terms**

| **Abbreviation** | **Term** |
|---|---|
| AAV | Adeno-associated virus |
| AAV9 | AAV vector of serotype 9 |
| AE(s) | Adverse event(s) |
| ALP | Alkaline phosphatase |
| ALT | Alanine aminotransferase |
| AST | Aspartate aminotransferase |
| BBB | Blood-brain barrier |
| BID | Twice a day |
| BP | Blood pressure |
| BSID | Bayley Scales of Infant and Toddler Development |
| BSL | Biosafety level |
| CB7 | Hybrid C4 and CB (chicken beta actin promoter) |
| CBC | Complete blood count |
| cDNA | Consensus DNA |
| CFR | Code of Federal Regulations |
| CI | Confidence interval |
| CMV | Cytomegalovirus |
| CNS | Central nervous system |
| CoA | Certificate of analysis |
| CRF | Case Report Form |
| CSF | Cerebrospinal fluid |
| CT | Computed tomography |
| CTA | Clinical Trial Agreement |
| CTCAE | Common Terminology Criteria for Adverse Events |
| CZ | Crystal Zenith^{®} |
| DLT(s) | Dose-limiting toxicity(ies) |
| DNA | Deoxyribonucleic acid |
| DRG | Dorsal root ganglia |
| EBV | Epstein-Barr virus |
| ECG | Electrocardiogram |
| EDC | Electronic Data Capture |
| eGFR | Estimated glomerular filtration rate |
| ELISA | Enzyme-Linked Immunosorbent Assay |
| ELISPOT | Enzyme-linked immunospot |
| EOS | End of Study |
| ERT | Enzyme replacement therapy |
| ET | Early Termination |
| FDA | US Food and Drug Administration |
| GAG(s) | Glycosaminoglycan(s) |
| GAN | Giant Axonal Neuropathy |
| GC | Genome copies |
| GCP | Good Clinical Practice |
| GLP | Good Laboratory Practice |
| GM3 | Monosialodihexosylganglioside |
| HDL | High-density lipoprotein |
| Hep | hepatitis |
| Hex | Hexosaminidase |
| hIDS | Human iduronate-2-sulfatase |
| HIPAA | Health Insurance Portability and Accounting Act |
| HIV | Human immunodeficiency virus |
| HSCT | Hematopoietic stem cell transplantation |
| I2S | Iduronate-2-sulfatase |
| IB | Investigator's Brochure |
| IC | Intracisternal(ly) |
| ICF | Informed Consent Form |
| ICH | International Council for Harmonisation |
| ICV | Intracerebroventricular |
| IDMC | Independent Data Monitoring Committee |
| *IDS* | Iduronate-2-sulfatase gene |
| IEC(s) | Independent Ethics Committee(s) |
| IgG | Immunoglobulin G |
| IND | Investigation New Drug |
| IP | Investigational product |
| IQ | Intelligence quotient |
| IRB | Institutional Review Board |
| IS | immune suppression / immunosuppression |
| IT | Intrathecal(ly) |
| ITR(s) | Inverted terminal repeat(s) |
| IV | Intravenous(ly) |
| KABC | Kaufman Assessment Battery for Children |
| KIDS | Kinder Infant Development Scale |
| KSPD | Kyoto Scale of Psychological Development |
| LDL | Low-density lipoprotein |
| LIMP2 | Lysosomal membrane protein |
| MED | Minimum effective dose |
| MedDRA | Medical Dictionary of Regulatory Activities |
| MMF | Mycophenolate mofetil |
| MPS I | Mucopolysaccharidosis type I |
| MPS II | Mucopolysaccharidosis type II |
| MPS III | Sanfilippo syndrome |
| MPS VII | Mucopolysaccharidosis type VII |
| MRI | Magnetic resonance imaging |
| MTD | Maximum tolerated dose |
| mTORC1 | Mammalian/mechanistic target of rapamycin complex 1 |
| N | Number in sample |
| NAB | Neutralizing antibody |
| NCI | National Cancer Institute |
| NHP(s) | Non-human primate(s) |
| NIH | National Institutes of Health |
| NOAEL | No-observable-adverse-effect level |
| PBMC(s) | Peripheral blood mononuclear cell(s) |
| PCR | Polymerase chain reaction |
| PD | Pharmacodynamic(s) |
| PgP | P-glycoprotein |
| PI | Principal Investigator |
| PML | Progressive multifocal leukoencephalopathy |
| PO | By mouth/orally |
| PT | Prothrombin time or Preferred Term |
| PTT | Partial thromboplastin time |
| PVAN | Polyoma virus-associated nephropathy |
| QD | Daily |
| qPCR | Quantitative polymerase chain reaction |
| RBC | Red blood cell |
| RG1 | Risk Group 1 |
| Construct 1 | Recombinant adeno-associated virus serotype 9 capsid containing human iduronate-2-sulfatase expression cassette |
| SAE(s) | Serious adverse event(s) |
| SAP | Statistical analysis plan |
| SDV | Source document verification |
| SMA | Spinal Muscular Atrophy |
| SOC | System Organ Class |
| SRT | Safety review trigger |
| TB | Tuberculosis |
| TEAE(s) | Treatment-emergent adverse event(s) |
| Treg | Regulatory T cell |
| ULN | Upper limit of normal |
| U.S. | United States |
| US | Ultrasound |
| USMs | Urgent safety measures |
| VZV | Varicella zoster virus |
| WBC | White blood cell (count) |
| WHO | World Health Organization |

### 6.5.3. INVESTIGATIONAL PLAN

### ENDPOINTS

### Primary Endpoints

- Safety through Week 24: AEs and SAEs

### Secondary Endpoints

- Safety through Week 104: AE reporting, laboratory evaluations, vital signs, electrocardiograms (ECGs), physical examinations, and neurologic assessments
- Biomarkers in CSF (GAGs, I2S activity), plasma (GAGs, I2S activity), and urine (GAGs)
- Neurodevelopmental parameters of cognitive, behavioral, and adaptive function:
   ▪ Bayley Scales of Infant and Toddler Development, 3rd Edition (BSID-III) (Bayley, 2005, Scales of Infant and Toddler Development, 3rd Ed. ) or Kaufman Assessment Battery for Children, 2nd Edition (KABC-II) (Kaufman, 2004, Kaufman Assessment Battery for Children, 2nd Ed.)
   ▪ Vineland Adaptive Behavior Scales, 2nd Edition, Comprehensive Interview Form (VABS-II) (Sparrow et al., 2005, Vineland Adaptive Behavior Scales, 2nd Ed. )
- Vector concentration in CSF, plasma, and urine by quantitative polymerase chain reaction (PCR) to Construct 1 DNA

### Exploratory Endpoints

- Immunogenicity measurements
   o Neutralizing antibody titers to AAV9 and binding antibody titers to I2S in CSF and serum
   ∘ Enzyme-linked immunospot (ELISPOT) assay: T-cell response to AAV9 and I2S
   ∘ Flow cytometry: AAV- and I2S-specific regulatory T cells
- CNS structural abnormalities assessed by MRI of the brain
- Liver and spleen size assessed by MRI of the abdomen
- Auditory capacity changes measured by auditory brainstem response (ABR) testing
- Plasma and urinary GAGss in subjects who temporarily discontinue IV ERT (ELAPRASE^{®})
- PedsQL (Version 4)
- Global impression of sleep scale

### STUDY DESIGN

This is a Phase I/II, first-in-human, multicenter, open-label, single arm dose escalation study of Construct 1. Approximately 6 pediatric subjects with severe MPS II could be enrolled into 2 dose cohorts, 1.3 × 10¹⁰ GC/g brain mass (Dose 1) or 6.5 × 10¹⁰ GC/g brain mass (Dose 2), and will receive a single dose of Construct 1 administered by IC injection. Safety will be the primary focus for the initial 24 weeks after treatment (primary study period). Following completion of the primary study period, subjects will continue to be assessed (safety and efficacy) for up to a total of 104 weeks following treatment with Construct 1. At the end of the study, all subjects will be invited to participate in a long-term follow-up study.

Potential subjects will be screened up to 35 days prior to dosing to determine eligibility for the study. Those subjects who meet the eligibility criteria will be admitted to the hospital between Day -2 and the morning of Day 1 (according to institutional practice), and baseline assessments will be performed pre-dose. Subjects will receive a single IC dose of Construct 1 on Day 1 and will remain in the hospital for approximately 30 to 36 hours after dosing for observation. Subsequent assessments in the primary study period (i.e., through Week 24) will be performed weekly through Week 4 and at Weeks 8, 12, 16, 20, and 24. After the primary study period, visits will be at Weeks 28, 32, 40, 48, 52, 56, 64, 78, and 104. The Week 12, 40, and 64 visits may be performed by a home health nurse. The Week 20 and 28 assessments will be limited to evaluation of AEs and concomitant therapies by telephone contact.

All subjects will initially receive IS in the study based on findings in the nonclinical studies. IS therapy will include corticosteroids (methylprednisolone 10 mg/kg IV once on Day 1 predose and oral prednisone starting at 0.5 mg/kg/day on Day 2 with gradual tapering and discontinuation by Week 12), tacrolimus (1 mg twice daily [BID] by mouth [PO] Day 2 to Week 24 with target blood level of 4-8 ng/mL and tapering over 8 weeks between Week 24 and 32), and sirolimus (a loading dose of 1 mg/m² every 4 hours x 3 doses on Day -2 and then from Day -1: sirolimus 0.5 mg/m²/day divided in twice a day dosing with target blood level of 4-8 ng/ml until Week 48). Neurologic assessments and tacrolimus/sirolimus blood level monitoring will be conducted. The doses of sirolimus and tacrolimus will be adjusted to maintain blood levels in the target range.

No IS therapy is planned after Week 48. If IS is required after Week 48 to control a clinically relevant immune response, the appropriate immunosuppressive regimen will be determined by the principal investigator (PI), in discussion with the Medical Monitor and Sponsor, as clinically indicated.

The safety and tolerability of Construct 1 will be monitored through assessment of AEs and serious adverse events (SAEs), chemistry, hematology, urinalysis, markers of CSF inflammation, immunogenicity, vector shedding (vector concentration), vital signs, electrocardiograms (ECGs), and physical examinations including neurological assessments.

Efficacy assessments will include neurocognitive and adaptive function, auditory capacity, brain MRI, liver and spleen size, measurements of levels of PD biomarkers in CSF, plasma, and urine.

### 6.5.4. SUBJECT POPULATION AND SELECTION

### SELECTION OF STUDY POPULATION

Approximately 6 pediatric subjects ages ≥ 4 months to < 5 years who have documented neurocognitive deficits due to MPS II or who have a genotype and family history consistent with an inherited form of severe MPS II will be treated with investigational product (IP).

### INCLUSION CRITERIA

To be eligible to participate in this study, a subject must meet all the following criteria:
1. The subject's legal guardian is (are) willing and able to provide written, signed informed consent after the nature of the study has been explained, and prior to any research-related procedures.
2. Is a male
3. Meets one of the following criteria:
   a. Has a documented diagnosis of MPS II *AND* is ≥ 4 months to < 5 years of age *AND* a has a neurocognitive testing score > 55 and ≤ 77 (BSID-III or KABC-II), *OR*
   b. Has a documented diagnosis of MPS II *AND* is ≥ 4 months and < 5 years of age *AND* has a decline of ≥ 1 standard deviation on sequential neurocognitive testing (BSID-III or KABC-II) and a testing score > 55, *OR*
   c. Has relative diagnosed with severe MPS II carrying the same *IDS* mutation as the subject AND in the opinion of a geneticist has inherited a severe form of MPS II
4. Has sufficient auditory and visual capacity, with or without aids, to complete the required protocol testing, and be compliant with wearing the aid, if applicable, on testing days.

### EXCLUSION CRITERIA

A subject who meets any of the following exclusion criteria will not be eligible to participate in the study:
1. Has a contraindication for an IC injection, including any of the following:
   a. Review of baseline MRI testing by the team of neuroradiologists/neurosurgeons participating in study (1 per site) shows a contraindication for an IC injection
   b. History of prior head/neck surgery, which resulted in a contraindication to IC injection, based on review of available information by the team of neuroradiologists/neurosurgeons participating in study
   c. Has any contraindication to computed tomography (CT), contrast agent or general anesthesia
   d. Has any contraindication to MRI or gadolinium
   e. Has estimated glomerular filtration rate (eGFR) <30 mL/min/1.73 m²
2. Has any condition that would contraindicate treatment with prednisone, tacrolimus, or sirolimus.
3. Has any neurocognitive deficit not attributable to MPS II or diagnosis of a neuropsychiatric condition that may in the opinion of the PI confound interpretation of study results.
4. Has any contraindication to lumbar puncture
5. Has a ventricular shunt
6. Has undergone hematopoietic stem cell transplantation (HSCT).
7. Has had prior treatment with an AAV-based gene therapy product.
8. Has received idursulfase via intrathecal (IT) administration
9. Has received IV idursulfase [ELAPRASE^{®}] and experienced a serious hypersensitivity reaction, including anaphylaxis, deemed related to IV idursulfase [ELAPRASE^{®}] administration.
10. Has received any investigational product within 30 days of Day 1 or 5 half-lives before signing of the Informed Consent Form (ICF), whichever is longer
11. Has any history of lymphoma or history of another cancer, other than squamous cell or basal cell carcinoma of the skin, that has not been in full remission for at least 3 months before screening.
12. Platelet count <100,000 per microliter (µL)
13. Has aminotransferase (ALT) or aspartate aminotransferase (AST) >3 × ULN or total bilirubin >1.5 × ULN at screening unless the subject has a previously known history of Gilbert's syndrome and a fractionated bilirubin that shows conjugated bilirubin <35% of total bilirubin.
14. Uncontrolled hypertension (systolic blood pressure [BP] >180 mmHg, diastolic BP > 100 mmHg) despite maximal medical treatment.
15. Has a history of human immunodeficiency virus (HIV) or hepatitis B or hepatitis C virus infection, or positive screening tests for hepatitis B surface antigen or hepatitis B core antibody, or hepatitis C or HIV antibodies.
16. Is a first-degree family member of a clinical site employee or any other individual involved with the conduct of the study or is a clinical site employee or other individual involved with the conduct of the study.
17. Has a clinically significant ECG abnormality that, in the opinion of the PI, would compromise the subject's safety.
18. Has a serious or unstable medical or psychological condition that, in the opinion of the PI, would compromise the subject's safety or successful participation in the study or interpretation of study results.
19. Has uncontrolled seizures that in opinion of the PI would put the subject at undue risk.

Exclusion criteria related to immunosuppressive therapy:
20. A history of a hypersensitivity reaction to tacrolimus, sirolimus, or prednisone
21. A history of a primary immunodeficiency (e.g., common variable immunodeficiency syndrome), splenectomy, or any underlying condition that predisposes the subject to infection
22. Herpes zoster (VZV), cytomegalovirus (CMV), or Epstein-Barr virus (EBV) infection that has not completely resolved at least 12 weeks prior to screening
23. Any infection requiring hospitalization or treatment with parenteral anti-infectives not resolved at least 8 weeks prior to Visit 2
24. Any active infection requiring oral anti-infectives (including antivirals) within 10 days prior to Visit 2
25. History of active tuberculosis (TB) or a positive Quantiferon-TB Gold test during screening
26. Any live vaccine within 8 weeks prior to signing the ICF
27. Major surgery within 8 weeks before signing the ICF or major surgery planned during the study period
28. Anticipate the need for adenoidectomy or tonsillectomy within 6 months of enrollment
29. Absolute neutrophil count <1.3 × 10³/µL
30. Any condition or laboratory abnormality that the PI believes would not be appropriate for immunosuppressive therapy

### 6.5.5. TREATMENTS

### TREATMENTS ADMINISTERED

The investigational product (IP), Construct 1 (see FIG. 5), will be given as a single dose IC administration. Two dose levels: 1.3 × 10¹⁰ GC/g brain mass (Dose 1) or 6.5 × 10¹⁰ GC/g brain mass (Dose 2). Total dose administered (total GC) will be adjusted to account for differences in brain size by age. Total volume of product administered will not exceed 5 mL.

No reference therapy will be administered during this study. IS therapy will be given in addition to IP, as described below.

### INVESTIGATIONAL PRODUCT

Construct 1 is a non-replicating recombinant AAV of serotype 9 capsid containing an hIDS expression cassette. See paragraph [0019].

| | |
|---|---|
| **Product** | Construct 1 |
| **Gene** | hIDS |
| **Control Elements:** | CB7 promoter, chicken beta actin intron, rabbit beta-globin polyadenylation signal |
| **AAV** | 9 |

| | |
|---|---|
| AAV = adeno-associated virus; CB = chicken beta-actin; hIDS = human iduronate-2-sulfatase | |

Construct 1 is a non-replicating recombinant AAV9 vector that allows for efficient expression of the human iduronate-2-sulfatase (hIDS) product in the central nervous system (CNS) following intrathecal (IT) administration. The vector genome contains an hIDS expression cassette flanked by AAV2 inverted terminal repeats (ITRs). Expression from the cassette is driven by a CB7 promoter, a hybrid between a cytomegalovirus (CMV) immediate-early enhancer and the chicken β-actin promoter. Transcription from this promoter is enhanced by the presence of the chicken β-actin intron (CI). The polyadenylation signal for the expression cassette is from the rabbit β-globin (RBG) gene. A schematic representation of Construct 1 is illustrated in FIG. 5.

The final IP is supplied as a frozen solution of the AAV vector active ingredient (AAV9.CB7.hIDS) in modified Elliotts B^{®} solution with 0.001% Pluronic^{®} F68, filled into 2-mL in CRYSTAL ZENITH^{®} (CZ) vials, and sealed with a latex- free rubber stopper and aluminum flip-off seal. Vials should be stored at ≤ -60°C. The concentration (in GC/mL) of each IP lot will be reported in the Certificate of Analysis (CoA). Detailed dosing instructions, based on the product concentration, will be provided in the Administration Manual.

### IMMUNOSUPPRESSIVE THERAPY

### Corticosteroids

- In the morning of vector administration (Day 1 predose), subjects will receive methylprednisolone 10mg/kg IV (maximum of 500 mg) over at least 30 minutes. The methylprednisolone should be administered before the lumbar puncture and IC injection of IP. Premedication with acetaminophen and an antihistamine is optional and at the discretion of the investigator.
- On Day 2, oral prednisone will be started with the goal to discontinue prednisone by Week 12. The dose of prednisone will be as follows:
   ∘ Day 2 to the end of Week 2: 0.5 mg/kg/day
   ∘ Week 3 and 4: 0.35 mg/kg/day
   ∘ Week 5-8: 0.2 mg/kg/day
   ∘ Week 9-12: 0.1 mg/kg
   ∘ Prednisone will be discontinued after Week 12. The exact dose of prednisone can be adjusted to the next higher clinically practical dose.

### Sirolimus

- 2 days prior to vector administration (Day -2): a loading dose of sirolimus 1 mg/m² every 4 hours x 3 doses will be administered
- From Day -1: sirolimus 0.5 mg/m²/day divided in twice a day dosing with target blood level of 4-8 ng/ml
- Sirolimus will be discontinued after the Week 48 visit.

### Tacrolimus

- Tacrolimus will be started on Day 2 (the day following IP administration) at a dose of 1 mg twice daily and adjusted to achieve a blood level 4-8 ng/mL for 24 Weeks.
- Starting at Week 24 visit, tacrolimus will be tapered off over 8 weeks. At Week 24 the dose will be decreased by approximately 50%. At Week 28 the dose will be further decreased by approximately 50%. Tacrolimus will be discontinued at Week 32.
- Tacrolimus and sirolimus blood level monitoring will be conducted. Dosing adjustments are discussed in the disclosure (e.g., in paragraphs [00124] - [00125]; [00220] - [00222]; Section 6).

### METHOD OF ASSIGNING SUBJECTS TO TREATMENT

Eligible subjects will be enrolled and assigned sequentially to a dose cohort with the initial 3 subjects assigned to get 1.3 × 10¹⁰ GC/g brain mass; the subsequent 3 subjects will be assigned to get 6.5 × 10¹⁰ GC/g brain mass pending review of safety data by the IDMC.

### DOSING CONSIDERATIONS

### INVESTIGATIONAL PRODUCT

A description of the plan to sequentially dose subjects, including review of safety data between individual subjects and after each cohort has been dosed at any dose level is disclosed in the disclosure (e.g., [00237] to [00265]; [00175] to [00186]).

### IMMUNOSUPPRESSIVE THERAPY

Prednisone dosing will start at 0.5 mg/kg/day and will be gradually tapered off by the Week 12 visit.

Tacrolimus dose adjustments will be made to maintain whole blood trough concentrations within 4 to 8 ng/mL for the first 24 Weeks. At Week 24 the dose will be decreased by approximately 50%. At Week 28 the dose will be further decreased by approximately 50%. Tacrolimus will be discontinued at Week 32. Sirolimus dose adjustments will be made to maintain whole blood trough concentrations within 4 to 8 ng/mL. In most subjects, dose adjustments can be based on the equation: new dose = current dose × (target concentration/current concentration). Subjects should continue on the new maintenance dose for at least 7 to 14 days before further dosage adjustment with concentration monitoring.

The following medications and procedures are prohibited:
- No IT ERT is allowed within 6 months of screening.
- Any investigational product within the 30 days or 5 half-lives, whichever is longer, prior to signing the ICF or at any time during the study (through Week 104)
- Live vaccines should be avoided while on sirolimus and/or tacrolimus
- Strong inhibitors of CYP3A4 and/or P-glycoprotein (PgP) (such as ketoconazole, voriconazole, itraconazole, posaconazole, erythromycin, telithromycin or clarithromycin) or strong inducers of CYP3A4 and or Pgp (such as rifampin or rifabutin) should be avoided while on sirolimus and/or tacrolimus
- Grapefruit juice inhibits CYP3A-enzymes resulting in increased tacrolimus and sirolimus whole blood trough concentrations. Subjects should avoid eating grapefruit or drinking grapefruit juice with tacrolimus and/or sirolimus.

### PERMITTED MEDICATIONS AND PROCEDURES

Subjects will be permitted to remain on a stable regimen of IV ERT as well as any supportive measures (e.g., physical therapy). According to local hospital standard of care, subjects will be permitted to receive medication to prevent claustrophobia during MRI and receive general anesthesia for lumbar puncture, MRI, and neuroconduction studies (ABRs or sensory evoked potentials).

Medications other than that described above, which are considered necessary for the subject's safety and wellbeing (e.g., for hypertension), may be given at the discretion of the Investigator in accordance with local standard of care and recorded in the appropriate sections of the CRF.

### 6.6 EXAMPLE 6: A Phase I/II Multicenter, Open-Label Study to Evaluate the Safety, Tolerability, and Pharmacodynamics of Construct 1 in Pediatric Subjects with MPS II (Hunter Syndrome)

As described above, Construct 1 is AAV9.CB7.hIDS (recombinant adeno-associated virus serotype 9 capsid containing human iduronate-2-sulfatase expression cassette). *See* paragraph [0019], FIG. 5, and Section 5.5 (Example 5).

Construct 1 will be delivered as a single intracisternal (IC) or intracerebroventricular (ICV) dose.

Two dose levels will be evaluated, 1.3 × 10¹⁰ genome copies (GC)/g brain mass (Dose 1) and 6.5 × 10¹⁰ GC/g brain mass (Dose 2). Total dose administered will account for estimated brain mass of study subjects based on their screening magnetic resonance imaging (MRI). The total volume of product administered will not exceed 10% of the total CSF volume (estimated to be ~50 mL in infant brain and ~150 mL in adult brain).

### 6.6.1. OBJECTIVES:

### PRIMARY OBJECTIVE

- To evaluate the safety and tolerability of Construct 1 through 24 weeks following a single IC, or ICV if IC is contraindicated, dose administered to pediatric subjects who have severe MPS II

### SECONDARY OBJECTIVES:

- To evaluate the long-term safety and tolerability of Construct 1
- To evaluate the effect of Construct 1 on biomarkers in cerebrospinal fluid (CSF), plasma, and urine
- To evaluate the effect of Construct 1 on neurodevelopmental parameters of cognitive, behavioral, and adaptive function
- To evaluate vector shedding in CSF, serum, and urine

### EXPLORATORY OBJECTIVES:

- To evaluate immunogenicity of Construct 1
- To explore the effect of Construct 1 on CNS imaging
- To explore the effect of Construct 1 on systemic manifestations of disease
- To explore the effect of Construct 1 on auditory capacity
- To explore the effect of Construct 1 on biomarkers in plasma and urine in subjects who temporarily discontinue intravenous (IV) ERT (ELAPRASE^{®})
- To explore the effect of Construct 1 on quality of life (QOL)
- To explore the effect of Construct 1 on sleep measures
- To explore the effect of Construct 1 on clinician reported outcome
- To explore the effect of Construct 1on caregiver reported outcome

### 6.6.2. STUDY DESIGN AND METHODOLOGY

This is a Phase I/II, first-in-human, multicenter, open-label, single arm dose escalation study of Construct 1. No control group is included. Approximately 6 pediatric subjects who have severe MPS II could be enrolled into 2 dose cohorts, 1.3 × 10¹⁰ GC/g brain mass (Dose 1) or 6.5 × 10¹⁰ GC/g brain mass (Dose 2) and will receive a single dose of Construct 1 administered by IC or ICV injection. Safety will be the primary focus for the initial 24 weeks after treatment (primary study period). Following completion of the primary study period, subjects will continue to be assessed (safety and efficacy) for up to a total of 104 weeks following treatment with Construct 1. At the end of the study, subjects will be invited to participate in a long-term follow-up study.

The first 3 eligible subjects will be enrolled into the Dose 1 cohort (1.3 × 10¹⁰ GC/g brain mass). After Construct 1 administration to the first subject, there will be an 8-week observation period for safety. The Internal Safety Committee (ISC) will review the safety data obtained during the first 8 weeks of the study according to the ISC Charter (including data obtained during the Week 8 visit) for this subject, and if there are no safety concerns, the 2nd subject may be enrolled. The same process will be used to enroll the 3rd subject. Informed consent and screening activities for the next subject may proceed during the observation period for the preceding subject.

If no safety review trigger (SRT) event is observed, all available safety data for the Dose 1 cohort obtained up to and including the Week 8 visit for the 3rd subject will be evaluated by the Independent Data Monitoring Committee (IDMC). If the decision is to proceed to the second dose cohort (6.5 × 10¹⁰ GC/g brain mass), the subsequent 2 subjects will follow the same dosing scheme as the initial dose cohort. The ISC will review all subject safety data obtained up to and including the Week 2 visit of the 2nd subject in the Dose 2 cohort and may determine that it is safe to proceed with dosing of the 3rd subject immediately after this assessment. All available safety data for the Dose 2 cohort will be evaluated by the IDMC after the Week 8 visit for the 3rd subject in the Dose 2 cohort. With approval of the IDMC, additional subjects may be dosed in a Dose 2 Expanded Cohort as long as study drug is available, and there is Sponsor approval and no safety event that warrants suspension of enrollment as per either the ISC or the IDMC. Each subject in the Expanded Cohort will be dosed in a staggered fashion at intervals of at least 2 weeks.

At any given IDMC meeting, whether planned at the conclusion of a dose cohort or called for by an SRT, the IDMC may recommend stopping the trial, dose additional subjects at the current dose, proceed to the next dose cohort, or proceed at a lower dose. Once 8 weeks of data are available from the last Dose 2 cohort subject, and if none of the Dose 2 cohort subjects had an SRT event, then enrollment in the study will be considered completed; enrollment into the Expanded Cohort may continue as stipulated above. Once the last subject enrolled in the Expanded Cohort completes the Week 2 visit, all safety data for the Expanded Cohort including data from the last subject's Week 2 visit will be evaluated by the IDMC.

If any event meets the criteria of a Stopping Rule, dosing of any new subjects will be suspended until a complete review of all safety data by REGENXBIO and the external IDMC has been performed.

Potential subjects will be screened up to 35 days prior to dosing to determine eligibility for the study; screening assessments performed after signing the Informed Consent Form (ICF) but outside of this window may be acceptable as determined by the principal investigator and approved by the Medical Monitor. Assessments performed outside the screening window will be repeated as deemed necessary by the Medical Monitor. Subjects may be rescreened for the study one time if initially failing to enroll; Sponsor approval to rescreen the subject will be required. Rescreening can occur after at least 3 months have elapsed from the time of the subject's initial screen failure.

Those subjects who meet the eligibility criteria will be admitted to the hospital between Day -2 and the morning of Day 1 (according to institutional practice), and baseline assessments will be performed pre-dose. Subjects will receive a single IC or ICV dose of Construct 1 on Day 1 and will remain in the hospital overnight and for approximately 1-2 days after dosing for observation. Subjects will be discharged after the principal investigator concludes that prolongation of hospitalization beyond two overnight stays is not necessary. Subsequent assessments in the primary study period (i.e., through Week 24) will be performed weekly through Week 4 and at Weeks 8, 12, 16, 20, and 24. After the primary study period, visits will be at Weeks 28, 32, 40, 48, 52, 56, 60, 64, 78, and 104. The Week 64 visit will be performed only for subjects who discontinue IV ERT. The Week 20 and 28 assessments will be limited to evaluation of adverse events (AEs) and concomitant therapies by telephone contact.

All subjects will initially receive immune suppression (IS) in the study to minimize the risk of any immune mediated reaction against tissues expressing the transgene as well as minimize any risk associated with the formation or increase of antibodies to *IDS* which may decrease efficacy. The IS regimen will include corticosteroids (methylprednisolone 10 mg/kg IV once on Day 1 predose and oral prednisone starting at 0.5 mg/kg/day on Day 2 with gradual tapering and discontinuation by Week 12), tacrolimus (0.05 mg/kg twice daily [BID] by mouth [PO] Day 2 to Week 24 with dose adjustments made to obtain a target blood level of 2-4 ng/ml and tapering over 8 weeks between Week 24 and 32) and sirolimus (a loading dose of 1 mg/m² every 4 hours x 3 doses on Day -2 and then from Day -1: sirolimus 0.5 mg/m²/day divided in BID dosing with target blood level of 1-3 ng/ml until Week 48). Neurologic assessments and tacrolimus/sirolimus blood level monitoring will be conducted. The doses of sirolimus and tacrolimus will be adjusted to maintain blood levels in the target range.

No IS therapy is planned after Week 48. If IS were required after Week 48 to control a clinically-relevant immune response, the appropriate immunosuppressive regimen will be determined by the principal investigator (PI), in discussion with the Medical Monitor and Sponsor, as clinically indicated.

Given the histopathological findings in the dorsal root ganglia and associated axonopathy observed in the nonclinical safety/toxicology studies and the potential safety risks with the IC administration procedure, close neurological monitoring, including focused neurological assessments and somatosensory evoked potential (SSEP) testing will be employed.

Since animal data suggest that there may be systemic benefits of IC and ICV Construct 1 administration, subjects who are on IV idursulfase (ELAPRASE^{®}) may be offered the option to discontinue ERT after the Week 52 visit. The decision to discontinue ERT will be at the clinical judgement of the PI and as agreed with the study sponsor. Additional information that may be useful for the decision to stop ERT are trough measurements (based on ERT dosing) of plasma I2S and plasma and urine GAGs up to the Week 52 visit, and measurement of the liver and spleen size by ultrasound. The Week 52, 56, 60, 64 and 78 visits will include additional monitoring of the subject's plasma I2S and plasma and urine GAGs levels in subjects who elect to discontinue ERT. Subjects who discontinue IV ERT will have an additional abdominal ultrasound at Week 64 to perform measurement of the liver and spleen size. IV ERT will be restarted if any of following criteria are met: increase in urinary GAGs levels 2 times above the level measured at the Week 52 visit, or an increase of liver diameter > 20% above the Week 52 value, or any change in other safety parameters deemed by the internal safety committee and/or the IDMC to warrant a restart of IV ERT. However, subjects may restart ERT at any time, if deemed necessary by the PI.

The safety and tolerability of Construct 1 will be monitored through assessment of AEs and serious adverse events (SAEs), chemistry, hematology, urinalysis, markers of CSF inflammation, immunogenicity, vector shedding (vector concentration), vital signs, electrocardiograms (ECGs), SSEP testing, and physical examinations including neurological assessments. Serial PCR (polymerase chain reaction) for detection of circulating viral genomes (EBV and CMV) will also be performed while subjects are receiving IS.

Efficacy assessments will include measurements of levels of pharmacodynamic (PD) biomarkers (GAGs and I2S in CSF and plasma, leukocyte I2S enzyme activity, and GAGs in urine), as well as on neurocognitive function, auditory capacity, brain MRI, liver and spleen size, and cardiac evaluation by echocardiogram. Neurocognitive or adaptive assessments performed as part of subjects' standard of care while participating in the trial may also be collected, as determined by the study sponsor after discussing with the site.

### 6.6.3. ENDPOINTS

### Primary Endpoints:

- Safety through Week 24: AEs and SAEs

### Secondary Endpoints:

- Safety through Week 104: AE reporting, laboratory evaluations, vital signs, ECGs, physical examinations, and neurologic assessments
- Biomarkers in CSF (GAGs, I2S), plasma (GAGs, I2S), and urine (GAGs)
- Neurodevelopmental parameters of cognitive, behavioral, and adaptive function:
   o Bayley Scales of Infant and Toddler Development, 3rd Edition (BSID-III) (Bayley, 2005, Scales of Infant and Toddler Development, 3rd Ed. ) or Kaufman Assessment Battery for Children, 2nd Edition (KABC-II) (Kaufman, 2004, Kaufman Assessment Battery for Children, 2nd Ed.)
   o Vineland Adaptive Behavior Scales, 2nd Edition, Expanded Interview Form (VABS-II) (Sparrow et al., 2005, Vineland Adaptive Behavior Scales, 2nd Ed. )
- Vector concentration in CSF, serum, and urine by quantitative polymerase chain reaction (PCR) to Construct 1 deoxyribonucleic acid (DNA)

### Exploratory Endpoints:

- Immunogenicity measurements
   o Neutralizing antibody titers to AAV9 and binding antibody titers to I2S in CSF and serum
   o Enzyme-linked immunospot (ELISPOT) assay: T-cell response to AAV9 and I2S
   o Flow cytometry: AAV- and I2S-specific regulatory T cells
- CNS structural abnormalities assessed by MRI of the brain
- Liver and spleen size assessed by ultrasound of the abdomen
- Cardiac evaluation by echocardiogram for valvular disease and left ventricular mass index
- Auditory capacity changes measured by auditory brainstem response (ABR) testing or behavioral audiometry and otoacoustic emissions testing
- Plasma and urinary GAGs in subjects who temporarily discontinue IV ERT (ELAPRASE^{®})
- PedsQL
- Sleep assessment (Ferreira et al., 2009, Sleep Medicine;10(4):457-463)
- Clinical Assessment of Disease
- Caregiver Assessment of Disease
- Activities of Daily Living (Tanjuakio et al. 2015, Mol Genet Metab, 114(2):161-169; Kato et al., 2007, Brain Dev, 29(5): 298-305)
- Burden of Illness

### 6.6.4. NUMBER OF SUBJECTS PLANNED AND STUDY DURATION

Up to 12 subjects will be enrolled.
- 3 subjects in Dose 1 cohort
- 3 subjects in Dose 2 cohort
- Up to 6 subjects in Dose 2 Expanded Cohort
- The IDMC may recommend adding additional subjects.

The total duration of the study will be 104 weeks post-dose with a primary safety evaluation time point of 24 weeks. Screening may take up to 35 days.

### 6.6.5. DIAGNOSIS AND CRITERIA FOR INCLUSION AND EXCLUSION:

The following information about the affected relative will be collected, if available, after relative informed consent has been obtained:
- Demographics (age, sex)
- Medical and surgical histories (e.g., cardiac disease, carpal tunnel syndrome, hip dysplasia or subluxation, etc.)
- Age at diagnosis of severe MPS II and how diagnosis was made
- Genotyping results
- Results of neurodevelopmental testing, including cognition, speech and language, fine motor and gross motor (if available and diagnosis of severe MPS II was done through neurodevelopmental testing)
- Results of brain MRI
- Treatment for MPS II, including ERT or HSCT
- Current concomitant medications

Subjects may be rescreened for the study one time if initially failing to enroll; Sponsor approval to rescreen the subject will be required. Rescreening can occur after at least 3 months have elapsed from the time of the subject's initial screen failure. Participants who are rescreened are required to sign a new ICF. All screening procedures will be repeated, with the exception of MRI, for which retesting will be decided by the neuroradiologist/neurosurgeon.

To be eligible to participate in this study, a subject must meet all the following inclusion criteria:
- 1. The subject's legal guardian(s) is (are) willing and able to provide written, signed informed consent after the nature of the study has been explained, and prior to any research-related procedures.
- 2. Is a male ≥ 4 months to <5 years of age
- 3. Meets any of the following criteria:
   o a. Has a documented diagnosis of MPS II AND has a neurocognitive testing score ≤ 77 (BSID-III or KABC-II), OR
   o b. Has a documented diagnosis of MPS II AND has a decline of ≥ 1 standard deviation on serial neurocognitive testing administered between 3 to 36 months apart (BSID-III or KABC-II), OR
   o c. Has a relative clinically diagnosed with severe MPS II who has the same IDS mutation as the subject AND the subject in the opinion of a geneticist has inherited a severe form of MPS II, OR
   o d. Has documented mutation(s) in IDS that in the opinion of a geneticist is always known to result in a neuronopathic phenotype [e.g., a complete deletion or large deletion (e.g., spanning ≥ 1 exon), or recombination] AND in the opinion of a clinician has a severe form of MPS II
- 4. Has sufficient auditory and visual capacity, with or without aids, to complete the required protocol testing, and be compliant with wearing the aid, if applicable, on testing days

Subjects who meet any of the following exclusion criteria will not be eligible to participate in the study:
- 1. Has a contraindication for an IC or ICV injection, including any of the following:
   o a. Review of baseline MRI testing by the team of neuroradiologists/neurosurgeons participating in study (1 neuroradiologist or neurosurgeon per site) shows a contraindication for an IC or an ICV injection
   ∘ b. History of prior head/neck surgery, which resulted in a contraindication to both IC and ICV injection, based on review of available information by the team of neuroradiologists/neurosurgeons participating in study
   ∘ c. Has any contraindication to computed tomography, contrast agent, or to general anesthesia
   o d. Has any contraindication to MRI or gadolinium
   o e. Has renal insufficiency as determined by an estimated glomerular filtration rate (eGFR) <30 mL/min/1.73 m², based on creatinine. If laboratory determines that creatinine is less than the lower limit of assay validation or detection then the lowest limit cutoff value will be used to estimate eGFR
   o f. Has previously experienced a clinically significant intracranial bleed that, in the opinion of the investigator and team of neuroradiologists/neurosurgeons, is a contraindication to IC and ICV injection
- 2. Has any condition that would contraindicate treatment with prednisone, tacrolimus or sirolimus
- 3. Has any neurocognitive deficit not attributable to MPS II or diagnosis of a neuropsychiatric condition that may in the opinion of the PI confound interpretation of study results
- 4. Has any contraindication to lumbar puncture
- 5. Has a (cerebral) ventricular shunt that in the opinion of the site neuroradiologist/neurosurgeon and through discussion with the Medical Monitor, may impact the administration and proper dosing of the subject
- 6. Has undergone HSCT
- 7. Has had prior treatment with an AAV-based gene therapy product
- 8. Has received idursulfase (ELAPRASE^{®}) via intrathecal (IT) administration within 4 months of signing the ICF
- 9. Has received idursulfase (ELAPRASE^{®}) IV and experienced a serious hypersensitivity reaction, including anaphylaxis, deemed related to IV idursulfase (ELAPRASE^{®}) administration. If currently receiving IV idursulfase and has had dose interruptions or a dose regimen different than weekly, then discussion with REGENXBIO Medical Monitor is needed.
- 10. Has received any investigational product within 30 days of Day 1 or 5 half-lives before signing of the ICF, whichever is longer
- 11. Has any history of lymphoma or history of another cancer, other than squamous cell or basal cell carcinoma of the skin, that has not been in full remission for at least 1 year before screening
- 12. Has a platelet count <100,000 per microliter (µL)
- 13. Has aminotransferase (ALT) or aspartate aminotransferase (AST) >3 × ULN or total bilirubin >1.5 × ULN at screening unless the subject has a previously known history of Gilbert's syndrome
- 14. Uncontrolled hypertension despite medical treatment, defined for children ≤ 17 years of age to be systolic blood pressure or diastolic blood pressure > 99th percentile plus 5mmHg based on normative standards for age, sex, and height
- 15. Has a history of human immunodeficiency virus (HIV) or hepatitis B or hepatitis C virus infection, or positive screening tests for hepatitis B surface antigen or hepatitis B core antibody, or hepatitis C or HIV antibodies
- 16. Is a first-degree family member of a clinical site employee or any other individual involved with the conduct of the study or is a clinical site employee or other individual involved with the conduct of the study
- 17. Has a clinically significant ECG abnormality that, in the opinion of the PI, would compromise the subject's safety
- 18. Has a serious or unstable medical or psychological condition that, in the opinion of the PI, would compromise the subject's safety or successful participation in the study or interpretation of study results
- 19. Has uncontrolled seizures that in opinion of the PI would put the subject at undue risk

Exclusion criteria related to immunosuppressive therapy:
- 20. Has a history of a hypersensitivity reaction to tacrolimus, sirolimus, or prednisone
- 21. Has a history of a primary immunodeficiency (e.g., common variable immunodeficiency syndrome), splenectomy, or any underlying condition that predisposes the subject to infection
- 22. Has herpes zoster (VZV), cytomegalovirus (CMV), or Epstein Barr virus (EBV) infection that has not completely resolved at least 12 weeks prior to screening
- 23. Has any infection requiring hospitalization or treatment with parenteral anti-infectives not resolved at least 8 weeks prior to Visit 2
- 24. Has any active infection requiring oral anti-infectives (including antivirals) within 10 days prior to Visit 2
- 25. Has a history of active tuberculosis (TB) or a positive Quantiferon-TB Gold test during screening
- 26. Has received any live vaccine within 4 weeks prior to Day -2
- 27. Had major surgery within 8 weeks before signing the ICF or major surgery planned during the study period
- 28. Anticipate the need for adenoidectomy or tonsillectomy within 6 months of enrollment. If adenoidectomy or tonsillectomy is anticipated it should be performed prior to screening.
- 29. Has an absolute neutrophil count <1.0 × 10³/µL
- 30. Has any condition or laboratory abnormality that the PI believes would not be appropriate for immunosuppressive therapy

### 6.6.6. PROPOSED DOSE

Construct 1 will be preferentially administered as a single IC injection, or as a single ICV injection should IC administration prove difficult or potentially unsafe, to allow direct delivery of the vector to the target tissue within the confined CSF compartment. Although cervical puncture (C1-C2) is a routine clinical procedure used for contrast administration for myelography, image-assisted suboccipital puncture is proposed as the IC clinical route of administration. This replicates the route of administration used in the nonclinical studies and is considered advantageous over the C1-C2 puncture in the intended patient population because patients with MPS II have a high incidence of abnormal narrowing of the C1-C2 IT space, which substantially increases the risks associated with a C1-C2 puncture. Prior to the procedure, each subject will have a magnetic resonance imaging (MRI) of the area reviewed by a team of neuroradiologists/neurosurgeons participating in the study. If it is not considered safe to proceed with an IC injection, then the subject will be considered for ICV injection. ICV injection is a commonly used route for ventriculoperitoneal shunt placement in pediatric and adult individuals and, more recently, CNS drug administration (Drake et al., 2000, Childs Nerv Syst. 16(10-11):800-804; Cohen et al., 2017, Pediatric Neurology, 67:23-25; Slavc et al., 2018, Mol Genetics and Metabolism, 124:184-188). Image-assisted single ICV injection, as proposed in this protocol, is comparable to stereotactic brain biopsy, which has also become a routine neurosurgical intervention with the advent of precise MRI and computed tomography (CT) technology.

From pharmacology studies conducted in MPS II mice with Construct 1, it has been shown that the biodistribution and transgene expression profiles of AAV9 vector-based products are comparable for ICV and IC routes, supporting the use of ICV as an alternative route of administration should IC administration prove difficult or potentially unsafe. Further details of the IC and ICV procedures are outlined in their respective Administration Manual.

The total volume of product administered will not exceed 10% of the total CSF volume (estimated to be ~50 mL in infant brain and ~150 mL in adult brain).

Because of the relatively rapid brain growth that occurs early in a developing child, the total dose of Construct 1 administered IC or ICV depends on the estimated brain mass derived from the study subject's screening MRI. The study subject's estimated brain volume from their MRI will be converted to brain mass and used to calculate the exact dose to be administered, as presented in Table 5, Section 5.3.2.

### 6.6.7. IMMUNOSUPPRESSIVE THERAPY

### Corticosteroids

- In the morning of vector administration (Day 1 predose), subjects will receive methylprednisolone 10mg/kg IV (maximum of 500 mg) over at least 30 minutes. The methylprednisolone should be administered before the lumbar puncture and IC or ICV injection of IP. Premedication with acetaminophen and an antihistamine is optional and at the discretion of the investigator.
- On Day 2, oral prednisone will be started with the goal to discontinue prednisone by Week 12. The dose of prednisone will be as follows:
   ∘ Day 2 to the end of Week 2: 0.5 mg/kg/day
   ∘ Week 3 and 4: 0.35 mg/kg/day
   ∘ Week 5-8: 0.2 mg/kg/day
   ∘ Week 9-12: 0.1 mg/kg
   ∘ Prednisone will be discontinued after Week 12. The exact dose of prednisone can be adjusted to the next higher clinically practical dose.

### Sirolimus

- 2 days prior to vector administration (Day -2): a loading dose of sirolimus 1 mg/m² every 4 hours x 3 doses will be administered
- From Day -1: sirolimus 0.5 mg/m²/day divided in twice a day dosing with target blood level of 1-3 ng/ml
- Sirolimus will be discontinued after the Week 48 visit.

### Tacrolimus

- Tacrolimus will be started on Day 2 (the day following IP administration) at a dose of 0.05mg/kg twice daily and adjusted to achieve a blood level 2-4 ng/mL for 24 Weeks.
- Starting at Week 24 visit, tacrolimus will be tapered off over 8 weeks. At Week 24, the dose will be decreased by approximately 50%. At Week 28, the dose will be further decreased by approximately 50%. Tacrolimus will be discontinued at Week 32.
- Tacrolimus and sirolimus blood level monitoring will be conducted.

### Dosing adjustments

Prednisone dosing will start at 0.5 mg/kg/day and will be gradually tapered off by the Week 12 visit.

Tacrolimus dose adjustments will be made to maintain whole blood trough concentrations within 2-4 ng/mL for the first 24 Weeks. At Week 24, the dose will be decreased by approximately 50%. At Week 28, the dose will be further decreased by approximately 50%. Tacrolimus will be discontinued at Week 32. Sirolimus dose adjustments will be made to maintain whole blood trough concentrations within 1-3 ng/mL. Dose adjustments should be performed by a clinical pharmacist. Subjects should continue on the new maintenance dose for at least 7 to 14 days before further dosage adjustment with concentration monitoring.

Pneumocystis carinii pneumonia (PCP) prophylaxis with trimethoprim/sulfamethoxazole (Bactrim^{™}; BACTRIM^{™} USPI, 2013) will be given three times a week (example dosing schedule; Monday, Wednesday, Friday) at a dose of 5 mg/kg beginning on Day -2 and continuing until Week 48. Refer to the prescribing information for risks associated with trimethoprim/sulfamethoxazole use (BACTRIM^{™} USPI, 2013). For patients with sulfa allergies, alternative medications can include pentamidine, dapsone, and atovaquone.

Antifungal prophylaxis is to be initiated if the ANC is < 500 mm³. The treatment regimen will be determined through local site standard of care in consultation with appropriate subspecialists.

The concomitant use of Rapamune with a calcineurin inhibitor may increase the risk of calcineurin inhibitor-induced thrombotic microangiopathies. Thrombotic microangiopathies (TMA) are a group of disorders characterized by thrombocytopenia, microangiopathic hemolytic anemia, and variable organ system involvement.

This may present severe thrombocytopenia (<30 × 10⁹/L), microangiopathic hemolytic anemia characterized by schistocytes on the blood smear, increased reticulocyte count (>120 × 10⁹/L), elevated lactate dehydrogenase level (LDH), and signs of skin and mucosal hemorrhage, weakness, and dyspnea. Treatment includes discontinuation of tacrolimus and possible initiation of plasma exchange.

If rising CMV or EBV viral genomes are detected during serial testing, the decision to decrease IS or begin antiviral therapy will be determined through local site standard of care in consultation with appropriate subspecialists.

### 6.6.8. Prohibited Medications and Procedures

The following medications and procedures are prohibited:
- No IT ERT is allowed within 4 months of signing the ICF.
- Any investigational product within the 30 days or 5 half-lives, whichever is longer, prior to signing the ICF or at any time during the study (through Week 104)
- Live vaccines should be avoided while on sirolimus and/or tacrolimus
- Strong inhibitors of CYP3A4 and/or P-glycoprotein (PgP) (such as ketoconazole, voriconazole, itraconazole, posaconazole, erythromycin, telithromycin or clarithromycin) or strong inducers of CYP3A4 and or Pgp (such as rifampin or rifabutin) should be avoided while on sirolimus and/or tacrolimus
- Grapefruit juice inhibits CYP3A-enzymes resulting in increased tacrolimus and sirolimus whole blood trough concentrations. Subjects should avoid eating grapefruit or drinking grapefruit juice with tacrolimus and/or sirolimus.

### 6.6.9. Permitted Medications and Procedures

Subjects will be permitted to remain on a stable regimen of IV ERT as well as any supportive measures (e.g., physical therapy). According to local hospital standard of care, subjects will be permitted to receive medication to prevent claustrophobia during MRI and receive general anesthesia for lumbar puncture, MRI, and neuroconduction studies (ABRs or SSEP).

Medications other than that described above, which are considered necessary for the subject's safety and wellbeing (e.g., for hypertension), may be given at the discretion of the Investigator in accordance with local standard of care and recorded in the appropriate sections of the CRF.

### 6.6.10. Efficacy Assessments

### Biomarkers

- CSF: GAGs, I2S
- Plasma: GAGs, I2S. For subjects treated with weekly IV ERT, plasma biomarkers are to be collected at trough, defined as at least 96 hours after ERT infusion up until the start of the subsequent infusion
- Leukocyte I2S enzyme activity. For subjects treated with weekly IV ERT, whole blood is to be collected at trough, defined as at least 96 hours after ERT infusion up until the start of the subsequent infusion
- Urine: GAGs. For subjects treated with weekly IV ERT, urine GAGs are to be collected at trough, defined as at least 96 hours after ERT infusion up until the start of the subsequent infusion

Neurodevelopmental parameters of cognitive, behavioral, and adaptive function:
- Bayley Scales of Infant and Toddler Development, 3rd Edition (BSID-III) or Kaufman Assessment Battery for Children, 2nd Edition (KABC-II)
- Vineland Adaptive Behavior Scales, 2nd Edition, Expanded Interview Form (VABS-II)
- Other neurocognitive or adaptive assessments performed as part of subjects' standard of care while participating in the trial may also be collected, as determined by the study sponsor after discussing with the site

### Imaging Assessments

- MRI of the brain will be performed at certain visits. Estimated glomerular filtration rate (eGFR) must be documented prior to the screening MRI with gadolinium. The investigator must consult with the Medical Monitor before proceeding with the screening MRI if the eGFR is < 30mL/min/1.73m²
- Abdominal ultrasounds will be performed at certain visits to document craniocaudal liver diameter at the midclavicular line (Kratzer et al., 2003, J. Ultrasound Med. 22:1155-1161) and spleen volume calculated using the prolate ellipsoid formula (0.52 x length x anteroposterior dimension x width)
- 2-D echocardiogram will be performed at certain visits to evaluate for valvular disease and left ventricular mass index.
- Auditory capacity changes will be measured by behavioral audiometry and otoacoustic emissions testing; if behavioral audiometry is not feasible, then ABR testing will be completed. Auditory capacity will be assessed at certain visits; an additional assessment is permitted at the Week 24 visit if deemed clinically necessary by the investigator.
- QOL assessments using PedsQL
- Sleep assessment
- Clinical Assessment of Disease
- Caregiver Assessment of Disease
- ADL
- Burden of Illness

### 6.6.11. Clinical Laboratory Tests

The following CSF safety laboratory and antibody tests will be assessed:
- Markers of CSF inflammation: CSF pressure, erythrocyte cell count, WBC count with differential, total protein, and glucose
- Immune Response Monitoring: neutralizing antibodies to AAV9 and binding antibodies to I2S
- Vector concentration (qPCR): Construct 1 concentrations

The following clinical laboratory tests will be assessed:
- Chemistry: glucose, blood urea nitrogen, creatinine, sodium, potassium, chloride, carbon dioxide, calcium, magnesium, phosphorus, total protein, albumin, total bilirubin, direct bilirubin, alkaline phosphatase (ALP), ALT, AST, gamma glutamyl-transferase, and creatine kinase
- Lipid panel: total cholesterol, low-density lipoprotein (LDL) cholesterol, high-density lipoprotein (HDL) cholesterol, and triglycerides
- Hematology: CBC with differential and platelet count, including hematocrit, hemoglobin, red blood cell (RBC), WBC, platelet, neutrophil, lymphocyte, monocyte, eosinophil, and basophil counts
- Coagulation: prothrombin time (PT) and partial thromboplastin time (PTT)
- Urinalysis: dipstick for glucose, ketones, protein, and blood (if warranted, a microscopic evaluation will be completed)
- Immunogenicity measurements: neutralizing antibodies to AAV9 and binding antibodies to I2S; ELISPOT assay: T-cell response to AAV9 and I2S; flow cytometry for AAV and I2S specific regulatory T cells
- Vector concentration (qPCR): Construct 1 concentrations in serum and urine
- Viral testing for serology and genome detection:
   ∘ VZV: Ab titer (baseline)
   ∘ EBV and CMV: PCR testing for viral genome (baseline and serially)
- Hepatitis B surface antigen, hepatitis B core antibody, anti-hepatitis C, and HIV: only for screening purposes
- Quantiferon-Gold_TB Plus: only for screening purposes.
- Plasma biomarkers
- Urine biomarkers

Laboratory reports will be made available to the PI in a timely manner to ensure appropriate clinical review. The PI is responsible for reviewing and signing all laboratory reports.

### 6.6.12. Vital Signs and Electrocardiogram

Assessment of vital signs (systolic/diastolic BP, pulse rate, temperature, and respiratory rate), head circumference, height, weight, and ECGs will be obtained/performed at visits.

### 6.6.13. Neurologic Assessments

Neurologic assessments should include the following:
- Level of consciousness
- Cranial nerve testing
- Motor function
- Sensory function
- Coordination and gait

SSEP testing will also be performed at selected time points. Additional details on protocol requirements for SSEP testing can be found in the SSEP Manual.

### 6.7 EXAMPLE 7: MPS II Murine Model Study

MPS II or Hunter syndrome is caused by a deficiency of iduronate-2-sulfatase (I2S) leading to an accumulation of glycosaminoglycans (GAGs) in tissues. Severe MPS II results in irreversible neurocognitive decline and behavioral symptoms that are not addressed by intravenously administered enzyme replacement therapy with recombinant I2S enzyme. Construct 1 is a recombinant adeno-associated virus serotype 9 capsid (AAV9) containing a human iduronate-2-sulfatase expression cassette (AAV9.CB7.hIDS). In an MPS II murine model, Construct 1 administered into the cerebrospinal fluid (CSF) demonstrated dose-dependent I2S activity, reduced GAG levels, amelioration of storage pathology in the central nervous system and improved neurobehavioral function. Vector distribution and reduced GAG levels were observed in peripheral organs, as well as normalization of liver size and weight.

### 6.8 EXAMPLE 8: A Phase 1/2, First-In-Human, Multicenter, Open-Label, Dose Escalation Trial

Mucopolysaccharidosis Type II (MPS II) is a rare, X-linked recessive disease caused by a deficiency in the lysosomal enzyme iduronate-2-sulfatase (I2S) leading to an accumulation of glycosaminoglycans (GAGs), including heparin sulfate (HS) in tissues which ultimately results in cell, tissue, and organ dysfunction. In severe forms of the disease, early developmental milestones may be met, but developmental delay is readily apparent by 18 to 24 months. Patients with MPS II continue to have significant difficulties despite the availability of systemic enzyme replacement therapy which does not address manifestations of the disease in the central nervous system (CNS) such as impaired cognitive development. Specific treatment to address the neurological manifestations of MPS II and prevent or stabilize cognitive decline remains a significant unmet medical need. Key biomarkers of I2S enzymatic activity in MPS II patients include its substrate HS, which has been shown to correlate with neurocognitive manifestations of the disorder.

In an ongoing phase 1/2, first-in-human, multicenter, open-label, dose escalation trial, Construct 1 has been administered as a one-time injection into the cisterna magna of participants with severe MPS II ages 4 months to 5 years. Construct 1 is designed to deliver the gene that encodes the I2S enzyme direct to the CNS using the AAV9 vector, aimed to provide a permanent source of secreted I2S beyond the blood-brain barrier, allowing for long-term cross correction of cells throughout the CNS.

Assessments include safety and tolerability up to 104 weeks; CSF, plasma and urine biomarkers; immunogenicity; neurodevelopmental scales (Bayley Scales of Infant and Toddler Development or Kaufman Assessment Battery for Children, and Vineland Adaptive Behavior Scales); audiometry; imaging of the brain, liver and spleen; and clinician- and patient-reported outcome measures.

Cohort 1 has completed enrollment with at least 1 patient completing 16 months of follow-up. In Cohort 1 of the Phase 1/2 study, three patients were dosed intracisternally at the ages of 5 months (Patient 1), 35 months (Patient 2), and 7 months (Patient 3) with 1.3x10¹⁰ genome copies per gram (GC/g) of brain mass. Safety follow-up post-administration of Construct 1 ranges from 12 weeks to 68 weeks. Construct 1 administration has been well tolerated, with no drug-related serious adverse events (SAEs) reported. Per protocol, patients received a 48 week immunosuppression regimen to minimize the potential for immune-mediated reactions and importantly, Patient 1 has completed the immunosuppression regimen.

In Cohort 1, CSF levels of HS, a key biomarker of I2S enzyme activity and a key GAG biomarker of neurocognitive decline in MPS II, showed consistent and durable reductions measured up to 48 weeks. HS has been measured in this Phase 1/2 study in the cerebral spinal fluid (CSF) following the administration of Construct 1. In MPS II patients, high amounts of HS accumulate in the CNS, closely correlating with neurocognitive decline. In the CSF of all three patients enrolled in Cohort 1, HS levels demonstrated a mean reduction of 33.3% from baseline to Week 8. Patient 1 demonstrated consistent decreases in HS levels in the CSF over time, with a 27.4% reduction from baseline at Week 8 and a 43.6% reduction from baseline at Week 48, the latest timepoint available. Patient 2 also demonstrated a decrease of HS levels in the CSF, with a 30.9% reduction from baseline to Week 8, the latest timepoint available. Patient 3 demonstrated a decrease in HS levels in the CSF with a reduction of 41.6% from baseline to Week 8, the latest timepoint available.

In addition, early neurodevelopmental parameters demonstrated ongoing skill acquisition. For the two patients who have progressed beyond Week 24, preliminary data indicates stability of neurocognitive development. Patient 1 continued to exhibit normal cognitive development as expected at Week 48, the time of last assessment. Patient 2 was diagnosed with a neurocognitive decline prior to dosing with Construct 1 and has remained developmentally delayed, but preliminary assessments suggest stable neurocognitive development since dosing.

Following review of the safety and efficacy data from all three patients in Cohort 1, the Independent Data Monitoring Committee for the Phase I/II study of Construct 1 approved the continuation and dose escalation into Cohort 2. Subsequently, Construct 1 was administered to the first patient in Cohort 2 at a dose of 6.5x10¹⁰ GC/g of brain mass. Cohort 2 is actively enrolling and dosing.

In conclusion, the interim data from the first cohort of the ongoing Phase 1/2 trial of Construct 1 for the treatment of MPS II show that: (1) Construct 1 has been well-tolerated in patients with MPS II following one-time intracisternal administration; (2) treatment with Construct 1 resulted in a consistent and sustained reduction in CSF levels of heparan sulfate, a key biomarker of I2S enzyme activity in MPS II, up to 48 weeks after administration of Construct 1; and (3) treatment with Construct 1 achieved early signs of neurocognitive stability in patients with previously diagnosed developmental delay and continued normal cognitive development in a younger patient.

### 6.9 EXAMPLE 9: A Phase VII Multicenter, Open-Label Study to Evaluate the Safety, Tolerability, and Pharmacodynamics of Construct 1 in Children 5 Years of Age and Older with MPS II (Hunter Syndrome)

### 6.9.1. SYNOPSIS

### STUDY DESIGN

This is a Phase I/II, multicenter, open-label, single arm study of Construct 1. No control group is included. Approximately 6 children (≥ 5 years to < 18 years of age) who have severe (neuronopathic) MPS II could be enrolled into a single dose cohort of 6.5 × 10¹⁰ GC/g brain mass and will receive a single dose of Construct 1 administered by IC or ICV injection.

### PRIMARY OBJECTIVES

- To evaluate the safety and tolerability of Construct 1 through 24 weeks following a single IC dose, or ICV dose if the IC route is contraindicated, administered to older (≥ 5 years) children who have neuronopathic MPS II

### SECONDARY OBJECTIVES

- To evaluate immunogenicity of Construct 1
- To explore the effect of Construct 1 on CNS imaging
- To explore the effect of Construct 1 on systemic manifestations of disease
- To explore the effect of Construct 1 on biomarkers in plasma and urine in participants who discontinue IV ERT (ELAPRASE^{®})
- To explore the effect of Construct 1 on quality of life (QOL)
- To explore the effect of Construct 1 on sleep measures
- To explore the effect of Construct 1 on clinician reported outcome
- To explore the effect of Construct 1 on caregiver reported outcome
- To use video recording to capture everyday function
- To explore the effect of Construct 1 on fine motor dexterity

### DIAGNOSIS AND MAIN CRITERIA FOR INCLUSION:

To be eligible to participate in this study, a participant must be a male ≥ 5 years to < 18 years of age with:
- a documented diagnosis of MPS II with neurocognitive decline, or
- a documented mutation(s) in IDS known to result in a neuronopathic form of MPS II, or
- a documented mutation(s) in IDS identical to that of a relative with neuronopathic MPS II.

In addition, the participant must be able to safely receive an IC or ICV injection and immunosuppression.

### INVESTIGATIONAL PRODUCT, DOSAGE AND MODE OF ADMINISTRATION

Construct 1: AAV9.CB7.hIDS (recombinant adeno-associated virus serotype 9 capsid containing human iduronate-2-sulfatase expression cassette). See paragraph [0019] and FIG. 5.

Product will be delivered as a single IC or ICV dose.

One dose level will be evaluated, 6.5 × 10¹⁰ GC/g brain mass. Total dose administered will account for estimated brain size of study participants based on their screening MRI. Total volume of product administered will not exceed 10% of estimated CSF volume.

### 6.9.2. CRITERIA FOR EVALUATION SAFETY AND EFFICACY

### PRIMARY ENDPOINTS

- Safety through Week 24: AEs and SAEs

### SECONDARY ENDPOINTS

- Safety through Week 104: AEs and SAEs
- Biomarkers in CSF (GAGs, I2S), plasma (GAGs, I2S), and urine (GAGs)
- Neurodevelopmental parameters of cognitive, behavioral, and adaptive function:
- Bayley Scales of Infant and Toddler Development, 3rd Edition (BSID-III) (Bayley, 2005, Scales of Infant and Toddler Development, 3rd Ed.)
- Mullen Scales of Early Learning (MSEL) (Mullen, Circle Pines, MN: American Guidance Service Inc.; 1995)
- Vineland Adaptive Behavior Scales, 2nd Edition, Expanded Interview Form (VABS-II) (Sparrow et al., 2005, Vineland Adaptive Behavior Scales, 2nd Ed.)

### EXPLORATORY ENDPOINTS

- Immunogenicity measurements
- Neutralizing antibody titers to AAV9 and binding antibody titers to I2S in CSF and serum
   - Enzyme-linked immunospot (ELISPOT) assay: T-cell response to AAV9 and I2S
- CNS structural abnormalities assessed by MRI of the brain
- Liver and spleen size assessed by ultrasound of the abdomen
- Cardiac evaluation by echocardiogram for valvular disease and left ventricular mass index
- Median nerve motor and sensory distal conduction velocity and latency
- Plasma and urinary GAGs in participants who discontinue IV ERT (ELAPRASE^{®})
- PedsQL
- Sleep assessment (Sleep Disturbance Scale for Children - SDSC) (Ferreira et al., 2009, Sleep Medicine;10(4):457-463)
- Clinician Global Impression of Severity and Clinician Global Impression of Change
- Caregiver Global Impression of Severity and Caregiver Global Impression of Change
- Activities of Daily Living (Tanjuakio et al. 2015, Mol Genet Metab, 114(2):161-169; Kato et al., 2007, Brain Dev, 29(5): 298-305)
- Pediatric Evaluation of Disability Inventory Computer Adaptive Test (PEDI CAT)
- 9-Hole Peg Test
- Functional performance as captured by video

### STATISTICAL METHODS

All data will be analyzed using descriptive statistics. Categorical variables will be summarized using frequencies and percentages. Continuous variables will be summarized using number of non-missing observations, mean, standard deviation, median, minimum, and maximum). Graphical displays will be presented as appropriate. Participant data listings will also be presented.

### SAMPLE SIZE AND POWER CALCULATION

No formal calculation was performed to determine sample size.

### 6.9.3. List of Abbreviations and Definitions of Terms

**Table 9: Abbreviations and Specialist Terms**

| **Abbreviation or Specialist Term** | **Explanation** |
|---|---|
| AAV | Adeno-associated virus |
| AAV9 | Adeno-associated virus serotype 9 |
| ABR | Auditory brainstem response (s) |
| ADL | Activities of Daily Living |
| AE | Adverse event |
| AESI | Adverse event of special interest |
| ALP | Alkaline phosphatase |
| ALT | Alanine aminotransferase |
| ANC | Absolute neutrophil count |
| APB | Abductor pollicis brevis |
| AST | Aspartate aminotransferase |
| BBB | Blood-brain barrier |
| BID | Twice a day |
| BP | Blood Pressure |
| BSID | Bayley Scales of Infant and Toddler Development |
| BSL | Biosafety level |
| BUN | Blood urea nitrogen |
| CB7 | Hybrid C4 and CB (chicken beta actin promoter) |
| CBC | Complete blood count |
| CFR | Code of Federal Regulations |
| CGI-C | Clinician Global Impression Scale for change |
| CGI-S | Clinician Global Impression Scale for severity |
| CI | Confidence Interval |
| CIOMS | Council for International Organizations of Medical Sciences |
| CMV | Cytomegalovirus |
| CNS | Central nervous system |
| CoA | Certificate of Analysis |
| CRF | Case report form |
| CRO | Contract research organization |
| CSF | Cerebrospinal fluid |
| CT | Computed tomography |
| CTA | Clinical Trial Agreement |
| CTCAE | Common Terminology Criteria for Adverse Events |
| CZ | Crystal Zenith^{®} |
| DLT | Dose-limiting toxicity |
| DNA | Deoxyribonucleic acid |
| DRG | Dorsal root ganglia |
| DS | Dermatan sulfate |
| ECG | Electrocardiogram |
| ED | Early discontinuation (visit) |
| EDC | Electronic data capture |
| eGFR | Estimated glomerular filtration rate |
| ELISA | Enzyme-Linked Immunosorbent Assay |
| ELISPOT | Enzyme-Linked immunospot |
| EOS | End of Study |
| ERT | Enzyme Replacement Therapy |
| EU | European Union |
| FDA | Food and Drug Administration |
| FIM | Family Impact Module |
| G | gram |
| GAG(s) | Glycosaminoglycan(s) |
| GC | Genome copies |
| GCP | Good Clinical Practice |
| GLP | Good Laboratory Practice |
| HBsAg | Hepatitis B surface antigen |
| HCV | Hepatitis C virus |
| Hep | Hepatitis |
| hIDS | Human iduronate-2-sulfatase gene |
| HIPAA | Health Insurance Portability and Accounting Act |
| HIV | Human immunodeficiency virus |
| HS | Heparan Sulfate |
| HSCT | Hematopoietic stem cell transplantation |
| IB | Investigator's brochure |
| IC | Intracisternal |
| ICF | Informed consent form |
| ICH | International Conference on Harmonisation |
| ICV | Intracerebroventricular |
| IDMC | Independent Data Monitoring Committee |
| IDS | Iduronate-2-sulfatase gene |
| IEC | Independent Ethics Committee (s) |
| IgG | Immunoglobulin G |
| IMP | Investigational medicinal product |
| IND | Investigational New Drug |
| INR | International normalized ratio |
| IP | Investigational Product |
| IRB/IEC | Institutional review board/independent ethics committee |
| IS | Immune Suppression |
| ISC | Internal Safety Committee |
| IT | Intrathecal(ly) |
| ITRs | Inverted terminal repeats |
| IV | Intravenous(ly) |
| LDH | Lactate dehydrogenase level |
| LDL | Low-density lipoprotein |
| LFT | Liver function tests |
| MED | Minimum effective dose |
| MedDRA | Medical Dictionary for Regulatory Activities |
| mL | Milliliter |
| MPS I | Mucopolysaccharidosis type II |
| MPS VII | Mucopolysaccharidosis type VII |
| MRI | Magnetic resonance imaging |
| MSEL | Mullen Scales of Early Learning |
| MTD | Maximum tolerated dose |
| NAb | Neutralizing antibody |
| NHP | Non-human primates |
| NIH | National Institutes of Health |
| NIMP | Non-investigational medicinal product |
| OAE | Otoacoustic emission |
| PBMC | Peripheral blood mononuclear cell |
| PCP | Pneumocystis carinii pneumonia |
| PCR | Polymerase chain reaction |
| PD | Pharmacodynamic |
| PEDI-CAT | Pediatric Evaluation of Disability Inventory Computer Adaptive Test |
| PHI | Protected health information |
| PI | Principal Investigator |
| PML | Progressive multifocal leukoencephalopathy |
| PK | Pharmacokinetic |
| PO | By mouth/ orally |
| PT | Prothrombin time, (MedDRA) preferred tenn |
| PTT | Partial thromboplastin time |
| PVAN | Polyoma virus-associated nephropathy |
| QD | Daily |
| QOL | Quality of Life |
| qPCR | Quantitative polymerase chain reaction |
| RBC | Red blood cell |
| Construct 1 | Recombinant adeno-associated virus serotype 9 capsid containing human iduronate-2-sulfatase expression cassette |
| RNA | Ribonucleic acid |
| SAE | Serious adverse event |
| SAP | Statistical Analysis Plan |
| SD | Standard deviation |
| SDSC | Sleep Disturbance Scale for Children |
| SDV | Source data verification |
| SNAP | Sensory nerve action potential |
| SOC | (MedDRA) System Organ Class |
| SRT | Safety Review Trigger |
| SSEP | Somatosensory evoked potential |
| SUSAR | Suspected unexpected serious adverse reaction |
| TB | Tuberculosis |
| TEAE | Treatment-emergent adverse event |
| TMA | Thrombotic microangiopathies |
| Treg | Regulatory T cell |
| ULN | Upper limit of normal |
| US | United States |
| VABS | Vineland Adaptive Behavior Scales |
| VAS | Visual Analog Scale |
| VZV | Varicella zoster virus |
| WBC | White blood cell |
| WHO | World Health Organization |

### 6.9.4. Overall Study Design

This is a Phase I/II, multicenter, open-label, single arm study of Construct 1. No control group is included. Approximately 6 children (≥ 5 years to < 18 years of age) who have severe (neuronopathic) MPS II could be enrolled into a single dose cohort of 6.5 × 10¹⁰ GC/g brain mass and will receive a single dose of Construct 1 administered by IC or ICV injection. Safety will be the primary focus for the initial 24 weeks after treatment (primary study period). Following completion of the primary study period, participants will continue to be assessed (safety and efficacy) for up to a total of 104 weeks following treatment with Construct 1. At the end of the study, participants will be invited to participate in a long-term follow-up study.

The first 2 eligible participants will be enrolled in a staggered fashion. After Construct 1 administration to the first participant, there will be an 8-week observation period for safety. The Internal Safety Committee (ISC) will review the safety data obtained during the first 8 weeks of the study according to the ISC Charter (including data obtained during the Week 8 visit) for this participant, and if there are no safety concerns, the 2^{nd} participant may be dosed. Informed consent and screening activities for the 2^{nd} participant may proceed during the observation period for the first participant.

If no safety review trigger (SRT) event is observed, all available safety data for the first 2 participants obtained up to and including the Week 8 visit for the 2^{nd} participant will be evaluated by the Independent Data Monitoring Committee (IDMC). If the decision is to proceed, the next 4 participants may be enrolled.

All available safety data will be evaluated by the IDMC after the Week 8 visit for the 6^{th} participant and at intervals stipulated within the study-specific IDMC Charter.

At any given IDMC meeting, whether planned or called for by an SRT, the IDMC may recommend stopping the trial, delaying the dosing of additional participants, or proceeding at a lower dose. Once 8 weeks of data are available from the 6^{th} participant, then enrollment in the study will be completed.

If any event meets the criteria of a prespecified Stopping Rule, dosing of any new participants will be suspended until a complete review of all safety data by REGENXBIO and the external IDMC has been performed.

Those participants who meet the eligibility criteria will be admitted to the hospital between Day -2 and the morning of Day 1 (according to institutional practice), and baseline assessments will be performed pre-dose. Participants will receive a single IC or ICV dose of Construct 1 on Day 1 and will remain in the hospital overnight after dosing for observation. Participants will be discharged after the principal investigator concludes that the participant is ready for discharge and prolongation of hospitalization is not necessary. Subsequent assessments in the primary study period (i.e., through Week 24) will be performed at Weeks 1, 2, 3, 4, 12, and 24. The Weeks 8 and 16 assessments will be limited to evaluation of adverse events (AEs) and concomitant therapies by telephone contact. After the primary study period, visits will be at Weeks 38, 52, 64, 78, and 104. The Week 30 visit will be performed only for participants who discontinue IV ERT starting at Week 24.

Because of the brain growth that occurs in children and differences in brain growth that may occur in MPS II, the total dose of Construct 1 administered IC or ICV will be calculated from the estimated brain mass derived from the study participant's screening MRI. The study participant's estimated brain volume from their MRI will be converted to brain mass and used to calculate the exact dose to be administered, as presented in Table 10.

**Table 10: Total Dose Administered by Brain Mass**

| **Brain Mass (in g)** | | **Target Brain Mass (in g)** | **Dose: Total GC (6.5 × 10¹⁰ GC/g brain mass)** |
|---|---|---|---|
| **Min** | **Max** | | |
| 801 | 900 | 850 | 5.5 × 10¹³ |
| 901 | 1050 | 975 | 6.3 × 10¹³ |
| 1051 | 1200 | 1125 | 7.3 × 10¹³ |
| 1201 | - | 1300 | 8.5 × 10¹³ |

1. Obtain participant brain volume in cm³ from screening MRI.
2. Convert participant's MRI brain volume to brain mass:
   brain mass = (brain volume in cm³) × 1.046 g/cm³ (brain density taken from (Hasgall et al, 2018,Tissue Properties, Density. [cited 04 November 2019]. In: IT'IS Database for Thermal and Electromagnetic Parameters of Biological Tissues, Version 4.0, 15 May 2018 [Internet]. Zurich: IT'IS Foundation. c2010).
3. Identify appropriate dose in Table 10 above.

Construct 1 is intended for investigational use only by selected investigators familiar with the information in the investigator's brochure for Construct 1 and experienced in conducting clinical trials. Construct 1 may only be administered to human participants enrolled in clinical trials sponsored/approved by the Sponsor and who have provided formal written consent.

### 6.9.5. Inclusion Criteria

Participants are eligible to be included in the study only if all of the following criteria apply:
1. The participant's legal guardian(s) is (are) willing and able to provide written, signed informed consent after the nature of the study has been explained, and prior to any research-related procedures.
2. Is a male ≥ 5 years to < 18 years of age
3. Meets any of the following criteria:
   a. Has a documented diagnosis of MPS II *AND* a neurocognitive testing score ≤ 1 ½ standard deviation (SD) from the test normative mean (BSID-III: 77 and MSEL Visual Reception: 35), *OR*
   b. Has a documented diagnosis of MPS II *AND* has a decline of ≥ 1 standard deviation on serial neurocognitive testing administered between 3 to 36 months apart (BSID-III Cognitive or MSEL Visual Reception), *OR*
   c. Has a relative clinically diagnosed with neuronopathic MPS II who has the same IDS mutation as the participant AND the participant in the opinion of a geneticist has inherited a neuronopathic form of MPS II, OR
   d. Has documented mutation(s) in *IDS* that in the opinion of a geneticist is known to result in a neuronopathic phenotype AND in the opinion of a clinician has a neuronopathic form of MPS II

### 6.9.6. Exclusion Criteria

Participants are excluded from the study if any of the following criteria apply:
1. Has a contraindication for an IC and ICV injection, including any of the following:
   a. Review of baseline MRI testing by the team of neuroradiologists/ neurosurgeons participating in study shows a contraindication for an IC and an ICV injection
   b. History of prior head/neck surgery, which resulted in a contraindication to both IC and ICV injection, based on review of available information by the team of neuroradiologists/neurosurgeons participating in study
   c. Has any contraindication to general anesthesia
   d. Has any contraindication to MRI or gadolinium
   e. Has renal insufficiency as determined by an estimated glomerular filtration rate (eGFR) <30 mL/min/1.73 m², based on creatinine. If laboratory determines that creatinine is less than the lower limit of assay validation or detection then the lowest limit cutoff value will be used to estimate eGFR.
   f. Has previously experienced a clinically significant intracranial bleed that, in the opinion of the investigator and team of neuroradiologists/neurosurgeons, is a contraindication to IC and ICV injection
   g. Has an elevated intracranial pressure (≥ 30cm H₂O)
2. Has any condition that would contraindicate treatment with prednisone, tacrolimus or sirolimus
3. Has any neurocognitive deficit not attributable to MPS II or diagnosis of a neuropsychiatric condition that may in the opinion of the PI confound interpretation of study results
4. Has any contraindication to lumbar puncture
5. Has a (cerebral) ventricular shunt that in the opinion of the site neuroradiologist/ neurosurgeon and through discussion with the Medical Monitor, may impact the administration and proper dosing of the participant
6. Has had prior treatment with an AAV-based gene therapy product
7. Is receiving idursulfase (ELAPRASE^{®}) via intrathecal (IT) administration at the time of ICF. The participant must agree to discontinue IT idursulfase for the duration of the study, starting immediately after signing the ICF
8. Has received idursulfase (ELAPRASE^{®}) IV and experienced a serious hypersensitivity reaction, including anaphylaxis, deemed related to IV idursulfase (ELAPRASE^{®}) administration
9. Is failing to respond to idursulfase (ELAPRASE^{®}) IV due to neutralizing anti-IDS antibodies, documented as increasing urine GAG levels; the participant may enroll if he has received immune modulation and is currently responsive to IV idursulfase (ELAPRASE^{®})
10. Has received any investigational product within 30 days of Day 1 or 5 half-lives before signing of the ICF, whichever is longer
11. Has any history of lymphoma or history of another cancer, other than squamous cell or basal cell carcinoma of the skin, that has not been in full remission for at least 1 year before screening
12. Has a platelet count <100,000 per microliter (µL)
13. Has aminotransferase (ALT) or aspartate aminotransferase (AST) >3 × ULN or total bilirubin >1.5 × ULN at screening unless the participant has a previously known history of Gilbert's syndrome
14. Has a history of human immunodeficiency virus (HIV) or hepatitis B or hepatitis C virus infection, or positive screening tests for hepatitis B surface antigen or hepatitis B core antibody, or hepatitis C or HIV antibodies
15. Is a first-degree family member of a clinical site employee or any other individual involved with the conduct of the study or is a clinical site employee or other individual involved with the conduct of the study
16. Has a clinically significant ECG abnormality that, in the opinion of the PI, would compromise the participant's safety
17. Has a serious or unstable medical or psychological condition that, in the opinion of the PI, would compromise the participant's safety in the study
18. Has uncontrolled seizures that in opinion of the PI would put the participant at undue risk
19. Has a history of a hypersensitivity reaction to tacrolimus, sirolimus, or prednisone
20. Has a history of a primary immunodeficiency (e.g., common variable immunodeficiency syndrome), splenectomy, or any underlying condition that predisposes the participant to infection
21. Has herpes zoster (VZV), cytomegalovirus (CMV), or Epstein-Barr virus (EBV) infection that has not completely resolved at least 12 weeks prior to screening
22. Has any infection requiring hospitalization or treatment with parenteral anti-infectives not resolved at least 8 weeks prior to Visit 2
23. Has any active infection requiring oral anti-infectives (including antivirals) within 10 days prior to Visit 2
24. Has a history of active tuberculosis (TB) or a positive Quantiferon-TB Gold test during screening
25. Has any live vaccine within 4 weeks prior to Day -2
26. Had major surgery within 8 weeks before signing the ICF or major surgery planned during the study period
27. Anticipate the need for adenoidectomy or tonsillectomy within 6 months of enrollment. If adenoidectomy or tonsillectomy is anticipated, it should be performed prior to screening.
28. Has an absolute neutrophil count <1.0 × 10³/µL
29. Has any condition or laboratory abnormality that the PI believes would not be appropriate for immunosuppressive therapy.

### 6.9.7. Immunosuppressive Therapy

### Corticosteroids

- In the morning of vector administration (Day 1 pre-dose), participants will receive methylprednisolone 10mg/kg IV (maximum of 500 mg) over at least 30 minutes. The methylprednisolone should be administered before the lumbar puncture and IC injection of IP. Premedication with acetaminophen and an antihistamine is optional and at the discretion of the investigator.
- On Day 2, oral prednisolone will be started with the goal to discontinue prednisolone by Week 12. The dose of prednisolone will be as follows:
   - Day 2 to the end of Week 2: 0.5 mg/kg/day
   - Week 3 and 4: 0.35 mg/kg/day
   - Week 5-8: 0.2 mg/kg/day
   - Week 9-12: 0.1 mg/kg
   - Prednisolone will be discontinued after Week 12. The exact dose of prednisolone can be adjusted to the next higher clinically practical dose.

### Sirolimus

- 2 days prior to vector administration (Day -2): a loading dose of sirolimus 1 mg/m² every 4 hours x 3 doses will be administered
- From Day -1: sirolimus 0.5 mg/m²/day divided in twice a day dosing with target blood level of 1-3 ng/ml
- Sirolimus will be discontinued after the Week 48 visit.

### Tacrolimus

- Tacrolimus will be started on Day 2 (the day following IP administration) at a dose of 0.05mg/kg twice daily and adjusted to achieve a blood level 2-4 ng/mL for 24 Weeks.
- Starting at Week 24 visit, tacrolimus will be tapered off over 8 weeks. At Week 24 the dose will be decreased by approximately 50%. At Week 28 the dose will be further decreased by approximately 50%. Tacrolimus will be discontinued at Week 32.
- Tacrolimus and sirolimus blood level monitoring will be conducted.

Tacrolimus dose adjustments will be made to maintain whole blood trough concentrations within 2-4 ng/mL for the first 24 Weeks. At Week 24 the dose will be decreased by approximately 50%. At Week 28 the dose will be further decreased by approximately 50%. Tacrolimus will be discontinued at Week 32. Sirolimus dose adjustments will be made to maintain whole blood trough concentrations within 1-3 ng/mL. Dose adjustments should be performed by a clinical pharmacist. Participants should continue on the new maintenance dose for at least 7 to 14 days before further dosage adjustment with concentration monitoring.

Immunosuppressant drug accountability, IS dispensation, sirolimus and tacrolimus whole blood trough concentrations will be completed by the investigator during routine study visits.

No IS therapy is planned after Week 48. If IS were required after Week 48 to control a clinically-relevant immune response, the appropriate immunosuppressive regimen will be determined by the PI, in discussion with the Medical Monitor and Sponsor, as clinically indicated.

Pneumocystis carinii pneumonia (PCP) prophylaxis with trimethoprim/sulfamethoxazole (Septra^{®}; Bactrim^{™}) (SEPTRA^{®} USPI, 2018; BACTRIM^{™} USPI, 2013) will be given three times a week (example dosing schedule; Monday, Wednesday, Friday) at a dose of 5 mg/kg beginning on Day -2 and continuing until Week 48. Refer to the prescribing information for risks associated with trimethoprim/sulfamethoxazole use (BACTRIM^{™} USPI, 2013). For patients with sulfa allergies, alternative medications can include pentamidine, dapsone, and atovaquone.

Antifungal prophylaxis is to be initiated if the ANC is < 500 mm³. The treatment regimen will be determined through local site standard of care in consultation with appropriate subspecialists.

The concomitant use of Rapamune with a calcineurin inhibitor may increase the risk of calcineurin inhibitor-induced thrombotic microangiopathies. Thrombotic microangiopathies (TMA) are a group of disorders characterized by thrombocytopenia, microangiopathic hemolytic anemia, and variable organ system involvement.

This may present severe thrombocytopenia (<30 × 10⁹/L), microangiopathic hemolytic anemia characterized by schistocytes on the blood smear, increased reticulocyte count (>120 × 10⁹/L), elevated lactate dehydrogenase level (LDH), and signs of skin and mucosal hemorrhage, weakness, and dyspnea. Treatment includes discontinuation of tacrolimus and possible initiation of plasma exchange.

If rising CMV or EBV viral genomes are detected during serial testing, the decision to decrease IS or begin antiviral therapy will be determined through local site standard of care in consultation with appropriate subspecialists.

### 6.10 EXAMPLE 10: A Phase I/II Multicenter, Open-Label Study to Evaluate the Safety, Tolerability, and Pharmacodynamics of Construct 1 in Pediatric Subjects with MPS II (Hunter Syndrome)

### 6.10.1. SYNOPSIS

### STUDY DESIGN

This is a Phase I/II, first-in-human, multicenter, open-label, single arm dose escalation study of Construct 1. No control group is included. Approximately 12 pediatric subjects who have severe MPS II could be enrolled into 3 dose cohorts, 1.3 × 10¹⁰ GC/g brain mass (Dose 1), 6.5 × 10¹⁰ GC/g brain mass (Dose 2), or 2.0 × 10¹¹ GC/g brain mass (Dose 3), and will receive a single dose of Construct 1 administered by IC or ICV injection. Safety will be the primary focus for the initial 24 weeks after treatment (primary study period). Following completion of the primary study period, subjects will continue to be assessed (safety and efficacy) for up to a total of 104 weeks following treatment with Construct 1. At the end of the study, subjects will be invited to participate in a long-term follow-up study.

The first 3 eligible subjects will be enrolled into the Dose 1 cohort (1.3 × 10¹⁰ GC/g brain mass). After Construct 1 administration to the first subject, there will be an 8-week observation period for safety. The Internal Safety Committee (ISC) will review the safety data obtained during the first 8 weeks of the study according to the ISC Charter (including data obtained during the Week 8 visit) for this subject, and if there are no safety concerns, the 2nd subject may be enrolled. The same process will be used to enroll the 3rd subject. Informed consent and screening activities for the next subject may proceed during the observation period for the preceding subject.

If no safety review trigger (SRT) event is observed, all available safety data for the Dose 1 cohort obtained up to and including the Week 8 visit for the 3rd subject will be evaluated by the Independent Data Monitoring Committee (IDMC). If the decision is to proceed to the second dose cohort (6.5 × 10¹⁰ GC/g brain mass), the subsequent 2 subjects will follow the same dosing scheme as the initial dose cohort. The ISC will review all subject safety data obtained up to and including the Week 2 visit of the 2nd subject in the Dose 2 cohort and may determine that it is safe to proceed with dosing of the 3rd subject immediately after this assessment. All available safety data for the Dose 2 cohort will be evaluated by the IDMC after the Week 8 visit for the 3rd subject in the Dose 2 cohort.

With approval of the IDMC, up to 6 subjects may be dosed in a Dose 2 Expanded Cohort as long as study drug is available, and there is Sponsor approval and no safety event that warrants suspension of enrollment as per either the ISC or the IDMC.

If no safety review trigger (SRT) event is observed and the IDMC provides approval, a third dose cohort (2.0 × 10¹¹ GC/g brain mass) will begin enrollment. The ISC will review all available safety data for the first subject dosed up to and including the Week 8 visit. If there are no safety concerns, the second subject will be dosed. The ISC will review all subject safety data obtained up to and including the Week 2 visit of the 2^{nd} subject in the Dose 3 cohort and may determine that it is safe to proceed with dosing of the 3^{rd} subject immediately after this assessment.

### PRIMARY OBJECTIVES

To evaluate the safety and tolerability of Construct 1 through 24 weeks following a single IC, or ICV if IC is contraindicated, dose administered to pediatric subjects who have severe MPS II

### SECONDARY OBJECTIVES

- To evaluate the long-term safety and tolerability of Construct 1
- To evaluate the effect of Construct 1 on biomarkers in CSF, plasma, and urine
- To evaluate the effect of Construct 1 on neurodevelopmental parameters of cognitive, behavioral, and adaptive function
- To evaluate vector shedding in CSF, serum, and urine

### EXPLORATORY OBJECTIVES

- To evaluate immunogenicity of Construct 1
- To explore the effect of Construct 1 on CNS imaging
- To explore the effect of Construct 1 on systemic manifestations of disease
- To explore the effect of Construct 1 on auditory capacity
- To explore the effect of Construct 1 on biomarkers in plasma and urine in subjects who temporarily discontinue IV ERT (ELAPRASE^{®})
- To explore the effect of Construct 1 on quality of life (QOL)
- To explore the effect of Construct 1 on sleep measures
- To explore the effect of Construct 1 on clinician reported outcome
- To explore the effect of Construct 1 on caregiver reported outcome

### DIAGNOSIS AND MAIN CRITERIA FOR INCLUSION:

To be eligible to participate in this study, a subject must meet all the following inclusion criteria:
**1.** The subject's legal guardian(s) is (are) willing and able to provide written, signed informed consent after the nature of the study has been explained, and prior to any research-related procedures.
**2.** Is a male ≥4 months to <5 years of age
**3.** Meets any of the following criteria:
   **a)** Has a documented diagnosis of MPS II AND has a neurocognitive testing score ≤77 (BSID-III or KABC-II), OR
   **b)** Has a documented diagnosis of MPS II AND has a decline of ≥1 standard deviation on serial neurocognitive testing administered between 3 to 36 months apart (BSID-III or KABC-II), OR
   **c)** Has a relative clinically diagnosed with severe MPS II who has the same IDS mutation as the subject AND the subject in the opinion of a geneticist has inherited a severe form of MPS II, OR
   **d)** Has documented mutation(s) in IDS that in the opinion of a geneticist is always known to result in a neuronopathic phenotype [e.g., a complete deletion or large deletion (e.g., spanning ≥1 exon), or recombination] AND in the opinion of a clinician has a severe form of MPS II
**4.** Has sufficient auditory and visual capacity, with or without aids, to complete the required protocol testing, and be compliant with wearing the aid, if applicable, on testing days

### DIAGNOSIS AND MAIN CRITERIA FOR EXCLUSION:

Subjects who meet any of the following exclusion criteria will not be eligible to participate in the study:
**1.** Has a contraindication for an IC or ICV injection, including any of the following:
   **a)** Review of baseline MRI testing by the team of neuroradiologists/neurosurgeons participating in study (1 neuroradiologist or neurosurgeon per site) shows a contraindication for an IC or an ICV injection
   **b)** History of prior head/neck surgery, which resulted in a contraindication to both IC and ICV injection, based on review of available information by the team of neuroradiologists/neurosurgeons participating in study
   **c)** Has any contraindication to computed tomography, contrast agent, or to general anesthesia
   **d)** Has any contraindication to MRI or gadolinium
   **e)** Has renal insufficiency as determined by an estimated glomerular filtration rate (eGFR) <30 mL/min/1.73 m2, based on creatinine. If laboratory determines that creatinine is less than the lower limit of assay validation or detection then the lowest limit cutoff value will be used to estimate eGFR
   **f)** Has previously experienced a clinically significant intracranial bleed that, in the opinion of the investigator and team of neuroradiologists/neurosurgeons, is a contraindication to IC and ICV injection
**2.** Has any condition that would contraindicate treatment with prednisone, tacrolimus or sirolimus
**3.** Has any neurocognitive deficit not attributable to MPS II or diagnosis of a neuropsychiatric condition that may in the opinion of the PI confound interpretation of study results
**4.** Has any contraindication to lumbar puncture
**5.** Has a (cerebral) ventricular shunt that in the opinion of the site neuroradiologist/neurosurgeon and through discussion with the Medical Monitor, may impact the administration and proper dosing of the subject
**6.** Has undergone HSCT
**7.** Has had prior treatment with an AAV-based gene therapy product
**8.** Has received idursulfase (ELAPRASE^{®}) via intrathecal (IT) administration within 4 months of signing the ICF
**9.** Has received idursulfase (ELAPRASE^{®}) IV and experienced a serious hypersensitivity reaction, including anaphylaxis, deemed related to IV idursulfase (ELAPRASE^{®}) administration. If currently receiving IV idursulfase and has had dose interruptions or a dose regimen different than weekly, then discussion with REGENXBIO Medical Monitor is needed.
**10.** Has received any investigational product within 30 days of Day 1 or 5 half-lives before signing of the ICF, whichever is longer
**11.** Has any history of lymphoma or history of another cancer, other than squamous cell or basal cell carcinoma of the skin, that has not been in full remission for at least 1 year before screening
**12.** Has a platelet count <100,000 per microliter (µL)
**13.** Has aminotransferase (ALT) or aspartate aminotransferase (AST) >3 × ULN or total bilirubin >1.5 × ULN at screening unless the subject has a previously known history of Gilbert's syndrome
**14.** Uncontrolled hypertension despite medical treatment, defined for children ≤ 17 years of age to be systolic blood pressure or diastolic blood pressure >99th percentile plus 5mmHg based on normative standards for age, sex, and height
**15.** Has a history of human immunodeficiency virus (HIV) or hepatitis B or hepatitis C virus infection, or positive screening tests for hepatitis B surface antigen or hepatitis B core antibody, or hepatitis C or HIV antibodies
**16.** Is a first-degree family member of a clinical site employee or any other individual involved with the conduct of the study or is a clinical site employee or other individual involved with the conduct of the study
**17.** Has a clinically significant ECG abnormality that, in the opinion of the PI, would compromise the subject's safety
**18.** Has a serious or unstable medical or psychological condition that, in the opinion of the PI, would compromise the subject's safety or successful participation in the study or interpretation of study results
**19.** Has uncontrolled seizures that in opinion of the PI would put the subject at undue risk

Exclusion criteria related to immunosuppressive therapy:
**20.** Has a history of a hypersensitivity reaction to tacrolimus, sirolimus, or prednisone
**21.** Has a history of a primary immunodeficiency (e.g., common variable immunodeficiency syndrome), splenectomy, or any underlying condition that predisposes the subject to infection
**22.** Has herpes zoster (VZV), cytomegalovirus (CMV), or Epstein-Barr virus (EBV) infection that has not completely resolved at least 12 weeks prior to screening
**23.** Has any infection requiring hospitalization or treatment with parenteral anti-infectives not resolved at least 8 weeks prior to Visit 2
**24.** Has any active infection requiring oral anti-infectives (including antivirals) within 10 days prior to Visit 2
**25.** Has a history of active tuberculosis (TB) or a positive Quantiferon-TB Gold test during screening
**26.** Has received any live vaccine within 4 weeks prior to Day 2
**27.** Had major surgery within 8 weeks before signing the ICF or major surgery planned during the study period
**28.** Anticipate the need for adenoidectomy or tonsillectomy within 6 months of enrollment. If adenoidectomy or tonsillectomy is anticipated it should be performed prior to screening.
**29.** Has an absolute neutrophil count <1.0 × 10³/µL
**30.** Has any condition or laboratory abnormality that the PI believes would not be appropriate for immunosuppressive therapy

### INVESTIGATIONAL PRODUCT

Construct 1: AAV9.CB7.hIDS (recombinant adeno-associated virus serotype 9 capsid containing human iduronate-2-sulfatase expression cassette). See paragraph [0019] and FIG. 5.

### DOSE

Construct 1 will be preferentially administered as a single IC injection, or as a single ICV injection should IC administration prove difficult or potentially unsafe, to allow direct delivery of the vector to the target tissue within the confined CSF compartment. Although cervical puncture (C1-C2) is a routine clinical procedure used for contrast administration for myelography, image-assisted suboccipital puncture is proposed as the IC clinical route of administration. This replicates the route of administration used in the nonclinical studies and is considered advantageous over the C1-C2 puncture in the intended patient population because patients with MPS II have a high incidence of abnormal narrowing of the C1-C2 IT space, which substantially increases the risks associated with a C1-C2 puncture. Prior to the procedure, each subject will have a magnetic resonance imaging (MRI) of the area reviewed by a team of neuroradiologists/neurosurgeons participating in the study. If it is not considered safe to proceed with an IC injection, then the subject will be considered for ICV injection.

**Table 11: Total Dose Administered by Brain Mass**

| **Brain Mass (in g)** | | **Target** | **Dose Levels** | |
|---|---|---|---|---|
| **Min** | **Max** | | **Dose 1 Total GC** (1.3 × 10¹⁰GC/g brain mass) | **Dose 2 Total GC** (6.5 × 10¹⁰GC/g brain mass) |
| - | 700 | 650 | 8.5 × 10¹² | 4.2 × 10¹³ |
| 701 | 800 | 750 | 9.8 × 10¹² | 4.9 × 10¹³ |
| 801 | 900 | 850 | 1.1 × 10¹³ | 5.5 × 10¹³ |
| 901 | 1050 | 975 | 1.3 × 10¹³ | 6.3 × 10¹³ |
| 1051 | 1200 | 1125 | 1.5× 10¹³ | 7.3 × 10¹³ |
| 1201 | - | 1300 | 1.7 × 10¹³ | 8.5 × 10¹³ |
| | | | | |
| **Total Dose Administered by Brain Mass Dose 3** | | | | |

| **Brain Mass (in g)** | | **Target** | **Dose 3 Total GC** (2.0 × 10¹¹ GC/g brain mass) | |
|---|---|---|---|---|
| **Min** | **Max** | | | |
| - | 474 | 450 | 9.0 x 10¹³ | |
| 475 | 524 | 500 | 1.0 × 10¹⁴ | |
| 525 | 574 | 550 | 1.1 × 10¹⁴ | |
| 575 | 624 | 600 | 1.2 × 10¹⁴ | |
| 625 | 674 | 650 | 1.3 × 10¹⁴ | |
| 675 | 724 | 700 | 1.4 × 10¹⁴ | |
| 725 | 774 | 750 | 1.5 × 10¹⁴ | |
| 775 | 824 | 800 | 1.6 × 10¹⁴ | |
| 825 | 874 | 850 | 1.7 × 10¹⁴ | |
| 875 | 924 | 900 | 1.8 × 10¹⁴ | |
| 925 | 974 | 950 | 1.9 × 10¹⁴ | |
| 975 | 1024 | 1000 | 2.0 × 10¹⁴ | |
| 1025 | 1074 | 1050 | 2.1 × 10¹⁴ | |
| 1075 | 1124 | 1100 | 2.2 × 10¹⁴ | |
| 1125 | 1174 | 1150 | 2.3 × 10¹⁴ | |
| 1175 | 1224 | 1200 | 2.4 × 10¹⁴ | |
| 1225 | 1274 | 1250 | 2.5 × 10¹⁴ | |
| 1275 | >1300 | 1300 | 2.6 × 10¹⁴ | |

**1.** Obtain subject brain volume in cm3 from screening MRI.
**2.** Convert subject's MRI brain volume to brain mass:
**3.** brain mass = (brain volume in cm³) × 1.046 g/cm³ (brain density taken from Hasgall et al, 2018,Tissue Properties, Density. [cited 04 November 2019]. In: IT'IS Database for Thermal and Electromagnetic Parameters of Biological Tissues, Version 4.0, 15 May 2018 [Internet]. Zurich: IT'IS Foundation. c2010).
**4.** Identify appropriate dose in Table 11 or above.

Construct 1 is intended for investigational use only by selected investigators familiar with the information in the investigator brochure for Construct 1 and experienced in conducting clinical trials. Construct 1 may only be administered to human subjects participating in clinical trials sponsored/approved by the Sponsor and who have provided formal written consent.

### 6.10.2. CRITERIA FOR EVALUATION SAFETY AND EFFICACY

### PRIMARY ENDPOINTS

- Safety through Week 24: AEs and SAEs

### SECONDARY ENDPOINTS

- Safety through Week 104: AEs and SAEs
- Biomarkers in CSF (GAGs, I2S), plasma (GAGs, I2S), and urine (GAGs)
- Neurodevelopmental parameters of cognitive, behavioral, and adaptive function:
   - Bayley Scales of Infant and Toddler Development, 3rd Edition (BSID-III) (Bayley, 2005, Scales of Infant and Toddler Development, 3rd Ed.)
   - Vineland Adaptive Behavior Scales, 2nd Edition, Expanded Interview Form (VABS-II) (Sparrow et al., 2005, Vineland Adaptive Behavior Scales, 2nd Ed.)
- Vector concentration in CSF, serum, and urine by quantitative polymerase chain reaction (PCR) to Construct 1 DNA

### EXPLORATORY ENDPOINTS

- Immunogenicity measurements
- Neutralizing antibody titers to AAV9 and binding antibody titers to I2S in CSF and serum
- Enzyme-linked immunospot (ELISPOT) assay: T-cell response to AAV9 and I2S
- Flow cytometry: AAV9 and I2S-specific regulatory T cells
- CNS structural abnormalities assessed by MRI of the brain
- Liver and spleen size assessed by ultrasound of the abdomen
- Cardiac evaluation by echocardiogram for valvular disease and left ventricular mass index
- Auditory capacity changes measured by auditory brainstem response (ABR) testing or behavioral audiometry and otoacoustic emissions testing
- Plasma and urinary GAGs in subjects who temporarily discontinue IV ERT (ELAPRASE^{®})
- PedsQL
- Sleep assessment (Ferreira et al., 2009, Sleep Medicine; 10(4):457-463)
- Clinical Assessment of Disease
- Caregiver Assessment of Disease
- Activities of Daily Living (ADL) (Tanjuakio et al. 2015, Mol Genet Metab, 114(2):161-169; Kato et al., 2007, Brain Dev, 29(5): 298-305)
- Burden of Illness

### 6.10.3. List of Abbreviations and Definitions of Terms

**Table 12: Abbreviations and Specialist Terms**

| **Abbreviation** | **Term** |
|---|---|
| AAV | Adeno-associated virus |
| AAV9 | AAV vector of serotype 9 |
| ABR | Auditory brainstem response(s) |
| ADL | Activities of Daily Living |
| AE(s) | Adverse event(s) |
| AESI | Adverse Event(s) of Special Interest |
| ALP | Alkaline phosphatase |
| ALT | Alanine aminotransferase |
| ANC | Absolute neutrophil count |
| AST | Aspartate aminotransferase |
| BBB | Blood-brain barrier |
| BID | Twice a day |
| BP | Blood pressure |
| BSID | Bayley Scales of Infant and Toddler Development |
| BSL | Biosafety level |
| CB7 | Hybrid C4 and CB (chicken beta actin promoter) |
| CBC | Complete blood count |
| cDNA | Consensus DNA |
| CFC | Contextual fear conditioning |
| CFR | Code of Federal Regulations |
| CI | Confidence interval |
| CMV | Cytomegalovirus |
| CNS | Central nervous system |
| CoA | Certificate of analysis |
| CRF | Case Report Form |
| CSF | Cerebrospinal fluid |
| CT | Computed tomography |
| CTA | Clinical Trial Agreement |
| CTCAE | Common Terminology Criteria for Adverse Events |
| CZ | Crystal Zenith^{®} |
| ddPCR | Digital droplet polymerase chain reaction |
| DLT(s) | Dose-limiting toxicity(ies) |
| DNA | Deoxyribonucleic acid |
| DRG | Dorsal root ganglia |
| DS | Dermatan sulfate |
| EBV | Epstein-Barr virus |
| ECG | Electrocardiogram |
| EDC | Electronic Data Capture |
| eGFR | Estimated glomerular filtration rate |
| ELISA | Enzyme-Linked Immunosorbent Assay |
| ELISPOT | Enzyme-linked immunospot |
| EOS | End of Study |
| ERT | Enzyme replacement therapy |
| ET | Early Termination |
| FDA | US Food and Drug Administration |
| g | gram |
| GAG(s) | Glycosaminoglycan(s) |
| GAN | Giant Axonal Neuropathy |
| GC | Genome copies |
| GCP | Good Clinical Practice |
| GLP | Good Laboratory Practice |
| GM3 | Monosialodihexosylganglioside |
| HD | High dose |
| HDL | High-density lipoprotein |
| Hep | Hepatitis |
| Hex | Hexosaminidase |
| hIDS | Human iduronate-2-sulfatase |
| HIPAA | Health Insurance Portability and Accounting Act |
| HIV | Human immunodeficiency virus |
| HS | Heparan sulfate |
| HSCT | Hematopoietic stem cell transplantation |
| I2S | Iduronate-2-sulfatase |
| IB | Investigator's Brochure |
| IC | Intracisternal(ly) |
| ICF | Informed Consent Form |
| ICH | International Council for Harmonisation |
| ICV | Intracerebroventricular |
| IDMC | Independent Data Monitoring Committee |
| IDS | Iduronate-2-sulfatase gene |
| IEC(s) | Independent Ethics Committee(s) |
| IgG | Immunoglobulin G |
| IND | Investigation New Drug |
| IP | Investigational product |
| IQ | Intelligence quotient |
| IRB | Institutional Review Board |
| IS | Immune suppression / immunosuppression |
| ISC | Internal Safety Committee |
| IT | Intrathecal(ly) |
| ITR(s) | Inverted terminal repeat(s) |
| IV | Intravenous(ly) |
| KABC | Kaufman Assessment Battery for Children |
| KIDS | Kinder Infant Development Scale |
| KSPD | Kyoto Scale of Psychological Development |
| LD | Low dose |
| LDH | Lactate dehydrogenase |
| LDL | Low-density lipoprotein |
| LIMP2 | Lysosomal membrane protein |
| MED | Minimum effective dose |
| MedDRA | Medical Dictionary of Regulatory Activities |
| mL | milliliter |
| MMF | Mycophenolate mofetil |
| MPS I | Mucopolysaccharidosis type I |
| MPS II | Mucopolysaccharidosis type II |
| MPS III | Sanfilippo syndrome |
| MPS VII | Mucopolysaccharidosis type VII |
| MRI | Magnetic resonance imaging |
| MTD | Maximum tolerated dose |
| mTORC 1 | Mammalian/mechanistic target of rapamycin complex 1 |
| N | Number in sample |
| NAb | Neutralizing antibody |
| NAGLU | N-acetyl-alpha-glucosaminidase |
| NCI | National Cancer Institute |
| NHP(s) | Non-human primate(s) |
| NIH | National Institutes of Health |
| NOAEL | No-observable-adverse-effect level |
| NOR | Novel object recognition |
| PBMC(s) | Peripheral blood mononuclear cell(s) |
| PCR | Polymerase chain reaction |
| PCP | Pneumocystis carinii pneumonia |
| PD | Pharmacodynamic(s) |
| PgP | P-glycoprotein |
| PI | Principal Investigator |
| PML | Progressive multifocal leukoencephalopathy |
| PNS | Peripheral nervous system |
| PO | By mouth/orally |
| PT | Prothrombin time or Preferred Term |
| PTT | Partial thromboplastin time |
| PVAN | Polyoma virus-associated nephropathy |
| QD | Daily |
| QOL | Quality of Life |
| qPCR | Quantitative polymerase chain reaction |
| RBC | Red blood cell |
| RG1 | Risk Group 1 |
| Construct 1 | Recombinant adeno-associated virus serotype 9 capsid containing human iduronate-2-sulfatase expression cassette |
| SAE(s) | Serious adverse event(s) |
| SAP | Statistical analysis plan |
| SDV | Source document verification |
| SMA | Spinal Muscular Atrophy |
| SOC | System Organ Class |
| SRT | Safety review trigger |
| SSEP | Somatosensory evoked potential |
| TB | Tuberculosis |
| TEAE(s) | Treatment-emergent adverse event(s) |
| TMA | Thrombotic microangiopathy |
| TPP | Thrombocytopenic purpura |
| Treg | Regulatory T cell |
| ULN | Upper limit of normal |
| US | United States |
| USMs | Urgent safety measures |
| VABS | Vineland Adaptive Behavior Scales |
| VZV | Varicella zoster virus |
| WBC | White blood cell (count) |
| WHO | World Health Organization |

### 6.10.4. Immunosuppressive Therapy

### Corticosteroids

- In the morning of vector administration (Day 1 pre-dose), participants will receive methylprednisolone 10mg/kg IV (maximum of 500 mg) over at least 30 minutes. The methylprednisolone should be administered before the lumbar puncture and IC injection of IP. Premedication with acetaminophen and an antihistamine is optional and at the discretion of the investigator.
- On Day 2, oral prednisolone will be started with the goal to discontinue prednisolone by Week 12. The dose of prednisolone will be as follows:
   - Day 2 to the end of Week 2: 0.5 mg/kg/day
   - Week 3 and 4: 0.35 mg/kg/day
   - Week 5-8: 0.2 mg/kg/day
   - Week 9-12: 0.1 mg/kg
   - Prednisolone will be discontinued after Week 12. The exact dose of prednisolone can be adjusted to the next higher clinically practical dose.
- Note: Prednisolone may be substituted for prednisone in a 1:1 conversion rate, at the discretion of the investigator.

### Sirolimus

- 2 days prior to vector administration (Day -2): a loading dose of sirolimus 1 mg/m² every 4 hours x 3 doses will be administered
- From Day -1: sirolimus 0.5 mg/m²/day divided in twice a day dosing with target blood level of 1-3 ng/ml
- Sirolimus will be discontinued after the Week 48 visit.

### Tacrolimus

- Tacrolimus will be started on Day 2 (the day following IP administration) at a dose of 0.05mg/kg twice daily and adjusted to achieve a blood level 2-4 ng/mL for 24 Weeks.
- Starting at Week 24 visit, tacrolimus will be tapered off over 8 weeks. At Week 24 the dose will be decreased by approximately 50%. At Week 28 the dose will be further decreased by approximately 50%. Tacrolimus will be discontinued at Week 32.
- Tacrolimus and sirolimus blood level monitoring will be conducted.

Prednisone dosing will start at 0.5 mg/kg/day and will be gradually tapered off by the Week 12 visit.

Tacrolimus dose adjustments will be made to maintain whole blood trough concentrations within 2-4 ng/mL for the first 24 Weeks. At Week 24, the dose will be decreased by approximately 50%. At Week 28, the dose will be further decreased by approximately 50%. Tacrolimus will be discontinued at Week 32. Sirolimus dose adjustments will be made to maintain whole blood trough concentrations within 1-3 ng/mL. Dose adjustments should be performed by a clinical pharmacist. Subjects should continue on the new maintenance dose for at least 7 to 14 days before further dosage adjustment with concentration monitoring.

Pneumocystis carinii pneumonia (PCP) prophylaxis with trimethoprim/sulfamethoxazole (Bactrim^{™}; BACTRIM^{™} USPI, 2013) will be given three times a week (example dosing schedule; Monday, Wednesday, Friday) at a dose of 5 mg/kg beginning on Day -2 and continuing until Week 48. Refer to the prescribing information for risks associated with trimethoprim/sulfamethoxazole use (BACTRIM^{™} USPI, 2013). For patients with sulfa allergies, alternative medications can include pentamidine, dapsone, and atovaquone.

Antifungal prophylaxis is to be initiated if the ANC is < 500 mm³. The treatment regimen will be determined through local site standard of care in consultation with appropriate subspecialists.

The concomitant use of Rapamune with a calcineurin inhibitor may increase the risk of calcineurin inhibitor-induced thrombotic microangiopathies. Thrombotic microangiopathies (TMA) are a group of disorders characterized by thrombocytopenia, microangiopathic hemolytic anemia, and variable organ system involvement.

This may present severe thrombocytopenia (<30 × 10⁹/L), microangiopathic hemolytic anemia characterized by schistocytes on the blood smear, increased reticulocyte count (>120 × 10⁹/L), elevated lactate dehydrogenase level (LDH), and signs of skin and mucosal hemorrhage, weakness, and dyspnea. Treatment includes discontinuation of tacrolimus and possible initiation of plasma exchange.

If rising CMV or EBV viral genomes are detected during serial testing, the decision to decrease IS or begin antiviral therapy will be determined through local site standard of care in consultation with appropriate subspecialists.

### EQUIVALENTS

Although the invention is described in detail with reference to specific embodiments thereof, it will be understood that variations which are functionally equivalent are within the scope of this invention. Indeed, various modifications of the invention in addition to those shown and described herein will become apparent to those skilled in the art from the foregoing description and accompanying drawings. Such modifications are intended to fall within the scope of the appended claims. Those skilled in the art will recognize, or be able to ascertain using no more than routine experimentation, many equivalents to the specific embodiments of the invention described herein. Such equivalents are intended to be encompassed by the following claims.

All publications, patents and patent applications mentioned in this specification are herein incorporated by reference into the specification to the same extent as if each individual publication, patent or patent application was specifically and individually indicated to be incorporated herein by reference in their entireties.

Also disclosed herein are *inter alia* the following items (said items are not claims).
1. A method for treating a human subject diagnosed with mucopolysaccharidosis type II (MPS II), comprising delivering to the cerebrospinal fluid (CSF) of the human subject a therapeutically effective amount of a glycosylated recombinant human iduronate-2-sulfatase (IDS) precursor produced by human neuronal or human glial cells, wherein the glycosylated recombinant human IDS precursor is delivered by administration of a recombinant nucleotide expression vector encoding human IDS, wherein the recombinant nucleotide expression vector is administered at a dose that is dependent on the human subject's brain mass, and wherein the brain mass is determined by brain magnetic resonance imaging (MRI) of the human subject's brain.
2. The method of item 1, wherein the glycosylated recombinant human IDS precursor is secreted at a detectable level.
3. The method of item 1 or 2, wherein the human neuronal or human glial cells carry at least one mutation in the endogenous gene encoding human IDS precursor.
4. The method of any one of items 1-3, wherein the human neuronal or human glial cells are transduced with a recombinant adeno-associated virus vector (rAAV).
5. The method of any one of items 1-4, wherein the glycosylated recombinant human IDS precursor is expressed under the control of a CB7 promoter.
6. The method of any one of items 1-5, wherein the glycosylated recombinant human IDS precursor is expressed from a cDNA encoding human IDS precursor.
7. The method of any one of items 1-6, wherein the glycosylated recombinant human IDS precursor is about 90 kDa as measured by polyacrylamide gel electrophoresis.
8. The method of any one of items 1-7, wherein the glycosylated recombinant human IDS precursor contains a formylglycine.
9. The method of any one of items 1-8, wherein the glycosylated recombinant human IDS precursor (a) is α2,6-sialylated; (b) does not contain detectable NeuGc; (c) does not contain detectable α-Gal antigen; (d) contains tyrosine-sulfation; and/or (e) is mannose-6-phosphorylated.
10. The method of any one of items 1-9, in which the glycosylated recombinant human IDS precursor comprises the amino acid sequence of SEQ ID NO. 1.
11. The method of any one of items 1-10, wherein the recombinant nucleotide expression vector is an AAV9 or AAVrh10 vector.
12. The method of any one of items 1-11, wherein the human subject's brain mass is converted from the human subject's brain volume by multiplying the human subject's brain volume in cm³ by a factor of 1.046 g/cm³, wherein the human subject's brain volume is obtained from the human subject's brain MRI.
13. The method of any one of items 1-12, wherein the recombinant nucleotide expression vector is administered at a dose of about 1.3 × 10¹⁰ GC/g brain mass as determined by MRI, or about 6.5 × 10¹⁰ GC/g brain mass as determined by MRI.
14. The method of any one of items 1-12, wherein the recombinant nucleotide expression vector is administered at a dose of about 2.0 × 10¹¹ GC/g brain mass as determined by MRI.
15. The method of any one of items 1-12, wherein the human subject is 5 years old or older and less than 18 years old.
16. The method of item 15, wherein the recombinant nucleotide expression vector is administered at a dose of about 6.5 × 10¹⁰ GC/g brain mass as determined by MRI.
17. The method of item 15, wherein the recombinant nucleotide expression vector is administered at a dose according to the table below:

| **Brain Mass (in g)** | | **Target Brain Mass (in g)** | **Dose: Total GC (6.5 × 10¹⁰ GC/g brain mass)** |
|---|---|---|---|
| **Min** | **Max** | | |
| 801 | 900 | 850 | 5.5 × 10¹³ |
| 901 | 1050 | 975 | 6.3 × 10¹³ |
| 1051 | 1200 | 1125 | 7.3 × 10¹³ |
| 1201 | - | 1300 | 8.5 × 10¹³ |

18. The method of any one of items 1-12, wherein the human subject is 4 months old or older and less than 5 years old.
19. The method of item 18, wherein the recombinant nucleotide expression vector is administered at a dose chosen from Dose 1 or Dose 2 according to the table below:

| **Brain Mass (in g)** | | **Target** | **Dose Levels** | |
|---|---|---|---|---|
| **Min** | **Max** | | **Dose 1** | **Dose 2** |
| | | | **Total GC** (1.3 × 10¹⁰ GC/g brain mass) | **Total GC** (6.5 × 10¹⁰ GC/g brain mass) |
| - | 700 | 650 | 8.5 × 10¹² | 4.2 × 10¹³ |
| 701 | 800 | 750 | 9.8 × 10¹² | 4.9 × 10¹³ |
| 801 | 900 | 850 | 1.1 × 10¹³ | 5.5 × 10¹³ |
| 901 | 1050 | 975 | 1.3 × 10¹³ | 6.3 × 10¹³ |
| 1051 | 1200 | 1125 | 1.5× 10¹³ | 7.3 × 10¹³ |
| 1201 | - | 1300 | 1.7 × 10¹³ | 8.5 × 10¹³ |

20. The method of item 18, wherein the recombinant nucleotide expression vector is administered at a dose according to the table below:

| **Brain Mass (in g)** | | **Target** | **Dose 3** |
|---|---|---|---|
| **Min** | **Max** | | **Total GC** (2.0 × 10¹¹ GC/g brain mass) |
| - | 474 | 450 | 9.0 x 10¹³ |
| 475 | 524 | 500 | 1.0 × 10¹⁴ |
| 525 | 574 | 550 | 1.1 × 10¹⁴ |
| 575 | 624 | 600 | 1.2 × 10¹⁴ |
| 625 | 674 | 650 | 1.3 × 10¹⁴ |
| 675 | 724 | 700 | 1.4 × 10¹⁴ |
| 725 | 774 | 750 | 1.5 × 10¹⁴ |
| 775 | 824 | 800 | 1.6 × 10¹⁴ |
| 825 | 874 | 850 | 1.7 × 10¹⁴ |
| 875 | 924 | 900 | 1.8 × 10¹⁴ |
| 925 | 974 | 950 | 1.9 × 10¹⁴ |
| 975 | 1024 | 1000 | 2.0 × 10¹⁴ |
| 1025 | 1074 | 1050 | 2.1 × 10¹⁴ |
| 1075 | 1124 | 1100 | 2.2 × 10¹⁴ |
| 1125 | 1174 | 1150 | 2.3 × 10¹⁴ |
| 1175 | 1224 | 1200 | 2.4 × 10¹⁴ |
| 1225 | 1274 | 1250 | 2.5 × 10¹⁴ |
| 1275 | >1300 | 1300 | 2.6 × 10¹⁴ |

21. The method of any one of items 1-20, wherein the recombinant nucleotide expression vector is administered via intracisternal (IC) administration.
22. The method of any one of items 1-20, wherein the recombinant nucleotide expression vector is administered via intracerebroventricular (ICV) administration.
23. The method of any one of items 1-22, wherein the recombinant nucleotide expression vector is administered at a volume that does not exceed 10% of the total cerebrospinal fluid volume of the human subject.
24. The method of any one of items 1-23, wherein the glycosylated recombinant human IDS precursor is delivered to lysosomes of cells in the CNS of the human subject.
25. The method of any one of items 1-24, further comprising administering an immune suppression therapy to the human subject before or concurrently with the human IDS precursor treatment and optionally continuing immune suppression therapy thereafter.
26. The method of item 25, wherein the immune suppression therapy comprises administering one or more corticosteroids, sirolimus, and/or tacrolimus.
27. The method of item 26, wherein the one or more corticosteroids are methylprednisolone and/or prednisone.
28. The method of any one of items 25-27, further comprising administering one or more antibiotics to the human subject before or concurrently with the immune suppression therapy.
29. The method of item 28, wherein the one or more antibiotics are trimethoprim, sulfamethoxazole, pentamidine, dapsone, and/or atovaquone.
30. The method of any one of items 25-29, further comprising administering one or more antifungal therapies to the human subject before or concurrently with the immune suppression therapy.
31. The method of any one of items 1-30, further comprising a step of measuring one or more of the following biomarkers after administration of the recombinant nucleotide expression vector: (a) level of glycosaminoglycans (GAGs) in CSF; (b) level of iduronate-2-sulfatase (I2S) in CSF; (c) level of GAGs in plasma; (d) level of I2S in plasma; (e) level of leukocyte I2S enzyme activity; and (f) level of GAGs in urine.
32. The method of item 31, wherein the GAGs in CSF comprise heparin sulfate in CSF.
33. The method of item 31, wherein the GAGs in CSF are heparin sulfate in CSF.
34. The method of any one of items 31-33, wherein the GAGs in plasma comprise heparin sulfate in plasma.
35. The method of any one of items 31-33, wherein the GAGs in plasma are heparin sulfate in plasma.
36. The method of any one of items 31-35, wherein the GAGs in urine comprise heparin sulfate in urine.
37. The method of any one of items 31-35, wherein the GAGs in urine are heparin sulfate in urine.
38. The method of any one of items 31-37, wherein the step of measuring comprises mearing level of heparin sulfate in CSF.
39. The method of any one of items 31-38, wherein the step of measuring comprises measuring level of leukocyte I2S enzyme activity.

## Claims

1. A glycosylated recombinant human iduronate-2-sulfatase (rhIDS) for use in a method for treating a human subject diagnosed with mucopolysaccharidosis type II (MPS II), wherein the method comprises delivering to the cerebrospinal fluid (CSF) of the human subject a therapeutically effective amount of the glycosylated rhIDS produced by human neuronal or human glial cells, wherein the glycosylated rhIDS is delivered by administration of a recombinant nucleotide expression vector encoding the rhIDS to the human subject, wherein the recombinant nucleotide expression vector is administered at a dose that is dependent on the human subject's brain mass, wherein the brain mass is determined by brain magnetic resonance imaging (MRI) of the human subject's brain, and wherein the recombinant nucleotide expression vector is administered at a dose of about 2.0 × 10¹¹ GC/g brain mass as determined by MRI.

2. The glycosylated rhIDS for use of claim 1, wherein the glycosylated rhIDS:
(a) is secreted at a detectable level;
(b) is expressed under the control of a CB7 promoter;
(c) is expressed from a cDNA encoding human IDS;
(d) is about 90 kDa as measured by polyacrylamide gel electrophoresis;
(e) contains a formylglycine;
(f) is α2,6-sialylated;
(g) does not contain detectable NeuGc;
(h) does not contain detectable α-Gal antigen;
(i) contains tyrosine-sulfation;
(j) is mannose-6-phosphorylated; and/or
(k) comprises the amino acid sequence of SEQ ID NO. 1

3. A recombinant nucleotide expression vector encoding a recombinant human iduronate-2-sulfatase (rhIDS) for use in a method for treating a human subject diagnosed with mucopolysaccharidosis type II (MPS II), wherein the method comprises administering the recombinant nucleotide expression vector to the cerebrospinal fluid (CSF) of the human subject at a dose that is dependent on the human subject's brain mass, wherein the brain mass is determined by brain magnetic resonance imaging (MRI) of the human subject's brain, and wherein the recombinant nucleotide expression vector is administered at a dose of about 2.0 × 10¹¹ GC/g brain mass as determined by MRI.

4. The glycosylated rhIDS for use of claim 1 or 2, or the recombinant nucleotide expression vector for use of claim 3, wherein the recombinant nucleotide expression vector is a recombinant adeno-associated virus vector (rAAV).

5. The glycosylated rhIDS for use of claim 4, or the recombinant nucleotide expression vector for use of claim 4, wherein the rAAV comprises an AAV9 or AAVrh10 capsid protein.

6. The glycosylated rhIDS for use of any one of claims 1, 2, 4 and 5, or the recombinant nucleotide expression vector for use of any one of claims 3 to 5, wherein the human subject's brain mass is converted from the human subject's brain volume by multiplying the human subject's brain volume in cm³ by a factor of 1.046 g/cm³, wherein the human subject's brain volume is obtained from the human subject's brain MRI.

7. The glycosylated rhIDS for use of any one of claims 1, 2 and 4 to 6, or the recombinant nucleotide expression vector for use of any one of claims 3 to 6, wherein the human subject is 5 years old or older and less than 18 years old.

8. The glycosylated rhIDS for use of any one of claims 1, 2 and 4 to 6, or the recombinant nucleotide expression vector for use of any one of claims 3 to 6, wherein the human subject is 4 months old or older and less than 5 years old.

9. The glycosylated rhIDS for use of claim 8, or the recombinant nucleotide expression vector for use of claim 8, wherein the recombinant nucleotide expression vector is administered to the human subject at a dose according to the table below:
| **Brain Mass (in g)** | | **Target Brain Mass (in g)** | **Dose 3 Total GC** |
|---|---|---|---|
| **Min** | **Max** | | (2.0 × 10¹¹ GC/g brain mass) |
| - | 474 | 450 | 9.0 x 10¹³ |
| 475 | 524 | 500 | 1.0 × 10¹⁴ |
| 525 | 574 | 550 | 1.1 × 10¹⁴ |
| 575 | 624 | 600 | 1.2 × 10¹⁴ |
| 625 | 674 | 650 | 1.3 × 10¹⁴ |
| 675 | 724 | 700 | 1.4 × 10¹⁴ |
| 725 | 774 | 750 | 1.5 × 10¹⁴ |
| 775 | 824 | 800 | 1.6 × 10¹⁴ |
| 825 | 874 | 850 | 1.7 × 10¹⁴ |
| 875 | 924 | 900 | 1.8 × 10¹⁴ |
| 925 | 974 | 950 | 1.9 × 10¹⁴ |
| 975 | 1024 | 1000 | 2.0 × 10¹⁴ |
| 1025 | 1074 | 1050 | 2.1 × 10¹⁴ |
| 1075 | 1124 | 1100 | 2.2 × 10¹⁴ |
| 1125 | 1174 | 1150 | 2.3 × 10¹⁴ |
| 1175 | 1224 | 1200 | 2.4 × 10¹⁴ |
| 1225 | 1274 | 1250 | 2.5 × 10¹⁴ |
| 1275 | >1300 | 1300 | 2.6 × 10¹⁴ |

10. The glycosylated rhIDS for use of any one of claims 1, 2 and 4 to 9, or the recombinant nucleotide expression vector for use of any one of claims 3 to 9, wherein the recombinant nucleotide expression vector is administered via intracisternal (IC) administration or via intracerebroventricular (ICV) administration.

11. The glycosylated rhIDS for use of any one of claims 1, 2 and 4 to 10, or the recombinant nucleotide expression vector for use of any one of claims 3 to 10, wherein the recombinant nucleotide expression vector is administered to the human subject at a volume that does not exceed 10% of the total cerebrospinal fluid volume of the human subject.

12. The glycosylated rhIDS for use of any one of claims 1, 2 and 4 to 11, or the recombinant nucleotide expression vector for use of any one of claims 3 to 11, wherein the method further comprises administering an immune suppression therapy to the human subject before or concurrently with the rhIDS treatment and optionally continuing the immune suppression therapy thereafter.

13. The glycosylated rhIDS for use of claim 12, or the recombinant nucleotide expression vector for use of claim 12, wherein the immune suppression therapy comprises administering one or more corticosteroids, sirolimus, and/or tacrolimus.

14. The glycosylated rhIDS for use of claim 12 or 13, or the recombinant nucleotide expression vector for use of claim 12 or 13, wherein the method further comprises administering one or more antibiotics and/or one or more antifungal therapies to the human subject before or concurrently with the immune suppression therapy, optionally wherein the one or more antibiotics are trimethoprim, sulfamethoxazole, pentamidine, dapsone, and/or atovaquone.

15. The glycosylated rhIDS for use of any one of claims 1, 2 and 4 to 14, or the recombinant nucleotide expression vector for use of any one of claims 3 to 14, wherein the method further comprises a step of measuring one or more of the following biomarkers after administration of the recombinant nucleotide expression vector: (a) level of glycosaminoglycans (GAGs) in CSF; (b) level of iduronate-2-sulfatase (I2S) in CSF; (c) level of GAGs in plasma; (d) level of I2S in plasma; (e) level of leukocyte I2S enzyme activity; and (f) level of GAGs in urine, optionally wherein:
(i) the GAGs in CSF comprise heparin sulfate in CSF;
(ii) the GAGs in CSF are heparin sulfate in CSF;
(iii) the GAGs in plasma comprise heparin sulfate in plasma;
(iv) the GAGs in plasma are heparin sulfate in plasma;
(v) the GAGs in urine comprise heparin sulfate in urine; and/or
(vi) the GAGs in urine are heparin sulfate in urine.
